# EUROPEAN PATENT SPECIFICATION

(11) **EP 3 262 063 B1**
(45) Date of publication and mention of the grant of the patent: **22.09.2021**
(21) Application number: 16706626.5
(22) Date of filing: 26.02.2016
(51) Int. Cl.: C07K 14/445

(54) **IMMUNOTHERAPEUTIC TARGETING OF PLACENTAL-LIKE CHONDROITIN SULFATE USING CHIMERIC ANTIGEN RECEPTORS (CARS) AND IMMUNOTHERAPEUTIC TARGETING OF CANCER USING CARS WITH SPLIT-PROTEIN BINDING SYSTEMS**
IMMUNTHERAPEUTISCHES TARGETING VON PLAZENTAARTIGEM CHONDROITINSULFAT MIT CHIMÄREN ANTIGENREZEPTOREN (CAR) UND IMMUNTHERAPEUTISCHES TARGETING VON KREBS MIT CAR MIT SPALTPROTEINBINDENDEN SYSTEMEN
CIBLAGE IMMUNOTHÉRAPEUTIQUE DE SULFATE DE CHONDROÏTINE DE TYPE PLACENTAIRE À L'AIDE DE RÉCEPTEURS D'ANTIGÈNES CHIMÉRIQUES (CAR) ETCIBLAGE IMMUNOTHÉRAPEUTIQUE DU CANCER À L'AIDE DE CAR AVEC DES SYSTÈMES DE LIAISON DE PROTÉINES DE DIVISION

(30) Priority: 26.02.2015 EP 15156736
(43) Date of publication of application: 03.01.2018
(73) Proprietor: Var2 Pharmaceuticals ApS, 2200 Copenhagen N (DK)
(72) Inventor: ZOCCA, Mai-Britt, 1266 Copenhagen K (DK)
(74) Representative: Inspicos P/S
(86) International application number: PCT/EP2016/054084
(87) International publication number: WO 2016/135291

(56) References cited:
- WO-A1-2013/117705
- US-A1- 2009 075 313
- C. GELDRES ET AL: "T Lymphocytes Redirected against the Chondroitin Sulfate Proteoglycan-4 Control the Growth of Multiple Solid Tumors both In Vitro and In Vivo", CLINICAL CANCER RESEARCH, vol. 20, no. 4, 13 December 2013 (2013-12-13), pages 962-971, XP055169325, ISSN: 1078-0432, DOI: 10.1158/1078-0432.CCR-13-2218
- SUJAN S SHEKHAWAT ET AL: "Split-protein systems: beyond binary protein-protein interactions", CURRENT OPINION IN CHEMICAL BIOLOGY, vol. 15, no. 6, 1 December 2011 (2011-12-01), pages 789-797, XP055268066, GB ISSN: 1367-5931, DOI: 10.1016/j.cbpa.2011.10.014
- LI LONG ET AL: "Structural Analysis and Optimization of the Covalent Association between SpyCatcher and a Peptide Tag", JOURNAL OF MOLECULAR BIOLOGY, ACADEMIC PRESS, UNITED KINGDOM, vol. 426, no. 2, 23 October 2013 (2013-10-23), pages 309-317, XP028549188, ISSN: 0022-2836, DOI: 10.1016/J.JMB.2013.10.021
- RAFIQ SARWISH ET AL: "Engineering strategies to overcome the current roadblocks in CAR T cell therapy", NATURE REVIEWS CLINICAL ONCOLOGY, NATURE, NY, US, vol. 17, no. 3, 17 December 2019 (2019-12-17), pages 147-167, XP037114979, ISSN: 1759-4774, DOI: 10.1038/S41571-019-0297-Y [retrieved on 2019-12-17]

### SEQUENCE LISTING

<110> VAR2 Pharmaceuticals ApS
<120> IMMUNOTHERAPEUTIC TARGETING OF PLACENTAL-LIKE CHONDROITIN SULFATE USING CHIMERIC ANTIGEN RECEPTORS (CARS) AND IMMUNOTHERAPEUTIC TARGETING OF CANCER USING CARS WITH SPLIT-PROTEIN BINDING SYSTEMS
<130> 20308PCT00
<160> 137
<170> PatentIn version 3.5
<210> 1
   <211> 640
   <212> PRT
   <213> Artificial
<220>
   <223> Artificial construct
<400> 1
   Asn Tyr Ile Lys Gly Asp Pro Tyr Phe Ala Glu Tyr Ala Thr Lys Leu
   1               5                   10                  15
   Ser Phe Ile Leu Asn Pro Ser Asp Ala Asn Asn Pro Ser Gly Glu Thr
               20                  25                  30
   Ala Asn His Asn Asp Glu Ala Cys Asn Cys Asn Glu Ser Gly Ile Ser
           35                  40                  45
   Ser Val Gly Gln Ala Gln Thr Ser Gly Pro Ser Ser Asn Lys Thr Cys
       50                  55                  60
   Ile Thr His Ser Ser Ile Lys Thr Asn Lys Lys Lys Glu Cys Lys Asp
   65                  70                  75                  80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Asp Ser Cys Asp Asn Lys Asn Gln Asp Glu Cys Gln Lys
       130                 135                 140
   Lys Leu Glu Lys Val Phe Ala Ser Leu Thr Asn Gly Tyr Lys Cys Asp
   145                 150                 155                 160
   Lys Cys Lys Ser Gly Thr Ser Arg Ser Lys Lys Lys Trp Ile Trp Lys
                   165                 170                 175
   Lys Ser Ser Gly Asn Glu Glu Gly Leu Gln Glu Glu Tyr Ala Asn Thr
               180                 185                 190
   Ile Gly Leu Pro Pro Arg Thr Gln Ser Leu Tyr Leu Gly Asn Leu Pro
           195                 200                 205
   Lys Leu Glu Asn Val Cys Glu Asp Val Lys Asp Ile Asn Phe Asp Thr
       210                 215                 220
   Lys Glu Lys Phe Leu Ala Gly Cys Leu Ile Val Ser Phe His Glu Gly
   225                 230                 235                 240
   Lys Asn Leu Lys Lys Arg Tyr Pro Gln Asn Lys Asn Ser Gly Asn Lys
                   245                 250                 255
   Glu Asn Leu Cys Lys Ala Leu Glu Tyr Ser Phe Ala Asp Tyr Gly Asp
               260                 265                 270
   Leu Ile Lys Gly Thr Ser Ile Trp Asp Asn Glu Tyr Thr Lys Asp Leu
           275                 280                 285
   Glu Leu Asn Leu Gln Asn Asn Phe Gly Lys Leu Phe Gly Lys Tyr Ile
       290                 295                 300
   Lys Lys Asn Asn Thr Ala Glu Gln Asp Thr Ser Tyr Ser Ser Leu Asp
   305                 310                 315                 320
   Glu Leu Arg Glu Ser Trp Trp Asn Thr Asn Lys Lys Tyr Ile Trp Thr
                   325                 330                 335
   Ala Met Lys His Gly Ala Glu Met Asn Ile Thr Thr Cys Asn Ala Asp
               340                 345                 350
   Gly Ser Val Thr Gly Ser Gly Ser Ser Cys Asp Asp Ile Pro Thr Ile
           355                 360                 365
   Asp Leu Ile Pro Gln Tyr Leu Arg Phe Leu Gln Glu Trp Val Glu Asn
       370                 375                 380
   Phe Cys Glu Gln Arg Gln Ala Lys Val Lys Asp Val Ile Thr Asn Cys
   385                 390                 395                 400
   Lys Ser Cys Lys Glu Ser Gly Asn Lys Cys Lys Thr Glu Cys Lys Thr
                   405                 410                 415
   Lys Cys Lys Asp Glu Cys Glu Lys Tyr Lys Lys Phe Ile Glu Ala Cys
               420                 425                 430
   Gly Thr Ala Gly Gly Gly Ile Gly Thr Ala Gly Ser Pro Trp Ser Lys
           435                 440                 445
   Arg Trp Asp Gln Ile Tyr Lys Arg Tyr Ser Lys His Ile Glu Asp Ala
       450                 455                 460
   Lys Arg Asn Arg Lys Ala Gly Thr Lys Asn Cys Gly Thr Ser Ser Thr
   465                 470                 475                 480
   Thr Asn Ala Ala Ala Ser Thr Asp Glu Asn Lys Cys Val Gln Ser Asp
                   485                 490                 495
   Ile Asp Ser Phe Phe Lys His Leu Ile Asp Ile Gly Leu Thr Thr Pro
               500                 505                 510
   Ser Ser Tyr Leu Ser Asn Val Leu Asp Asp Asn Ile Cys Gly Ala Asp
           515                 520                 525
   Lys Ala Pro Trp Thr Thr Tyr Thr Thr Tyr Thr Thr Thr Glu Lys Cys
       530                 535                 540
   Asn Lys Glu Arg Asp Lys Ser Lys Ser Gln Ser Ser Asp Thr Leu Val
   545                 550                 555                 560
   Val Val Asn Val Pro Ser Pro Leu Gly Asn Thr Pro Tyr Arg Tyr Lys
                   565                 570                 575
   Tyr Ala Cys Gln Cys Lys Ile Pro Thr Asn Glu Glu Thr Cys Asp Asp
               580                 585                 590
   Arg Lys Glu Tyr Met Asn Gln Trp Ser Cys Gly Ser Ala Arg Thr Met
           595                 600                 605
   Lys Arg Gly Tyr Lys Asn Asp Asn Tyr Glu Leu Cys Lys Tyr Asn Gly
       610                 615                 620
   Val Asp Val Lys Pro Thr Thr Val Arg Ser Asn Ser Ser Lys Leu Asp
   625                 630                 635                 640
<210> 2
   <211> 341
   <212> PRT
   <213> Plasmodium falciparum
<400> 2
   Lys Cys Asp Lys Cys Lys Ser Gly Thr Ser Arg Ser Arg Lys Ile Trp
   1               5                   10                  15
   Thr Trp Arg Lys Ser Ser Gly Asn Lys Glu Gly Leu Gln Glu Glu Tyr
               20                  25                  30
   Ala Asn Thr Ile Gly Leu Ser Pro Arg Thr Gln Leu Leu Tyr Leu Gly
           35                  40                  45
   Asn Leu Arg Lys Leu Glu Asn Val Cys Glu Asp Val Thr Asp Ile Asn
       50                  55                  60
   Phe Asp Thr Lys Glu Lys Phe Leu Ala Gly Cys Leu Ile Ala Ala Phe
   65                  70                  75                  80
   His Glu Gly Lys Asn Leu Lys Lys Arg Tyr Leu Glu Lys Lys Lys Gly
                   85                  90                  95
   Asp Asn Asn Ser Lys Leu Cys Lys Asp Leu Lys Tyr Ser Phe Ala Asp
               100                 105                 110
   Tyr Gly Asp Leu Ile Lys Gly Thr Ser Ile Trp Asp Asn Asp Phe Thr
           115                 120                 125
   Lys Asp Leu Glu Leu Asn Leu Gln Gln Ile Phe Gly Lys Leu Phe Arg
       130                 135                 140
   Lys Tyr Ile Lys Lys Lys Asn Ile Ser Thr Glu Gln Asp Thr Ser Tyr
   145                 150                 155                 160
   Ser Ser Leu Asp Glu Leu Arg Glu Ser Trp Trp Asn Thr Asn Lys Lys
                   165                 170                 175
   Tyr Ile Trp Leu Ala Met Lys His Gly Ala Gly Met Asn Ser Thr Thr
               180                 185                 190
   Cys Ser Cys Ser Gly Asp Ser Ser Ser Gly Glu Asn Gln Thr Asn Ser
           195                 200                 205
   Cys Asp Asp Ile Pro Thr Ile Asp Leu Ile Pro Gln Tyr Leu Arg Phe
       210                 215                 220
   Leu Gln Glu Trp Val Glu His Phe Cys Glu Gln Arg Gln Ala Lys Val
   225                 230                 235                 240
   Lys Asp Val Ile Thr Asn Cys Asn Ser Cys Lys Glu Ser Gly Gly Thr
   245 250 255
   Cys Asn Ser Asp Cys Glu Lys Lys Cys Lys Asn Lys Cys Asp Ala Tyr
   260 265 270
   Lys Thr Phe Ile Glu Asp Cys Lys Gly Val Gly Gly Thr Gly Thr Ala
   275 280 285
   Gly Ser Ser Trp Val Lys Arg Trp Tyr Gln Ile Tyr Met Arg Tyr Ser
   290 295 300
   Lys Tyr Ile Glu Asp Ala Lys Arg Asn Arg Lys Ala Gly Thr Lys Ser
   305 310 315 320
   Cys Gly Thr Ser Ser Thr Thr Asn Val Ser Val Ser Thr Asp Glu Asn
   325 330 335
   Lys Cys Val Gln Ser
   340
<210> 3
   <211> 656
   <212> PRT
   <213> Artificial
<220>
   <223> Artificial construct
<400> 3
   Asp Tyr Ile Lys Gly Asp Pro Tyr Phe Ala Glu Tyr Ala Thr Lys Leu
   1               5                   10                  15
   Ser Phe Ile Leu Asn Ser Ser Asp Ala Asn Asn Pro Ser Gly Glu Thr
               20                  25                  30
   Ala Asn His Asn Asp Glu Val Cys Asn Pro Asn Glu Ser Glu Ile Ser
           35                  40                  45
   Ser Val Gly Gln Ala Gln Thr Ser Asp Pro Ser Ser Asn Lys Thr Cys
       50                  55                  60
   Asn Thr His Ser Ser Ile Lys Ala Asn Lys Lys Lys Val Cys Lys His
   65                  70                  75                  80
   Val Lys Leu Gly Ile Asn Asn Asn Asp Lys Val Leu Arg Val Cys Val
                   85                  90                  95
   Ile Glu Asp Thr Ser Leu Ser Gly Val Glu Asn Cys Cys Phe Lys Asp
               100                 105                 110
   Leu Leu Gly Ile Leu Gln Glu Asn Cys Ser Asp Asn Lys Ser Gly Ser
           115                 120                 125
   Ser Ser Asn Gly Ser Cys Asn Asn Lys Asn Gln Glu Ala Cys Glu Lys
       130                 135                 140
   Asn Leu Glu Lys Val Leu Ala Ser Leu Thr Asn Cys Tyr Lys Cys Asp
   145                 150                 155                 160
   Lys Cys Lys Ser Gly Thr Ser Thr Val Asn Lys Asn Trp Ile Trp Lys
                   165                 170                 175
   Lys Ser Ser Gly Asn Lys Glu Gly Leu Gln Lys Glu Tyr Ala Asn Thr
               180                 185                 190
   Ile Gly Leu Pro Pro Arg Thr His Ser Leu Tyr Leu Gly Asn Leu Pro
           195                 200                 205
   Lys Leu Glu Asn Val Cys Glu Asp Val Lys Asp Ile Asn Phe Asp Thr
       210                 215                 220
   Lys Glu Lys Phe Leu Ala Gly Cys Leu Ile Ala Ala Phe His Glu Gly
   225                 230                 235                 240
   Lys Asn Leu Lys Lys Arg Tyr Pro Gln Asn Lys Asn Asp Asp Asn Asn
                   245                 250                 255
   Ser Lys Leu Cys Lys Ala Leu Glu Tyr Ser Phe Ala Asp Tyr Gly Asp
               260                 265                 270
   Leu Ile Lys Gly Thr Ser Ile Trp Asp Asn Glu Tyr Thr Lys Asp Leu
           275                 280                 285
   Glu Leu Asn Leu Gln Gln Ile Phe Gly Lys Leu Phe Arg Lys Tyr Ile
       290                 295                 300
   Lys Lys Asn Ile Ser Thr Glu Gln Asp Thr Leu Tyr Ser Ser Leu Asp
   305                 310                 315                 320
   Glu Leu Arg Glu Ser Trp Trp Asn Thr Asn Lys Lys Tyr Ile Trp Leu
                   325                 330                 335
   Ala Met Lys His Gly Ala Gly Met Asn Ile Thr Thr Cys Cys Gly Asp
               340                 345                 350
   Gly Ser Val Thr Gly Ser Gly Ser Ser Cys Asp Asp Ile Pro Thr Ile
   355 360 365
   Asp Leu Ile Pro Gln Tyr Leu Arg Phe Leu Gln Glu Trp Val Glu His
   370 375 380
   Phe Cys Lys Gln Arg Gln Glu Lys Val Lys Asp Val Ile Asn Ser Cys
   385 390 395 400
   Asn Ser Cys Lys Asn Thr Ser Ser Lys Thr Lys Leu Gly Asp Thr Cys
   405 410 415
   Asn Ser Asp Cys Glu Lys Lys Cys Lys Ile Glu Cys Glu Lys Tyr Lys
   420 425 430
   Lys Phe Ile Glu Glu Cys Arg Thr Ala Val Gly Gly Thr Ala Gly Ser
   435 440 445
   Ser Trp Ser Lys Arg Trp Asp Gln Ile Tyr Lys Met Tyr Ser Lys His
   450 455 460
   Ile Glu Asp Ala Lys Arg Asn Arg Lys Ala Gly Thr Lys Asn Cys Gly
   465 470 475 480
   Ile Thr Thr Gly Thr Ile Ser Gly Glu Ser Ser Gly Ala Asn Ser Gly
   485 490 495
   Val Thr Thr Thr Glu Asn Lys Cys Val Gln Ser Asp Ile Asp Ser Phe
   500 505 510
   Phe Lys His Leu Ile Asp Ile Gly Leu Thr Thr Pro Ser Ser Tyr Leu
   515 520 525
   Ser Ile Val Leu Asp Asp Asn Ile Cys Gly Asp Asp Lys Ala Pro Trp
   530 535 540
   Thr Thr Tyr Thr Thr Tyr Thr Thr Tyr Thr Thr Thr Glu Lys Cys Asn
   545 550 555 560
   Lys Glu Arg Asp Lys Ser Lys Ser Gln Gln Ser Asn Thr Ser Val Val
   565 570 575
   Val Asn Val Pro Ser Pro Leu Gly Asn Thr Pro His Gly Tyr Lys Tyr
   580 585 590
   Ala Cys Gln Cys Lys Ile Pro Thr Asn Glu Glu Thr Cys Asp Asp Arg
   595 600 605
   Lys Glu Tyr Met Asn Gln Trp Ile Ser Asp Thr Ser Lys Asn Pro Lys
   610 615 620
   Gly Ser Gly Ser Thr Asn Asn Asp Tyr Glu Leu Tyr Thr Tyr Asn Gly
   625 630 635 640
   Val Lys Glu Thr Lys Leu Pro Lys Lys Leu Asn Ser Pro Lys Leu Asp
   645 650 655
<210> 4
   <211> 643
   <212> PRT
   <213> Artificial
<220>
   <223> Artificial construct
<400> 4
   Asp Tyr Ile Lys Asp Asp Pro Tyr Ser Lys Glu Tyr Thr Thr Lys Leu
   1               5                   10                  15
   Ser Phe Ile Leu Asn Ser Ser Asp Ala Asn Thr Ser Ser Gly Glu Thr
               20                  25                  30
   Ala Asn His Asn Asp Glu Ala Cys Asn Cys Asn Glu Ser Glu Ile Ser
           35                  40                  45
   Ser Val Gly Gln Ala Gln Thr Ser Gly Pro Ser Ser Asn Lys Thr Cys
       50                  55                  60
   Ile Thr His Ser Phe Ile Lys Ala Asn Lys Lys Lys Val Cys Lys Asp
   65                  70                  75                  80
   Val Lys Leu Gly Val Arg Glu Asn Asp Lys Val Leu Arg Val Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Gly Ser Cys Asn Asn Lys Asn Gln Asp Glu Cys Gln Lys
       130                 135                 140
   Lys Leu Glu Lys Val Phe Val Ser Leu Thr Asn Gly Tyr Lys Cys Asp
   145                 150                 155                 160
   Lys Cys Lys Ser Gly Thr Ser Thr Val Asn Lys Lys Trp Ile Trp Lys
                   165                 170                 175
   Lys Ser Ser Gly Asn Glu Lys Gly Leu Gln Lys Glu Tyr Ala Asn Thr
               180                 185                 190
   Ile Gly Leu Pro Pro Arg Thr Gln Ser Leu Tyr Leu Gly Asn Leu Pro
           195                 200                 205
   Lys Leu Gly Asn Val Cys Glu Asp Val Thr Asp Ile Asn Phe Asp Thr
       210                 215                 220
   Lys Glu Lys Phe Leu Ala Gly Cys Leu Ile Ala Ala Phe His Glu Gly
   225                 230                 235                 240
   Lys Asn Leu Lys Ile Ser His Glu Lys Lys Lys Gly Asp Asn Gly Lys
                   245                 250                 255
   Lys Leu Cys Lys Ala Leu Glu Tyr Ser Phe Ala Asp Tyr Gly Asp Leu
               260                 265                 270
   Ile Lys Gly Thr Ser Ile Trp Asp Asn Glu Tyr Thr Lys Asp Leu Glu
           275                 280                 285
   Leu Asn Leu Gln Lys Ala Phe Gly Lys Leu Phe Gly Lys Tyr Ile Lys
       290                 295                 300
   Lys Asn Ile Ala Ser Asp Glu Asn Thr Ser Tyr Ser Ser Leu Asp Glu
   305                 310                 315                 320
   Leu Arg Glu Ser Trp Trp Asn Thr Asn Lys Lys Tyr Ile Trp Thr Ala
                   325                 330                 335
   Met Lys His Gly Ala Glu Met Asn Ser Thr Met Cys Asn Ala Asp Gly
               340                 345                 350
   Ser Val Thr Gly Ser Gly Ser Ser Cys Asp Asp Ile Pro Thr Thr Asp
           355                 360                 365
   Phe Ile Pro Gln Tyr Leu Arg Phe Leu Gln Glu Trp Val Glu His Phe
       370                 375                 380
   Cys Lys Gln Arg Gln Glu Lys Val Asn Ala Val Ile Glu Asn Cys Asn
   385                 390                 395                 400
   Ser Cys Lys Asn Thr Ser Gly Glu Arg Lys Ile Gly Gly Thr Cys Asn
                   405                 410                 415
   Gly Asp Cys Lys Thr Glu Cys Lys Asn Lys Cys Glu Ala Tyr Lys Asn
   420 425 430
   Phe Ile Glu Asp Cys Lys Gly Gly Asp Gly Thr Ala Gly Ser Ser Trp
   435 440 445
   Val Lys Arg Trp Asp Gln Ile Tyr Lys Arg Tyr Ser Lys His Ile Glu
   450 455 460
   Asp Ala Lys Arg Asn Arg Lys Ala Gly Thr Lys Ser Cys Gly Pro Ser
   465 470 475 480
   Ser Ile Thr Asn Ala Ser Val Ser Thr Asp Glu Asn Lys Cys Val Gln
   485 490 495
   Ser Asp Ile Asp Ser Phe Phe Lys His Leu Ile Asp Ile Gly Leu Thr
   500 505 510
   Thr Pro Ser Ser Tyr Leu Ser Ile Val Leu Asp Glu Asn Asn Cys Gly
   515 520 525
   Glu Asp Asn Ala Pro Trp Thr Thr Tyr Thr Thr Tyr Thr Thr Thr Glu
   530 535 540
   Lys Cys Asn Lys Asp Lys Lys Lys Ser Lys Ser Gln Ser Cys Asn Thr
   545 550 555 560
   Ala Val Val Val Asn Val Pro Ser Pro Leu Gly Asn Thr Pro His Glu
   565 570 575
   Tyr Lys Tyr Ala Cys Gln Cys Lys Ile Pro Thr Thr Glu Glu Thr Cys
   580 585 590
   Asp Asp Arg Lys Glu Tyr Met Asn Gln Trp Ile Ser Asp Thr Ser Lys
   595 600 605
   Lys Gln Lys Gly Ser Gly Ser Thr Asn Asn Asp Tyr Glu Leu Tyr Thr
   610 615 620
   Tyr Thr Gly Val Lys Glu Thr Lys Leu Pro Lys Lys Leu Asn Ser Pro
   625 630 635 640
   Lys Leu Asp
<210> 5
   <211> 640
   <212> PRT
   <213> Artificial
<220>
   <223> Artificial construct
<400> 5
   Ser Tyr Val Lys Asn Asp Pro Tyr Ser Lys Glu Tyr Val Thr Lys Leu
   1               5                   10                  15
   Ser Phe Ile Leu Asn Pro Ser Asp Ala Asn Asn Pro Ser Gly Glu Thr
               20                  25                  30
   Ala Asn His Asn Asp Glu Ala Cys Asn Pro Asn Glu Ser Glu Ile Ala
           35                  40                  45
   Ser Val Gly Gln Ala Gln Thr Ser Asp Arg Leu Ser Gln Lys Ala Cys
       50                  55                  60
   Ile Thr His Ser Phe Ile Gly Ala Asn Lys Lys Ile Val Cys Lys Asp
   65                  70                  75                  80
   Val Lys Leu Gly Val Arg Glu Lys Asp Lys Asp Leu Lys Ile Cys Val
                   85                  90                  95
   Ile Glu Asp Asp Ser Leu Arg Gly Val Glu Asn Cys Cys Phe Lys Asp
               100                 105                 110
   Leu Leu Gly Ile Leu Gln Glu Asn Cys Ser Asp Asn Lys Ser Gly Ser
           115                 120                 125
   Ser Ser Asn Gly Ser Cys Asn Asn Lys Asn Gln Asp Glu Cys Gln Lys
       130                 135                 140
   Lys Leu Asp Glu Ala Leu Ala Ser Leu His Asn Gly Tyr Lys Cys Asp
   145                 150                 155                 160
   Lys Cys Lys Ser Gly Thr Ser Arg Ser Lys Lys Ile Trp Thr Trp Arg
                   165                 170                 175
   Lys Phe Pro Gly Asn Gly Glu Gly Leu Gln Lys Glu Tyr Ala Asn Thr
               180                 185                 190
   Ile Gly Leu Pro Pro Arg Thr Gln Ser Leu Tyr Leu Gly Asn Leu Arg
           195                 200                 205
   Lys Leu Glu Asn Val Cys Lys Gly Val Thr Asp Ile Asn Phe Asp Thr
       210                 215                 220
   Lys Glu Lys Phe Leu Ala Gly Cys Leu Ile Ala Ala Phe His Glu Gly
   225 230 235 240
   Lys Asn Leu Lys Ile Ser Asn Lys Lys Lys Asn Asp Asp Asn Gly Lys
   245 250 255
   Lys Leu Cys Lys Asp Leu Lys Tyr Ser Phe Ala Asp Tyr Gly Asp Leu
   260 265 270
   Ile Lys Gly Thr Ser Ile Trp Asp Asn Glu Tyr Thr Lys Asp Leu Glu
   275 280 285
   Leu Asn Leu Gln Lys Ile Phe Gly Lys Leu Phe Arg Lys Tyr Ile Lys
   290 295 300
   Lys Asn Ile Ala Ser Asp Glu Asn Thr Leu Tyr Ser Ser Leu Asp Glu
   305 310 315 320
   Leu Arg Glu Ser Trp Trp Asn Thr Asn Lys Lys Tyr Ile Trp Leu Ala
   325 330 335
   Met Lys His Gly Thr Thr Cys Ser Ser Gly Ser Gly Asp Asn Gly Asp
   340 345 350
   Gly Ser Val Thr Gly Ser Gly Ser Ser Cys Asp Asp Met Ser Thr Ile
   355 360 365
   Asp Leu Ile Pro Gln Tyr Leu Arg Phe Leu Gln Glu Trp Val Glu His
   370 375 380
   Phe Cys Lys Gln Arg Gln Glu Lys Val Lys Asp Val Ile Glu Asn Cys
   385 390 395 400
   Lys Ser Cys Lys Asn Thr Ser Gly Glu Arg Ile Ile Gly Gly Thr Cys
   405 410 415
   Gly Ser Asp Cys Lys Thr Lys Cys Lys Gly Glu Cys Asp Ala Tyr Lys
   420 425 430
   Asn Phe Ile Glu Glu Cys Lys Arg Gly Asp Gly Thr Ala Gly Ser Pro
   435 440 445
   Trp Ser Lys Arg Trp Asp Gln Ile Tyr Met Arg Tyr Ser Lys Tyr Ile
   450 455 460
   Glu Asp Ala Lys Arg Asn Arg Lys Ala Gly Thr Lys Asn Cys Gly Thr
   465 470 475 480
   Ser Ser Thr Thr Asn Ala Ala Glu Asn Lys Cys Val Gln Ser Asp Ile
   485 490 495
   Asp Ser Phe Phe Lys His Leu Ile Asp Ile Gly Leu Thr Thr Pro Ser
   500 505 510
   Ser Tyr Leu Ser Ile Val Leu Asp Glu Asn Ile Cys Gly Asp Asp Lys
   515 520 525
   Ala Pro Trp Thr Thr Tyr Thr Thr Tyr Thr Thr Thr Glu Lys Cys Asn
   530 535 540
   Lys Glu Thr Asp Lys Ser Lys Ser Gln Ser Cys Asn Thr Ala Val Val
   545 550 555 560
   Val Asn Val Pro Ser Pro Leu Gly Asn Thr Pro His Gly Tyr Lys Tyr
   565 570 575
   Ala Cys Glu Cys Lys Ile Pro Thr Thr Glu Glu Thr Cys Asp Asp Arg
   580 585 590
   Lys Glu Tyr Met Asn Gln Trp Ile Ser Asp Thr Ser Lys Lys Pro Lys
   595 600 605
   Gly Gly Arg Ser Thr Asn Asn Asp Tyr Glu Leu Tyr Thr Tyr Asn Gly
   610 615 620
   Val Lys Glu Thr Lys Leu Pro Lys Lys Ser Ser Ser Ser Lys Leu Asp
   625 630 635 640
<210> 6
   <211> 358
   <212> PRT
   <213> Plasmodium falciparum
<400> 6
   Lys Cys Glu Lys Cys Lys Ser Glu Gln Ser Lys Lys Asn Asn Asn Ile
   1 5 10 15
   Trp Ile Trp Arg Lys Phe Pro Gly Asn Gly Glu Gly Leu Gln Lys Glu
   20 25 30
   Tyr Ala Asn Thr Ile Gly Leu Pro Pro Arg Thr His Ser Leu Tyr Leu
   35 40 45
   Gly Asn Leu Pro Lys Leu Glu Asn Val Cys Lys Asp Val Lys Asp Ile
   50 55 60
   Asn Phe Asp Thr Lys Glu Lys Phe Leu Ala Gly Cys Leu Ile Ala Ala
   65                  70                  75                  80
   Phe His Glu Gly Lys Asn Leu Lys Thr Thr Tyr Pro Gln Asn Lys Asn
                   85                  90                  95
   Ala Asp Asn Asn Ser Lys Leu Cys Lys Asp Leu Lys Tyr Ser Phe Ala
               100                 105                 110
   Asp Tyr Gly Asp Leu Ile Lys Gly Thr Ser Ile Trp Asp Asn Asp Phe
           115                 120                 125
   Thr Lys Asp Leu Glu Leu Asn Leu Gln Lys Ile Phe Gly Lys Leu Phe
       130                 135                 140
   Arg Lys Tyr Ile Lys Lys Asn Ile Ala Ser Asp Glu Asn Thr Leu Tyr
   145                 150                 155                 160
   Ser Ser Leu Asp Glu Leu Arg Glu Ser Trp Trp Asn Thr Asn Lys Lys
                   165                 170                 175
   Tyr Ile Trp Leu Ala Met Lys His Gly Ala Glu Met Asn Ser Thr Met
               180                 185                 190
   Cys Asn Gly Asp Gly Ser Val Thr Gly Ser Ser Asp Ser Gly Ser Thr
           195                 200                 205
   Thr Cys Ser Gly Asp Asn Gly Ser Ile Ser Cys Asp Asp Ile Pro Thr
       210                 215                 220
   Ile Asp Leu Ile Pro Gln Tyr Leu Arg Phe Leu Gln Glu Trp Val Glu
   225                 230                 235                 240
   His Phe Cys Lys Gln Arg Gln Glu Lys Val Lys Pro Val Ile Glu Asn
                   245                 250                 255
   Cys Lys Ser Cys Lys Asn Thr Ser Gly Glu Arg Ile Ile Gly Gly Thr
               260                 265                 270
   Cys Gly Ser Asp Cys Glu Lys Lys Cys Lys Gly Glu Cys Asp Ala Tyr
           275                 280                 285
   Lys Lys Phe Ile Glu Glu Cys Lys Gly Gly Gly Gly Gly Thr Gly Thr
       290                 295                 300
   Ala Gly Ser Pro Trp Ser Lys Arg Trp Asp Gln Ile Tyr Lys Arg Tyr
   305                 310                 315                 320
   Ser Lys Tyr Ile Glu Asp Ala Lys Arg Asn Arg Lys Ala Gly Thr Lys
                   325                 330                 335
   Ser Cys Gly Pro Ser Ser Thr Thr Asn Ala Ala Ala Ser Thr Thr Glu
               340                 345                 350
   Ser Lys Cys Val Gln Ser
           355
<210> 7
   <211> 333
   <212> PRT
   <213> Plasmodium falciparum
<400> 7
   Lys Cys Asp Lys Cys Lys Ser Glu Gln Ser Lys Lys Asn Asn Lys Asn
   1               5                   10                  15
   Trp Ile Trp Lys Gln Phe Pro Gly Asn Gly Glu Gly Leu Gln Lys Glu
               20                  25                  30
   Tyr Ala Asn Thr Ile Gly Leu Pro Pro Arg Thr His Ser Leu Tyr Leu
           35                  40                  45
   Gly Asn Leu Pro Lys Leu Glu Asn Val Cys Lys Gly Val Thr Asp Ile
       50                  55                  60
   Asn Phe Asp Thr Lys Glu Lys Phe Leu Ala Gly Cys Leu Ile Ala Ala
   65                  70                  75                  80
   Phe His Glu Gly Lys Asn Leu Lys Thr Ser His Glu Lys Lys Lys Gly
                   85                  90                  95
   Asp Asn Gly Lys Lys Leu Cys Lys Asp Leu Lys Tyr Ser Phe Ala Asp
               100                 105                 110
   Tyr Gly Asp Leu Ile Lys Gly Thr Ser Ile Trp Asp Asn Asp Phe Thr
           115                 120                 125
   Lys Asp Leu Glu Leu Asn Leu Gln Gln Ile Phe Gly Lys Leu Phe Arg
       130                 135                 140
   Lys Tyr Ile Lys Lys Asn Ile Ser Ala Glu Gln Asp Thr Ser Tyr Ser
   145                 150                 155                 160
   Ser Leu Asp Glu Leu Arg Glu Ser Trp Trp Asn Thr Asn Lys Lys Tyr
                   165                 170                 175
   Ile Trp Leu Ala Met Lys His Gly Thr Thr Cys Ser Ser Gly Ser Gly
               180                 185                 190
   Asp Asn Gly Asp Gly Ser Val Thr Gly Ser Gly Ser Ser Cys Asp Asp
           195                 200                 205
   Met Pro Thr Thr Asp Phe Ile Pro Gln Tyr Leu Arg Phe Leu Gln Glu
       210                 215                 220
   Trp Val Glu His Phe Cys Lys Gln Arg Gln Glu Lys Val Asn Ala Val
   225                 230                 235                 240
   Ile Thr Asn Cys Lys Ser Cys Lys Glu Ser Gly Gly Thr Cys Asn Ser
                   245                 250                 255
   Asp Cys Glu Lys Lys Cys Lys Asp Glu Cys Glu Lys Tyr Lys Lys Phe
               260                 265                 270
   Ile Glu Glu Cys Arg Thr Ala Ala Asp Gly Thr Ala Gly Ser Ser Trp
           275                 280                 285
   Ser Lys Arg Trp Asp Gln Ile Tyr Lys Met Tyr Ser Lys His Ile Glu
       290                 295                 300
   Asp Ala Lys Arg Asn Arg Lys Ala Gly Thr Lys Asn Cys Gly Thr Ser
   305                 310                 315                 320
   Ser Thr Thr Asn Ala Ala Glu Asn Lys Cys Val Gln Ser
                   325                 330
<210> 8
   <211> 269
   <212> PRT
   <213> Plasmodium falciparum
<400> 8
   Asp Tyr Ile Lys Asp Asp Pro Tyr Ser Lys Glu Tyr Thr Thr Lys Leu
   1               5                   10                  15
   Ser Phe Ile Leu Asn Ser Ser Asp Ala Asn Thr Ser Ser Gly Glu Thr
               20                  25                  30
   Ala Asn His Asn Asp Glu Ala Cys Asn Cys Asn Glu Ser Glu Ile Ala
           35                  40                  45
   Ser Val Glu Gln Ala Ser Ile Ser Asp Arg Ser Ser Gln Lys Ala Tyr
       50                  55                  60
   Ile Thr His Ser Ser Ile Lys Thr Asn Lys Lys Lys Val Cys Lys Tyr
   65 70 75 80
   Val Lys Leu Gly Ile Asn Asn Asn Asp Lys Val Leu Arg Val Cys Val
   85 90 95
   Ile Glu Asp Thr Ser Leu Ser Gly Val Glu Asn Cys Cys Phe Lys Asp
   100 105 110
   Leu Leu Gly Ile Leu Gln Glu Asn Cys Ser Asp Asn Lys Arg Gly Ser
   115 120 125
   Ser Phe Asn Asp Ser Cys Asn Asn Asn Asn Glu Glu Ala Cys Gln Lys
   130 135 140
   Lys Leu Glu Lys Val Leu Ala Ser Leu Thr Asn Gly Tyr Lys Cys Glu
   145 150 155 160
   Lys Cys Lys Ser Gly Thr Ser Arg Ser Lys Lys Lys Trp Ile Trp Lys
   165 170 175
   Lys Ser Ser Gly Lys Glu Gly Gly Leu Gln Lys Glu Tyr Ala Asn Thr
   180 185 190
   Ile Gly Leu Pro Pro Arg Thr Gln Ser Leu Tyr Leu Gly Asn Leu Pro
   195 200 205
   Lys Leu Glu Asn Val Cys Lys Gly Val Thr Asp Ile Asn Phe Asp Thr
   210 215 220
   Lys Glu Lys Phe Leu Ala Gly Cys Leu Ile Ala Ala Phe His Glu Gly
   225 230 235 240
   Lys Asn Leu Lys Pro Ser His Gln Asn Lys Asn Asp Asp Asn Asn Ser
   245 250 255
   Lys Leu Cys Lys Asp Leu Lys Tyr Ser Phe Ala Asp Tyr
   260 265
<210> 9
   <211> 333
   <212> PRT
   <213> Plasmodium falciparum
<400> 9
   Lys Cys Asp Lys Cys Lys Ser Gly Thr Ser Arg Ser Lys Lys Lys Trp
   1 5 10 15
   Thr Trp Arg Lys Ser Ser Gly Asn Lys Glu Gly Leu Gln Lys Glu Tyr
               20                  25                  30
   Ala Asn Thr Ile Gly Leu Pro Pro Arg Thr His Ser Leu Tyr Leu Gly
           35                  40                  45
   Asn Leu Arg Lys Leu Glu Asn Val Cys Glu Asp Val Thr Asp Ile Asn
       50                  55                  60
   Phe Asp Thr Lys Glu Lys Phe Leu Ala Gly Cys Leu Ile Ala Ala Phe
   65                  70                  75                  80
   His Glu Gly Lys Asn Leu Lys Thr Thr Tyr Pro Gln Asn Lys Asn Asp
                   85                  90                  95
   Asp Asn Asn Ser Lys Leu Cys Lys Ala Leu Lys Tyr Ser Phe Ala Asp
               100                 105                 110
   Tyr Gly Asp Leu Ile Lys Gly Thr Ser Ile Trp Asp Asn Asp Phe Thr
           115                 120                 125
   Lys Asp Leu Glu Leu Asn Leu Gln Lys Ile Phe Gly Lys Leu Phe Arg
       130                 135                 140
   Lys Tyr Ile Lys Lys Asn Ile Ser Thr Glu Gln His Thr Ser Tyr Ser
   145                 150                 155                 160
   Ser Leu Asp Glu Leu Arg Glu Ser Trp Trp Asn Thr Asn Lys Lys Tyr
                   165                 170                 175
   Ile Trp Leu Ala Met Lys His Gly Ala Glu Met Asn Gly Thr Thr Cys
               180                 185                 190
   Ser Cys Ser Gly Asp Ser Ser Asp Asp Ile Pro Thr Ile Asp Leu Ile
           195                 200                 205
   Pro Gln Tyr Leu Arg Phe Leu Gln Glu Trp Val Glu His Phe Cys Lys
       210                 215                 220
   Gln Arg Gln Ala Lys Val Asn Ala Val Ile Asn Ser Cys Asn Ser Cys
   225                 230                 235                 240
   Lys Asn Thr Ser Gly Glu Arg Lys Leu Gly Gly Thr Cys Gly Ser Glu
                   245                 250                 255
   Cys Lys Thr Glu Cys Lys Asn Lys Cys Asp Ala Tyr Lys Glu Phe Ile
                   260                 265                 270
   Asp Gly Thr Gly Ser Gly Gly Gly Thr Gly Thr Ala Gly Ser Ser Trp
           275                 280                 285
   Val Lys Arg Trp Asp Gln Ile Tyr Lys Arg Tyr Ser Lys Tyr Ile Glu
       290                 295                 300
   Asp Ala Lys Arg Asn Arg Lys Ala Gly Ser Lys Asn Cys Gly Thr Ser
   305                 310                 315                 320
   Ser Thr Thr Asn Ala Ala Glu Ser Lys Cys Val Gln Ser
                   325                 330
<210> 10
   <211> 650
   <212> PRT
   <213> Artificial
<220>
   <223> Artificial construct
<400> 10
   Ser Tyr Val Lys Asn Asn Pro Tyr Ser Ala Glu Tyr Val Thr Lys Leu
   1               5                   10                  15
   Ser Phe Ile Leu Asn Ser Ser Asp Ala Asn Thr Ser Ser Glu Thr Pro
               20                  25                  30
   Ser Lys Tyr Tyr Asp Glu Val Cys Asn Cys Asn Glu Ser Glu Ile Ser
           35                  40                  45
   Ser Val Gly Gln Ala Gln Thr Ser Gly Pro Ser Ser Asn Lys Thr Cys
       50                  55                  60
   Ile Thr His Ser Ser Ile Lys Thr Asn Lys Lys Lys Val Cys Lys Asp
   65                  70                  75                  80
   Val Lys Leu Gly Ile Asn Asn Asn Asp Lys Val Leu Arg Val Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Lys Asn Gln Ser Gly
           115                 120                 125
   Ser Ser Ser Asn Gly Ser Cys Asn Asn Lys Asn Gln Asp Glu Cys Gln
       130                 135                 140
   Lys Lys Leu Glu Lys Val Phe Ala Ser Leu Thr Asn Gly Tyr Lys Cys
   145                 150                 155                 160
   Asp Lys Cys Lys Ser Gly Thr Ser Arg Ser Lys Lys Lys Trp Ile Trp
                   165                 170                 175
   Arg Lys Ser Ser Gly Asn Glu Glu Gly Leu Gln Lys Glu Tyr Ala Asn
               180                 185                 190
   Thr Ile Gly Leu Pro Pro Arg Thr Gln Ser Leu Tyr Leu Gly Asn Leu
           195                 200                 205
   Arg Lys Leu Glu Asn Val Cys Lys Gly Val Thr Asp Ile Asn Phe Asp
       210                 215                 220
   Thr Lys Glu Lys Phe Leu Ala Gly Cys Leu Ile Ala Ala Phe His Glu
   225                 230                 235                 240
   Gly Lys Asn Leu Lys Thr Thr Tyr Pro Gln Asn Lys Lys Lys Leu Cys
                   245                 250                 255
   Lys Asp Leu Lys Tyr Ser Phe Ala Asp Tyr Gly Asp Leu Ile Lys Gly
               260                 265                 270
   Thr Ser Ile Trp Asp Asn Glu Tyr Thr Lys Asp Leu Glu Leu Asn Leu
           275                 280                 285
   Gln Lys Ala Phe Gly Lys Leu Phe Arg Lys Tyr Ile Lys Lys Asn Ile
       290                 295                 300
   Ser Thr Glu Gln His Thr Leu Tyr Ser Ser Leu Asp Glu Leu Arg Glu
   305                 310                 315                 320
   Ser Trp Trp Asn Thr Asn Lys Lys Tyr Ile Trp Leu Ala Met Lys His
                   325                 330                 335
   Gly Ala Gly Met Asn Ser Thr Thr Cys Cys Gly Asp Gly Ser Val Thr
               340                 345                 350
   Gly Ser Gly Ser Ser Cys Asp Asp Ile Pro Thr Ile Asp Leu Ile Pro
           355                 360                 365
   Gln Tyr Leu Arg Phe Leu Gln Glu Trp Val Glu His Phe Cys Lys Gln
       370                 375                 380
   Arg Gln Glu Lys Val Asn Ala Val Ile Glu Asn Cys Asn Ser Cys Lys
   385 390 395 400
   Glu Cys Gly Asp Thr Cys Asn Gly Glu Cys Lys Thr Glu Cys Glu Lys
   405 410 415
   Lys Cys Lys Ile Glu Cys Glu Lys Tyr Lys Thr Phe Ile Glu Glu Cys
   420 425 430
   Val Thr Ala Val Gly Gly Thr Ser Gly Ser Pro Trp Ser Lys Arg Trp
   435 440 445
   Asp Gln Ile Tyr Lys Arg Tyr Ser Lys Tyr Ile Glu Asp Ala Lys Arg
   450 455 460
   Asn Arg Lys Ala Gly Thr Lys Asn Cys Gly Ile Thr Thr Gly Thr Ile
   465 470 475 480
   Ser Gly Glu Ser Ser Gly Ala Asn Ser Gly Val Thr Thr Thr Glu Asn
   485 490 495
   Lys Cys Val Gln Ser Asp Ile Asp Ser Phe Phe Lys His Leu Ile Asp
   500 505 510
   Ile Gly Leu Thr Thr Pro Ser Ser Tyr Leu Ser Ile Val Leu Asp Asp
   515 520 525
   Asn Ile Cys Gly Ala Asp Asn Ala Pro Trp Thr Thr Tyr Thr Thr Tyr
   530 535 540
   Thr Thr Tyr Thr Thr Thr Lys Asn Cys Asp Ile Lys Lys Lys Thr Pro
   545 550 555 560
   Lys Ser Gln Pro Ile Asn Thr Ser Val Val Val Asn Val Pro Ser Pro
   565 570 575
   Leu Gly Asn Thr Pro His Gly Tyr Lys Tyr Ala Cys Gln Cys Lys Ile
   580 585 590
   Pro Thr Thr Glu Glu Ser Cys Asp Asp Arg Lys Glu Tyr Met Asn Gln
   595 600 605
   Trp Ile Ile Asp Thr Ser Lys Lys Gln Lys Gly Ser Gly Ser Thr Asn
   610 615 620
   Asn Asp Tyr Glu Leu Tyr Thr Tyr Asn Gly Val Lys Glu Thr Lys Leu
   625 630 635 640
   Pro Lys Lys Ser Ser Ser Ser Lys Leu Asp
   645 650
<210> 11
   <211> 643
   <212> PRT
   <213> Artificial
<220>
   <223> Artificial construct
<400> 11
   Ser Tyr Val Lys Asp Asp Pro Tyr Ser Ala Glu Tyr Val Thr Lys Leu
   1               5                   10                  15
   Ser Phe Ile Leu Asn Ser Ser Asp Ala Asn Thr Ser Ser Glu Thr Pro
               20                  25                  30
   Ser Lys Tyr Tyr Asp Glu Val Cys Asn Cys Asn Glu Ser Glu Ile Ser
           35                  40                  45
   Ser Val Gly Gln Ala Gln Thr Ser Gly Pro Ser Ser Asn Lys Thr Cys
       50                  55                  60
   Ile Thr His Ser Ser Ile Lys Thr Asn Lys Lys Lys Val Cys Lys Asp
   65                  70                  75                  80
   Val Lys Leu Gly Ile Asn Asn Asn Asp Lys Val Leu Arg Val Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Lys Asn Gln Ser Gly
           115                 120                 125
   Ser Ser Ser Asn Gly Ser Cys Asn Asn Lys Asn Gln Asp Glu Cys Gln
       130                 135                 140
   Lys Lys Leu Glu Lys Val Phe Ala Ser Leu Thr Asn Gly Tyr Lys Cys
   145                 150                 155                 160
   Asp Lys Cys Lys Ser Gly Thr Ser Arg Ser Lys Lys Lys Trp Ile Trp
                   165                 170                 175
   Arg Lys Ser Ser Gly Asn Glu Glu Gly Leu Gln Lys Glu Tyr Ala Asn
               180                 185                 190
   Thr Ile Gly Leu Pro Pro Arg Thr Gln Ser Leu Tyr Leu Gly Asn Leu
           195                 200                 205
   Pro Lys Leu Glu Asn Val Cys Lys Gly Val Thr Asp Ile Ile Tyr Asp
       210                 215                 220
   Thr Lys Glu Lys Phe Leu Ser Gly Cys Leu Ile Ala Ala Phe His Glu
   225                 230                 235                 240
   Gly Lys Asn Leu Lys Thr Ser His Glu Lys Lys Asn Asp Asp Asn Gly
                   245                 250                 255
   Lys Lys Leu Cys Lys Ala Leu Glu Tyr Ser Phe Ala Asp Tyr Gly Asp
               260                 265                 270
   Leu Ile Lys Gly Thr Ser Ile Trp Asp Asn Asp Phe Thr Lys Asp Leu
           275                 280                 285
   Glu Leu Asn Leu Gln Lys Ile Phe Gly Lys Leu Phe Arg Lys Tyr Ile
       290                 295                 300
   Lys Lys Asn Asn Thr Ala Glu Gln Asp Thr Ser Tyr Ser Ser Leu Asp
   305                 310                 315                 320
   Glu Leu Arg Glu Ser Trp Trp Asn Thr Asn Lys Lys Tyr Ile Trp Thr
                   325                 330                 335
   Ala Met Lys His Gly Ala Gly Met Asn Ser Thr Thr Cys Ser Gly Asp
               340                 345                 350
   Gly Ser Val Thr Gly Ser Gly Ser Ser Cys Asp Asp Met Pro Thr Ile
           355                 360                 365
   Asp Leu Ile Pro Gln Tyr Leu Arg Phe Leu Gln Glu Trp Val Glu His
       370                 375                 380
   Phe Cys Lys Gln Arg Gln Glu Lys Val Lys Asp Val Ile Thr Asn Cys
   385                 390                 395                 400
   Asn Ser Cys Lys Glu Cys Gly Asp Thr Cys Asn Gly Glu Cys Lys Thr
                   405                 410                 415
   Glu Cys Lys Thr Lys Cys Lys Gly Glu Cys Glu Lys Tyr Lys Asn Phe
               420                 425                 430
   Ile Glu Glu Cys Asn Gly Thr Ala Asp Gly Gly Thr Ser Gly Ser Ser
           435                 440                 445
   Trp Ser Lys Arg Trp Asp Gln Ile Tyr Lys Arg Tyr Ser Lys Tyr Ile
       450                 455                 460
   Glu Asp Ala Lys Arg Asn Arg Lys Ala Gly Thr Lys Asn Cys Gly Thr
   465                 470                 475                 480
   Ser Ser Thr Thr Asn Ala Ala Ala Ser Thr Thr Glu Asn Lys Cys Val
                   485                 490                 495
   Gln Ser Asp Ile Asp Ser Phe Phe Lys His Leu Ile Asp Ile Gly Leu
               500                 505                 510
   Thr Thr Pro Ser Ser Tyr Leu Ser Asn Val Leu Asp Asp Asn Ile Cys
           515                 520                 525
   Gly Glu Asp Lys Ala Pro Trp Thr Thr Tyr Thr Thr Tyr Thr Thr Lys
       530                 535                 540
   Asn Cys Asp Ile Gln Lys Lys Thr Pro Lys Pro Gln Ser Cys Asp Thr
   545                 550                 555                 560
   Leu Val Val Val Asn Val Pro Ser Pro Leu Gly Asn Thr Pro His Gly
                   565                 570                 575
   Tyr Lys Tyr Val Cys Glu Cys Lys Ile Pro Thr Thr Glu Glu Thr Cys
               580                 585                 590
   Asp Asp Arg Lys Glu Tyr Met Asn Gln Trp Ile Ile Asp Thr Ser Lys
           595                 600                 605
   Lys Gln Lys Gly Ser Gly Ser Thr Asn Asn Asp Tyr Glu Leu Tyr Thr
       610                 615                 620
   Tyr Asn Gly Val Gln Ile Lys Gln Ala Ala Gly Thr Leu Lys Asn Ser
   625                 630                 635                 640
   Lys Leu Asp
<210> 12
   <211> 269
   <212> PRT
   <213> Plasmodium falciparum
<400> 12
   Asn Tyr Ile Lys Gly Asp Pro Tyr Ser Ala Glu Tyr Ala Thr Lys Leu
   1               5                   10                  15
   Ser Phe Ile Leu Asn Ser Ser Asp Thr Glu Asn Ala Ser Glu Lys Ile
               20                  25                  30
   Gln Lys Asn Asn Asp Glu Val Cys Asn Cys Asn Glu Ser Glu Ile Ala
           35                  40                  45
   Ser Val Glu Gln Ala Pro Ile Ser Asp Arg Ser Ser Gln Lys Ala Cys
       50                  55                  60
   Ile Thr His Ser Ser Ile Lys Ala Asn Lys Lys Lys Val Cys Lys His
   65                  70                  75                  80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Ser Gly Ser
           115                 120                 125
   Ser Ser Asn Gly Ser Cys Asn Asn Asn Asn Glu Glu Ile Cys Gln Lys
       130                 135                 140
   Lys Leu Glu Lys Val Leu Ala Ser Leu Thr Asn Gly Tyr Lys Cys Asp
   145                 150                 155                 160
   Lys Cys Lys Ser Gly Thr Ser Thr Val Asn Lys Asn Trp Ile Trp Lys
                   165                 170                 175
   Lys Tyr Ser Gly Lys Glu Gly Gly Leu Gln Glu Glu Tyr Ala Asn Thr
               180                 185                 190
   Ile Gly Leu Pro Pro Arg Thr Gln Ser Leu Tyr Leu Gly Asn Leu Pro
           195                 200                 205
   Lys Leu Glu Asn Val Cys Glu Asp Val Lys Asp Ile Asn Phe Asp Thr
       210                 215                 220
   Lys Glu Lys Phe Leu Ala Gly Cys Leu Ile Ala Ala Phe His Glu Gly
   225                 230                 235                 240
   Lys Asn Leu Lys Thr Ser Asn Lys Lys Lys Asn Asp Asp Asn Asn Ser
                   245                 250                 255
   Lys Leu Cys Lys Ala Leu Lys Tyr Ser Phe Ala Asp Tyr
               260                 265
<210> 13
   <211> 344
   <212> PRT
   <213> Plasmodium falciparum
<400> 13
   Lys Cys Asp Lys Cys Lys Ser Gly Thr Ser Thr Val Asn Lys Lys Trp
   1               5                   10                  15
   Ile Trp Lys Lys Tyr Ser Gly Thr Glu Gly Gly Leu Gln Glu Glu Tyr
               20                  25                  30
   Ala Asn Thr Ile Ala Leu Pro Pro Arg Thr Gln Ser Leu Tyr Leu Gly
           35                  40                  45
   Asn Leu Pro Lys Leu Glu Asn Val Cys Lys Asp Val Thr Asp Ile Asn
       50                  55                  60
   Phe Asp Thr Lys Glu Lys Phe Leu Ala Gly Cys Leu Ile Ala Ala Phe
   65                  70                  75                  80
   His Glu Gly Lys Asn Leu Lys Thr Thr Tyr Leu Glu Lys Lys Lys Gly
                   85                  90                  95
   Asp Asn Gly Lys Lys Asn Asp Asp Asn Asn Ser Lys Leu Cys Lys Ala
               100                 105                 110
   Leu Lys Tyr Ser Phe Ala Asp Tyr Gly Asp Leu Ile Lys Gly Thr Ser
           115                 120                 125
   Ile Trp Asp Asn Asp Phe Thr Lys Asp Leu Glu Leu Asn Leu Gln Gln
       130                 135                 140
   Ile Phe Gly Lys Leu Phe Arg Lys Tyr Ile Lys Lys Asn Ile Ala Ser
   145                 150                 155                 160
   Asp Glu Asn Thr Leu Tyr Ser Ser Leu Asp Glu Leu Arg Glu Ser Trp
                   165                 170                 175
   Trp Asn Thr Asn Lys Lys Tyr Ile Trp Leu Ala Met Lys His Gly Ala
               180                 185                 190
   Gly Met Asn Ser Thr Met Cys Asn Ala Asp Gly Ser Val Thr Gly Ser
           195                 200                 205
   Gly Ser Ser Cys Asp Asp Ile Pro Thr Ile Asp Leu Ile Pro Gln Tyr
       210                 215                 220
   Leu Arg Phe Leu Gln Glu Trp Val Glu His Phe Cys Lys Gln Arg Gln
   225 230 235 240
   Ala Lys Val Lys Asp Val Ile Thr Asn Cys Asn Ser Cys Lys Glu Cys
   245 250 255
   Gly Gly Thr Cys Asn Gly Glu Cys Lys Thr Glu Cys Glu Lys Lys Cys
   260 265 270
   Lys Gly Glu Cys Asp Ala Tyr Lys Lys Phe Ile Glu Glu Cys Lys Gly
   275 280 285
   Lys Ala Asp Glu Gly Thr Ser Gly Ser Ser Trp Ser Lys Arg Trp Asp
   290 295 300
   Gln Ile Tyr Lys Arg Tyr Ser Lys Tyr Ile Glu Asp Ala Lys Arg Asn
   305 310 315 320
   Arg Lys Ala Gly Thr Lys Asn Cys Gly Pro Ser Ser Thr Thr Ser Thr
   325 330 335
   Ala Glu Ser Lys Cys Val Gln Ser
   340
<210> 14
   <211> 334
   <212> PRT
   <213> Plasmodium falciparum
<400> 14
   Lys Cys Asp Lys Cys Lys Ser Glu Gln Ser Lys Lys Asn Asn Asn Ile
   1 5 10 15
   Trp Ile Trp Lys Lys Ser Ser Gly Thr Glu Gly Gly Leu Gln Lys Glu
   20 25 30
   Tyr Ala Asn Thr Ile Ala Leu Pro Pro Arg Thr Gln Ser Leu Tyr Leu
   35 40 45
   Gly Asn Leu Arg Lys Leu Glu Asn Val Cys Glu Asp Val Lys Asp Ile
   50 55 60
   Asn Phe Asp Thr Lys Glu Lys Phe Leu Ala Gly Cys Leu Ile Ala Ala
   65 70 75 80
   Phe His Glu Gly Lys Asn Leu Lys Lys Arg Tyr Leu Glu Lys Lys Asn
   85 90 95
   Gly Asp Asn Asn Ser Lys Leu Cys Lys Ala Leu Lys Tyr Ser Phe Ala
               100                 105                 110
   Asp Tyr Gly Asp Leu Ile Lys Gly Thr Ser Ile Trp Asp Asn Glu Tyr
           115                 120                 125
   Thr Lys Asp Leu Glu Leu Asn Leu Gln Lys Ile Phe Gly Lys Leu Phe
       130                 135                 140
   Arg Lys Tyr Ile Lys Lys Asn Asn Thr Ala Glu Gln His Thr Ser Tyr
   145                 150                 155                 160
   Ser Ser Leu Asp Glu Leu Arg Glu Ser Trp Trp Asn Thr Asn Lys Lys
                   165                 170                 175
   Tyr Ile Trp Leu Ala Met Lys His Gly Thr Thr Cys Ser Ser Gly Ser
               180                 185                 190
   Gly Asp Asn Gly Ser Ile Ser Cys Asp Asp Ile Pro Thr Ile Asp Leu
           195                 200                 205
   Ile Pro Gln Tyr Leu Arg Phe Leu Gln Glu Trp Val Glu His Phe Cys
       210                 215                 220
   Glu Gln Arg Gln Gly Lys Val Asn Ala Val Ile Glu Asn Cys Asn Ser
   225                 230                 235                 240
   Cys Lys Asn Thr Ser Ser Lys Thr Lys Leu Gly Gly Thr Cys Asn Gly
                   245                 250                 255
   Glu Cys Lys Thr Glu Cys Lys Gly Glu Cys Asp Ala Tyr Lys Glu Phe
               260                 265                 270
   Ile Glu Lys Cys Lys Gly Thr Ala Ala Glu Gly Thr Ser Gly Ser Ser
           275                 280                 285
   Trp Val Lys Arg Trp Tyr Gln Ile Tyr Met Arg Tyr Ser Lys Tyr Ile
       290                 295                 300
   Glu Asp Ala Lys Arg Asn Arg Lys Ala Gly Thr Lys Asn Cys Gly Thr
   305                 310                 315                 320
   Ser Ser Thr Thr Ser Thr Ala Glu Ser Lys Cys Val Gln Ser
                   325                 330
<210> 15
   <211> 332
   <212> PRT
   <213> Plasmodium falciparum
<400> 15
   Lys Cys Asp Lys Cys Lys Ser Glu Gln Ser Lys Lys Asn Asn Asn Ile
   1               5                   10                  15
   Trp Ile Trp Lys Lys Ser Ser Gly Thr Glu Gly Gly Leu Gln Lys Glu
               20                  25                  30
   Tyr Ala Asn Thr Ile Ala Leu Pro Pro Arg Thr Gln Ser Leu Tyr Leu
           35                  40                  45
   Gly Asn Leu Arg Lys Leu Glu Asn Val Cys Glu Asp Val Lys Asp Ile
       50                  55                  60
   Asn Phe Asp Thr Lys Glu Lys Phe Leu Ala Gly Cys Leu Ile Ala Ala
   65                  70                  75                  80
   Phe His Glu Gly Lys Asn Leu Lys Lys Arg Tyr Leu Glu Lys Lys Asn
                   85                  90                  95
   Gly Asp Asn Asn Ser Lys Leu Cys Lys Ala Leu Lys Tyr Ser Phe Ala
               100                 105                 110
   Asp Tyr Gly Asp Leu Ile Lys Gly Thr Ser Ile Trp Asp Asn Glu Tyr
           115                 120                 125
   Thr Lys Asp Leu Glu Leu Asn Leu Gln Lys Ile Phe Gly Lys Leu Phe
       130                 135                 140
   Arg Lys Tyr Ile Lys Lys Asn Asn Thr Ala Glu Gln Asp Thr Ser Tyr
   145                 150                 155                 160
   Ser Ser Leu Asp Glu Leu Arg Glu Ser Trp Trp Asn Thr Asn Lys Lys
                   165                 170                 175
   Tyr Ile Trp Thr Ala Met Lys His Gly Thr Thr Cys Ser Ser Gly Ser
               180                 185                 190
   Gly Asp Asn Gly Ser Ile Ser Cys Asp Asp Ile Pro Thr Ile Asp Leu
           195                 200                 205
   Ile Pro Gln Tyr Leu Arg Phe Leu Gln Glu Trp Val Glu His Phe Cys
       210                 215                 220
   Glu Gln Arg Gln Glu Lys Val Lys Asp Val Ile Lys Asn Cys Asn Ser
   225                 230                 235                 240
   Cys Lys Glu Cys Gly Gly Thr Cys Asn Gly Glu Cys Lys Thr Glu Cys
                   245                 250                 255
   Lys Asn Lys Cys Lys Asp Glu Cys Asp Ala Tyr Lys Lys Phe Ile Glu
               260                 265                 270
   Glu Cys Glu Gly Lys Ala Ala Glu Gly Thr Ser Gly Ser Ser Trp Ser
           275                 280                 285
   Lys Arg Trp Asp Gln Ile Tyr Lys Arg Tyr Ser Lys Tyr Ile Glu Asp
       290                 295                 300
   Ala Lys Arg Asn Arg Lys Ala Gly Thr Lys Asn Cys Gly Thr Ser Ser
   305                 310                 315                 320
   Thr Thr Ser Thr Ala Glu Asn Lys Cys Val Gln Ser
                   325                 330
<210> 16
   <211> 267
   <212> PRT
   <213> Plasmodium falciparum
<400> 16
   Asn Tyr Ile Lys Asp Asp Pro Tyr Ser Ala Glu Tyr Thr Thr Lys Leu
   1               5                   10                  15
   Ser Phe Ile Leu Asn Ser Ser Asp Thr Glu Asn Ala Ser Glu Lys Ile
               20                  25                  30
   Gln Lys Asn Asn Asp Glu Val Cys Asn Pro Asn Glu Ser Gly Ile Ala
           35                  40                  45
   Cys Val Glu Leu Ala Gln Thr Ser Gly Ser Ser Ser Asn Lys Thr Cys
       50                  55                  60
   Asn Thr His Ser Phe Ile Lys Ala Asn Lys Lys Lys Val Cys Lys Asp
   65                  70                  75                  80
   Val Lys Leu Gly Ile Asn Lys Lys Asp Lys Asp Leu Lys Ile Cys Val
                   85                  90                  95
   Ile Glu Asp Asp Ser Leu Arg Gly Val Asp Asn Cys Cys Cys Gln Asp
               100                 105                 110
   Leu Leu Gly Ile Leu Gln Glu Asn Cys Ser Asp Lys Asn Gln Ser Gly
           115                 120                 125
   Ser Ser Ser Asn Gly Ser Cys Asn Asn Lys Asn Gln Glu Ala Cys Gln
   130 135 140
   Lys Lys Leu Glu Asn Val Phe Ala Ser Leu Thr Asn Gly Tyr Lys Cys
   145 150 155 160
   Glu Lys Cys Lys Ser Glu Gln Ser Lys Lys Asn Asn Lys Asn Trp Ile
   165 170 175
   Trp Lys Lys Tyr Ser Val Lys Glu Glu Gly Leu Gln Lys Glu Tyr Ala
   180 185 190
   Asn Thr Ile Ala Leu Pro Pro Arg Thr Gln Ser Leu Tyr Leu Gly Asn
   195 200 205
   Leu Pro Lys Leu Gly Asn Val Cys Lys Gly Val Thr Asp Ile Asn Phe
   210 215 220
   Asp Thr Lys Glu Lys Phe Leu Ala Gly Cys Leu Ile Ala Ala Phe His
   225 230 235 240
   Glu Gly Lys Asn Leu Lys Thr Thr Tyr Leu Gln Asn Lys Lys Lys Leu
   245 250 255
   Cys Lys Ala Leu Lys Tyr Ser Phe Ala Asp Tyr
   260 265
<210> 17
   <211> 263
   <212> PRT
   <213> Plasmodium falciparum
<400> 17
   Asp Tyr Ile Lys Gly Asp Pro Tyr Phe Ala Glu Tyr Ala Thr Lys Leu
   1 5 10 15
   Ser Phe Ile Leu Asn Ser Ser Asp Ala Asn Thr Ser Ser Gly Glu Thr
   20 25 30
   Ala Asn His Asn Asp Glu Ala Cys Asn Pro Asn Glu Ser Glu Ile Ala
   35 40 45
   Ser Val Glu Gln Ala Ser Ile Ser Asp Arg Ser Ser Gln Lys Ala Cys
   50 55 60
   Asn Thr His Ser Ser Ile Lys Ala Asn Lys Lys Lys Glu Cys Lys His
   65 70 75 80
   Val Lys Leu Gly Val Arg Glu Asn Asp Lys Asp Leu Lys Ile Cys Val
   85 90 95
   Ile Glu Asp Thr Ser Leu Ser Gly Val Asp Asn Cys Cys Cys Gln Asp
   100 105 110
   Leu Leu Gly Ile Leu Gln Glu Asn Cys Ser Asp Asn Lys Arg Gly Ser
   115 120 125
   Ser Ser Asn Gly Ser Cys Asp Lys Asn Ser Glu Glu Ile Cys Gln Lys
   130 135 140
   Lys Leu Asp Glu Ala Leu Ala Ser Leu His Asn Gly Tyr Lys Asn Gln
   145 150 155 160
   Lys Cys Lys Ser Glu Gln Ser Lys Lys Asn Lys Asn Lys Trp Ile Trp
   165 170 175
   Lys Lys Ser Ser Gly Asn Glu Lys Gly Leu Gln Lys Glu Tyr Ala Asn
   180 185 190
   Thr Ile Gly Leu Pro Pro Arg Thr Gln Ser Leu Tyr Leu Gly Asn Leu
   195 200 205
   Pro Lys Leu Glu Asn Val Cys Glu Asp Val Thr Asp Ile Asn Phe Asp
   210 215 220
   Thr Lys Glu Lys Phe Leu Ala Gly Cys Leu Ile Ala Ala Phe His Glu
   225 230 235 240
   Gly Lys Asn Leu Lys Thr Thr Tyr Pro Gln Asn Lys Asn Asp Asp Asn
   245 250 255
   Gly Lys Lys Leu Cys Lys Asp
   260
<210> 18
   <211> 338
   <212> PRT
   <213> Plasmodium falciparum
<400> 18
   Lys Cys Asp Lys Cys Lys Ser Glu Gln Ser Lys Lys Asn Asn Asn Ile
   1 5 10 15
   Trp Ile Trp Lys Lys Ser Ser Gly Asn Lys Lys Gly Leu Gln Lys Glu
   20 25 30
   Tyr Ala Asn Thr Ile Gly Leu Pro Pro Arg Thr Gln Ser Leu Tyr Leu
           35                  40                  45
   Gly Asn Leu Pro Lys Leu Glu Asn Val Cys Lys Asp Val Thr Asp Ile
       50                  55                  60
   Asn Phe Asp Thr Lys Glu Lys Phe Leu Ala Gly Cys Leu Ile Ala Ala
   65                  70                  75                  80
   Phe His Glu Gly Lys Asn Leu Lys Ile Ser Asn Glu Lys Lys Asn Asp
                   85                  90                  95
   Asp Asn Gly Lys Lys Leu Cys Lys Asp Leu Lys Tyr Ser Phe Ala Asp
               100                 105                 110
   Tyr Gly Asp Leu Ile Lys Gly Thr Ser Ile Trp Asp Asn Glu Tyr Thr
           115                 120                 125
   Lys Asp Leu Glu Leu Asn Leu Gln Asn Asn Phe Gly Lys Leu Phe Arg
       130                 135                 140
   Lys Tyr Ile Lys Lys Asn Asn Thr Ala Glu Gln His Thr Leu Tyr Ser
   145                 150                 155                 160
   Ser Leu Asp Glu Leu Arg Glu Ser Trp Trp Asn Thr Asn Lys Lys Tyr
                   165                 170                 175
   Ile Trp Leu Ala Met Lys His Gly Thr Thr Cys Ser Ser Gly Ser Gly
               180                 185                 190
   Asp Asn Gly Asp Gly Ser Val Thr Gly Ser Gly Ser Ser Cys Asp Asp
           195                 200                 205
   Met Ser Thr Ile Asp Leu Ile Pro Gln Tyr Leu Arg Phe Leu Gln Glu
       210                 215                 220
   Trp Val Glu His Phe Cys Lys Gln Arg Gln Glu Lys Val Asn Ala Val
   225                 230                 235                 240
   Ile Glu Asn Cys Asn Ser Cys Lys Asn Thr Ser Ser Lys Thr Lys Leu
                   245                 250                 255
   Gly Gly Thr Cys Asn Gly Glu Cys Lys Thr Glu Cys Glu Lys Lys Cys
               260                 265                 270
   Lys Asp Glu Cys Glu Lys Tyr Lys Glu Phe Ile Glu Glu Cys Lys Arg
   275 280 285
   Gly Asp Gly Thr Ala Gly Ser Pro Trp Val Lys Arg Trp Asp Gln Ile
   290 295 300
   Tyr Met Arg Tyr Ser Lys Tyr Ile Glu Asp Ala Lys Arg Asn Arg Lys
   305 310 315 320
   Ala Gly Thr Lys Ser Cys Gly Thr Ser Ala Ala Glu Asn Lys Cys Val
   325 330 335
   Gln Ser
<210> 19
   <211> 341
   <212> PRT
   <213> Plasmodium falciparum
<400> 19
   Lys Cys Asp Lys Cys Lys Ser Glu Gln Ser Lys Lys Asn Asn Asn Ile
   1 5 10 15
   Trp Ile Trp Lys Lys Ser Ser Gly Asp Glu Lys Gly Leu Gln Lys Glu
   20 25 30
   Tyr Ala Asn Thr Ile Ala Leu Pro Pro Arg Thr Gln Ser Leu Tyr Leu
   35 40 45
   Gly Asn Leu Pro Lys Leu Glu Asn Val Cys Lys Asp Val Thr Asp Ile
   50 55 60
   Asn Phe Asp Thr Lys Glu Lys Phe Leu Ala Gly Cys Leu Ile Ala Ala
   65 70 75 80
   Phe His Glu Gly Lys Asn Leu Lys Thr Ser His Gln Asn Lys Asn Ala
   85 90 95
   Asp Asn Gly Lys Lys Asn Asp Asp Asn Gly Lys Lys Leu Cys Lys Ala
   100 105 110
   Leu Lys Tyr Ser Phe Ala Asp Tyr Gly Asp Leu Ile Lys Gly Thr Ser
   115 120 125
   Ile Trp Asp Asn Glu Tyr Thr Lys Asp Leu Glu Leu Asn Leu Gln Gln
   130 135 140
   Ile Phe Gly Lys Leu Phe Arg Lys Tyr Ile Lys Arg Asn Asn Thr Ala
   145 150 155 160
   Glu Gln His Thr Leu Tyr Ser Ser Leu Asp Glu Leu Arg Glu Ser Trp
   165 170 175
   Trp Asn Thr Asn Lys Lys Tyr Ile Trp Leu Ala Met Lys His Gly Thr
   180 185 190
   Thr Cys Ser Ser Gly Ser Gly Asp Asn Gly Asp Gly Ser Val Thr Gly
   195 200 205
   Ser Gly Ser Ser Cys Asp Asp Met Ser Thr Ile Asp Leu Ile Pro Gln
   210 215 220
   Tyr Leu Arg Phe Leu Gln Glu Trp Val Glu His Phe Cys Lys Gln Arg
   225 230 235 240
   Gln Glu Lys Val Lys Asp Val Ile Thr Asn Cys Asn Ser Cys Lys Glu
   245 250 255
   Cys Gly Gly Thr Cys Gly Ser Asp Cys Lys Thr Lys Cys Glu Ala Tyr
   260 265 270
   Lys Lys Phe Ile Glu Glu Cys Asn Gly Thr Ala Asp Gly Gly Thr Ser
   275 280 285
   Gly Ser Ser Trp Ser Lys Arg Trp Asp Gln Ile Tyr Lys Arg Tyr Ser
   290 295 300
   Lys Tyr Ile Glu Asp Ala Lys Arg Asn Arg Lys Ala Gly Thr Lys Asn
   305 310 315 320
   Cys Gly Pro Ser Ser Gly Ala Asn Ser Gly Val Thr Thr Thr Glu Asn
   325 330 335
   Lys Cys Val Gln Ser
   340
<210> 20
   <211> 352
   <212> PRT
   <213> Plasmodium falciparum
<400> 20
   Lys Cys Glu Lys Cys Glu Ser Glu Gln Ser Lys Lys Asn Asn Lys Tyr
   1               5                   10                  15
   Trp Ile Trp Lys Lys Ser Ser Gly Asn Gly Glu Gly Leu Gln Glu Glu
               20                  25                  30
   Tyr Ala Asn Thr Ile Ala Leu Pro Pro Arg Thr His Ser Leu Cys Leu
           35                  40                  45
   Val Cys Leu His Glu Lys Glu Gly Lys Lys Thr Gln Glu Leu Lys Asn
       50                  55                  60
   Ile Arg Thr Asn Ser Glu Leu Leu Lys Glu Arg Ile Ile Ala Ala Phe
   65                  70                  75                  80
   His Glu Gly Lys Asn Leu Lys Thr Ser Pro Gln Asn Lys Asn Asp Asn
                   85                  90                  95
   Gly Lys Lys Leu Cys Lys Asp Leu Lys Tyr Ser Phe Ala Asp Tyr Gly
               100                 105                 110
   Asp Leu Ile Lys Gly Thr Ser Ile Trp Asp Asn Glu Tyr Thr Lys Asp
           115                 120                 125
   Leu Glu Leu Asn Leu Gln Lys Ile Phe Gly Lys Leu Phe Arg Lys Tyr
       130                 135                 140
   Ile Lys Lys Asn Asn Thr Ala Glu Gln His Thr Leu Tyr Ser Ser Leu
   145                 150                 155                 160
   Asp Glu Leu Arg Glu Ser Trp Trp Asn Thr Asn Lys Lys Tyr Ile Trp
                   165                 170                 175
   Leu Ala Met Lys His Gly Ala Gly Met Asn Ser Thr Met Cys Asn Ala
               180                 185                 190
   Asp Gly Ser Val Thr Gly Ser Ser Asp Ser Gly Ser Thr Thr Cys Cys
           195                 200                 205
   Gly Asp Asn Gly Ser Ile Ser Cys Asp Asp Met Pro Thr Ile Asp Leu
       210                 215                 220
   Ile Pro Gln Tyr Leu Arg Phe Leu Gln Glu Trp Val Glu His Phe Cys
   225                 230                 235                 240
   Glu Gln Arg Gln Glu Lys Val Asn Ala Val Ile Thr Asn Cys Lys Ser
                   245                 250                 255
   Cys Lys Glu Cys Gly Gly Thr Cys Asn Ser Asp Cys Glu Lys Lys Cys
               260                 265                 270
   Lys Ala Tyr Lys Glu Phe Ile Glu Lys Cys Lys Gly Gly Gly Thr Glu
   275 280 285
   Gly Thr Ser Gly Ser Ser Trp Ser Lys Arg Trp Asp Gln Ile Tyr Lys
   290 295 300
   Arg His Ser Lys His Ile Glu Asp Ala Lys Arg Asn Arg Lys Ala Gly
   305 310 315 320
   Thr Lys Asn Cys Gly Ile Thr Thr Gly Thr Ile Ser Gly Glu Ser Ser
   325 330 335
   Gly Ala Asn Ser Gly Val Thr Thr Thr Glu Asn Lys Cys Val Gln Ser
   340 345 350
<210> 21
   <211> 344
   <212> PRT
   <213> Plasmodium falciparum
<400> 21
   Lys Cys Asp Lys Cys Lys Ser Gly Thr Ser Arg Ser Arg Lys Ile Trp
   1 5 10 15
   Thr Trp Arg Lys Phe Arg Gly Asn Gly Glu Gly Leu Gln Lys Glu Tyr
   20 25 30
   Ala Asn Thr Ile Gly Leu Ser Pro Arg Thr Gln Leu Leu Tyr Leu Val
   35 40 45
   Cys Leu His Glu Lys Gly Lys Lys Thr Gln Glu Leu Lys Asn Ile Ser
   50 55 60
   Thr Asn Ser Glu Leu Leu Lys Glu Trp Ile Ile Ala Ala Phe His Glu
   65 70 75 80
   Gly Lys Asn Leu Lys Thr Thr Tyr Pro Gln Lys Lys Asn Asp Asp Asn
   85 90 95
   Gly Lys Lys Leu Cys Lys Ala Leu Lys Tyr Ser Phe Ala Asp Tyr Gly
   100 105 110
   Asp Leu Ile Lys Gly Thr Ser Ile Trp Asp Asn Asp Phe Thr Lys Asp
   115 120 125
   Leu Glu Leu Asn Leu Gln Lys Ile Phe Gly Lys Leu Phe Arg Lys Tyr
   130 135 140
   Ile Lys Lys Asn Ile Ala Ser Asp Glu Asn Thr Ser Tyr Ser Ser Leu
   145                 150                 155                 160
   Asp Glu Leu Arg Glu Ser Trp Trp Asn Thr Asn Lys Lys Tyr Ile Trp
                   165                 170                 175
   Thr Ala Met Lys His Gly Ala Gly Met Asn Gly Thr Thr Cys Cys Gly
               180                 185                 190
   Asp Gly Ser Val Thr Gly Ser Ser Asp Ser Gly Ser Thr Thr Cys Cys
           195                 200                 205
   Gly Asp Gly Ser Val Thr Gly Ser Gly Ser Ser Cys Asp Asp Ile Pro
       210                 215                 220
   Thr Ile Asp Leu Ile Pro Gln Tyr Leu Arg Phe Leu Gln Glu Trp Val
   225                 230                 235                 240
   Glu His Phe Cys Glu Gln Arg Gln Glu Lys Val Lys Asp Val Ile Thr
                   245                 250                 255
   Asn Cys Lys Ser Cys Lys Glu Ser Glu Lys Lys Cys Lys Asn Lys Cys
               260                 265                 270
   Asp Ala Tyr Lys Glu Phe Ile Asp Gly Thr Gly Ser Gly Gly Gly Thr
           275                 280                 285
   Gly Thr Ala Gly Ser Ser Trp Ser Lys Arg Trp Asp Gln Ile Tyr Met
       290                 295                 300
   Arg Tyr Ser Lys Tyr Ile Glu Asp Ala Lys Arg Asn Arg Lys Ala Gly
   305                 310                 315                 320
   Thr Lys Asn Cys Gly Thr Ser Ser Gly Ala Asn Ser Gly Val Thr Thr
                   325                 330                 335
   Thr Glu Asn Lys Cys Val Gln Ser
               340
<210> 22
   <211> 350
   <212> PRT
   <213> Plasmodium falciparum
<400> 22
   Lys Cys Glu Lys Cys Lys Ser Glu Gln Ser Lys Lys Asn Asn Lys Ile
   1               5                   10                  15
   Trp Thr Trp Arg Lys Phe Pro Gly Asn Gly Glu Gly Leu Gln Lys Glu
               20                  25                  30
   Tyr Ala Asn Thr Ile Gly Leu Ser Pro Arg Thr Gln Leu Leu Tyr Leu
           35                  40                  45
   Val Cys Leu His Glu Lys Gly Lys Lys Thr Gln His Lys Thr Ile Ser
       50                  55                  60
   Thr Asn Ser Glu Leu Leu Lys Glu Trp Ile Ile Ala Ala Phe His Glu
   65                  70                  75                  80
   Gly Lys Asn Leu Lys Lys Arg Tyr Leu Glu Lys Lys Lys Gly Asp Asn
                   85                  90                  95
   Asn Ser Lys Leu Cys Lys Asp Leu Lys Tyr Ser Phe Ala Asp Tyr Gly
               100                 105                 110
   Asp Leu Ile Lys Gly Thr Ser Ile Trp Asp Asn Asp Phe Thr Lys Asp
           115                 120                 125
   Leu Glu Leu Asn Leu Gln Gln Ile Phe Gly Lys Leu Phe Arg Lys Tyr
       130                 135                 140
   Ile Lys Lys Asn Ile Ala Ser Asp Glu Asn Thr Ser Tyr Ser Ser Leu
   145                 150                 155                 160
   Asp Glu Leu Arg Glu Ser Trp Trp Asn Thr Asn Lys Lys Tyr Ile Trp
                   165                 170                 175
   Thr Ala Met Lys His Gly Ala Gly Met Asn Ser Thr Met Cys Asn Gly
               180                 185                 190
   Asp Gly Ser Val Thr Gly Ser Ser Asp Ser Gly Ser Thr Thr Cys Ser
           195                 200                 205
   Gly Asp Asn Gly Ser Ile Ser Cys Asp Asp Ile Pro Thr Ile Asp Leu
       210                 215                 220
   Ile Pro Gln Tyr Leu Arg Phe Leu Gln Glu Trp Val Glu His Phe Cys
   225                 230                 235                 240
   Glu Gln Arg Gln Glu Lys Val Lys Asp Val Ile Lys Asn Cys Asn Ser
                   245                 250                 255
   Cys Lys Glu Cys Gly Gly Thr Cys Asn Gly Glu Cys Lys Thr Glu Cys
               260                 265                 270
   Lys Asn Lys Cys Lys Asp Glu Cys Glu Lys Tyr Lys Asn Phe Ile Glu
   275 280 285
   Val Cys Thr Gly Gly Asp Gly Thr Ala Gly Ser Pro Trp Ser Lys Arg
   290 295 300
   Trp Tyr Gln Ile Tyr Met Arg Tyr Ser Lys Tyr Ile Glu Asp Ala Lys
   305 310 315 320
   Arg Asn Arg Lys Ala Gly Thr Lys Ser Cys Gly Thr Ser Ser Gly Ala
   325 330 335
   Asn Ser Gly Val Thr Thr Thr Glu Ser Lys Cys Val Gln Ser
   340 345 350
<210> 23
   <211> 359
   <212> PRT
   <213> Plasmodium falciparum
<400> 23
   Lys Cys Glu Lys Cys Lys Ser Glu Gln Ser Lys Lys Asn Asn Lys Asn
   1 5 10 15
   Trp Ile Trp Arg Lys Phe Pro Gly Asn Gly Glu Gly Leu Gln Lys Glu
   20 25 30
   Tyr Ala Asn Thr Ile Gly Leu Pro Pro Arg Thr His Ser Leu Tyr Leu
   35 40 45
   Val Cys Leu His Glu Lys Gly Lys Lys Thr Gln Glu Leu Lys Asn Ile
   50 55 60
   Arg Thr Asn Ser Glu Leu Leu Lys Glu Trp Ile Ile Ala Ala Phe His
   65 70 75 80
   Glu Gly Lys Asn Leu Lys Lys Arg Tyr His Gln Asn Asn Asn Ser Gly
   85 90 95
   Asn Lys Lys Lys Leu Cys Lys Ala Leu Glu Tyr Ser Phe Ala Asp Tyr
   100 105 110
   Gly Asp Leu Ile Lys Gly Thr Ser Ile Trp Asp Asn Glu Tyr Thr Lys
   115 120 125
   Asp Leu Glu Leu Asn Leu Gln Gln Ile Phe Gly Lys Leu Phe Arg Lys
   130 135 140
   Tyr Ile Lys Lys Asn Ile Ser Thr Glu Gln Asp Thr Leu Tyr Ser Ser
   145 150 155 160
   Leu Asp Glu Leu Arg Glu Ser Trp Trp Asn Thr Asn Lys Lys Tyr Ile
   165 170 175
   Trp Leu Ala Met Lys His Gly Ala Gly Met Asn Ser Thr Thr Cys Cys
   180 185 190
   Gly Asp Gly Ser Val Thr Gly Ser Ser Asp Ser Gly Ser Thr Thr Cys
   195 200 205
   Ser Gly Asp Asn Gly Ser Ile Ser Cys Asp Asp Met Pro Thr Ile Asp
   210 215 220
   Leu Ile Pro Gln Tyr Leu Arg Phe Leu Gln Glu Trp Val Glu His Phe
   225 230 235 240
   Cys Glu Gln Arg Gln Glu Lys Val Lys Asp Val Ile Glu Asn Cys Lys
   245 250 255
   Ser Cys Lys Asn Thr Ser Gly Glu Arg Ile Ile Gly Gly Thr Cys Asn
   260 265 270
   Gly Glu Cys Lys Thr Glu Cys Glu Lys Lys Cys Lys Ala Ala Cys Glu
   275 280 285
   Ala Tyr Lys Thr Phe Ile Glu Glu Cys Glu Gly Lys Ala Ala Glu Gly
   290 295 300
   Thr Ser Gly Ser Ser Trp Ser Lys Arg Trp Tyr Gln Ile Tyr Met Arg
   305 310 315 320
   Tyr Ser Lys Tyr Ile Glu Asp Ala Lys Arg Asn Arg Lys Ala Gly Thr
   325 330 335
   Lys Asn Cys Gly Lys Ser Ser Gly Ala Asn Ser Gly Val Thr Thr Thr
   340 345 350
   Glu Asn Lys Cys Val Gln Ser
   355
<210> 24
   <211> 270
   <212> PRT
   <213> Plasmodium falciparum
<400> 24
   Asn Tyr Ile Lys Asp Asp Pro Tyr Ser Lys Glu Tyr Val Thr Lys Leu
   1               5                   10                  15
   Ser Phe Ile Pro Asn Ser Ser Asp Ala Asn Asn Pro Ser Gly Glu Thr
               20                  25                  30
   Ala Asn His Asn Asp Glu Val Cys Asn Pro Asn Glu Ser Glu Ile Ser
           35                  40                  45
   Ser Val Glu His Ala Gln Thr Ser Val Leu Leu Ser Gln Lys Ala Tyr
       50                  55                  60
   Ile Thr His Ser Ser Ile Lys Ala Asn Lys Lys Lys Val Cys Lys Tyr
   65                  70                  75                  80
   Val Lys Leu Gly Val Arg Glu Asn Asp Lys Asp Leu Lys Ile Cys Val
                   85                  90                  95
   Ile Glu Asp Asp Ser Leu Arg Gly Val Glu Asn Cys Cys Phe Lys Asp
               100                 105                 110
   Phe Leu Arg Ile Leu Gln Glu Asn Cys Ser Asp Asn Lys Arg Glu Ser
           115                 120                 125
   Ser Ser Asn Gly Ser Cys Asn Asn Asn Asn Glu Glu Ala Cys Glu Lys
       130                 135                 140
   Asn Leu Asp Glu Ala Leu Ala Ser Leu Thr Asn Cys Tyr Lys Asn Gln
   145                 150                 155                 160
   Lys Cys Lys Ser Gly Thr Ser Thr Val Asn Asn Asn Lys Trp Ile Trp
                   165                 170                 175
   Lys Lys Ser Ser Gly Lys Glu Gly Gly Leu Gln Lys Glu Tyr Ala Asn
               180                 185                 190
   Thr Ile Gly Leu Pro Pro Arg Thr Gln Ser Leu Cys Leu Val Val Cys
           195                 200                 205
   Leu Asp Glu Lys Glu Gly Lys Thr Gln Glu Leu Lys Asn Ile Arg Thr
       210                 215                 220
   Asn Ser Glu Leu Leu Lys Glu Trp Ile Ile Ala Ala Phe His Glu Gly
   225                 230                 235                 240
   Lys Asn Leu Lys Lys Arg Tyr His Gln Asn Lys Asn Asp Asp Asn Asn
                   245                 250                 255
   Ser Lys Leu Cys Lys Ala Leu Lys Tyr Ser Phe Ala Asp Tyr
   260 265 270
<210> 25
   <211> 334
   <212> PRT
   <213> Plasmodium falciparum
<400> 25
   Lys Cys Asp Lys Cys Lys Ser Glu Gln Ser Lys Lys Asn Asn Lys Tyr
   1 5 10 15
   Trp Ile Trp Lys Lys Tyr Ser Val Lys Glu Gly Gly Leu Gln Lys Glu
   20 25 30
   Tyr Ala Asn Thr Ile Ala Leu Pro Pro Arg Thr Gln Ser Leu Cys Leu
   35 40 45
   Val Val Cys Leu Asp Glu Lys Glu Gly Lys Thr Gln Glu Leu Lys Asn
   50 55 60
   Ile Arg Thr Asn Ser Glu Leu Leu Lys Glu Arg Ile Ile Ala Ala Phe
   65 70 75 80
   His Glu Gly Lys Asn Leu Lys Thr Tyr His Glu Lys Lys Lys Gly Asp
   85 90 95
   Asp Gly Lys Lys Leu Cys Lys Asp Leu Lys Tyr Ser Phe Ala Asp Tyr
   100 105 110
   Gly Asp Leu Ile Lys Gly Thr Ser Ile Trp Asp Asn Asp Phe Thr Lys
   115 120 125
   Asp Leu Glu Leu Asn Leu Gln Lys Ile Phe Gly Lys Leu Phe Arg Lys
   130 135 140
   Tyr Ile Lys Lys Asn Asn Thr Ala Glu Gln His Thr Ser Tyr Ser Ser
   145 150 155 160
   Leu Asp Glu Leu Arg Glu Ser Trp Trp Asn Thr Asn Lys Lys Tyr Ile
   165 170 175
   Trp Thr Ala Met Lys His Gly Ala Glu Met Asn Gly Thr Thr Cys Ser
   180 185 190
   Cys Ser Gly Asp Ser Ser Asn Asp Ile Pro Thr Ile Asp Leu Ile Pro
   195 200 205
   Gln Tyr Leu Arg Phe Leu Gln Glu Trp Val Glu His Phe Cys Glu Gln
       210             215             220
   Arg Gln Ala Lys Val Asn Ala Val Ile Lys Asn Cys Lys Ser Cys Lys
   225             230             235             240
   Glu Cys Gly Gly Thr Cys Asn Gly Glu Cys Lys Thr Glu Cys Lys Thr
                   245             250             255
   Lys Cys Lys Gly Glu Cys Glu Lys Tyr Lys Glu Phe Ile Glu Lys Cys
               260             265             270
   Glu Gly Gln Ala Ala Glu Gly Thr Ser Gly Ser Ser Trp Ser Lys Arg
           275             280             285
   Trp Tyr Gln Ile Tyr Met Arg Tyr Ser Lys Tyr Ile Glu Asp Ala Lys
       290             295             300
   Arg Asn Arg Lys Ala Gly Thr Lys Asn Cys Gly Thr Ser Ser Gly Ala
   305             310             315             320
   Asn Ser Gly Val Thr Thr Thr Glu Asn Lys Cys Val Gln Ser
                   325             330
<210> 26
   <211> 351
   <212> PRT
   <213> Plasmodium falciparum
<400> 26
   Lys Cys Asp Lys Cys Lys Ser Glu Gln Ser Lys Lys Asn Asn Lys Asn
   1               5                   10                  15
   Trp Ile Trp Lys Lys Tyr Ser Gly Thr Glu Gly Gly Leu Gln Lys Glu
               20                  25                  30
   Tyr Ala Asn Thr Ile Ala Leu Pro Pro Arg Thr Gln Ser Leu Tyr Leu
           35                  40                  45
   Val Cys Leu His Glu Lys Glu Glu Lys Thr Gln Glu Leu Lys Asn Ile
       50                  55                  60
   Ser Thr Asn Ser Glu Leu Leu Lys Glu Trp Ile Ile Ala Ala Phe His
   65                  70                  75                  80
   Glu Gly Lys Asn Leu Lys Ile Ser Pro Gln Asn Lys Asn Asp Asn Gly
                   85                  90                  95
   Lys Asn Leu Cys Lys Asp Leu Lys Tyr Ser Phe Ala Asp Tyr Gly Asp
               100                 105                 110
   Leu Ile Lys Gly Thr Ser Ile Trp Asp Asn Asp Phe Thr Lys Asp Leu
           115                 120                 125
   Glu Leu Asn Leu Gln Gln Ile Phe Gly Lys Leu Phe Arg Lys Tyr Ile
       130                 135                 140
   Lys Lys Asn Asn Thr Ala Glu Gln Asp Thr Leu Tyr Ser Ser Leu Asp
   145                 150                 155                 160
   Glu Leu Arg Glu Ser Trp Trp Asn Thr Asn Lys Lys Tyr Ile Trp Thr
                   165                 170                 175
   Ala Met Lys His Gly Ala Gly Met Asn Gly Thr Thr Cys Cys Gly Asp
               180                 185                 190
   Gly Ser Val Thr Gly Ser Ser Asp Ser Gly Ser Thr Thr Cys Cys Gly
           195                 200                 205
   Asp Gly Ser Val Thr Gly Ser Gly Ser Ser Cys Asp Asp Ile Pro Thr
       210                 215                 220
   Ile Asp Leu Ile Pro Gln Tyr Leu Arg Phe Leu Gln Glu Trp Val Glu
   225                 230                 235                 240
   His Phe Cys Glu Gln Arg Gln Ala Lys Val Lys Asp Val Ile Lys Asn
                   245                 250                 255
   Cys Asn Ser Cys Lys Glu Cys Gly Gly Thr Cys Asn Gly Glu Cys Lys
               260                 265                 270
   Thr Glu Cys Glu Lys Lys Cys Lys Gly Glu Cys Glu Ala Tyr Lys Lys
           275                 280                 285
   Phe Ile Glu Lys Cys Asn Gly Gly Gly Gly Glu Gly Thr Ser Gly Ser
       290                 295                 300
   Ser Trp Ser Lys Arg Trp Asp Gln Ile Tyr Met Arg Tyr Ser Lys Tyr
   305                 310                 315                 320
   Ile Glu Asp Ala Lys Arg Asn Arg Lys Ala Gly Thr Lys Asn Cys Gly
                   325                 330                 335
   Thr Ser Ser Thr Thr Asn Ala Ala Glu Asn Lys Cys Val Gln Ser
   340 345 350
<210> 28
   <211> 327
   <212> PRT
   <213> Plasmodium falciparum
<400> 28
   Lys Cys Asp Lys Cys Lys Ser Gly Thr Ser Thr Val Asn Lys Lys Trp
   1               5                   10                  15
   Ile Trp Lys Lys Tyr Ser Gly Lys Glu Gly Gly Leu Gln Lys Glu Tyr
               20                  25                  30
   Ala Asn Thr Ile Gly Leu Pro Pro Arg Thr Gln Ser Leu Cys Leu Val
           35                  40                  45
   Cys Leu His Glu Lys Glu Gly Lys Thr Gln Glu Leu Lys Asn Ile Ser
       50                  55                  60
   Thr Asn Ser Glu Leu Leu Lys Glu Trp Ile Ile Ala Ala Phe His Glu
   65 70 75 80
   Gly Lys Asn Leu Lys Ile Ser Asn Lys Lys Lys Asn Asp Asp Asn Gly
   85 90 95
   Lys Lys Leu Cys Lys Asp Leu Lys Tyr Ser Phe Ala Asp Tyr Gly Asp
   100 105 110
   Leu Ile Lys Gly Thr Ser Ile Trp Asp Asn Asp Phe Thr Lys Asp Leu
   115 120 125
   Glu Leu Asn Leu Gln Lys Ile Phe Gly Lys Leu Phe Arg Lys Tyr Ile
   130 135 140
   Lys Lys Asn Asn Thr Ala Glu Gln Asp Thr Leu Tyr Ser Ser Leu Asp
   145 150 155 160
   Glu Leu Arg Glu Ser Trp Trp Asn Thr Asn Lys Lys Tyr Ile Trp Thr
   165 170 175
   Ala Met Lys His Gly Ala Gly Met Asn Ser Thr Thr Cys Ser Cys Ser
   180 185 190
   Gly Asp Ser Ser Asn Asp Ile Pro Thr Ile Asp Leu Ile Pro Gln Tyr
   195 200 205
   Leu Arg Phe Leu Gln Glu Trp Val Glu His Phe Cys Lys Gln Arg Gln
   210 215 220
   Glu Lys Val Asn Ala Val Ile Thr Asn Cys Lys Ser Cys Lys Glu Ser
   225 230 235 240
   Gly Gly Thr Cys Asn Ser Asp Cys Glu Lys Lys Cys Lys Ile Glu Cys
   245 250 255
   Glu Lys Tyr Lys Asn Phe Ile Glu Lys Cys Val Thr Ala Ala Gly Gly
   260 265 270
   Thr Ser Gly Ser Ser Trp Ser Lys Arg Trp Asp Gln Ile Tyr Lys Met
   275 280 285
   Tyr Ser Lys Tyr Ile Glu Asp Ala Lys Arg Asn Arg Lys Ala Gly Thr
   290 295 300
   Lys Asn Cys Gly Pro Ser Ser Thr Thr Asn Ala Ala Ala Ser Thr Asp
   305 310 315 320
   Glu Asn Lys Cys Val Gln Ser
   325
<210> 29
   <211> 628
   <212> PRT
   <213> Artificial
<220>
   <223> Artificial construct
<400> 29
   Asn Tyr Ile Lys Gly Asp Pro Tyr Phe Ala Glu Tyr Ala Thr Lys Leu
   1               5                   10                  15
   Ser Phe Ile Leu Asn Ser Ser Asp Thr Glu Asn Ala Ser Glu Thr Pro
               20                  25                  30
   Ser Lys Tyr Tyr Asp Glu Ala Cys Asn Cys Asn Glu Ser Glu Ile Ala
           35                  40                  45
   Ser Val Gly Gln Ala Gln Thr Ser Gly Pro Ser Ser Asn Lys Thr Cys
       50                  55                  60
   Ile Thr His Ser Ser Ile Lys Thr Asn Lys Lys Lys Glu Cys Lys Asp
   65                  70                  75                  80
   Val Lys Leu Gly Ile Asn Asn Asn Asp Lys Val Leu Arg Val Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Gly Ser Cys Asp Lys Asn Ser Glu Glu Ile Cys Gln Lys
       130                 135                 140
   Lys Leu Glu Lys Val Phe Ala Ser Leu Thr Asn Gly Tyr Lys Cys Asp
   145                 150                 155                 160
   Lys Cys Lys Ser Gly Thr Ser Arg Ser Lys Lys Lys Trp Ile Trp Lys
                   165                 170                 175
   Lys Ser Ser Gly Asn Glu Glu Gly Leu Gln Lys Glu Tyr Ala Asn Thr
               180                 185                 190
   Ile Gly Leu Pro Pro Arg Thr Gln Ser Leu Cys Leu Val Cys Leu His
               195                 200                 205
   Glu Lys Glu Gly Lys Thr Gln His Lys Thr Ile Ser Thr Asn Ser Glu
       210                 215                 220
   Leu Leu Lys Glu Trp Ile Ile Ala Ala Phe His Glu Gly Lys Asn Leu
   225                 230                 235                 240
   Lys Thr Ser His Glu Lys Lys Asn Asp Asp Asn Gly Lys Lys Leu Cys
                   245                 250                 255
   Lys Ala Leu Glu Tyr Ser Phe Ala Asp Tyr Gly Asp Leu Ile Lys Gly
               260                 265                 270
   Thr Ser Ile Trp Asp Asn Glu Tyr Thr Lys Asp Leu Glu Leu Asn Leu
           275                 280                 285
   Gln Lys Ile Phe Gly Lys Leu Phe Arg Lys Tyr Ile Lys Lys Asn Asn
       290                 295                 300
   Thr Ala Glu Gln His Thr Ser Tyr Ser Ser Leu Asp Glu Leu Arg Glu
   305                 310                 315                 320
   Ser Trp Trp Asn Thr Asn Lys Lys Tyr Ile Trp Thr Ala Met Lys His
                   325                 330                 335
   Gly Ala Gly Met Asn Gly Thr Thr Cys Ser Cys Ser Gly Asp Ser Ser
               340                 345                 350
   Asn Asp Met Pro Thr Ile Asp Leu Ile Pro Gln Tyr Leu Arg Phe Leu
           355                 360                 365
   Gln Glu Trp Val Glu His Phe Cys Lys Gln Arg Gln Glu Lys Val Asn
       370                 375                 380
   Ala Val Ile Glu Asn Cys Asn Ser Cys Lys Glu Ser Gly Gly Thr Cys
   385                 390                 395                 400
   Asn Ser Asp Cys Lys Thr Glu Cys Lys Asn Lys Cys Glu Ala Tyr Lys
                   405                 410                 415
   Glu Phe Ile Glu Asp Cys Lys Gly Gly Gly Thr Gly Thr Ala Gly Ser
               420                 425                 430
   Pro Trp Ser Lys Arg Trp Asp Gln Ile Tyr Lys Arg Tyr Ser Lys His
           435                 440                 445
   Ile Glu Asp Ala Lys Arg Asn Arg Lys Ala Gly Thr Lys Asn Cys Gly
   450 455 460
   Thr Ser Ser Thr Thr Asn Ala Ala Ala Ser Thr Asp Glu Asn Lys Cys
   465 470 475 480
   Val Gln Ser Asp Val Asp Ser Phe Phe Lys His Leu Ile Asp Ile Gly
   485 490 495
   Leu Thr Thr Pro Ser Ser Tyr Leu Ser Asn Val Leu Asp Asp Asn Ile
   500 505 510
   Cys Gly Ala Asp Lys Ala Pro Trp Thr Thr Tyr Thr Thr Tyr Thr Thr
   515 520 525
   Thr Lys Asn Cys Asp Ile Gln Lys Lys Thr Pro Lys Ser Gln Ser Cys
   530 535 540
   Asp Thr Leu Val Val Val Asn Val Pro Ser Pro Leu Gly Asn Thr Pro
   545 550 555 560
   His Glu Tyr Lys Tyr Ala Cys Glu Cys Lys Ile Pro Thr Thr Glu Glu
   565 570 575
   Thr Cys Asp Asp Arg Lys Glu Tyr Met Asn Gln Trp Ser Cys Gly Ser
   580 585 590
   Ala Gln Thr Val Arg Gly Arg Ser Gly Lys Asp Asp Tyr Glu Leu Tyr
   595 600 605
   Thr Tyr Asn Gly Val Lys Glu Thr Lys Pro Leu Gly Thr Leu Lys Asn
   610 615 620
   Ser Lys Leu Asp
   625
<210> 30
   <211> 350
   <212> PRT
   <213> Plasmodium falciparum
<400> 30
   Lys Cys Glu Lys Cys Lys Ser Glu Gln Ser Lys Lys Asn Asn Lys Asn
   1 5 10 15
   Trp Ile Trp Arg Lys Phe Arg Gly Thr Glu Gly Gly Leu Gln Glu Glu
   20 25 30
   Tyr Ala Asn Thr Ile Gly Leu Pro Pro Arg Thr Gln Ser Leu Cys Leu
   35 40 45
   Val Val Cys Leu Asp Glu Lys Gly Lys Lys Thr Gln Glu Leu Lys Asn
   50 55 60
   Ile Arg Thr Asn Ser Glu Leu Leu Lys Glu Trp Ile Ile Ala Ala Phe
   65 70 75 80
   His Glu Gly Lys Asn Leu Lys Pro Ser His Gln Asn Lys Asn Ser Gly
   85 90 95
   Asn Lys Glu Asn Leu Cys Lys Ala Leu Lys Tyr Ser Phe Ala Asp Tyr
   100 105 110
   Gly Asp Leu Ile Lys Gly Thr Ser Ile Trp Asp Asn Asp Phe Thr Lys
   115 120 125
   Asp Leu Glu Leu Asn Leu Gln Lys Ile Phe Gly Lys Leu Phe Arg Lys
   130 135 140
   Tyr Ile Lys Lys Asn Asn Thr Ala Glu Gln His Thr Ser Tyr Ser Ser
   145 150 155 160
   Leu Asp Glu Leu Arg Glu Ser Trp Trp Asn Thr Asn Lys Lys Tyr Ile
   165 170 175
   Trp Thr Ala Met Lys His Gly Ala Glu Met Asn Gly Thr Thr Cys Asn
   180 185 190
   Ala Asp Gly Ser Val Thr Gly Ser Ser Asp Ser Gly Ser Thr Thr Cys
   195 200 205
   Ser Gly Asp Asn Gly Ser Ile Ser Cys Asp Asp Ile Pro Thr Ile Asp
   210 215 220
   Leu Ile Pro Gln Tyr Leu Arg Phe Leu Gln Glu Trp Val Glu His Phe
   225 230 235 240
   Cys Lys Gln Arg Gln Glu Lys Val Asn Ala Val Ile Asn Ser Cys Asn
   245 250 255
   Ser Cys Lys Asn Thr Ser Ser Lys Thr Lys Leu Gly Asp Thr Cys Asn
   260 265 270
   Ser Asp Cys Lys Thr Lys Cys Lys Ile Glu Cys Glu Lys Tyr Lys Thr
   275 280 285
   Phe Ile Glu Lys Cys Val Thr Ala Ala Gly Gly Thr Ser Gly Ser Pro
       290                 295                 300
   Trp Ser Lys Arg Trp Asp Gln Ile Tyr Lys Arg Tyr Ser Lys Tyr Ile
   305                 310                 315                 320
   Glu Asp Ala Lys Arg Asn Arg Lys Ala Gly Thr Lys Asn Cys Gly Pro
                   325                 330                 335
   Ser Ser Thr Thr Ser Thr Ala Glu Ser Lys Cys Val Gln Ser
               340                 345                 350
<210> 31
   <211> 330
   <212> PRT
   <213> Plasmodium falciparum
<400> 31
   Lys Cys Asp Lys Cys Lys Ser Glu Gln Ser Lys Lys Asn Asn Lys Asn
   1               5                   10                  15
   Trp Ile Trp Arg Lys Tyr Ser Gly Asn Gly Glu Gly Leu Gln Lys Glu
               20                  25                  30
   Tyr Ala Asn Thr Ile Gly Leu Pro Pro Arg Thr His Ser Leu Tyr Leu
           35                  40                  45
   Val Cys Leu His Glu Lys Glu Gly Lys Thr Gln Glu Leu Lys Asn Ile
       50                  55                  60
   Arg Thr Asn Ser Glu Leu Leu Lys Glu Trp Ile Ile Ala Ala Phe His
   65                  70                  75                  80
   Glu Gly Lys Asn Leu Lys Thr Thr Tyr Leu Glu Asn Lys Asn Asp Glu
                   85                  90                  95
   Asn Lys Lys Lys Leu Cys Lys Ala Leu Lys Tyr Ser Phe Ala Asp Tyr
               100                 105                 110
   Gly Asp Leu Ile Lys Gly Thr Ser Ile Trp Asp Asn Asp Phe Thr Lys
           115                 120                 125
   Asp Leu Glu Leu Asn Leu Gln Lys Ile Phe Gly Lys Leu Phe Arg Lys
       130                 135                 140
   Tyr Ile Lys Lys Asn Ile Ala Ser Asp Glu Asn Thr Leu Tyr Ser Ser
   145                 150                 155                 160
   Leu Asp Glu Leu Arg Glu Ser Trp Trp Asn Thr Asn Lys Lys Tyr Ile
   165 170 175
   Trp Thr Ala Met Lys His Gly Ala Glu Met Asn Gly Thr Thr Cys Ser
   180 185 190
   Ser Gly Ser Gly Asp Asn Gly Ser Ile Ser Cys Asp Asp Ile Pro Thr
   195 200 205
   Ile Asp Leu Ile Pro Gln Tyr Leu Arg Phe Leu Gln Glu Trp Val Gly
   210 215 220
   His Phe Cys Lys Gln Arg Gln Glu Lys Val Asn Ala Val Ile Thr Asn
   225 230 235 240
   Cys Asn Ser Cys Lys Glu Ser Gly Gly Thr Cys Asn Ser Asp Cys Glu
   245 250 255
   Lys Lys Cys Lys Ile Glu Cys Glu Lys Tyr Lys Lys Phe Ile Glu Glu
   260 265 270
   Cys Arg Thr Ala Ala Gly Gly Thr Ser Gly Ser Pro Trp Ser Lys Arg
   275 280 285
   Trp Asp Gln Ile Tyr Lys Met Tyr Ser Lys Tyr Ile Glu Asp Ala Lys
   290 295 300
   Arg Asn Arg Lys Ala Gly Thr Lys Asn Cys Gly Pro Ser Ser Thr Thr
   305 310 315 320
   Ser Thr Ala Glu Ser Lys Cys Val Gln Ser
   325 330
<210> 32
   <211> 334
   <212> PRT
   <213> Plasmodium falciparum
<400> 32
   Lys Cys Asp Lys Cys Lys Ser Glu Gln Ser Lys Lys Asn Asn Lys Asn
   1 5 10 15
   Trp Ile Trp Arg Lys Tyr Ser Gly Asn Gly Glu Gly Leu Gln Lys Glu
   20 25 30
   Tyr Ala Asn Thr Ile Gly Leu Pro Pro Arg Thr His Ser Leu Tyr Leu
   35 40 45
   Val Cys Leu His Glu Lys Glu Gly Lys Thr Gln His Lys Thr Ile Ser
       50                  55                  60
   Thr Asn Ser Glu Leu Leu Lys Glu Trp Ile Ile Ala Ala Phe His Glu
   65                  70                  75                  80
   Gly Lys Asn Leu Lys Lys Arg Tyr Pro Gln Asn Asn Asn Ser Gly Asn
                   85                  90                  95
   Lys Lys Lys Leu Cys Lys Asp Leu Lys Tyr Ser Phe Ala Asp Tyr Gly
               100                 105                 110
   Asp Leu Ile Lys Gly Thr Ser Ile Trp Asp Asn Glu Tyr Thr Lys Asp
           115                 120                 125
   Leu Glu Leu Asn Leu Gln Lys Ala Phe Gly Lys Leu Phe Arg Lys Tyr
       130                 135                 140
   Ile Lys Lys Asn Ile Ala Ser Asp Glu Asn Thr Leu Tyr Ser Ser Leu
   145                 150                 155                 160
   Asp Glu Leu Arg Glu Ser Trp Trp Asn Thr Asn Lys Lys Tyr Ile Trp
                   165                 170                 175
   Leu Ala Met Lys His Gly Ala Glu Met Asn Gly Thr Met Cys Asn Ala
               180                 185                 190
   Asp Gly Ser Val Thr Gly Ser Gly Ser Ser Cys Asp Asp Met Ser Thr
           195                 200                 205
   Ile Asp Leu Ile Pro Gln Tyr Leu Arg Phe Leu Gln Glu Trp Val Glu
       210                 215                 220
   His Phe Cys Glu Gln Arg Gln Ala Lys Val Lys Asp Val Ile Asn Ser
   225                 230                 235                 240
   Cys Lys Ser Cys Lys Glu Ser Gly Asp Thr Cys Asn Ser Asp Cys Glu
                   245                 250                 255
   Lys Lys Cys Lys Asn Lys Cys Asp Ala Tyr Lys Thr Phe Ile Glu Glu
               260                 265                 270
   Phe Cys Thr Ala Asp Gly Gly Thr Ala Gly Ser Pro Trp Ser Lys Arg
           275                 280                 285
   Trp Asp Gln Ile Tyr Lys Arg Tyr Ser Lys Tyr Ile Glu Asp Ala Lys
       290                 295                 300
   Arg Asn Arg Lys Ala Gly Thr Lys Asn Cys Gly Thr Ser Ser Gly Ala
   305 310 315 320
   Asn Ser Gly Val Thr Thr Thr Glu Asn Lys Cys Val Gln Ser
   325 330
<210> 33
   <211> 350
   <212> PRT
   <213> Plasmodium falciparum
<400> 33
   Lys Cys Asp Lys Cys Lys Ser Gly Thr Ser Thr Val Asn Lys Asn Trp
   1 5 10 15
   Ile Trp Lys Lys Tyr Ser Gly Lys Glu Glu Gly Leu Gln Lys Glu Tyr
   20 25 30
   Ala Asn Thr Ile Ala Leu Pro Pro Arg Thr His Ser Leu Tyr Leu Val
   35 40 45
   Cys Leu His Glu Lys Gly Lys Lys Thr Gln Glu Leu Lys Asn Ile Arg
   50 55 60
   Thr Asn Ser Glu Leu Leu Lys Glu Trp Ile Ile Ala Ala Phe His Glu
   65 70 75 80
   Gly Lys Asn Leu Lys Thr Ser Pro Gln Asn Asn Asn Ser Gly Asn Lys
   85 90 95
   Lys Lys Leu Cys Lys Ala Leu Lys Tyr Ser Phe Ala Asp Tyr Gly Asp
   100 105 110
   Leu Ile Lys Gly Thr Ser Ile Trp Asp Asn Asp Phe Thr Lys Asp Leu
   115 120 125
   Glu Leu Asn Leu Gln Lys Ile Phe Gly Lys Leu Phe Arg Lys Tyr Ile
   130 135 140
   Lys Lys Asn Asn Thr Ala Glu Gln His Thr Ser Tyr Ser Ser Leu Asp
   145 150 155 160
   Glu Leu Arg Glu Ser Trp Trp Asn Thr Asn Lys Lys Tyr Ile Trp Leu
   165 170 175
   Ala Met Lys His Gly Ala Glu Met Asn Gly Thr Thr Cys Cys Gly Asp
   180 185 190
   Gly Ser Val Thr Gly Ser Ser Asp Ser Gly Ser Thr Thr Cys Ser Gly
           195                 200                 205
   Asp Asn Gly Ser Ile Ser Cys Asp Asp Met Pro Thr Thr Asp Phe Ile
       210                 215                 220
   Pro Gln Tyr Leu Arg Phe Leu Gln Glu Trp Val Glu His Phe Cys Lys
   225                 230                 235                 240
   Gln Arg Gln Glu Lys Val Lys His Val Met Glu Ser Cys Lys Ser Cys
                   245                 250                 255
   Lys Glu Cys Gly Asp Thr Cys Asn Gly Glu Cys Lys Thr Glu Cys Glu
               260                 265                 270
   Lys Lys Cys Lys Asn Lys Cys Glu Ala Tyr Lys Thr Phe Ile Glu Lys
           275                 280                 285
   Cys Val Ser Ala Asp Gly Gly Thr Ser Gly Ser Ser Trp Ser Lys Arg
       290                 295                 300
   Trp Asp Gln Ile Tyr Met Arg Tyr Ser Lys Tyr Ile Glu Asp Ala Lys
   305                 310                 315                 320
   Arg Asn Arg Lys Ala Gly Thr Lys Asn Cys Gly Thr Ser Ser Thr Thr
                   325                 330                 335
   Asn Ala Ala Ala Ser Thr Ala Glu Asn Lys Cys Val Gln Ser
               340                 345                 350
<210> 34
   <211> 647
   <212> PRT
   <213> Artificial
<220>
   <223> Artificial construct
<400> 34
   Asp Tyr Ile Lys Asp Asp Pro Tyr Ser Ala Glu Tyr Ala Thr Lys Leu
   1               5                   10                  15
   Ser Phe Ile Leu Asn Pro Ser Asp Ala Asn Thr Ser Ser Gly Glu Thr
               20                  25                  30
   Ala Asn His Asn Asp Glu Val Cys Asn Cys Asn Glu Ser Glu Ile Ala
           35                  40                  45
   Ser Val Glu Leu Ala Pro Ile Ser Asp Ser Ser Ser Asn Lys Thr Cys
       50                  55                  60
   Ile Thr His Ser Phe Ile Gly Ala Asn Lys Lys Lys Glu Cys Lys Asp
   65                  70                  75                  80
   Val Lys Leu Gly Val Arg Glu Lys Asp Lys Asp Leu Lys Ile Cys Val
                   85                  90                  95
   Ile Glu Asp Asp Ser Leu Arg Gly Val Glu Asn Cys Cys Cys Gln Asp
               100                 105                 110
   Leu Leu Gly Ile Leu Gln Glu Asn Cys Ser Asp Asn Lys Ser Gly Ser
           115                 120                 125
   Ser Ser Asn Gly Ser Cys Asp Lys Asn Ser Glu Asp Glu Cys Gln Lys
       130                 135                 140
   Lys Leu Glu Asn Val Phe Ala Ser Leu Lys Asn Gly Tyr Lys Cys Asp
   145                 150                 155                 160
   Lys Cys Lys Ser Gly Thr Ser Thr Val Asn Lys Lys Trp Ile Trp Arg
                   165                 170                 175
   Lys Tyr Ser Gly Asn Gly Glu Gly Leu Gln Lys Glu Tyr Ala Asn Thr
               180                 185                 190
   Ile Gly Leu Pro Pro Arg Thr His Ser Leu Tyr Leu Val Cys Leu His
           195                 200                 205
   Glu Lys Glu Gly Lys Thr Gln His Lys Thr Ile Ser Thr Asn Ser Glu
       210                 215                 220
   Leu Leu Lys Glu Trp Ile Ile Ala Ala Phe His Glu Gly Lys Asn Leu
   225                 230                 235                 240
   Lys Thr Ser His Gln Asn Asn Asn Ser Gly Asn Lys Lys Lys Leu Cys
                   245                 250                 255
   Lys Ala Leu Lys Tyr Ser Phe Ala Asp Tyr Gly Asp Leu Ile Lys Gly
               260                 265                 270
   Thr Ser Ile Trp Asp Asn Asp Phe Thr Lys Asp Leu Glu Leu Asn Leu
           275                 280                 285
   Gln Lys Ile Phe Gly Lys Leu Phe Arg Lys Tyr Ile Lys Lys Asn Ile
       290                 295                 300
   Ala Ser Asp Glu Asn Thr Ser Tyr Ser Ser Leu Asp Glu Leu Arg Glu
   305 310 315 320
   Ser Trp Trp Asn Thr Asn Lys Lys Tyr Ile Trp Leu Ala Met Lys His
   325 330 335
   Gly Ala Glu Met Asn Ser Thr Met Cys Asn Gly Asp Gly Ser Val Thr
   340 345 350
   Gly Ser Ser Asp Ser Gly Ser Thr Thr Cys Ser Gly Asp Asn Gly Ser
   355 360 365
   Ile Ser Cys Asp Asp Ile Pro Thr Ile Asp Leu Ile Pro Gln Tyr Leu
   370 375 380
   Arg Phe Leu Gln Glu Trp Val Glu His Phe Cys Lys Gln Arg Gln Glu
   385 390 395 400
   Lys Val Lys Asp Val Ile Thr Asn Cys Lys Ser Cys Lys Glu Ser Gly
   405 410 415
   Asp Thr Cys Asn Ser Asp Cys Glu Lys Lys Cys Lys Asn Lys Cys Glu
   420 425 430
   Ala Tyr Lys Lys Phe Ile Glu Glu Arg Arg Thr Ala Ala Gln Gly Thr
   435 440 445
   Ala Glu Ser Ser Trp Val Lys Arg Trp Asp Gln Ile Tyr Met Arg Tyr
   450 455 460
   Ser Lys Tyr Ile Glu Asp Ala Lys Arg Asn Arg Lys Ala Gly Thr Lys
   465 470 475 480
   Ser Cys Gly Pro Ser Ser Thr Thr Asn Ala Ala Ala Ser Thr Ala Glu
   485 490 495
   Asn Lys Cys Val Gln Ser Asp Ile Asp Ser Phe Phe Lys His Leu Ile
   500 505 510
   Asp Ile Gly Leu Thr Thr Pro Ser Ser Tyr Leu Ser Ile Val Leu Asp
   515 520 525
   Asp Asn Ile Cys Gly Ala Asp Asn Ala Pro Trp Thr Thr Tyr Thr Thr
   530 535 540
   Tyr Thr Thr Thr Lys Asn Cys Asp Ile Lys Lys Lys Thr Pro Lys Pro
   545 550 555 560
   Gln Ser Cys Asp Thr Leu Val Val Val Asn Val Pro Ser Pro Leu Gly
   565 570 575
   Asn Thr Pro His Glu Tyr Lys Tyr Ala Cys Gln Cys Arg Thr Pro Asn
   580 585 590
   Lys Gln Glu Ser Cys Asp Asp Arg Lys Glu Tyr Met Asn Gln Trp Ser
   595 600 605
   Ser Gly Ser Ala Gln Thr Val Arg Gly Arg Ser Thr Asn Asn Asp Tyr
   610 615 620
   Glu Leu Tyr Thr Tyr Asn Gly Val Lys Glu Thr Lys Pro Leu Gly Thr
   625 630 635 640
   Leu Lys Asn Ser Lys Leu Asp
   645
<210> 35
   <211> 341
   <212> PRT
   <213> Plasmodium falciparum
<400> 35
   Lys Cys Asp Lys Cys Lys Ser Gly Thr Ser Thr Val Asn Lys Lys Trp
   1               5                   10                  15
   Ile Trp Arg Lys Ser Ser Gly Asn Lys Glu Gly Leu Gln Lys Glu Tyr
               20                  25                  30
   Ala Asn Thr Ile Gly Leu Pro Pro Arg Thr Gln Ser Leu Tyr Leu Gly
           35                  40                  45
   Asn Leu Pro Lys Leu Glu Asn Val Cys Glu Asp Val Lys Asp Ile Asn
       50                  55                  60
   Phe Asp Thr Lys Glu Lys Phe Leu Ala Gly Cys Leu Ile Val Ser Phe
   65                  70                  75                  80
   His Glu Gly Lys Asn Leu Lys Thr Ser His Glu Lys Lys Asn Asp Asp
                   85                  90                  95
   Asn Gly Lys Lys Leu Cys Lys Ala Leu Glu Tyr Ser Phe Ala Asp Tyr
               100                 105                 110
   Gly Asp Leu Ile Lys Gly Thr Ser Ile Trp Asp Asn Glu Tyr Thr Lys
           115                 120                 125
   Asp Leu Glu Leu Asn Leu Gln Lys Ile Phe Gly Lys Leu Phe Arg Lys
       130                 135                 140
   Tyr Ile Lys Lys Asn Asn Thr Ala Glu Gln Asp Thr Ser Tyr Ser Ser
   145                 150                 155                 160
   Leu Asp Glu Leu Arg Glu Ser Trp Trp Asn Thr Asn Lys Lys Tyr Ile
                   165                 170                 175
   Trp Thr Ala Met Lys His Gly Ala Gly Met Asn Ile Thr Thr Cys Cys
               180                 185                 190
   Gly Asp Gly Ser Ser Gly Glu Asn Gln Thr Asn Ser Cys Asp Asp Ile
           195                 200                 205
   Pro Thr Ile Asp Leu Ile Pro Gln Tyr Leu Arg Phe Leu Gln Glu Trp
       210                 215                 220
   Val Glu His Phe Cys Lys Gln Arg Gln Glu Lys Val Asn Ala Val Val
   225                 230                 235                 240
   Thr Asn Cys Lys Ser Cys Lys Glu Ser Gly Gly Thr Cys Asn Gly Glu
                   245                 250                 255
   Cys Lys Thr Lys Cys Lys Asn Lys Cys Glu Val Tyr Lys Thr Phe Ile
               260                 265                 270
   Asp Asn Val Gly Asp Gly Thr Ala Gly Ser Pro Trp Val Lys Arg Trp
           275                 280                 285
   Asp Gln Ile Tyr Lys Arg Tyr Ser Lys His Ile Glu Asp Ala Lys Arg
       290                 295                 300
   Asn Arg Lys Ala Gly Thr Lys Asn Cys Gly Ile Thr Thr Gly Thr Ile
   305                 310                 315                 320
   Ser Gly Glu Ser Ser Gly Ala Thr Ser Gly Val Thr Thr Thr Glu Asn
                   325                 330                 335
   Lys Cys Val Gln Ser
               340
<210> 36
   <211> 632
   <212> PRT
   <213> Artificial
<220>
   <223> Artificial construct
<400> 36
   Asn Tyr Ile Lys Asp Asp Pro Tyr Ser Lys Glu Tyr Val Thr Lys Leu
   1               5                   10                  15
   Ser Phe Ile Pro Asn Ser Ser Asp Ala Asn Thr Ser Ser Glu Lys Ile
               20                  25                  30
   Gln Lys Asn Asn Asp Glu Val Cys Asn Pro Asn Glu Ser Gly Ile Ser
           35                  40                  45
   Ser Val Glu Gln Ala Gln Thr Ser Gly Pro Ser Ser Asn Lys Thr Cys
       50                  55                  60
   Ile Thr His Ser Ser Ile Lys Ala Asn Lys Lys Lys Glu Cys Lys Asp
   65                  70                  75                  80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Asp Ser Cys Asp Asn Lys Asn Gln Asp Glu Cys Gln Lys
       130                 135                 140
   Lys Leu Asp Glu Ala Leu Glu Ser Leu His Asn Gly Tyr Lys Asn Gln
   145                 150                 155                 160
   Lys Cys Lys Ser Gly Thr Ser Thr Val Asn Lys Lys Trp Ile Trp Lys
                   165                 170                 175
   Lys Ser Ser Gly Asn Lys Glu Gly Leu Gln Lys Glu Tyr Ala Asn Thr
               180                 185                 190
   Ile Gly Leu Pro Pro Arg Thr Gln Ser Leu Tyr Leu Gly Asn Leu Pro
           195                 200                 205
   Lys Leu Glu Asn Val Ser Lys Gly Val Thr Asp Ile Ile Tyr Asp Thr
       210                 215                 220
   Lys Glu Lys Phe Leu Ala Gly Cys Leu Ile Val Ser Phe His Glu Gly
   225 230 235 240
   Lys Asn Leu Lys Thr Ser His Glu Lys Lys Asn Asp Asp Asn Gly Lys
   245 250 255
   Lys Leu Cys Lys Ala Leu Glu Tyr Ser Phe Ala Asp Tyr Gly Asp Leu
   260 265 270
   Ile Lys Gly Thr Ser Ile Trp Asp Asn Glu Tyr Thr Lys Asp Leu Glu
   275 280 285
   Leu Asn Leu Gln Lys Ala Phe Gly Lys Leu Phe Arg Lys Tyr Ile Lys
   290 295 300
   Lys Asn Ile Ser Ala Glu Gln Asp Thr Ser Tyr Ser Ser Leu Asp Glu
   305 310 315 320
   Leu Arg Glu Ser Trp Trp Asn Thr Asn Lys Lys Tyr Ile Trp Ile Ala
   325 330 335
   Met Lys His Gly Ala Gly Met Asn Gly Thr Thr Cys Cys Gly Asp Gly
   340 345 350
   Ser Ser Gly Glu Asn Gln Thr Asn Ser Cys Asp Asp Ile Pro Thr Ile
   355 360 365
   Asp Leu Ile Pro Gln Tyr Leu Arg Phe Leu Gln Glu Trp Val Glu His
   370 375 380
   Phe Cys Glu Gln Arg Gln Ala Lys Val Lys Asp Val Ile Thr Asn Cys
   385 390 395 400
   Lys Ser Cys Lys Asn Thr Ser Gly Glu Arg Lys Ile Gly Gly Thr Cys
   405 410 415
   Asn Gly Glu Cys Lys Thr Lys Cys Lys Asn Lys Cys Glu Ala Tyr Lys
   420 425 430
   Thr Phe Ile Glu His Cys Lys Gly Gly Asp Gly Thr Ala Gly Ser Ser
   435 440 445
   Trp Val Lys Arg Trp Asp Gln Ile Tyr Lys Arg Tyr Ser Lys His Ile
   450 455 460
   Glu Asp Ala Lys Arg Asn Arg Lys Ala Gly Thr Lys Ser Cys Gly Thr
   465 470 475 480
   Ser Thr Ala Glu Asn Lys Cys Val Gln Ser Asp Ile Asp Ser Phe Phe
                   485                 490                 495
   Lys His Leu Ile Asp Ile Gly Leu Thr Thr Pro Ser Ser Tyr Leu Ser
               500                 505                 510
   Ile Val Leu Asp Glu Asn Asn Cys Gly Glu Asp Lys Ala Pro Trp Thr
           515                 520                 525
   Thr Tyr Thr Thr Thr Lys Asn Cys Asp Ile Gln Lys Asp Lys Ser Lys
       530                 535                 540
   Ser Gln Ser Ser Asp Thr Leu Val Val Val Asn Val Pro Ser Pro Leu
   545                 550                 555                 560
   Gly Asn Thr Pro His Gly Tyr Lys Tyr Ala Cys Gln Cys Lys Ile Pro
                   565                 570                 575
   Thr Thr Glu Glu Thr Cys Asp Asp Arg Lys Glu Tyr Met Asn Gln Trp
               580                 585                 590
   Ser Cys Gly Ser Ala Arg Thr Met Lys Arg Gly Tyr Lys Asn Asp Asn
           595                 600                 605
   Tyr Glu Leu Cys Lys Tyr Asn Gly Val Asp Val Lys Pro Thr Thr Val
       610                 615                 620
   Arg Ser Ser Ser Thr Lys Leu Asp
   625                 630
<210> 37
   <211> 639
   <212> PRT
   <213> Artificial
<220>
   <223> Artificial construct
<400> 37
   Asp Tyr Ile Lys Gly Asp Pro Tyr Ser Ala Glu Tyr Val Thr Lys Leu
   1               5                   10                  15
   Ser Phe Ile Pro Asn Ser Ser Asp Ala Asn Asn Pro Ser Glu Lys Ile
               20                  25                  30
   Gln Lys Asn Asn Asp Glu Val Cys Asn Cys Asn Glu Ser Glu Ile Ser
           35                  40                  45
   Ser Val Gly Gln Ala Ser Ile Ser Asp Pro Ser Ser Asn Lys Thr Cys
       50                  55                  60
   Asn Thr His Ser Ser Ile Lys Ala Asn Lys Lys Lys Val Cys Lys Asp
   65                  70                  75                  80
   Val Lys Leu Gly Val Arg Glu Asn Asp Lys Val Leu Lys Ile Cys Val
                   85                  90                  95
   Ile Glu His Thr Ser Leu Arg Gly Val Asp Asn Cys Cys Phe Lys Asp
               100                 105                 110
   Leu Leu Gly Ile Leu Gln Glu Pro Arg Ile Asp Lys Asn Gln Ser Gly
           115                 120                 125
   Ser Ser Ser Asn Gly Ser Cys Asp Lys Asn Ser Glu Glu Ala Cys Glu
       130                 135                 140
   Lys Asn Leu Glu Lys Val Leu Ala Ser Leu Thr Asn Gly Tyr Lys Cys
   145                 150                 155                 160
   Asp Lys Cys Lys Ser Gly Thr Ser Arg Ser Lys Lys Lys Trp Ile Trp
                   165                 170                 175
   Lys Lys Tyr Ser Gly Lys Glu Gly Gly Leu Gln Glu Glu Tyr Ala Asn
               180                 185                 190
   Thr Ile Gly Leu Pro Pro Arg Thr Gln Ser Leu Cys Leu Val Val Cys
           195                 200                 205
   Leu Asp Glu Lys Glu Gly Lys Thr Gln Glu Leu Lys Asn Ile Ser Thr
       210                 215                 220
   Asn Ser Glu Leu Leu Lys Glu Trp Ile Ile Ala Ala Phe Pro Glu Gly
   225                 230                 235                 240
   Lys Asn Leu Lys Pro Ser Pro Glu Lys Lys Lys Gly Asp Asn Gly Lys
                   245                 250                 255
   Lys Leu Cys Lys Asp Leu Lys Tyr Ser Phe Ala Asp Tyr Gly Asp Leu
               260                 265                 270
   Ile Lys Gly Thr Ser Ile Trp Asp Asn Glu Tyr Thr Lys Asp Leu Glu
           275                 280                 285
   Leu Asn Leu Gln Lys Ile Phe Gly Lys Leu Phe Arg Lys Tyr Ile Lys
       290                 295                 300
   Lys Asn Ile Ala Ser Asp Glu Asn Thr Leu Tyr Ser Ser Leu Asp Glu
   305                 310                 315                 320
   Leu Arg Glu Ser Trp Trp Asn Thr Asn Lys Lys Tyr Ile Trp Leu Ala
                   325                 330                 335
   Met Lys His Gly Ala Gly Met Asn Ser Thr Met Cys Asn Ala Asp Gly
               340                 345                 350
   Ser Val Thr Gly Ser Gly Ser Ser Cys Asp Asp Met Pro Thr Ile Asp
           355                 360                 365
   Leu Ile Pro Gln Tyr Leu Arg Phe Leu Gln Glu Trp Val Glu His Phe
       370                 375                 380
   Cys Lys Gln Arg Gln Glu Lys Val Lys Pro Val Ile Glu Asn Cys Asn
   385                 390                 395                 400
   Ser Cys Lys Asn Thr Ser Ser Glu Arg Lys Ile Gly Gly Thr Cys Asn
                   405                 410                 415
   Ser Asp Cys Lys Thr Glu Cys Lys Asn Lys Cys Glu Val Tyr Lys Lys
               420                 425                 430
   Phe Ile Glu Asp Cys Lys Gly Gly Asp Gly Thr Ala Gly Ser Ser Trp
           435                 440                 445
   Ser Lys Arg Trp Asp Gln Ile Tyr Lys Arg Tyr Ser Lys Tyr Ile Glu
       450                 455                 460
   Asp Ala Lys Arg Asn Arg Lys Ala Gly Thr Lys Asn Cys Gly Pro Ser
   465                 470                 475                 480
   Ser Thr Thr Asn Ala Ala Glu Asn Lys Cys Val Gln Ser Asp Ile Asp
                   485                 490                 495
   Ser Phe Phe Lys His Leu Ile Asp Ile Gly Leu Thr Thr Pro Ser Ser
               500                 505                 510
   Tyr Leu Ser Thr Val Leu Asp Asp Asn Ile Cys Gly Glu Asp Asn Ala
           515                 520                 525
   Pro Trp Thr Thr Tyr Thr Thr Tyr Thr Thr Thr Lys Asn Cys Asp Lys
       530                 535                 540
   Asp Lys Lys Lys Ser Lys Ser Gln Ser Cys Asp Thr Leu Val Val Val
   545                 550                 555                 560
   Asn Val Pro Ser Pro Leu Gly Asn Thr Pro His Glu Tyr Lys Tyr Ala
   565 570 575
   Cys Glu Cys Arg Thr Pro Asn Lys Gln Glu Ser Cys Asp Asp Arg Lys
   580 585 590
   Glu Tyr Met Asn Gln Trp Ile Ser Asp Asn Thr Lys Asn Pro Lys Gly
   595 600 605
   Ser Gly Ser Gly Lys Asp Tyr Tyr Glu Leu Tyr Thr Tyr Asn Gly Val
   610 615 620
   Asp Val Lys Pro Thr Thr Val Arg Ser Ser Ser Thr Lys Leu Asp
   625 630 635
<210> 38
   <211> 655
   <212> PRT
   <213> Artificial
<220>
   <223> Artificial construct
<400> 38
   Asp Tyr Ile Lys Gly Asp Pro Tyr Phe Ala Glu Tyr Ala Thr Lys Leu
   1 5 10 15
   Ser Phe Ile Leu Asn Ser Ser Asp Ala Asn Thr Ser Ser Gly Glu Thr
   20 25 30
   Ala Asn His Asn Asp Glu Ala Cys Asn Cys Asn Glu Ser Glu Ile Ser
   35 40 45
   Ser Val Glu His Ala Ser Ile Ser Asp Pro Ser Ser Asn Lys Thr Cys
   50 55 60
   Asn Thr His Ser Ser Ile Lys Ala Asn Lys Lys Lys Val Cys Lys His
   65 70 75 80
   Val Lys Leu Gly Val Arg Glu Asn Asp Lys Asp Leu Arg Val Cys Val
   85 90 95
   Ile Glu His Thr Ser Leu Ser Gly Val Glu Asn Cys Cys Phe Lys Asp
   100 105 110
   Phe Leu Arg Ile Leu Gln Glu Asn Cys Ser Asp Asn Lys Ser Gly Ser
   115 120 125
   Ser Ser Asn Gly Ser Cys Asp Lys Asn Asn Glu Glu Ala Cys Glu Lys
       130                 135                 140
   Asn Leu Glu Lys Val Phe Ala Ser Leu Thr Asn Cys Tyr Lys Cys Glu
   145                 150                 155                 160
   Lys Cys Lys Ser Glu Gln Ser Lys Lys Asn Asn Lys Lys Trp Thr Trp
                   165                 170                 175
   Arg Lys Ser Ser Gly Asn Lys Gly Gly Leu Gln Glu Glu Tyr Ala Asn
               180                 185                 190
   Thr Ile Gly Leu Pro Pro Arg Thr Gln Ser Leu Cys Leu Val Val Cys
           195                 200                 205
   Leu Asp Glu Lys Glu Gly Lys Lys Thr Gln Glu Leu Lys Asn Ile Arg
       210                 215                 220
   Thr Asn Ser Glu Leu Leu Lys Glu Trp Ile Ile Ala Ala Phe His Glu
   225                 230                 235                 240
   Gly Lys Asn Leu Lys Pro Ser His Glu Lys Lys Asn Asp Asp Asn Gly
                   245                 250                 255
   Lys Lys Asn Asp Asp Asn Asn Ser Lys Leu Cys Lys Asp Leu Lys Tyr
               260                 265                 270
   Ser Phe Ala Asp Tyr Gly Asp Leu Ile Lys Gly Thr Ser Ile Trp Asp
           275                 280                 285
   Asn Glu Tyr Thr Lys Asp Leu Glu Leu Asn Leu Gln Lys Ile Phe Gly
       290                 295                 300
   Lys Leu Phe Arg Lys Tyr Ile Lys Lys Asn Ile Ala Ser Asp Glu Asn
   305                 310                 315                 320
   Thr Leu Tyr Ser Ser Leu Asp Glu Leu Arg Glu Ser Trp Trp Asn Thr
                   325                 330                 335
   Asn Lys Lys Tyr Ile Trp Leu Ala Met Lys His Gly Ala Glu Met Asn
               340                 345                 350
   Gly Thr Thr Cys Asn Ala Asp Gly Ser Val Thr Gly Ser Gly Ser Ser
           355                 360                 365
   Cys Asp Asp Ile Pro Thr Ile Asp Leu Ile Pro Gln Tyr Leu Arg Phe
       370                 375                 380
   Leu Gln Glu Trp Val Glu His Phe Cys Lys Gln Arg Gln Ala Lys Val
   385 390 395 400
   Lys Asp Val Ile Glu Asn Cys Lys Ser Cys Lys Glu Ser Gly Asn Lys
   405 410 415
   Cys Lys Thr Glu Cys Lys Asn Lys Cys Glu Ala Tyr Lys Lys Phe Ile
   420 425 430
   Glu Asn Cys Lys Gly Gly Asp Gly Thr Ala Gly Ser Ser Trp Val Lys
   435 440 445
   Arg Trp Asp Gln Ile Tyr Met Arg Tyr Ser Lys Tyr Ile Glu Asp Ala
   450 455 460
   Lys Arg Asn Arg Lys Ala Gly Thr Lys Asn Cys Gly Pro Ser Ser Ile
   465 470 475 480
   Thr Asn Val Ser Ala Ser Thr Asp Glu Asn Lys Cys Val Gln Ser Asp
   485 490 495
   Ile Asp Ser Phe Phe Lys His Leu Ile Asp Ile Gly Leu Thr Thr Pro
   500 505 510
   Ser Ser Tyr Leu Ser Ile Val Leu Asp Asp Asn Ile Cys Gly Asp Asp
   515 520 525
   Lys Ala Pro Trp Thr Thr Tyr Thr Thr Tyr Thr Thr Tyr Thr Thr Tyr
   530 535 540
   Thr Thr Tyr Thr Thr Tyr Thr Thr Tyr Thr Thr Thr Lys Asn Cys Asp
   545 550 555 560
   Lys Glu Arg Asp Lys Ser Lys Ser Gln Ser Cys Asn Thr Ala Val Val
   565 570 575
   Val Asn Val Pro Ser Pro Leu Gly Asn Thr Pro His Glu Tyr Lys Tyr
   580 585 590
   Ala Cys Glu Cys Arg Thr Pro Ser Asn Lys Glu Leu Cys Asp Asp Arg
   595 600 605
   Lys Glu Tyr Met Asn Gln Trp Ser Ser Gly Ser Ala Gln Thr Val Arg
   610 615 620
   Asp Arg Ser Gly Lys Asp Tyr Tyr Glu Leu Tyr Thr Tyr Asn Gly Val
   625 630 635 640
   Lys Glu Thr Lys Leu Pro Lys Lys Leu Asn Ser Ser Lys Leu Asp
   645 650 655
<210> 39
   <211> 347
   <212> PRT
   <213> Plasmodium falciparum
<400> 39
   Lys Cys Asp Lys Cys Lys Ser Glu Gln Ser Lys Lys Asn Asn Lys Tyr
   1 5 10 15
   Trp Ile Trp Lys Lys Ser Ser Val Lys Glu Glu Gly Leu Gln Lys Glu
   20 25 30
   Tyr Ala Asn Thr Ile Ala Leu Pro Pro Arg Thr His Ser Leu Cys Leu
   35 40 45
   Val Val Cys Leu Asp Glu Lys Gly Lys Lys Thr Gln Glu Leu Lys Asn
   50 55 60
   Ile Ser Thr Asn Ser Glu Leu Leu Lys Glu Arg Ile Ile Ala Ala Phe
   65 70 75 80
   His Glu Gly Lys Asn Leu Lys Thr Thr Tyr Leu Glu Lys Lys Asn Ala
   85 90 95
   Asp Asn Asn Ser Lys Leu Cys Lys Ala Leu Lys Tyr Ser Phe Ala Asp
   100 105 110
   Tyr Gly Asp Leu Ile Lys Gly Thr Ser Ile Trp Asp Asn Glu Tyr Thr
   115 120 125
   Lys Asp Leu Glu Leu Asn Leu Gln Gln Ile Phe Gly Lys Leu Phe Arg
   130 135 140
   Lys Tyr Ile Lys Lys Asn Asn Thr Ala Glu Gln His Thr Leu Tyr Ser
   145 150 155 160
   Ser Leu Asp Glu Leu Arg Glu Ser Trp Trp Asn Thr Asn Lys Lys Tyr
   165 170 175
   Ile Trp Leu Ala Met Lys His Gly Ala Gly Met Asn Gly Thr Thr Cys
   180 185 190
   Cys Gly Asp Gly Ser Val Thr Gly Ser Ser Asp Ser Gly Ser Thr Thr
   195 200 205
   Cys Ser Gly Asp Asn Gly Ser Ile Ser Cys Asp Asp Met Pro Thr Thr
       210                 215                 220
   Asp Phe Ile Pro Gln Tyr Leu Arg Phe Leu Gln Glu Trp Val Glu His
   225                 230                 235                 240
   Phe Cys Lys Gln Arg Gln Glu Lys Val Lys Asp Val Ile Glu Asn Cys
                   245                 250                 255
   Asn Ser Cys Lys Asn Asn Leu Gly Lys Thr Glu Ile Asn Glu Lys Cys
               260                 265                 270
   Lys Thr Glu Cys Lys Asn Lys Cys Glu Ala Tyr Lys Asn Phe Ile Glu
           275                 280                 285
   Lys Phe Cys Thr Ala Asp Gly Gly Thr Ser Gly Ser Pro Trp Ser Lys
       290                 295                 300
   Arg Trp Asp Gln Ile Tyr Lys Arg Tyr Ser Lys Tyr Ile Glu Asp Ala
   305                 310                 315                 320
   Lys Arg Asn Arg Lys Ala Gly Thr Lys Asn Cys Gly Thr Ser Ser Thr
                   325                 330                 335
   Thr Ser Thr Ala Glu Asn Lys Cys Val Gln Ser
               340                 345
<210> 40
   <211> 335
   <212> PRT
   <213> Plasmodium falciparum
<400> 40
   Lys Cys Glu Lys Cys Lys Ser Gly Thr Ser Thr Val Asn Lys Tyr Trp
   1               5                   10                  15
   Ile Trp Arg Lys Ser Ser Gly Asn Lys Glu Gly Leu Gln Lys Glu Tyr
               20                  25                  30
   Ala Asn Thr Ile Ala Leu Pro Pro Arg Thr His Ser Leu Cys Leu Val
           35                  40                  45
   Val Cys Leu Asp Glu Lys Glu Gly Lys Thr Gln Glu Leu Lys Asn Ile
       50                  55                  60
   Ser Thr Asn Ser Glu Leu Leu Lys Glu Arg Ile Ile Ala Ala Phe His
   65                  70                  75                  80
   Glu Gly Glu Asn Leu Lys Thr Ser His Glu Lys Lys Lys Gly Asp Asp
                   85                  90                  95
   Gly Lys Lys Asn Ala Asp Asn Asn Ser Lys Leu Cys Lys Ala Leu Lys
               100                 105                 110
   Tyr Ser Phe Ala Asp Tyr Gly Asp Leu Ile Lys Gly Thr Ser Ile Trp
           115                 120                 125
   Asp Asn Glu Tyr Thr Lys Asp Leu Glu Leu Asn Leu Gln Lys Ile Phe
       130                 135                 140
   Gly Lys Leu Phe Arg Lys Tyr Ile Lys Lys Asn Ile Ala Ser Asp Glu
   145                 150                 155                 160
   Asn Thr Ser Tyr Ser Ser Leu Asp Glu Leu Arg Glu Ser Trp Trp Asn
                   165                 170                 175
   Thr Asn Lys Lys Tyr Ile Trp Leu Ala Met Lys His Gly Ala Gly Met
               180                 185                 190
   Asn Gly Thr Thr Cys Ser Cys Ser Gly Asp Ser Ser Asp Asp Met Pro
           195                 200                 205
   Thr Thr Asp Phe Ile Pro Gln Tyr Leu Arg Phe Leu Gln Glu Trp Val
       210                 215                 220
   Glu His Phe Cys Lys Gln Arg Gln Glu Asn Val Asn Ala Val Ile Glu
   225                 230                 235                 240
   Asn Cys Asn Ser Cys Lys Glu Cys Gly Gly Thr Cys Asn Ser Asp Cys
                   245                 250                 255
   Glu Lys Lys Cys Lys Thr Glu Cys Lys Asn Lys Cys Glu Ala Tyr Lys
               260                 265                 270
   Asn Phe Ile Glu Lys Phe Cys Thr Ala Asp Gly Gly Thr Ser Gly Tyr
           275                 280                 285
   Ser Trp Ser Lys Arg Trp Asp Gln Ile Tyr Lys Arg Tyr Ser Lys Tyr
       290                 295                 300
   Ile Glu Asp Ala Lys Arg Asn Arg Lys Ala Gly Thr Lys Ser Cys Gly
   305                 310                 315                 320
   Thr Ser Ser Thr Thr Ser Thr Ala Glu Ser Lys Cys Val Gln Ser
   325 330 335
<210> 41
   <211> 667
   <212> PRT
   <213> Artificial
<220>
   <223> Artificial construct
<400> 41
   Ser Tyr Val Lys Asn Asn Pro Tyr Ser Lys Glu Tyr Val Thr Lys Leu
   1               5                   10                  15
   Ser Phe Ile Leu Asn Pro Ser Asp Ala Asn Asn Pro Ser Glu Thr Pro
               20                  25                  30
   Ser Lys Tyr Tyr Asp Glu Val Cys Asn Cys Asn Glu Ser Gly Ile Ala
           35                  40                  45
   Cys Val Gly Gln Ala Gln Thr Ser Gly Pro Ser Ser Asn Lys Thr Cys
       50                  55                  60
   Ile Thr His Ser Phe Ile Gly Ala Asn Lys Lys Lys Val Cys Lys Asp
   65                  70                  75                  80
   Val Lys Leu Gly Val Arg Glu Lys Asp Lys Asp Leu Lys Ile Cys Val
                   85                  90                  95
   Ile Glu Asp Thr Tyr Leu Ser Gly Val Asp Asn Cys Cys Phe Lys Asp
               100                 105                 110
   Phe Leu Gly Met Leu Gln Glu Asn Cys Ser Asp Asn Lys Ser Gly Ser
           115                 120                 125
   Ser Ser Asn Gly Ser Cys Asn Asn Lys Asn Gln Asp Glu Cys Glu Lys
       130                 135                 140
   Asn Leu Asp Glu Ala Leu Ala Ser Leu Thr Asn Gly Tyr Lys Cys Glu
   145                 150                 155                 160
   Lys Cys Lys Ser Gly Thr Ser Thr Val Asn Lys Tyr Trp Ile Trp Arg
                   165                 170                 175
   Lys Ser Ser Gly Asn Lys Glu Gly Leu Gln Lys Glu Tyr Ala Asn Thr
               180                 185                 190
   Ile Ala Leu Pro Pro Arg Thr His Ser Leu Cys Leu Val Val Cys Leu
           195                 200                 205
   Asp Glu Lys Glu Gly Lys Thr Gln His Lys Thr Ile Ser Thr Asn Ser
       210                 215                 220
   Glu Leu Leu Lys Glu Trp Ile Ile Ala Ala Phe His Glu Gly Lys Asn
   225                 230                 235                 240
   Leu Lys Thr Ser His Glu Lys Lys Lys Gly Asp Asp Gly Lys Lys Asn
                   245                 250                 255
   Ala Asp Asn Asn Ser Lys Leu Cys Lys Ala Leu Lys Tyr Ser Phe Ala
               260                 265                 270
   Asp Tyr Gly Asp Leu Ile Lys Gly Thr Ser Ile Trp Asp Asn Asp Phe
           275                 280                 285
   Thr Lys Asp Leu Glu Leu Asn Leu Gln Lys Ile Phe Gly Lys Leu Phe
       290                 295                 300
   Arg Lys Tyr Ile Lys Lys Asn Ile Ala Ser Asp Glu Asn Thr Ser Tyr
   305                 310                 315                 320
   Ser Ser Leu Asp Glu Leu Arg Glu Ser Trp Trp Asn Thr Asn Lys Lys
                   325                 330                 335
   Tyr Ile Trp Leu Ala Met Lys His Gly Ala Gly Met Asn Ser Thr Thr
               340                 345                 350
   Cys Cys Gly Asp Gly Ser Val Thr Gly Ser Ser Asp Ser Gly Ser Thr
           355                 360                 365
   Thr Cys Cys Gly Asp Gly Ser Val Thr Gly Ser Gly Ser Ser Cys Asp
       370                 375                 380
   Asp Met Pro Thr Thr Asp Phe Ile Pro Gln Tyr Leu Arg Phe Leu Gln
   385                 390                 395                 400
   Glu Trp Val Glu His Phe Cys Lys Gln Arg Gln Glu Asn Val Asn Ala
                   405                 410                 415
   Val Ile Glu Asn Cys Asn Ser Cys Lys Glu Cys Gly Gly Thr Cys Asn
               420                 425                 430
   Ser Asp Cys Glu Lys Lys Cys Lys Thr Glu Cys Lys Gly Glu Cys Asp
           435                 440                 445
   Ala Tyr Lys Glu Phe Ile Glu Lys Cys Asn Gly Gly Ala Ala Glu Gly
   450 455 460
   Thr Ser Gly Ser Ser Trp Ser Lys Arg Trp Asp Gln Ile Tyr Lys Arg
   465 470 475 480
   Tyr Ser Lys Tyr Ile Glu Asp Ala Lys Arg Asn Arg Lys Ala Gly Thr
   485 490 495
   Lys Asn Cys Gly Thr Ser Ser Thr Thr Ser Thr Ala Glu Ser Lys Cys
   500 505 510
   Val Gln Ser Asp Ile Asp Ser Phe Phe Lys His Leu Ile Asp Ile Gly
   515 520 525
   Leu Thr Thr Pro Ser Ser Tyr Leu Ser Ile Val Leu Asp Glu Asn Ile
   530 535 540
   Cys Gly Ala Asp Asn Ala Pro Trp Thr Thr Tyr Thr Thr Tyr Thr Thr
   545 550 555 560
   Tyr Thr Thr Tyr Thr Thr Thr Glu Lys Cys Asn Lys Glu Thr Asp Lys
   565 570 575
   Ser Lys Leu Gln Gln Cys Asn Thr Ser Val Val Val Asn Val Pro Ser
   580 585 590
   Pro Leu Gly Asn Thr Pro His Gly Tyr Lys Tyr Val Cys Glu Cys Arg
   595 600 605
   Thr Pro Asn Lys Gln Glu Thr Cys Asp Asp Arg Lys Glu Tyr Met Asn
   610 615 620
   Gln Trp Ile Ser Asp Asn Thr Lys Asn Pro Lys Gly Ser Arg Ser Thr
   625 630 635 640
   Asn Asn Asp Tyr Glu Leu Tyr Thr Tyr Asn Gly Val Gln Ile Lys Pro
   645 650 655
   Thr Thr Val Arg Ser Asn Ser Thr Lys Leu Asp
   660 665
<210> 42
   <211> 348
   <212> PRT
   <213> Plasmodium falciparum
<400> 42
   Lys Cys Asp Lys Cys Lys Ser Glu Gln Ser Lys Lys Asn Asn Lys Asn
   1               5                   10                  15
   Trp Ile Trp Lys Lys Ser Ser Gly Asn Glu Lys Gly Leu Gln Lys Glu
               20                  25                  30
   Tyr Ala Asn Thr Ile Gly Leu Pro Pro Arg Thr Gln Ser Leu Cys Leu
           35                  40                  45
   Val Val Cys Leu Asp Glu Lys Glu Gly Lys Thr Gln Glu Leu Lys Asn
       50                  55                  60
   Ile Arg Thr Asn Ser Glu Leu Leu Lys Glu Trp Ile Ile Ala Ala Phe
   65                  70                  75                  80
   His Glu Gly Lys Asn Leu Lys Thr Ser His Glu Lys Lys Lys Gly Asp
                   85                  90                  95
   Asn Asn Ser Lys Leu Cys Lys Asp Leu Lys Tyr Ser Phe Ala Asp Tyr
               100                 105                 110
   Gly Asp Leu Ile Lys Gly Thr Ser Ile Trp Asp Asn Glu Tyr Thr Lys
           115                 120                 125
   Asp Leu Glu Leu Asn Leu Gln Asn Asn Phe Gly Lys Leu Phe Arg Lys
       130                 135                 140
   Tyr Ile Lys Lys Asn Ile Ala Ser Asp Glu Asn Thr Ser Tyr Ser Ser
   145                 150                 155                 160
   Leu Asp Glu Leu Arg Glu Ser Trp Trp Asn Thr Asn Lys Lys Tyr Ile
                   165                 170                 175
   Trp Leu Ala Met Lys His Gly Ala Gly Met Asn Ser Thr Thr Cys Ser
               180                 185                 190
   Ser Gly Ser Gly Ser Thr Thr Cys Ser Ser Gly Ser Gly Ser Thr Thr
           195                 200                 205
   Cys Ser Ser Gly Ser Gly Asp Ser Cys Asp Asp Met Pro Thr Ile Asp
       210                 215                 220
   Leu Ile Pro Gln Tyr Leu Arg Phe Leu Gln Glu Trp Val Glu His Phe
   225                 230                 235                 240
   Cys Lys Gln Arg Gln Glu Lys Val Asn Ala Val Ile Lys Asn Cys Asn
                   245                 250                 255
   Ser Cys Lys Glu Ser Gly Gly Thr Cys Asn Gly Glu Cys Lys Thr Glu
               260                 265                 270
   Cys Lys Asn Lys Cys Glu Ala Tyr Lys Thr Phe Ile Glu Glu Phe Cys
           275                 280                 285
   Thr Ala Asp Gly Gly Thr Ser Gly Ser Pro Trp Ser Lys Arg Trp Asp
       290                 295                 300
   Gln Ile Tyr Lys Met Tyr Ser Lys His Ile Glu Asp Ala Lys Arg Asn
   305                 310                 315                     320
   Arg Lys Ala Gly Thr Lys Asn Cys Gly Pro Ser Ser Thr Thr Asn Val
                   325                 330                 335
   Ser Val Ser Thr Asp Glu Asn Lys Cys Val Gln Ser
               340                 345
<210> 43
   <211> 652
   <212> PRT
   <213> Artificial
<220>
   <223> Artificial construct
<400> 43
   Asp Tyr Ile Lys Asp Asp Pro Tyr Phe Ala Glu Tyr Val Thr Lys Leu
   1               5                   10                  15
   Ser Phe Ile Leu Asn Ser Ser Asp Ala Asn Asn Pro Ser Gly Glu Thr
               20                  25                  30
   Ala Asn His Asn Asp Glu Val Cys Asn Pro Asn Glu Ser Gly Ile Ala
           35                  40                  45
   Ser Val Glu Gln Ala Gln Thr Ser Asp Pro Ser Ser Asn Lys Thr Cys
       50                  55                  60
   Asn Thr His Ser Ser Ile Lys Ala Asn Lys Lys Lys Val Cys Lys His
   65                  70                  75                  80
   Val Lys Leu Gly Val Arg Glu Asn Asp Lys Asp Leu Lys Ile Cys Val
                   85                  90                  95
   Ile Glu His Thr Ser Leu Ser Gly Val Glu Asn Cys Cys Cys Gln Asp
               100                 105                 110
   Phe Leu Arg Ile Leu Gln Glu Asn Cys Ser Asp Asn Lys Ser Gly Ser
   115 120 125
   Ser Ser Asn Gly Ser Cys Asn Asn Lys Asn Gln Glu Ala Cys Glu Lys
   130 135 140
   Asn Leu Glu Lys Val Leu Ala Ser Leu Thr Asn Cys Tyr Lys Cys Asp
   145 150 155 160
   Lys Cys Lys Ser Glu Gln Ser Lys Lys Asn Asn Lys Asn Trp Ile Trp
   165 170 175
   Lys Lys Ser Ser Gly Asn Glu Lys Gly Leu Gln Lys Glu Tyr Ala Asn
   180 185 190
   Thr Ile Gly Leu Pro Pro Arg Thr Gln Ser Leu Cys Leu Val Val Cys
   195 200 205
   Leu Asp Glu Lys Glu Gly Lys Thr Gln Glu Leu Lys Asn Ile Arg Thr
   210 215 220
   Asn Ser Glu Leu Leu Lys Glu Trp Ile Ile Ala Ala Phe His Glu Gly
   225 230 235 240
   Lys Asn Leu Lys Lys Arg Tyr Pro Gln Asn Lys Asn Asp Asp Asn Asn
   245 250 255
   Ser Lys Leu Cys Lys Asp Leu Lys Tyr Ser Phe Ala Asp Tyr Gly Asp
   260 265 270
   Leu Ile Lys Gly Thr Ser Ile Trp Asp Asn Glu Tyr Thr Lys Asp Leu
   275 280 285
   Glu Leu Asn Leu Gln Asn Asn Phe Gly Lys Leu Phe Arg Lys Tyr Ile
   290 295 300
   Lys Lys Asn Ile Ser Thr Glu Gln Asp Thr Leu Tyr Ser Ser Leu Asp
   305 310 315 320
   Glu Leu Arg Glu Ser Trp Trp Asn Thr Asn Lys Lys Tyr Ile Trp Leu
   325 330 335
   Ala Met Lys His Gly Ala Gly Met Asn Ser Thr Thr Cys Ser Ser Gly
   340 345 350
   Ser Gly Ser Thr Thr Cys Ser Ser Gly Ser Gly Ser Thr Thr Cys Ser
   355 360 365
   Ser Gly Ser Gly Asp Ser Cys Asp Asp Met Pro Thr Thr Asp Phe Ile
       370                 375                 380
   Pro Gln Tyr Leu Arg Phe Leu Gln Glu Trp Val Glu His Phe Cys Lys
   385                 390                 395                 400
   Gln Arg Gln Glu Lys Val Asn Ala Val Ile Lys Asn Cys Asn Ser Cys
                   405                 410                 415
   Lys Glu Ser Gly Gly Thr Cys Asn Gly Glu Cys Lys Thr Glu Cys Lys
               420                 425                 430
   Asn Lys Cys Glu Ala Tyr Lys Thr Phe Ile Glu Glu Phe Cys Thr Ala
           435                 440                 445
   Asp Gly Gly Thr Ser Gly Ser Pro Trp Ser Lys Arg Trp Asp Gln Ile
       450                 455                 460
   Tyr Lys Met Tyr Ser Lys His Ile Glu Asp Ala Lys Arg Asn Arg Lys
   465                 470                 475                 480
   Ala Gly Thr Lys Asn Cys Gly Pro Ser Ser Thr Thr Asn Val Ser Val
                   485                 490                 495
   Ser Thr Asp Glu Asn Lys Cys Val Gln Ser Asp Ile Asp Ser Phe Phe
               500                 505                 510
   Lys His Leu Ile Asp Ile Gly Leu Thr Thr Pro Ser Ser Tyr Leu Ser
           515                 520                 525
   Ile Val Leu Asp Asp Asn Ile Cys Gly Glu Asp Lys Ala Pro Trp Thr
       530                 535                 540
   Thr Tyr Thr Thr Tyr Thr Thr Thr Lys Lys Cys Asn Lys Glu Thr Asp
   545                 550                 555                 560
   Lys Ser Lys Ser Gln Ser Cys Asn Thr Ala Val Val Val Asn Val Pro
                   565                 570                 575
   Ser Pro Leu Gly Asn Thr Pro His Gly Tyr Lys Tyr Ala Cys Glu Cys
               580                 585                 590
   Lys Ile Pro Thr Thr Glu Glu Thr Cys Asp Asp Arg Lys Glu Tyr Met
           595                 600                 605
   Asn Gln Trp Ile Ile Asp Thr Ser Lys Lys Gln Lys Gly Ser Gly Ser
       610                 615                 620
   Gly Lys Asp Asp Tyr Glu Leu Tyr Thr Tyr Asn Gly Val Asp Val Lys
   625 630 635 640
   Pro Thr Thr Val Arg Ser Asn Ser Thr Lys Leu Asp
   645 650
<210> 44
   <211> 628
   <212> PRT
   <213> Artificial
<220>
   <223> Artificial construct
<400> 44
   Asp Tyr Ile Lys Asp Asp Pro Tyr Ser Ala Gln Tyr Thr Thr Lys Leu
   1 5 10 15
   Ser Phe Ile Leu Asn Pro Ser Asp Ala Asn Thr Ser Ser Glu Lys Ile
   20 25 30
   Gln Lys Asn Asn Asp Glu Ala Cys Asn Cys Asn Glu Ser Gly Ile Ser
   35 40 45
   Ser Val Gly Gln Ala Gln Thr Ser Gly Pro Ser Ser Asn Lys Thr Cys
   50 55 60
   Ile Thr His Ser Ser Ile Lys Ala Asn Lys Lys Lys Val Cys Lys Asp
   65 70 75 80
   Val Lys Leu Gly Ile Asn Asn Asn Asp Lys Val Leu Arg Val Cys Val
   85 90 95
   Ile Glu Asp Thr Ser Leu Ser Gly Val Asp Asn Cys Cys Cys Gln Asp
   100 105 110
   Leu Leu Gly Ile Leu Gln Glu Asn Cys Ser Asp Asn Lys Arg Gly Ser
   115 120 125
   Ser Ser Asn Gly Ser Cys Asn Asn Asn Asn Glu Glu Ala Cys Glu Lys
   130 135 140
   Asn Leu Asp Glu Ala Pro Ala Ser Leu His Asn Gly Tyr Lys Asn Gln
   145 150 155 160
   Lys Cys Lys Ser Gly Thr Ser Arg Ser Lys Lys Lys Trp Ile Trp Lys
   165 170 175
   Lys Ser Ser Gly Asn Glu Lys Gly Leu Gln Glu Glu Tyr Ala Asn Thr
   180 185 190
   Ile Gly Leu Pro Pro Arg Thr Gln Ser Leu Cys Leu Val Cys Leu His
   195 200 205
   Glu Lys Glu Gly Lys Thr Gln His Lys Thr Ile Ser Thr Asn Ser Glu
   210 215 220
   Leu Leu Lys Glu Trp Ile Ile Ala Ala Phe His Glu Gly Lys Asn Leu
   225 230 235 240
   Lys Thr Ser His Glu Lys Lys Asn Asp Asp Asn Gly Lys Lys Leu Cys
   245 250 255
   Lys Ala Leu Glu Tyr Ser Phe Ala Asp Tyr Gly Asp Leu Ile Lys Gly
   260 265 270
   Thr Ser Ile Trp Asp Asn Glu Tyr Thr Lys Asp Leu Glu Leu Asn Leu
   275 280 285
   Gln Lys Ala Phe Gly Lys Leu Phe Arg Lys Tyr Ile Lys Lys Asn Asn
   290 295 300
   Thr Ala Glu Gln Asp Thr Ser Tyr Ser Ser Leu Asp Glu Leu Arg Glu
   305 310 315 320
   Ser Trp Trp Asn Thr Asn Lys Lys Tyr Ile Trp Ile Ala Met Lys His
   325 330 335
   Gly Ala Gly Met Asn Gly Thr Thr Cys Ser Cys Ser Gly Asp Ser Ser
   340 345 350
   Asn Asp Met Pro Thr Ile Asp Leu Ile Pro Gln Tyr Leu Arg Phe Leu
   355 360 365
   Gln Glu Trp Val Glu His Phe Cys Glu Gln Arg Gln Ala Lys Val Lys
   370 375 380
   Asp Val Ile Thr Asn Cys Lys Ser Cys Lys Glu Ser Gly Asn Lys Cys
   385 390 395 400
   Lys Thr Glu Cys Lys Thr Lys Cys Lys Asp Glu Cys Glu Lys Tyr Lys
   405 410 415
   Thr Phe Ile Glu Asp Cys Asn Gly Gly Gly Thr Gly Thr Ala Gly Ser
   420 425 430
   Ser Trp Val Lys Arg Trp Asp Gln Ile Tyr Lys Arg Tyr Ser Lys His
           435                 440                 445
   Ile Glu Asp Ala Lys Arg Asn Arg Lys Ala Gly Thr Lys Asn Cys Gly
       450                 455                 460
   Pro Ser Ser Ile Thr Asn Ala Ala Ala Ser Thr Asp Glu Asn Lys Cys
   465                 470                 475                 480
   Val Gln Ser Asp Ile Asp Ser Phe Phe Lys His Leu Ile Asp Ile Gly
                   485                 490                 495
   Leu Thr Thr Pro Ser Ser Tyr Leu Ser Asn Val Leu Asp Glu Asn Ser
               500                 505                 510
   Cys Gly Asp Asp Lys Ala Pro Trp Thr Thr Tyr Thr Thr Tyr Thr Thr
           515                 520                 525
   Thr Lys Asn Cys Asp Ile Gln Lys Asp Lys Ser Lys Ser Gln Pro Ile
       530                 535                 540
   Asn Thr Ser Val Val Val Asn Val Pro Ser Pro Leu Gly Asn Thr Pro
   545                 550                 555                 560
   Tyr Arg Tyr Lys Tyr Ala Cys Glu Cys Lys Ile Pro Thr Thr Glu Glu
                   565                 570                 575
   Ser Cys Asp Asp Arg Lys Glu Tyr Met Asn Gln Trp Ser Cys Gly Ser
               580                 585                 590
   Ala Arg Thr Met Lys Arg Gly Tyr Lys Asn Asp Asn Tyr Glu Leu Cys
           595                 600                 605
   Lys Tyr Asn Gly Val Asp Val Lys Pro Thr Thr Val Arg Ser Asn Ser
       610                 615                 620
   Ser Lys Leu Asp
   625
<210> 45
   <211> 653
   <212> PRT
   <213> Artificial
<220>
   <223> Artificial construct
<400> 45
   Asp Tyr Ile Lys Gly Asp Pro Tyr Phe Ala Glu Tyr Ala Thr Lys Leu
   1               5                   10                  15
   Ser Phe Ile Leu Asn Pro Ser Asp Thr Glu Asn Ala Ser Glu Thr Pro
               20                  25                  30
   Ser Lys Tyr Tyr Asp Glu Ala Cys Asn Pro Asn Glu Ser Glu Ile Ala
           35                  40                  45
   Ser Val Glu Gln Ala Gln Thr Ser Gly Pro Ser Ser Asn Lys Thr Cys
       50                  55                  60
   Ile Thr His Ser Ser Ile Lys Thr Asn Lys Lys Lys Glu Cys Lys Asp
   65                  70                  75                  80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Phe Lys Asp
               100                 105                 110
   Leu Leu Gly Ile Leu Gln Glu Asn Cys Ser Asp Asn Lys Arg Gly Ser
           115                 120                 125
   Ser Ser Asn Asp Ser Cys Asn Asn Asn Asn Glu Glu Ala Cys Glu Lys
       130                 135                 140
   Asn Leu Asp Glu Ala Leu Ala Ser Leu Thr Asn Gly Tyr Lys Cys Asp
   145                 150                 155                 160
   Lys Cys Lys Ser Gly Thr Ser Thr Val Asn Lys Lys Trp Thr Trp Arg
                   165                 170                 175
   Lys Ser Ser Gly Asn Glu Glu Gly Leu Gln Lys Glu Tyr Ala Asn Thr
               180                 185                 190
   Ile Gly Leu Pro Pro Arg Thr Gln Ser Leu Cys Leu Val Cys Leu His
           195                 200                 205
   Glu Lys Glu Gly Lys Thr Lys His Lys Thr Ile Ser Thr Asn Ser Glu
       210                 215                 220
   Leu Leu Lys Glu Trp Ile Ile Ala Ala Phe His Glu Gly Lys Asn Leu
   225                 230                 235                 240
   Lys Thr Ser His Glu Lys Lys Asn Asp Asp Asn Gly Lys Lys Leu Cys
                   245                 250                 255
   Lys Ala Leu Glu Tyr Ser Phe Ala Asp Tyr Gly Asp Leu Ile Lys Gly
               260                 265                 270
   Thr Ser Ile Trp Asp Asn Glu Tyr Thr Lys Asp Leu Glu Leu Asn Leu
           275                 280                 285
   Gln Lys Ala Phe Gly Lys Leu Phe Arg Lys Tyr Ile Lys Lys Asn Asn
       290                 295                 300
   Thr Ala Glu Gln Asp Thr Ser Tyr Ser Ser Leu Asp Glu Leu Arg Glu
   305                 310                 315                 320
   Ser Trp Trp Asn Thr Asn Lys Lys Tyr Ile Trp Thr Ala Met Lys His
                   325                 330                 335
   Gly Ala Glu Met Asn Gly Thr Thr Cys Ser Ser Gly Ser Gly Asp Asn
               340                 345                 350
   Gly Asp Ser Ser Ile Thr Gly Ser Ser Asp Ser Gly Ser Thr Thr Cys
           355                 360                 365
   Ser Gly Asp Asn Gly Ser Ile Ser Cys Asp Asp Ile Pro Thr Thr Asp
       370                 375                 380
   Phe Ile Pro Gln Tyr Leu Arg Phe Leu Gln Glu Trp Val Glu His Phe
   385                 390                 395                 400
   Cys Glu Gln Arg Gln Ala Lys Val Lys Asp Val Ile Asn Ser Cys Asn
                   405                 410                 415
   Ser Cys Asn Glu Ser Gly Gly Thr Cys Asn Gly Glu Cys Lys Thr Lys
               420                 425                 430
   Cys Lys Asp Glu Cys Glu Lys Tyr Lys Lys Phe Ile Glu Asp Cys Asn
           435                 440                 445
   Gly Gly Asp Gly Thr Ala Gly Ser Ser Trp Val Lys Arg Trp Asp Gln
       450                 455                 460
   Ile Tyr Lys Arg Tyr Ser Lys His Ile Glu Asp Ala Lys Arg Asn Arg
   465                 470                 475                 480
   Lys Ala Gly Thr Lys Asn Cys Gly Pro Ser Ser Ile Thr Asn Ala Ala
                   485                 490                 495
   Ala Ser Thr Asp Glu Asn Lys Cys Val Gln Ser Asp Val Asp Ser Phe
               500                 505                 510
   Phe Lys His Leu Ile Asp Ile Gly Leu Thr Thr Pro Ser Ser Tyr Leu
           515                 520                 525
   Ser Ile Val Leu Asp Glu Asn Ser Cys Gly Asp Asp Lys Ala Pro Trp
       530                 535                 540
   Thr Thr Tyr Thr Thr Tyr Thr Thr Thr Glu Lys Cys Asn Lys Glu Arg
   545                 550                 555                 560
   Asp Lys Ser Lys Ser Gln Ser Ser Asp Thr Leu Val Val Val Asn Val
                   565                 570                 575
   Pro Ser Pro Leu Gly Asn Thr Pro His Glu Tyr Lys Tyr Ala Cys Glu
               580                 585                 590
   Cys Lys Ile Pro Thr Asn Glu Glu Thr Cys Asp Asp Arg Lys Asp Tyr
           595                 600                 605
   Met Asn Gln Trp Ile Ser Asp Thr Ser Lys Lys Gln Lys Gly Ser Gly
       610                 615                 620
   Ser Gly Lys Asp Tyr Tyr Glu Leu Tyr Thr Tyr Asn Gly Val Gln Ile
   625                 630                 635                 640
   Lys Gln Ala Ala Gly Arg Ser Ser Ser Thr Lys Leu Asp
                   645                 650
<210> 46
   <211> 490
   <212> PRT
   <213> Plasmodium falciparum
<400> 46
   Lys Cys Asp Lys Cys Lys Ser Glu Gln Ser Lys Lys Asn Asn Asn Lys
   1               5                   10                  15
   Trp Ile Trp Lys Lys Tyr Ser Gly Asn Gly Glu Gly Leu Gln Lys Glu
               20                  25                  30
   Tyr Ala Asn Thr Ile Gly Leu Pro Pro Arg Thr Gln Ser Leu Cys Leu
           35                  40                  45
   Val Cys Leu His Glu Lys Glu Gly Lys Thr Gln His Lys Thr Ile Ser
       50                  55                  60
   Thr Asn Ser Glu Leu Leu Lys Glu Trp Ile Ile Ala Ala Phe His Glu
   65                  70                  75                  80
   Gly Lys Asn Leu Lys Lys Arg Tyr Pro Gln Asn Lys Asn Asp Asp Asn
                   85                  90                  95
   Asn Ser Lys Leu Cys Lys Ala Leu Glu Tyr Ser Phe Ala Asp Tyr Gly
               100                 105                 110
   Asp Leu Ile Lys Gly Thr Ser Ile Trp Asp Asn Glu Tyr Thr Lys Asp
           115                 120                 125
   Leu Glu Leu Asn Leu Gln Lys Ala Phe Gly Lys Leu Phe Arg Lys Tyr
       130                 135                 140
   Ile Lys Lys Asn Asn Thr Ala Glu Gln Asp Thr Ser Tyr Ser Ser Leu
   145                 150                 155                 160
   Asp Glu Leu Arg Glu Ser Trp Trp Asn Thr Asn Lys Lys Tyr Ile Trp
                   165                 170                 175
   Thr Ala Met Lys His Gly Ala Glu Met Asn Gly Thr Thr Cys Ser Ser
               180                 185                 190
   Gly Ser Gly Asp Asn Gly Asp Ser Ser Cys Asp Asp Ile Pro Thr Ile
           195                 200                 205
   Asp Leu Ile Pro Gln Tyr Leu Arg Phe Leu Gln Glu Trp Val Glu His
       210                 215                 220
   Phe Cys Lys Gln Arg Gln Ala Lys Val Lys Asp Val Ile Asn Ser Cys
   225                 230                 235                 240
   Asn Ser Cys Lys Asn Thr Ser Gly Glu Arg Lys Ile Gly Gly Thr Cys
                   245                 250                 255
   Asn Ser Asp Cys Glu Lys Lys Cys Lys Val Ala Cys Asp Ala Tyr Lys
               260                 265                 270
   Thr Phe Ile Glu Glu Cys Arg Thr Ala Val Gly Gly Thr Ala Gly Ser
           275                 280                 285
   Ser Trp Val Lys Arg Trp Asp Gln Ile Tyr Lys Arg Tyr Ser Lys His
       290                 295                 300
   Ile Glu Asp Ala Lys Arg Asn Arg Lys Ala Gly Thr Lys Asn Cys Gly
   305                 310                 315                 320
   Pro Ser Ser Thr Thr Asn Ala Ala Glu Asn Lys Cys Val Gln Ser Asp
                   325                 330                 335
   Ile Asp Ser Phe Phe Lys His Leu Ile Asp Ile Gly Leu Thr Thr Pro
               340                 345                 350
   Ser Ser Tyr Leu Ser Asn Val Leu Asp Glu Asn Ser Cys Gly Ala Asp
           355                 360                 365
   Lys Ala Pro Trp Thr Thr Tyr Thr Thr Tyr Thr Thr Tyr Thr Thr Tyr
       370                 375                 380
   Thr Thr Tyr Thr Thr Thr Glu Lys Cys Asn Lys Glu Arg Asp Lys Ser
   385                 390                 395                 400
   Lys Ser Gln Gln Ser Asn Thr Ser Val Val Val Asn Val Pro Ser Pro
                   405                 410                 415
   Leu Gly Asn Thr Pro His Glu Tyr Lys Tyr Ala Cys Glu Cys Lys Ile
               420                 425                 430
   Pro Thr Thr Glu Glu Thr Cys Asp Asp Arg Lys Glu Tyr Met Asn Gln
           435                 440                 445
   Trp Ile Ile Asp Asn Thr Lys Asn Pro Lys Gly Ser Gly Ser Thr Asp
       450                 455                 460
   Asn Asp Tyr Glu Leu Tyr Thr Tyr Asn Gly Val Gln Ile Lys Gln Ala
   465                 470                 475                 480
   Ala Gly Arg Ser Ser Ser Thr Lys Leu Asp
                   485                 490
<210> 47
   <211> 335
   <212> PRT
   <213> Plasmodium falciparum
<400> 47
   Lys Cys Glu Lys Cys Lys Ser Gly Thr Ser Thr Val Asn Asn Lys Trp
   1               5                   10                  15
   Ile Trp Arg Lys Ser Ser Gly Lys Glu Gly Gly Leu Gln Lys Glu Tyr
               20                  25                  30
   Ala Asn Thr Ile Gly Leu Pro Pro Arg Thr Gln Ser Leu Tyr Leu Gly
           35                  40                  45
   Asn Leu Pro Lys Leu Glu Asn Val Cys Lys Gly Val Thr Asp Ile Ile
       50                  55                  60
   Tyr Asp Thr Lys Glu Lys Phe Leu Ser Gly Cys Leu Ile Ala Ala Phe
   65                  70                  75                  80
   His Glu Gly Lys Asn Leu Lys Thr Thr Tyr Leu Glu Lys Lys Asn Asp
                   85                  90                  95
   Asp Asn Gly Lys Lys Leu Cys Lys Ala Leu Glu Tyr Ser Phe Ala Asp
               100                 105                 110
   Tyr Gly Asp Leu Ile Lys Gly Thr Ser Ile Trp Asp Asn Glu Tyr Thr
           115                 120                 125
   Lys Asp Leu Glu Leu Asn Leu Gln Lys Ile Phe Gly Lys Leu Phe Arg
       130                 135                 140
   Lys Tyr Ile Lys Lys Asn Asn Thr Ala Glu Gln Asp Thr Ser Tyr Ser
   145                 150                 155                 160
   Ser Leu Asp Glu Leu Arg Glu Ser Trp Trp Asn Thr Asn Lys Lys Tyr
                   165                 170                 175
   Ile Trp Ile Ala Met Lys His Gly Ala Gly Met Asn Gly Thr Thr Cys
               180                 185                 190
   Ser Ser Gly Ser Gly Asp Ser Ser Asn Asp Ile Pro Thr Thr Asp Phe
           195                 200                 205
   Ile Pro Gln Tyr Leu Arg Phe Leu Gln Glu Trp Val Glu Asn Phe Cys
       210                 215                 220
   Glu Gln Arg Gln Ala Lys Val Lys Pro Val Ile Glu Asn Cys Asn Ser
   225                 230                 235                 240
   Cys Lys Glu Ser Gly Gly Thr Cys Asn Gly Glu Cys Lys Thr Lys Cys
                   245                 250                 255
   Lys Val Ala Cys Asp Ala Tyr Lys Lys Phe Ile Asp Gly Thr Gly Ser
               260                 265                 270
   Gly Gly Gly Ser Arg Pro Thr Gly Ile Ala Gly Ser Ser Trp Ser Lys
           275                 280                 285
   Arg Trp Asp Gln Ile Tyr Lys Arg Tyr Ser Lys His Ile Glu Asp Ala
       290                 295                 300
   Lys Arg Asn Arg Lys Ala Gly Thr Lys Asn Cys Gly Pro Ser Ser Ile
   305 310 315 320
   Thr Asn Val Ser Val Ser Thr Asp Glu Asn Lys Cys Val Gln Ser
   325 330 335
<210> 48
   <211> 637
   <212> PRT
   <213> Artificial
<220>
   <223> Artificial construct
<400> 48
   Asn Tyr Ile Lys Asp Asp Pro Tyr Ser Lys Glu Tyr Val Thr Lys Leu
   1               5                   10                  15
   Ser Phe Ile Pro Asn Ser Ser Asp Ala Asn Thr Ser Ser Glu Lys Ile
               20                  25                  30
   Gln Lys Asn Asn Asp Glu Val Cys Asn Pro Asn Glu Ser Gly Ile Ser
           35                  40                  45
   Ser Val Glu Gln Ala Gln Thr Ser Asp Pro Ser Ser Asn Lys Thr Cys
       50                  55                  60
   Ile Thr His Ser Ser Ile Lys Ala Asn Lys Lys Lys Glu Cys Lys Asp
   65                  70                  75                  80
   Val Lys Leu Gly Val Arg Glu Asn Asp Lys Asp Leu Lys Ile Cys Val
                   85                  90                  95
   Ile Glu His Thr Ser Leu Ser Gly Val Asp Asn Cys Cys Phe Lys Asp
               100                 105                 110
   Phe Leu Arg Met Leu Gln Glu Pro Arg Ile Asp Lys Asn Gln Arg Gly
           115                 120                 125
   Ser Ser Ser Asn Gly Ser Cys Asp Lys Asn Ser Glu Glu Ala Cys Glu
       130                 135                 140
   Lys Asn Leu Asp Glu Ala Leu Ala Ser Leu Thr Asn Gly Tyr Lys Cys
   145                 150                 155                 160
   Asp Lys Cys Lys Ser Glu Gln Ser Lys Lys Asn Asn Asn Lys Trp Ile
                   165                 170                 175
   Trp Lys Lys Phe Pro Gly Lys Glu Gly Gly Leu Gln Glu Glu Tyr Ala
   180 185 190
   Asn Thr Ile Gly Leu Pro Pro Arg Thr Gln Tyr Leu Cys Leu Val Val
   195 200 205
   Cys Leu Asp Glu Lys Glu Gly Lys Thr Gln Glu Leu Lys Asn Ile Arg
   210 215 220
   Thr Asn Ser Glu Leu Leu Lys Glu Trp Ile Ile Ala Ala Phe His Glu
   225 230 235 240
   Gly Lys Asn Leu Lys Thr Thr Tyr Pro Gln Lys Lys Asn Asp Asp Asn
   245 250 255
   Gly Lys Lys Leu Cys Lys Asp Leu Lys Tyr Ser Phe Ala Asp Tyr Gly
   260 265 270
   Asp Leu Ile Lys Gly Thr Ser Ile Trp Asp Asn Glu Tyr Thr Lys Asn
   275 280 285
   Val Glu Leu Asn Leu Gln Asn Asn Phe Gly Lys Leu Phe Arg Lys Tyr
   290 295 300
   Ile Lys Lys Asn Asn Thr Ala Glu Gln Asp Thr Ser Tyr Ser Ser Leu
   305 310 315 320
   Asp Glu Leu Arg Glu Ser Trp Trp Asn Thr Asn Lys Lys Tyr Ile Trp
   325 330 335
   Leu Ala Met Lys His Gly Ala Glu Met Asn Ser Thr Thr Cys Cys Gly
   340 345 350
   Asp Gly Ser Val Thr Gly Ser Gly Ser Ser Cys Asp Asp Ile Pro Thr
   355 360 365
   Ile Asp Leu Ile Pro Gln Tyr Leu Arg Phe Leu Gln Glu Trp Val Glu
   370 375 380
   His Phe Cys Lys Gln Arg Gln Ala Lys Val Lys Asp Val Ile Thr Asn
   385 390 395 400
   Cys Asn Ser Cys Lys Glu Ser Gly Asn Lys Cys Lys Thr Glu Cys Lys
   405 410 415
   Asn Lys Cys Lys Asp Glu Cys Glu Lys Tyr Lys Lys Phe Ile Glu Ala
   420 425 430
   Cys Gly Thr Ala Val Gly Gly Thr Gly Thr Ala Gly Ser Pro Trp Ser
   435 440 445
   Lys Arg Trp Asp Gln Ile Tyr Lys Arg Tyr Ser Lys His Ile Glu Asp
   450 455 460
   Ala Lys Arg Asn Arg Lys Ala Gly Thr Lys Asn Cys Gly Pro Ser Ser
   465 470 475 480
   Thr Thr Asn Ala Ala Glu Asn Lys Cys Val Gln Ser Asp Ile Asp Ser
   485 490 495
   Phe Phe Lys His Leu Ile Asp Ile Gly Leu Thr Thr Pro Ser Ser Tyr
   500 505 510
   Leu Ser Ile Val Leu Asp Asp Asn Ile Cys Gly Ala Asp Lys Ala Pro
   515 520 525
   Trp Thr Thr Tyr Thr Thr Tyr Thr Thr Glu Asn Cys Asp Ile Gln Lys
   530 535 540
   Lys Thr Pro Lys Ser Gln Ser Cys Asp Thr Leu Val Val Val Asn Val
   545 550 555 560
   Pro Ser Pro Leu Gly Asn Thr Pro His Gly Tyr Lys Tyr Ala Cys Gln
   565 570 575
   Cys Arg Thr Pro Asn Lys Gln Glu Ser Cys Asp Asp Arg Lys Glu Tyr
   580 585 590
   Met Asn Gln Trp Ile Ile Asp Asn Thr Lys Asn Pro Lys Gly Ser Gly
   595 600 605
   Ser Gly Lys Asp Tyr Tyr Glu Leu Cys Lys Tyr Asn Gly Val Lys Glu
   610 615 620
   Thr Lys Pro Leu Gly Thr Leu Lys Asn Ser Lys Leu Asp
   625 630 635
<210> 49
   <211> 330
   <212> PRT
   <213> Plasmodium falciparum
<400> 49
   Lys Cys Asp Lys Cys Lys Ser Glu Gln Ser Lys Lys Asn Asn Asn Lys
   1 5 10 15
   Trp Ile Trp Arg Lys Phe Pro Gly Lys Glu Gly Gly Leu Gln Lys Glu
   20 25 30
   Tyr Ala Asn Thr Ile Gly Leu Pro Pro Arg Thr Gln Ser Leu Cys Leu
   35 40 45
   Val Cys Leu His Glu Lys Glu Gly Lys Thr Gln His Lys Thr Ile Ser
   50 55 60
   Thr Asn Ser Glu Leu Leu Lys Glu Trp Ile Ile Ala Ala Phe His Glu
   65 70 75 80
   Gly Lys Asn Leu Lys Thr Thr Tyr Leu Glu Lys Lys Asn Ala Glu Asn
   85 90 95
   Lys Lys Lys Leu Cys Lys Ala Leu Lys Tyr Ser Phe Ala Asp Tyr Gly
   100 105 110
   Asp Leu Ile Lys Gly Thr Ser Ile Trp Asp Asn Glu Tyr Thr Lys Asp
   115 120 125
   Leu Glu Leu Asn Leu Gln Lys Ile Phe Gly Lys Leu Phe Arg Lys Tyr
   130 135 140
   Ile Lys Lys Asn Asn Thr Ala Glu Gln Asp Thr Ser Tyr Ser Ser Leu
   145 150 155 160
   Asp Glu Leu Arg Glu Ser Trp Trp Asn Thr Asn Lys Lys Tyr Ile Trp
   165 170 175
   Thr Ala Met Lys His Gly Ala Gly Met Asn Gly Thr Met Cys Asn Ala
   180 185 190
   Asp Gly Ser Val Thr Gly Ser Gly Ser Ser Cys Asp Asp Met Pro Thr
   195 200 205
   Thr Asp Phe Ile Pro Gln Tyr Leu Arg Phe Leu Gln Glu Trp Val Glu
   210 215 220
   His Phe Cys Lys Gln Arg Gln Ala Lys Val Lys Asp Val Ile Glu Asn
   225 230 235 240
   Cys Lys Ser Cys Lys Glu Ser Gly Asn Lys Cys Lys Thr Glu Cys Lys
   245 250 255
   Asn Lys Cys Asp Ala Tyr Lys Thr Phe Ile Glu Glu Cys Gly Thr Ala
   260 265 270
   Val Gly Gly Thr Ala Gly Ser Ser Trp Val Lys Arg Trp Asp Gln Ile
           275                 280                 285
   Tyr Lys Arg Tyr Ser Lys His Ile Glu Asp Ala Lys Arg Asn Arg Lys
       290                 295                 300
   Ala Gly Thr Lys Asn Cys Gly Thr Ser Ser Thr Thr Asn Ala Ala Ala
   305                 310                 315                 320
   Ser Thr Ala Glu Asn Lys Cys Val Gln Ser
                   325                 330
<210> 50
   <211> 269
   <212> PRT
   <213> Plasmodium falciparum
<400> 50
   Asn Tyr Ile Lys Asp Asp Pro Tyr Ser Lys Glu Tyr Val Thr Lys Leu
   1               5                   10                  15
   Ser Phe Ile Leu Asn Ser Ser Asp Ala Glu Asn Ala Ser Glu Thr Pro
               20                  25                  30
   Ser Lys Tyr Tyr Asp Glu Ala Cys Asn Cys Asn Glu Ser Gly Ile Ser
           35                  40                  45
   Ser Val Glu Gln Ala Ser Ile Ser Asp Arg Ser Ser Gln Lys Ala Cys
       50                  55                  60
   Asn Thr His Ser Phe Ile Gly Ala Asn Lys Lys Lys Val Cys Lys His
   65                  70                  75                  80
   Val Lys Leu Gly Val Arg Glu Asn Asp Lys Asp Leu Lys Ile Cys Val
                   85                  90                  95
   Ile Glu Asp Asp Ser Leu Arg Gly Val Glu Asn Cys Cys Phe Lys Asp
               100                 105                 110
   Phe Leu Arg Met Leu Gln Glu Pro Arg Ile Asp Lys Asn Gln Arg Gly
           115                 120                 125
   Ser Ser Ser Asn Asp Ser Cys Asn Asn Asn Asn Glu Glu Ala Cys Glu
       130                 135                 140
   Lys Asn Leu Asp Glu Ala Leu Ala Ser Leu His Asn Gly Tyr Lys Asn
   145                 150                 155                 160
   Gln Lys Cys Lys Ser Glu Gln Ser Lys Lys Asn Asn Asn Lys Trp Ile
   165 170 175
   Trp Lys Lys Ser Ser Gly Lys Glu Gly Gly Leu Gln Lys Glu Tyr Ala
   180 185 190
   Asn Thr Ile Gly Leu Pro Pro Arg Thr Gln Ser Leu Cys Leu Val Cys
   195 200 205
   Leu His Glu Lys Glu Gly Lys Thr Gln His Lys Thr Ile Ser Thr Asn
   210 215 220
   Ser Glu Leu Leu Lys Glu Trp Ile Ile Asp Ala Phe His Glu Gly Lys
   225 230 235 240
   Asn Leu Lys Thr Thr Tyr Leu Glu Lys Lys Lys Gly Asp Asn Gly Lys
   245 250 255
   Lys Leu Cys Lys Ala Leu Lys Tyr Ser Phe Ala Asp Tyr
   260 265
<210> 51
   <211> 347
   <212> PRT
   <213> Plasmodium falciparum
<400> 51
   Lys Cys Asp Lys Cys Lys Ser Glu Gln Ser Lys Lys Asn Asn Lys Asn
   1 5 10 15
   Trp Ile Trp Lys Lys Ser Ser Gly Lys Glu Gly Gly Leu Gln Lys Glu
   20 25 30
   Tyr Ala Asn Thr Ile Ala Leu Pro Pro Arg Thr Gln Ser Leu Cys Leu
   35 40 45
   Val Val Cys Leu His Glu Lys Glu Gly Lys Thr Gln His Lys Thr Ile
   50 55 60
   Ser Thr Asn Ser Glu Leu Leu Lys Glu Trp Ile Ile Asp Ala Phe His
   65 70 75 80
   Glu Gly Lys Asn Leu Lys Thr Thr Tyr Leu Glu Lys Gln Asn Ala Asp
   85 90 95
   Asn Gly Lys Lys Asn Ala Asp Asn Asn Ser Lys Leu Cys Lys Asp Leu
   100 105 110
   Lys Tyr Ser Phe Ala Asp Tyr Gly Asp Leu Ile Lys Gly Thr Ser Ile
   115 120 125
   Trp Asp Asn Glu Tyr Thr Lys Asp Leu Glu Leu Asn Leu Gln Gln Ile
   130 135 140
   Phe Gly Lys Leu Phe Arg Lys Tyr Ile Lys Lys Asn Ile Ala Ser Asp
   145 150 155 160
   Glu Asn Thr Leu Tyr Ser Ser Leu Asp Glu Leu Arg Glu Ser Trp Trp
   165 170 175
   Asn Thr Asn Lys Lys Tyr Ile Trp Thr Ala Met Lys His Gly Ala Glu
   180 185 190
   Met Asn Gly Thr Thr Cys Ser Ser Gly Ser Gly Asp Ser Ser Ser Gly
   195 200 205
   Glu Asn Gln Thr Asn Ser Cys Asp Asp Ile Pro Thr Ile Asp Leu Ile
   210 215 220
   Pro Gln Tyr Leu Arg Phe Leu Gln Glu Trp Val Glu His Phe Cys Glu
   225 230 235 240
   Gln Arg Gln Ala Lys Val Lys Asp Val Ile Thr Asn Cys Lys Ser Cys
   245 250 255
   Lys Glu Ser Gly Gly Thr Cys Asn Ser Asp Cys Lys Thr Lys Cys Lys
   260 265 270
   Gly Glu Cys Glu Lys Tyr Lys Lys Phe Ile Glu Lys Cys Lys Gly Gly
   275 280 285
   Gly Thr Glu Gly Thr Ser Gly Ser Ser Trp Val Lys Arg Trp Tyr Gln
   290 295 300
   Ile Tyr Met Arg Tyr Ser Lys Tyr Ile Glu Asp Ala Lys Arg Asn Arg
   305 310 315 320
   Lys Ala Gly Thr Lys Ser Cys Gly Thr Ser Ser Gly Ala Asn Ser Gly
   325 330 335
   Val Thr Thr Thr Glu Ser Lys Cys Val Gln Ser
   340 345
<210> 52
   <211> 269
   <212> PRT
   <213> Plasmodium falciparum
<400> 52
   Asp Tyr Ile Lys Asp Asp Pro Tyr Ser Lys Glu Tyr Thr Thr Lys Leu
   1               5                   10                  15
   Ser Phe Ile Leu Asn Ser Ser Asp Ala Asn Thr Ser Ser Glu Lys Ile
               20                  25                  30
   Gln Lys Asn Asn Asp Glu Val Cys Asn Pro Asn Glu Ser Glu Ile Ser
           35                  40                  45
   Ser Val Glu Gln Ala Gln Thr Ser Arg Pro Ser Ser Asn Lys Thr Cys
       50                  55                  60
   Ile Thr His Ser Ser Ile Lys Ala Asn Lys Lys Lys Val Cys Lys Asp
   65                  70                  75                  80
   Val Lys Leu Gly Val Arg Glu Asn Asp Lys Val Leu Arg Val Cys Val
                   85                  90                  95
   Ile Glu His Thr Ser Leu Ser Gly Val Glu Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Gly Ser Cys Asp Lys Asn Ser Glu Glu Ala Cys Glu Lys
       130                 135                 140
   Asn Leu Asp Glu Ala Leu Ala Ser Leu Thr Asn Cys Tyr Lys Asn Gln
   145                 150                 155                 160
   Lys Cys Lys Ser Glu Gln Ser Lys Lys Asn Asn Asn Lys Trp Ile Trp
                   165                 170                 175
   Lys Lys Ser Ser Gly Asn Glu Lys Gly Leu Gln Lys Glu Tyr Ala Asn
               180                 185                 190
   Thr Ile Gly Leu Pro Pro Arg Thr Gln Ser Leu Cys Leu Val Cys Leu
           195                 200                 205
   His Glu Lys Glu Gly Lys Thr Gln Glu Leu Lys Asn Ile Ser Thr Asn
       210                 215                 220
   Ser Glu Leu Leu Lys Glu Trp Ile Ile Ala Ala Phe His Glu Gly Lys
   225                 230                 235                 240
   Asn Leu Lys Thr Thr Tyr Pro Gln Asn Lys Asn Asp Asp Asn Gly Lys
   245 250 255
   Lys Leu Phe Lys Asp Leu Lys Tyr Ser Phe Ala Asp Tyr
   260 265
<210> 53
   <211> 646
   <212> PRT
   <213> Artificial
<220>
   <223> Artificial construct
<400> 53
   Asp Tyr Ile Lys Asp Asp Pro Tyr Ser Lys Glu Tyr Thr Thr Lys Leu
   1 5 10 15
   Ser Phe Ile Leu Asn Ser Ser Asp Ala Asn Thr Ser Ser Glu Lys Ile
   20 25 30
   Gln Lys Asn Asn Asp Glu Val Cys Asn Pro Asn Glu Ser Glu Ile Ser
   35 40 45
   Ser Val Glu Gln Ala Gln Thr Ser Arg Pro Ser Ser Asn Lys Thr Cys
   50 55 60
   Ile Thr His Ser Ser Ile Lys Ala Asn Lys Lys Lys Val Cys Lys Asp
   65 70 75 80
   Val Lys Leu Gly Val Arg Glu Asn Asp Lys Val Leu Arg Val Cys Val
   85 90 95
   Ile Glu His Thr Ser Leu Ser Gly Val Glu Asn Cys Cys Cys Gln Asp
   100 105 110
   Leu Leu Gly Ile Leu Gln Glu Asn Cys Ser Asp Asn Lys Arg Gly Ser
   115 120 125
   Ser Ser Asn Gly Ser Cys Asp Lys Asn Ser Glu Glu Ala Cys Glu Lys
   130 135 140
   Asn Leu Asp Glu Ala Leu Ala Ser Leu Thr Asn Cys Tyr Lys Asn Gln
   145 150 155 160
   Lys Cys Lys Ser Glu Gln Ser Lys Lys Asn Asn Asn Lys Trp Ile Trp
   165 170 175
   Lys Lys Ser Ser Gly Lys Glu Gly Gly Leu Gln Lys Glu Tyr Ala Asn
               180                 185                 190
   Thr Ile Gly Leu Pro Pro Arg Thr Gln Ser Leu Tyr Leu Gly Asn Leu
           195                 200                 205
   Pro Lys Leu Glu Asn Val Cys Lys Gly Val Thr Asp Ile Asn Phe Asp
       210                 215                 220
   Thr Lys Glu Lys Phe Leu Ala Gly Cys Leu Ile Ala Ala Phe His Glu
   225                 230                 235                 240
   Gly Lys Asn Leu Lys Thr Thr Tyr Leu Glu Lys Lys Asn Asp Asp Asn
                   245                 250                 255
   Gly Lys Lys Leu Cys Lys Ala Leu Glu Tyr Ser Phe Ala Asp Tyr Gly
               260                 265                 270
   Asp Leu Ile Lys Gly Thr Ser Ile Trp Asp Asn Glu Tyr Thr Lys Asp
           275                 280                 285
   Leu Glu Leu Asn Leu Gln Lys Ala Phe Gly Lys Leu Phe Arg Lys Tyr
       290                 295                 300
   Ile Lys Lys Asn Asn Thr Ala Glu Gln Asp Thr Ser Tyr Ser Ser Leu
   305                 310                 315                 320
   Asp Glu Leu Arg Glu Ser Trp Trp Asn Thr Asn Lys Lys Tyr Ile Trp
                   325                 330                 335
   Thr Ala Met Lys His Gly Ala Gly Met Asn Gly Thr Thr Cys Ser Ser
               340                 345                 350
   Gly Ser Gly Asp Ser Ser Asn Asp Ile Pro Thr Thr Asp Phe Ile Pro
           355                 360                 365
   Gln Tyr Leu Arg Phe Leu Gln Glu Trp Val Glu Asn Phe Cys Glu Gln
       370                 375                 380
   Arg Gln Ala Lys Val Lys Asp Val Ile Glu Asn Cys Asn Ser Cys Lys
   385                 390                 395                 400
   Asn Thr Ser Gly Glu Arg Lys Ile Gly Asp Thr Cys Asn Ser Asp Cys
                   405                 410                 415
   Glu Lys Lys Cys Lys Asp Glu Cys Glu Lys Tyr Lys Lys Phe Ile Glu
               420                 425                 430
   Asp Cys Lys Gly Gly Asp Gly Thr Ala Gly Ser Ser Trp Val Lys Arg
           435                 440                 445
   Trp Asp Gln Ile Tyr Lys Arg Tyr Ser Lys His Ile Glu Asp Ala Lys
       450                 455                 460
   Arg Asn Arg Lys Ala Gly Thr Lys Asn Cys Gly Ile Thr Thr Gly Thr
   465                 470                 475                 480
   Ile Ser Gly Glu Ser Ser Gly Ala Thr Ser Gly Val Thr Thr Thr Glu
                   485                 490                 495
   Asn Lys Cys Val Gln Ser Asp Ile Asp Ser Phe Phe Lys His Leu Ile
               500                 505                 510
   Asp Ile Gly Leu Thr Thr Pro Ser Ser Tyr Leu Ser Asn Val Leu Asp
           515                 520                 525
   Asp Asn Ile Cys Gly Glu Asp Asn Ala Pro Trp Thr Thr Tyr Thr Thr
       530                 535                 540
   Tyr Thr Thr Glu Lys Cys Asn Lys Glu Thr Asp Lys Ser Lys Ser Gln
   545                 550                 555                 560
   Gln Ser Asn Thr Ala Val Val Val Asn Val Pro Ser Pro Leu Gly Asn
                   565                 570                 575
   Thr Pro His Gly Tyr Lys Tyr Ala Cys Glu Cys Lys Ile Pro Thr Thr
               580                 585                 590
   Glu Glu Thr Cys Asp Asp Arg Lys Glu Tyr Met Asn Gln Trp Ser Cys
           595                 600                 605
   Gly Ser Ala Gln Thr Val Arg Asp Arg Ser Gly Lys Asp Asp Tyr Glu
       610                 615                 620
   Leu Cys Lys Tyr Asn Gly Val Gln Ile Lys Gln Ala Ala Gly Thr Leu
   625                 630                 635                 640
   Lys Asn Ser Lys Leu Asp
                   645
<210> 54
   <211> 632
   <212> PRT
   <213> Plasmodium falciparum
<210> 55
   <211> 2730
   <212> PRT
   <213> Plasmodium falciparum
<400> 55
   Met Asp Lys Ser Ser Ile Ala Asn Lys Ile Glu Ala Tyr Leu Gly Ala
   1 5 10 15
   Lys Ser Asp Asp Ser Lys Ile Asp Gln Ser Leu Lys Ala Asp Pro Ser
   20 25 30
   Glu Val Gln Tyr Tyr Gly Ser Gly Gly Asp Gly Tyr Tyr Leu Arg Lys
   35 40 45
   Asn Ile Cys Lys Ile Thr Val Asn His Ser Asp Ser Gly Thr Asn Asp
   50 55 60
   Pro Cys Asp Arg Ile Pro Pro Pro Tyr Gly Asp Asn Asp Gln Trp Lys
   65 70 75 80
   Cys Ala Ile Ile Leu Ser Lys Val Ser Glu Lys Pro Glu Asn Val Phe
                   85                  90                  95
   Val Pro Pro Arg Arg Gln Arg Met Cys Ile Asn Asn Leu Glu Lys Leu
               100                 105                 110
   Asn Val Asp Lys Ile Arg Asp Lys His Ala Phe Leu Ala Asp Val Leu
           115                 120                 125
   Leu Thr Ala Arg Asn Glu Gly Glu Arg Ile Val Gln Asn His Pro Asp
       130                 135                 140
   Thr Asn Ser Ser Asn Val Cys Asn Ala Leu Glu Arg Ser Phe Ala Asp
   145                 150                 155                 160
   Ile Ala Asp Ile Ile Arg Gly Thr Asp Leu Trp Lys Gly Thr Asn Ser
                   165                 170                 175
   Asn Leu Glu Gln Asn Leu Lys Gln Met Phe Ala Lys Ile Arg Glu Asn
               180                 185                 190
   Asp Lys Val Leu Gln Asp Lys Tyr Pro Lys Asp Gln Asn Tyr Arg Lys
           195                 200                 205
   Leu Arg Glu Asp Trp Trp Asn Ala Asn Arg Gln Lys Val Trp Glu Val
       210                 215                 220
   Ile Thr Cys Gly Ala Arg Ser Asn Asp Leu Leu Ile Lys Arg Gly Trp
   225                 230                 235                 240
   Arg Thr Ser Gly Lys Ser Asn Gly Asp Asn Lys Leu Glu Leu Cys Arg
                   245                 250                 255
   Lys Cys Gly His Tyr Glu Glu Lys Val Pro Thr Lys Leu Asp Tyr Val
               260                 265                 270
   Pro Gln Phe Leu Arg Trp Leu Thr Glu Trp Ile Glu Asp Phe Tyr Arg
           275                 280                 285
   Glu Lys Gln Asn Leu Ile Asp Asp Met Glu Arg His Arg Glu Glu Cys
       290                 295                 300
   Thr Ser Glu Asp His Lys Ser Lys Glu Gly Thr Ser Tyr Cys Ser Thr
   305                 310                 315                 320
   Cys Lys Asp Lys Cys Lys Lys Tyr Cys Glu Cys Val Lys Lys Trp Lys
                   325                 330                 335
   Ser Glu Trp Glu Asn Gln Lys Asn Lys Tyr Thr Glu Leu Tyr Gln Gln
   340 345 350
   Asn Lys Asn Glu Thr Ser Gln Lys Asn Thr Ser Arg Tyr Asp Asp Tyr
   355 360 365
   Val Lys Asp Phe Phe Lys Lys Leu Glu Ala Asn Tyr Ser Ser Leu Glu
   370 375 380
   Asn Tyr Ile Lys Gly Asp Pro Tyr Phe Ala Glu Tyr Ala Thr Lys Leu
   385 390 395 400
   Ser Phe Ile Leu Asn Ser Ser Asp Ala Asn Asn Pro Ser Glu Lys Ile
   405 410 415
   Gln Lys Asn Asn Asp Glu Val Cys Asn Cys Asn Glu Ser Gly Ile Ala
   420 425 430
   Ser Val Glu Gln Glu Gln Ile Ser Asp Pro Ser Ser Asn Lys Thr Cys
   435 440 445
   Ile Thr His Ser Ser Ile Lys Ala Asn Lys Lys Lys Val Cys Lys His
   450 455 460
   Val Lys Leu Gly Val Arg Glu Asn Asp Lys Asp Leu Arg Val Cys Val
   465 470 475 480
   Ile Glu His Thr Ser Leu Ser Gly Val Glu Asn Cys Cys Cys Gln Asp
   485 490 495
   Phe Leu Arg Ile Leu Gln Glu Asn Cys Ser Asp Asn Lys Ser Gly Ser
   500 505 510
   Ser Ser Asn Gly Ser Cys Asn Asn Lys Asn Gln Glu Ala Cys Glu Lys
   515 520 525
   Asn Leu Glu Lys Val Leu Ala Ser Leu Thr Asn Cys Tyr Lys Cys Asp
   530 535 540
   Lys Cys Lys Ser Glu Gln Ser Lys Lys Asn Asn Lys Asn Trp Ile Trp
   545 550 555 560
   Lys Lys Ser Ser Gly Lys Glu Gly Gly Leu Gln Lys Glu Tyr Ala Asn
   565 570 575
   Thr Ile Gly Leu Pro Pro Arg Thr Gln Ser Leu Cys Leu Val Val Cys
   580 585 590
   Leu Asp Glu Lys Gly Lys Lys Thr Gln Glu Leu Lys Asn Ile Arg Thr
   595 600 605
   Asn Ser Glu Leu Leu Lys Glu Trp Ile Ile Ala Ala Phe His Glu Gly
   610 615 620
   Lys Asn Leu Lys Pro Ser His Glu Lys Lys Asn Asp Asp Asn Gly Lys
   625 630 635 640
   Lys Leu Cys Lys Ala Leu Glu Tyr Ser Phe Ala Asp Tyr Gly Asp Leu
   645 650 655
   Ile Lys Gly Thr Ser Ile Trp Asp Asn Glu Tyr Thr Lys Asp Leu Glu
   660 665 670
   Leu Asn Leu Gln Lys Ile Phe Gly Lys Leu Phe Arg Lys Tyr Ile Lys
   675 680 685
   Lys Asn Asn Thr Ala Glu Gln Asp Thr Ser Tyr Ser Ser Leu Asp Glu
   690 695 700
   Leu Arg Glu Ser Trp Trp Asn Thr Asn Lys Lys Tyr Ile Trp Leu Ala
   705 710 715 720
   Met Lys His Gly Ala Gly Met Asn Ser Thr Thr Cys Cys Gly Asp Gly
   725 730 735
   Ser Val Thr Gly Ser Gly Ser Ser Cys Asp Asp Ile Pro Thr Ile Asp
   740 745 750
   Leu Ile Pro Gln Tyr Leu Arg Phe Leu Gln Glu Trp Val Glu His Phe
   755 760 765
   Cys Lys Gln Arg Gln Glu Lys Val Lys Pro Val Ile Glu Asn Cys Lys
   770 775 780
   Ser Cys Lys Glu Ser Gly Gly Thr Cys Asn Gly Glu Cys Lys Thr Glu
   785 790 795 800
   Cys Lys Asn Lys Cys Glu Val Tyr Lys Lys Phe Ile Glu Asp Cys Lys
   805 810 815
   Gly Gly Asp Gly Thr Ala Gly Ser Ser Trp Val Lys Arg Trp Asp Gln
   820 825 830
   Ile Tyr Lys Arg Tyr Ser Lys Tyr Ile Glu Asp Ala Lys Arg Asn Arg
   835 840 845
   Lys Ala Gly Thr Lys Asn Cys Gly Pro Ser Ser Thr Thr Asn Ala Ala
   850 855 860
   Glu Asn Lys Cys Val Gln Ser Asp Ile Asp Ser Phe Phe Lys His Leu
   865 870 875 880
   Ile Asp Ile Gly Leu Thr Thr Pro Ser Ser Tyr Leu Ser Ile Val Leu
   885 890 895
   Asp Asp Asn Ile Cys Gly Ala Asp Lys Ala Pro Trp Thr Thr Tyr Thr
   900 905 910
   Thr Tyr Thr Thr Thr Glu Lys Cys Asn Lys Glu Thr Asp Lys Ser Lys
   915 920 925
   Leu Gln Gln Cys Asn Thr Ala Val Val Val Asn Val Pro Ser Pro Leu
   930 935 940
   Gly Asn Thr Pro His Gly Tyr Lys Tyr Ala Cys Gln Cys Lys Ile Pro
   945 950 955 960
   Thr Asn Glu Glu Thr Cys Asp Asp Arg Lys Glu Tyr Met Asn Gln Trp
   965 970 975
   Ser Cys Gly Ser Ala Arg Thr Met Lys Arg Gly Tyr Lys Asn Asp Asn
   980 985 990
   Tyr Glu Leu Cys Lys Tyr Asn Gly Val Asp Val Lys Pro Thr Thr Val
   995 1000 1005
   Arg Ser Asn Ser Ser Lys Leu Asp Asp Lys Asp Val Thr Phe Phe
   1010 1015 1020
   Asn Leu Phe Glu Gln Trp Asn Lys Glu Ile Gln Tyr Gln Ile Glu
   1025 1030 1035
   Gln Tyr Met Thr Asn Thr Lys Ile Ser Cys Asn Asn Glu Lys Asn
   1040 1045 1050
   Val Leu Ser Arg Val Ser Asp Glu Ala Ala Gln Pro Lys Phe Ser
   1055 1060 1065
   Asp Asn Glu Arg Asp Arg Asn Ser Ile Thr His Glu Asp Lys Asn
   1070 1075 1080
   Cys Lys Glu Lys Cys Lys Cys Tyr Ser Leu Trp Ile Glu Lys Ile
   1085 1090 1095
   Asn Asp Gln Trp Asp Lys Gln Lys Asp Asn Tyr Asn Lys Phe Gln
   1100 1105 1110
   Arg Lys Gln Ile Tyr Asp Ala Asn Lys Gly Ser Gln Asn Lys Lys
   1115 1120 1125
   Val Val Ser Leu Ser Asn Phe Leu Phe Phe Ser Cys Trp Glu Glu
   1130 1135 1140
   Tyr Ile Gln Lys Tyr Phe Asn Gly Asp Trp Ser Lys Ile Lys Asn
   1145 1150 1155
   Ile Gly Ser Asp Thr Phe Glu Phe Leu Ile Lys Lys Cys Gly Asn
   1160 1165 1170
   Asp Ser Gly Asp Gly Glu Thr Ile Phe Ser Glu Lys Leu Asn Asn
   1175 1180 1185
   Ala Glu Lys Lys Cys Lys Glu Asn Glu Ser Thr Asn Asn Lys Met
   1190 1195 1200
   Lys Ser Ser Glu Thr Ser Cys Asp Cys Ser Glu Pro Ile Tyr Ile
   1205 1210 1215
   Arg Gly Cys Gln Pro Lys Ile Tyr Asp Gly Lys Ile Phe Pro Gly
   1220 1225 1230
   Lys Gly Gly Glu Lys Gln Trp Ile Cys Lys Asp Thr Ile Ile His
   1235 1240 1245
   Gly Asp Thr Asn Gly Ala Cys Ile Pro Pro Arg Thr Gln Asn Leu
   1250 1255 1260
   Cys Val Gly Glu Leu Trp Asp Lys Arg Tyr Gly Gly Arg Ser Asn
   1265 1270 1275
   Ile Lys Asn Asp Thr Lys Glu Ser Leu Lys Gln Lys Ile Lys Asn
   1280 1285 1290
   Ala Ile Gln Lys Glu Thr Glu Leu Leu Tyr Glu Tyr His Asp Lys
   1295 1300 1305
   Gly Thr Ala Ile Ile Ser Arg Asn Pro Met Lys Gly Gln Lys Glu
   1310 1315 1320
   Lys Glu  Glu Lys Asn Asn Asp  Ser Asn Gly Leu Pro  Lys Gly Phe
       1325                 1330                 1335
   Cys His  Ala Val Gln Arg Ser  Phe Ile Asp Tyr Lys  Asn Met Ile
       1340                 1345                 1350
   Leu Gly  Thr Ser Val Asn Ile  Tyr Glu Tyr Ile Gly  Lys Leu Gln
       1355                 1360                 1365
   Glu Asp  Ile Lys Lys Ile Ile  Glu Lys Gly Thr Thr  Lys Gln Asn
       1370                 1375                 1380
   Gly Lys  Thr Val Gly Ser Gly  Ala Glu Asn Val Asn  Ala Trp Trp
       1385                 1390                 1395
   Lys Gly  Ile Glu Gly Glu Met  Trp Asp Ala Val Arg  Cys Ala Ile
       1400                 1405                 1410
   Thr Lys  Ile Asn Lys Lys Gln  Lys Lys Asn Gly Thr  Phe Ser Ile
       1415                 1420                 1425
   Asp Glu  Cys Gly Ile Phe Pro  Pro Thr Gly Asn Asp  Glu Asp Gln
       1430                 1435                 1440
   Ser Val  Ser Trp Phe Lys Glu  Trp Ser Glu Gln Phe  Cys Ile Glu
       1445                 1450                 1455
   Arg Leu  Gln Tyr Glu Lys Asn  Ile Arg Asp Ala Cys  Thr Asn Asn
       1460                 1465                 1470
   Gly Gln  Gly Asp Lys Ile Gln  Gly Asp Cys Lys Arg  Lys Cys Glu
       1475                 1480                 1485
   Glu Tyr  Lys Lys Tyr Ile Ser  Glu Lys Lys Gln Glu  Trp Asp Lys
       1490                 1495                 1500
   Gln Lys  Thr Lys Tyr Glu Asn  Lys Tyr Val Gly Lys  Ser Ala Ser
       1505                 1510                 1515
   Asp Leu  Leu Lys Glu Asn Tyr  Pro Glu Cys Ile Ser  Ala Asn Phe
       1520                 1525                 1530
   Asp Phe  Ile Phe Asn Asp Asn  Ile Glu Tyr Lys Thr  Tyr Tyr Pro
       1535                 1540                 1545
   Tyr Gly  Asp Tyr Ser Ser Ile  Cys Ser Cys Glu Gln  Val Lys Tyr
       1550                 1555                 1560
   Tyr Glu Tyr Asn Asn Ala Glu Lys Lys Asn Asn Lys Ser Leu Cys
   1565 1570 1575
   His Glu Lys Gly Asn Asp Arg Thr Trp Ser Lys Lys Tyr Ile Lys
   1580 1585 1590
   Lys Leu Glu Asn Gly Arg Thr Leu Glu Gly Val Tyr Val Pro Pro
   1595 1600 1605
   Arg Arg Gln Gln Leu Cys Leu Tyr Glu Leu Phe Pro Ile Ile Ile
   1610 1615 1620
   Lys Asn Lys Asn Asp Ile Thr Asn Ala Lys Lys Glu Leu Leu Glu
   1625 1630 1635
   Thr Leu Gln Ile Val Ala Glu Arg Glu Ala Tyr Tyr Leu Trp Lys
   1640 1645 1650
   Gln Tyr His Ala His Asn Asp Thr Thr Tyr Leu Ala His Lys Lys
   1655 1660 1665
   Ala Cys Cys Ala Ile Arg Gly Ser Phe Tyr Asp Leu Glu Asp Ile
   1670 1675 1680
   Ile Lys Gly Asn Asp Leu Val His Asp Glu Tyr Thr Lys Tyr Ile
   1685 1690 1695
   Asp Ser Lys Leu Asn Glu Ile Phe Asp Ser Ser Asn Lys Asn Asp
   1700 1705 1710
   Ile Glu Thr Lys Arg Ala Arg Thr Asp Trp Trp Glu Asn Glu Ala
   1715 1720 1725
   Ile Ala Val Pro Asn Ile Thr Gly Ala Asn Lys Ser Asp Pro Lys
   1730 1735 1740
   Thr Ile Arg Gln Leu Val Trp Asp Ala Met Gln Ser Gly Val Arg
   1745 1750 1755
   Lys Ala Ile Asp Glu Glu Lys Glu Lys Lys Lys Pro Asn Glu Asn
   1760 1765 1770
   Phe Pro Pro Cys Met Gly Val Gln His Ile Gly Ile Ala Lys Pro
   1775 1780 1785
   Gln Phe Ile Arg Trp Leu Glu Glu Trp Thr Asn Glu Phe Cys Glu
   1790 1795 1800
   Lys Tyr Thr Lys Tyr Phe Glu Asp Met Lys Ser Asn Cys Asn Leu
   1805 1810 1815
   Arg Lys Gly Ala Asp Asp Cys Asp Asp Asn Ser Asn Ile Glu Cys
   1820 1825 1830
   Lys Lys Ala Cys Ala Asn Tyr Thr Asn Trp Leu Asn Pro Lys Arg
   1835 1840 1845
   Ile Glu Trp Asn Gly Met Ser Asn Tyr Tyr Asn Lys Ile Tyr Arg
   1850 1855 1860
   Lys Ser Asn Lys Glu Ser Glu Asp Gly Lys Asp Tyr Ser Met Ile
   1865 1870 1875
   Met Glu Pro Thr Val Ile Asp Tyr Leu Asn Lys Arg Cys Asn Gly
   1880 1885 1890
   Glu Ile Asn Gly Asn Tyr Ile Cys Cys Ser Cys Lys Asn Ile Gly
   1895 1900 1905
   Glu Asn Ser Thr Ser Gly Thr Val Asn Lys Lys Leu Gln Lys Lys
   1910 1915 1920
   Glu Thr Gln Cys Glu Asp Asn Lys Gly Pro Leu Asp Leu Met Asn
   1925 1930 1935
   Lys Val Leu Asn Lys Met Asp Pro Lys Tyr Ser Glu His Lys Met
   1940 1945 1950
   Lys Cys Thr Glu Val Tyr Leu Glu His Val Glu Glu Gln Leu Lys
   1955 1960 1965
   Glu Ile Asp Asn Ala Ile Lys Asp Tyr Lys Leu Tyr Pro Leu Asp
   1970 1975 1980
   Arg Cys Phe Asp Asp Lys Ser Lys Met Lys Val Cys Asp Leu Ile
   1985 1990 1995
   Gly Asp Ala Ile Gly Cys Lys His Lys Thr Lys Leu Asp Glu Leu
   2000 2005 2010
   Asp Glu Trp Asn Asp Val Asp Met Arg Asp Pro Tyr Asn Lys Tyr
   2015 2020 2025
   Lys Gly  Val Leu Ile Pro Pro  Arg Arg Arg Gln Leu  Cys Phe Ser
       2030                 2035                 2040
   Arg Ile  Val Arg Gly Pro Ala  Asn Leu Arg Asn Leu  Lys Glu Phe
       2045                 2050                 2055
   Lys Glu  Glu Ile Leu Lys Gly  Ala Gln Ser Glu Gly  Lys Phe Leu
       2060                 2065                 2070
   Gly Asn  Tyr Tyr Asn Glu Asp  Lys Asp Lys Glu Lys  Ala Leu Glu
       2075                 2080                 2085
   Ala Met  Lys Asn Ser Phe Tyr  Asp Tyr Glu Tyr Ile  Ile Lys Gly
       2090                 2095                 2100
   Ser Asp  Met Leu Thr Asn Ile  Gln Phe Lys Asp Ile  Lys Arg Lys
       2105                 2110                 2115
   Leu Asp  Arg Leu Leu Glu Lys  Glu Thr Asn Asn Thr  Glu Lys Val
       2120                 2125                 2130
   Asp Asp  Trp Trp Glu Thr Asn  Lys Lys Ser Ile Trp  Asn Ala Met
       2135                 2140                 2145
   Leu Cys  Gly Tyr Lys Lys Ser  Gly Asn Lys Ile Ile  Asp Pro Ser
       2150                 2155                 2160
   Trp Cys  Thr Ile Pro Thr Thr  Glu Thr Pro Pro Gln  Phe Leu Arg
       2165                 2170                 2175
   Trp Ile  Lys Glu Trp Gly Thr  Asn Val Cys Ile Gln  Lys Glu Glu
       2180                 2185                 2190
   His Lys  Glu Tyr Val Lys Ser  Lys Cys Ser Asn Val  Thr Asn Leu
       2195                 2200                 2205
   Gly Ala  Gln Glu Ser Glu Ser  Lys Asn Cys Thr Ser  Glu Ile Lys
       2210                 2215                 2220
   Lys Tyr  Gln Glu Trp Ser Arg  Lys Arg Ser Ile Gln  Trp Glu Ala
       2225                 2230                 2235
   Ile Ser  Glu Gly Tyr Lys Lys  Tyr Lys Gly Met Asp  Glu Phe Lys
       2240                 2245                 2250
   Asn Thr  Phe Lys Asn Ile Lys  Glu Pro Asp Ala Asn  Glu Pro Asn
       2255                 2260                 2265
   Ala Asn Glu Tyr Leu Lys Lys His Cys Ser Lys Cys Pro Cys Gly
   2270 2275 2280
   Phe Asn Asp Met Gln Glu Ile Thr Lys Tyr Thr Asn Ile Gly Asn
   2285 2290 2295
   Glu Ala Phe Lys Gln Ile Lys Glu Gln Val Asp Ile Pro Ala Glu
   2300 2305 2310
   Leu Glu Asp Val Ile Tyr Arg Leu Lys His His Glu Tyr Asp Lys
   2315 2320 2325
   Gly Asn Asp Tyr Ile Cys Asn Lys Tyr Lys Asn Ile Asn Val Asn
   2330 2335 2340
   Met Lys Lys Asn Asn Asp Asp Thr Trp Thr Asp Leu Val Lys Asn
   2345 2350 2355
   Ser Ser Asp Ile Asn Lys Gly Val Leu Leu Pro Pro Arg Arg Lys
   2360 2365 2370
   Asn Leu Phe Leu Lys Ile Asp Glu Ser Asp Ile Cys Lys Tyr Lys
   2375 2380 2385
   Arg Asp Pro Lys Leu Phe Lys Asp Phe Ile Tyr Ser Ser Ala Ile
   2390 2395 2400
   Ser Glu Val Glu Arg Leu Lys Lys Val Tyr Gly Glu Ala Lys Thr
   2405 2410 2415
   Lys Val Val His Ala Met Lys Tyr Ser Phe Ala Asp Ile Gly Ser
   2420 2425 2430
   Ile Ile Lys Gly Asp Asp Met Met Glu Asn Asn Ser Ser Asp Lys
   2435 2440 2445
   Ile Gly Lys Ile Leu Gly Asp Gly Val Gly Gln Asn Glu Lys Arg
   2450 2455 2460
   Lys Lys Trp Trp Asp Met Asn Lys Tyr His Ile Trp Glu Ser Met
   2465 2470 2475
   Leu Cys Gly Tyr Lys His Ala Tyr Gly Asn Ile Ser Glu Asn Asp
   2480 2485 2490
   Arg Lys Met Leu Asp Ile Pro Asn Asn Asp Asp Glu His Gln Phe
   2495 2500 2505
   Leu Arg Trp Phe Gln Glu Trp Thr Glu Asn Phe Cys Thr Lys Arg
   2510 2515 2520
   Asn Glu Leu Tyr Glu Asn Met Val Thr Ala Cys Asn Ser Ala Lys
   2525 2530 2535
   Cys Asn Thr Ser Asn Gly Ser Val Asp Lys Lys Glu Cys Thr Glu
   2540 2545 2550
   Ala Cys Lys Asn Tyr Ser Asn Phe Ile Leu Ile Lys Lys Lys Glu
   2555 2560 2565
   Tyr Gln Ser Leu Asn Ser Gln Tyr Asp Met Asn Tyr Lys Glu Thr
   2570 2575 2580
   Lys Ala Glu Lys Lys Glu Ser Pro Glu Tyr Phe Lys Asp Lys Cys
   2585 2590 2595
   Asn Gly Glu Cys Ser Cys Leu Ser Glu Tyr Phe Lys Asp Glu Thr
   2600 2605 2610
   Arg Trp Lys Asn Pro Tyr Glu Thr Leu Asp Asp Thr Glu Val Lys
   2615 2620 2625
   Asn Asn Cys Met Cys Lys Pro Pro Pro Pro Ala Ser Asn Asn Thr
   2630 2635 2640
   Ser Asp Ile Leu Gln Lys Thr Ile Pro Phe Gly Ile Ala Leu Ala
   2645 2650 2655
   Leu Gly Ser Ile Ala Phe Leu Phe Met Lys Lys Lys Pro Lys Thr
   2660 2665 2670
   Pro Val Asp Leu Leu Arg Val Leu Asp Ile Pro Lys Gly Asp Tyr
   2675 2680 2685
   Gly Ile Pro Thr Pro Lys Ser Ser Asn Arg Tyr Ile Pro Tyr Ala
   2690 2695 2700
   Ser Asp Arg Tyr Lys Gly Lys Thr Tyr Ile Tyr Met Glu Gly Asp
   2705 2710 2715
   Thr Ser Gly Asp Asp Asp Lys Tyr Ile Trp Asp Leu
   2720 2725 2730
<210> 56
   <211> 2734
   <212> PRT
   <213> Artificial
<220>
   <223> Artificial construct
<400> 56
   Met Asp Ser Thr Ser Thr Ile Ala Asn Lys Ile Glu Glu Tyr Leu Gly
   1               5                   10                  15
   Ala Lys Ser Asp Asp Ser Lys Ile Asp Glu Leu Leu Lys Ala Asp Pro
               20                  25                  30
   Ser Glu Val Glu Tyr Tyr Arg Ser Gly Gly Asp Gly Asp Tyr Leu Lys
           35                  40                  45
   Asn Asn Ile Cys Lys Ile Thr Val Asn His Ser Asp Ser Gly Lys Tyr
       50                  55                  60
   Asp Pro Cys Glu Lys Lys Leu Pro Pro Tyr Asp Asp Asn Asp Gln Trp
   65                  70                  75                  80
   Lys Cys Gln Gln Asn Ser Ser Asp Gly Ser Gly Lys Pro Glu Asn Ile
                   85                  90                  95
   Cys Val Pro Pro Arg Arg Glu Arg Leu Cys Thr Tyr Asn Leu Glu Asn
               100                 105                 110
   Leu Lys Phe Asp Lys Ile Arg Asp Asn Asn Ala Phe Leu Ala Asp Val
           115                 120                 125
   Leu Leu Thr Ala Arg Asn Glu Gly Glu Lys Ile Val Gln Asn His Pro
       130                 135                 140
   Asp Thr Asn Ser Ser Asn Val Cys Asn Ala Leu Glu Arg Ser Phe Ala
   145                 150                 155                 160
   Asp Leu Ala Asp Ile Ile Arg Gly Thr Asp Gln Trp Lys Gly Thr Asn
                   165                 170                 175
   Ser Asn Leu Glu Lys Asn Leu Lys Gln Met Phe Ala Lys Ile Arg Glu
               180                 185                 190
   Asn Asp Lys Val Leu Gln Asp Lys Tyr Pro Lys Asp Gln Lys Tyr Thr
           195                 200                 205
   Lys Leu Arg Glu Ala Trp Trp Asn Ala Asn Arg Gln Lys Val Trp Glu
       210                 215                 220
   Val Ile Thr Cys Gly Ala Arg Ser Asn Asp Leu Leu Ile Lys Arg Gly
   225 230 235 240
   Trp Arg Thr Ser Gly Lys Ser Asp Arg Lys Lys Asn Phe Glu Leu Cys
   245 250 255
   Arg Lys Cys Gly His Tyr Glu Lys Glu Val Pro Thr Lys Leu Asp Tyr
   260 265 270
   Val Pro Gln Phe Leu Arg Trp Leu Thr Glu Trp Ile Glu Asp Phe Tyr
   275 280 285
   Arg Glu Lys Gln Asn Leu Ile Asp Asp Met Glu Arg His Arg Glu Glu
   290 295 300
   Cys Thr Arg Glu Asp His Lys Ser Lys Glu Gly Thr Ser Tyr Cys Ser
   305 310 315 320
   Thr Cys Lys Asp Lys Cys Lys Lys Tyr Cys Glu Cys Val Lys Lys Trp
   325 330 335
   Lys Thr Glu Trp Glu Asn Gln Glu Asn Lys Tyr Lys Asp Leu Tyr Glu
   340 345 350
   Gln Asn Lys Asn Lys Thr Ser Gln Lys Asn Thr Ser Arg Tyr Asp Asp
   355 360 365
   Tyr Val Lys Asp Phe Phe Glu Lys Leu Glu Ala Asn Tyr Ser Ser Leu
   370 375 380
   Glu Asn Tyr Ile Lys Gly Asp Pro Tyr Phe Ala Glu Tyr Ala Thr Lys
   385 390 395 400
   Leu Ser Phe Ile Leu Asn Pro Ser Asp Ala Asn Asn Pro Ser Gly Glu
   405 410 415
   Thr Ala Asn His Asn Asp Glu Ala Cys Asn Cys Asn Glu Ser Gly Ile
   420 425 430
   Ser Ser Val Gly Gln Ala Gln Thr Ser Gly Pro Ser Ser Asn Lys Thr
   435 440 445
   Cys Ile Thr His Ser Ser Ile Lys Thr Asn Lys Lys Lys Glu Cys Lys
   450 455 460
   Asp Val Lys Leu Gly Val Arg Glu Asn Asp Lys Asp Leu Lys Ile Cys
   465 470 475 480
   Val Ile Glu Asp Thr Ser Leu Ser Gly Val Asp Asn Cys Cys Cys Gln
   485 490 495
   Asp Leu Leu Gly Ile Leu Gln Glu Asn Cys Ser Asp Asn Lys Arg Gly
   500 505 510
   Ser Ser Ser Asn Asp Ser Cys Asp Asn Lys Asn Gln Asp Glu Cys Gln
   515 520 525
   Lys Lys Leu Glu Lys Val Phe Ala Ser Leu Thr Asn Gly Tyr Lys Cys
   530 535 540
   Asp Lys Cys Lys Ser Gly Thr Ser Arg Ser Lys Lys Lys Trp Ile Trp
   545 550 555 560
   Lys Lys Ser Ser Gly Asn Glu Glu Gly Leu Gln Glu Glu Tyr Ala Asn
   565 570 575
   Thr Ile Gly Leu Pro Pro Arg Thr Gln Ser Leu Tyr Leu Gly Asn Leu
   580 585 590
   Pro Lys Leu Glu Asn Val Cys Glu Asp Val Lys Asp Ile Asn Phe Asp
   595 600 605
   Thr Lys Glu Lys Phe Leu Ala Gly Cys Leu Ile Val Ser Phe His Glu
   610 615 620
   Gly Lys Asn Leu Lys Lys Arg Tyr Pro Gln Asn Lys Asn Ser Gly Asn
   625 630 635 640
   Lys Glu Asn Leu Cys Lys Ala Leu Glu Tyr Ser Phe Ala Asp Tyr Gly
   645 650 655
   Asp Leu Ile Lys Gly Thr Ser Ile Trp Asp Asn Glu Tyr Thr Lys Asp
   660 665 670
   Leu Glu Leu Asn Leu Gln Asn Asn Phe Gly Lys Leu Phe Gly Lys Tyr
   675 680 685
   Ile Lys Lys Asn Asn Thr Ala Glu Gln Asp Thr Ser Tyr Ser Ser Leu
   690 695 700
   Asp Glu Leu Arg Glu Ser Trp Trp Asn Thr Asn Lys Lys Tyr Ile Trp
   705 710 715 720
   Thr Ala Met Lys His Gly Ala Glu Met Asn Ile Thr Thr Cys Asn Ala
   725 730 735
   Asp Gly Ser Val Thr Gly Ser Gly Ser Ser Cys Asp Asp Ile Pro Thr
   740 745 750
   Ile Asp Leu Ile Pro Gln Tyr Leu Arg Phe Leu Gln Glu Trp Val Glu
   755 760 765
   Asn Phe Cys Glu Gln Arg Gln Ala Lys Val Lys Asp Val Ile Thr Asn
   770 775 780
   Cys Lys Ser Cys Lys Glu Ser Gly Asn Lys Cys Lys Thr Glu Cys Lys
   785 790 795 800
   Thr Lys Cys Lys Asp Glu Cys Glu Lys Tyr Lys Lys Phe Ile Glu Ala
   805 810 815
   Cys Gly Thr Ala Gly Gly Gly Ile Gly Thr Ala Gly Ser Pro Trp Ser
   820 825 830
   Lys Arg Trp Asp Gln Ile Tyr Lys Arg Tyr Ser Lys His Ile Glu Asp
   835 840 845
   Ala Lys Arg Asn Arg Lys Ala Gly Thr Lys Asn Cys Gly Thr Ser Ser
   850 855 860
   Thr Thr Asn Ala Ala Ala Ser Thr Asp Glu Asn Lys Cys Val Gln Ser
   865 870 875 880
   Asp Ile Asp Ser Phe Phe Lys His Leu Ile Asp Ile Gly Leu Thr Thr
   885 890 895
   Pro Ser Ser Tyr Leu Ser Asn Val Leu Asp Asp Asn Ile Cys Gly Ala
   900 905 910
   Asp Lys Ala Pro Trp Thr Thr Tyr Thr Thr Tyr Thr Thr Thr Glu Lys
   915 920 925
   Cys Asn Lys Glu Arg Asp Lys Ser Lys Ser Gln Ser Ser Asp Thr Leu
   930 935 940
   Val Val Val Asn Val Pro Ser Pro Leu Gly Asn Thr Pro Tyr Arg Tyr
   945 950 955 960
   Lys Tyr Ala Cys Gln Cys Lys Ile Pro Thr Asn Glu Glu Thr Cys Asp
   965 970 975
   Asp Arg Lys Glu Tyr Met Asn Gln Trp Ser Cys Gly Ser Ala Arg Thr
   980 985 990
   Met Lys Arg Gly Tyr Lys Asn Asp Asn Tyr Glu Leu Cys Lys Tyr Asn
   995 1000 1005
   Gly Val Asp Val Lys Pro Thr Thr Val Arg Ser Asn Ser Ser Lys
   1010 1015 1020
   Leu Asp Gly Asn Asp Val Thr Phe Phe Asn Leu Phe Glu Gln Trp
   1025 1030 1035
   Asn Lys Glu Ile Gln Tyr Gln Ile Glu Gln Tyr Met Thr Asn Ala
   1040 1045 1050
   Asn Ile Ser Cys Ile Asp Glu Lys Glu Val Leu Asp Ser Val Ser
   1055 1060 1065
   Asp Glu Gly Thr Pro Lys Val Arg Gly Gly Tyr Glu Asp Gly Arg
   1070 1075 1080
   Asn Asn Asn Thr Asp Gln Gly Thr Asn Cys Lys Glu Lys Cys Lys
   1085 1090 1095
   Cys Tyr Lys Leu Trp Ile Glu Lys Ile Asn Asp Gln Trp Gly Lys
   1100 1105 1110
   Gln Lys Asp Asn Tyr Asn Lys Phe Arg Ser Lys Gln Ile Tyr Asp
   1115 1120 1125
   Ala Asn Lys Gly Ser Gln Asn Lys Lys Val Val Ser Leu Ser Asn
   1130 1135 1140
   Phe Leu Phe Phe Ser Cys Trp Glu Glu Tyr Ile Gln Lys Tyr Phe
   1145 1150 1155
   Asn Gly Asp Trp Ser Lys Ile Lys Asn Ile Gly Ser Asp Thr Phe
   1160 1165 1170
   Glu Phe Leu Ile Lys Lys Cys Gly Asn Asn Ser Ala His Gly Glu
   1175 1180 1185
   Glu Ile Phe Asn Glu Lys Leu Lys Asn Ala Glu Lys Lys Cys Lys
   1190 1195 1200
   Glu Asn Glu Ser Thr Asp Thr Asn Ile Asn Lys Ser Glu Thr Ser
   1205 1210 1215
   Cys Asp Leu Asn Ala Thr Asn Tyr Ile Arg Gly Cys Gln Ser Lys
   1220 1225 1230
   Thr Tyr Asp Gly Lys Ile Phe Pro Gly Lys Gly Gly Glu Lys Gln
   1235 1240 1245
   Trp Ile Cys Lys Asp Thr Ile Ile His Gly Asp Thr Asn Gly Ala
   1250 1255 1260
   Cys Ile Pro Pro Arg Thr Gln Asn Leu Cys Val Gly Glu Leu Trp
   1265 1270 1275
   Asp Lys Ser Tyr Gly Gly Arg Ser Asn Ile Lys Asn Asp Thr Lys
   1280 1285 1290
   Glu Leu Leu Lys Glu Lys Ile Lys Asn Ala Ile His Lys Glu Thr
   1295 1300 1305
   Glu Leu Leu Tyr Glu Tyr His Asp Thr Gly Thr Ala Ile Ile Ser
   1310 1315 1320
   Lys Asn Asp Lys Lys Gly Gln Lys Gly Lys Asn Asp Pro Asn Gly
   1325 1330 1335
   Leu Pro Lys Gly Phe Cys His Ala Val Gln Arg Ser Phe Ile Asp
   1340 1345 1350
   Tyr Lys Asn Met Ile Leu Gly Thr Ser Val Asn Ile Tyr Glu His
   1355 1360 1365
   Ile Gly Lys Leu Gln Glu Asp Ile Lys Lys Ile Ile Glu Lys Gly
   1370 1375 1380
   Thr Pro Gln Gln Lys Asp Lys Ile Gly Gly Val Gly Ser Ser Thr
   1385 1390 1395
   Glu Asn Val Asn Ala Trp Trp Lys Gly Ile Glu Arg Glu Met Trp
   1400 1405 1410
   Asp Ala Val Arg Cys Ala Ile Thr Lys Ile Asn Lys Lys Asn Asn
   1415 1420 1425
   Asn Ser Ile Phe Asn Gly Asp Glu Cys Gly Val Ser Pro Pro Thr
   1430 1435 1440
   Gly Asn Asp Glu Asp Gln Ser Val Ser Trp Phe Lys Glu Trp Gly
   1445 1450 1455
   Glu Gln Phe Cys Ile Glu Arg Leu Arg Tyr Glu Gln Asn Ile Arg
   1460 1465 1470
   Glu Ala Cys Thr Ile Asn Gly Lys Asn Glu Lys Lys Cys Ile Asn
   1475 1480 1485
   Ser Lys Ser Gly Gln Gly Asp Lys Ile Gln Gly Ala Cys Lys Arg
   1490 1495 1500
   Lys Cys Glu Lys Tyr Lys Lys Tyr Ile Ser Glu Lys Lys Gln Glu
   1505 1510 1515
   Trp Asp Lys Gln Lys Thr Lys Tyr Glu Asn Lys Tyr Val Gly Lys
   1520 1525 1530
   Ser Ala Ser Asp Leu Leu Lys Glu Asn Tyr Pro Glu Cys Ile Ser
   1535 1540 1545
   Ala Asn Phe Asp Phe Ile Phe Asn Asp Asn Ile Glu Tyr Lys Thr
   1550 1555 1560
   Tyr Tyr Pro Tyr Gly Asp Tyr Ser Ser Ile Cys Ser Cys Glu Gln
   1565 1570 1575
   Val Lys Tyr Tyr Lys Tyr Asn Asn Ala Glu Lys Lys Asn Asn Lys
   1580 1585 1590
   Ser Leu Cys Tyr Glu Lys Asp Asn Asp Met Thr Trp Ser Lys Lys
   1595 1600 1605
   Tyr Ile Lys Lys Leu Glu Asn Gly Arg Ser Leu Glu Gly Val Tyr
   1610 1615 1620
   Val Pro Pro Arg Arg Gln Gln Leu Cys Leu Tyr Glu Leu Phe Pro
   1625 1630 1635
   Ile Ile Ile Lys Asn Glu Glu Gly Met Glu Lys Ala Lys Glu Glu
   1640 1645 1650
   Leu Leu Glu Thr Leu Gln Ile Val Ala Glu Arg Glu Ala Tyr Tyr
   1655 1660 1665
   Leu Trp Lys Gln Tyr Asn Pro Thr Gly Lys Gly Ile Asp Asp Ala
   1670 1675 1680
   Asn Lys Lys Ala Cys Cys Ala Ile Arg Gly Ser Phe Tyr Asp Leu
   1685 1690 1695
   Glu Asp Ile Ile Lys Gly Asn Asp Leu Val His Asp Glu Tyr Thr
   1700 1705 1710
   Lys Tyr Ile Asp Ser Lys Leu Asn Glu Ile Phe Gly Ser Ser Asp
   1715 1720 1725
   Thr Asn Asp Ile Asp Thr Lys Arg Ala Arg Thr Asp Trp Trp Glu
   1730 1735 1740
   Asn Glu Thr Ile Thr Asn Gly Thr Asp Arg Lys Thr Ile Arg Gln
   1745 1750 1755
   Leu Val Trp Asp Ala Met Gln Ser Gly Val Arg Tyr Ala Val Glu
   1760 1765 1770
   Glu Lys Asn Glu Asn Phe Pro Leu Cys Met Gly Val Glu His Ile
   1775 1780 1785
   Gly Ile Ala Lys Pro Gln Phe Ile Arg Trp Leu Glu Glu Trp Thr
   1790 1795 1800
   Asn Glu Phe Cys Glu Lys Tyr Thr Lys Tyr Phe Glu Asp Met Lys
   1805 1810 1815
   Ser Lys Cys Asp Pro Pro Lys Arg Ala Asp Thr Cys Gly Asp Asn
   1820 1825 1830
   Ser Asn Ile Glu Cys Lys Lys Ala Cys Ala Asn Tyr Thr Asn Trp
   1835 1840 1845
   Leu Asn Pro Lys Arg Ile Glu Trp Asn Gly Met Ser Asn Tyr Tyr
   1850 1855 1860
   Asn Lys Ile Tyr Arg Lys Ser Asn Lys Glu Ser Glu Gly Gly Lys
   1865 1870 1875
   Asp Tyr Ser Met Ile Met Ala Pro Thr Val Ile Asp Tyr Leu Asn
   1880 1885 1890
   Lys Arg Cys His Gly Glu Ile Asn Gly Asn Tyr Ile Cys Cys Ser
   1895 1900 1905
   Cys Lys Asn Ile Gly Ala Tyr Asn Thr Thr Ser Gly Thr Val Asn
   1910 1915 1920
   Lys Lys Leu Gln Lys Lys Glu Thr Glu Cys Glu Glu Glu Lys Gly
   1925 1930 1935
   Pro Leu Asp Leu Met Asn Glu Val Leu Asn Lys Met Asp Lys Lys
   1940 1945 1950
   Tyr Ser Ala His Lys Met Lys Cys Thr Glu Val Tyr Leu Glu His
   1955 1960 1965
   Val Glu Glu Gln Leu Asn Glu Ile Asp Asn Ala Ile Lys Asp Tyr
   1970 1975 1980
   Lys Leu Tyr Pro Leu Asp Arg Cys Phe Asp Asp Gln Thr Lys Met
   1985 1990 1995
   Lys Val Cys Asp Leu Ile Ala Asp Ala Ile Gly Cys Lys Asp Lys
   2000 2005 2010
   Thr Lys Leu Asp Glu Leu Asp Glu Trp Asn Asp Met Asp Leu Arg
   2015 2020 2025
   Gly Thr Tyr Asn Lys His Lys Gly Val Leu Ile Pro Pro Arg Arg
   2030 2035 2040
   Arg Gln Leu Cys Phe Ser Arg Ile Val Arg Gly Pro Ala Asn Leu
   2045 2050 2055
   Arg Ser Leu Asn Glu Phe Lys Glu Glu Ile Leu Lys Gly Ala Gln
   2060 2065 2070
   Ser Glu Gly Lys Phe Leu Gly Asn Tyr Tyr Lys Glu His Lys Asp
   2075 2080 2085
   Lys Glu Lys Ala Leu Glu Ala Met Lys Asn Ser Phe Tyr Asp Tyr
   2090 2095 2100
   Glu Asp Ile Ile Lys Gly Thr Asp Met Leu Thr Asn Ile Glu Phe
   2105 2110 2115
   Lys Asp Ile Lys Ile Lys Leu Asp Arg Leu Leu Glu Lys Glu Thr
   2120 2125 2130
   Asn Asn Thr Lys Lys Ala Glu Asp Trp Trp Lys Thr Asn Lys Lys
   2135 2140 2145
   Ser Ile Trp Asn Ala Met Leu Cys Gly Tyr Lys Lys Ser Gly Asn
   2150 2155 2160
   Lys Ile Ile Asp Pro Ser Trp Cys Thr Ile Pro Thr Thr Glu Thr
   2165 2170 2175
   Pro Pro Gln Phe Leu Arg Trp Ile Lys Glu Trp Gly Thr Asn Val
   2180 2185 2190
   Cys Ile Gln Lys Gln Glu His Lys Glu Tyr Val Lys Ser Lys Cys
   2195 2200 2205
   Ser Asn Val Thr Asn Leu Gly Ala Gln Ala Ser Glu Ser Asn Asn
   2210 2215 2220
   Cys Thr Ser Glu Ile Lys Lys Tyr Gln Glu Trp Ser Arg Lys Arg
   2225 2230 2235
   Ser Ile Arg Trp Glu Thr Ile Ser Lys Arg Tyr Lys Lys Tyr Lys
   2240 2245 2250
   Arg Met Asp Ile Leu Lys Asp Val Lys Glu Pro Asp Ala Asn Thr
   2255 2260 2265
   Tyr Leu Arg Glu His Cys Ser Lys Cys Pro Cys Gly Phe Asn Asp
   2270 2275 2280
   Met Glu Glu Met Asn Asn Asn Glu Asp Asn Glu Lys Glu Ala Phe
   2285 2290 2295
   Lys Gln Ile Lys Glu Gln Val Lys Ile Pro Ala Glu Leu Glu Asp
   2300 2305 2310
   Val Ile Tyr Arg Ile Lys His His Glu Tyr Asp Lys Gly Asn Asp
   2315 2320 2325
   Tyr Ile Cys Asn Lys Tyr Lys Asn Ile His Asp Arg Met Lys Lys
   2330 2335 2340
   Asn Asn Gly Asn Phe Val Thr Asp Asn Phe Val Lys Lys Ser Trp
   2345 2350 2355
   Glu Ile Ser Asn Gly Val Leu Ile Pro Pro Arg Arg Lys Asn Leu
   2360 2365 2370
   Phe Leu Tyr Ile Asp Pro Ser Lys Ile Cys Glu Tyr Lys Lys Asp
   2375 2380 2385
   Pro Lys Leu Phe Lys Asp Phe Ile Tyr Trp Ser Ala Phe Thr Glu
   2390 2395 2400
   Val Glu Arg Leu Lys Lys Ala Tyr Gly Gly Ala Arg Ala Lys Val
   2405 2410 2415
   Val His Ala Met Lys Tyr Ser Phe Thr Asp Ile Gly Ser Ile Ile
   2420 2425 2430
   Lys Gly Asp Asp Met Met Glu Lys Asn Ser Ser Asp Lys Ile Gly
   2435 2440 2445
   Lys Ile Leu Gly Asp Thr Asp Gly Gln Asn Glu Lys Arg Lys Lys
   2450 2455 2460
   Trp Trp Asp Met Asn Lys Tyr His Ile Trp Glu Ser Met Leu Cys
   2465 2470 2475
   Gly Tyr Arg Glu Ala Glu Gly Asp Thr Glu Thr Asn Glu Asn Cys
   2480 2485 2490
   Arg Phe Pro Asp Ile Glu Ser Val Pro Gln Phe Leu Arg Trp Phe
   2495 2500 2505
   Gln Glu Trp Ser Glu Asn Phe Cys Asp Arg Arg Gln Lys Leu Tyr
   2510 2515 2520
   Asp Lys Leu Asn Ser Glu Cys Ile Ser Ala Glu Cys Thr Asn Gly
   2525 2530 2535
   Ser Val Asp Asn Ser Lys Cys Thr His Ala Cys Val Asn Tyr Lys
   2540 2545 2550
   Asn Tyr Ile Leu Thr Lys Lys Thr Glu Tyr Glu Ile Gln Thr Asn
   2555 2560 2565
   Lys Tyr Asp Asn Glu Phe Lys Asn Lys Asn Ser Asn Asp Lys Asp
   2570 2575 2580
   Ala Pro Asp Tyr Leu Lys Glu Lys Cys Asn Asp Asn Lys Cys Glu
   2585 2590 2595
   Cys Leu Asn Lys His Ile Asp Asp Lys Asn Lys Thr Trp Lys Asn
   2600 2605 2610
   Pro Tyr Glu Thr Leu Glu Asp Thr Phe Lys Ser Lys Cys Asp Cys
   2615 2620 2625
   Pro Lys Pro Leu Pro Ser Pro Ile Lys Pro Asp Asp Leu Pro Pro
       2630                2635                2640
   Gln Ala Asp Glu Pro Phe Asp Pro Thr Ile Leu Gln Thr Thr Ile
       2645                2650                2655
   Pro Phe Gly Ile Ala Leu Ala Leu Gly Ser Ile Ala Phe Leu Phe
       2660                2665                2670
   Met Lys Val Ile Tyr Ile Tyr Ile Tyr Val Cys Cys Ile Cys Met
       2675                2680                2685
   Tyr Val Cys Met Tyr Val Cys Met Tyr Val Cys Met Tyr Val Cys
       2690                2695                2700
   Met Tyr Val Cys Met His Val Cys Met Leu Cys Val Tyr Val Ile
       2705                2710                2715
   Tyr Val Phe Lys Ile Cys Ile Tyr Ile Glu Lys Glu Lys Arg Lys
       2720                2725                2730
   Lys
<210> 57
   <211> 58
   <212> PRT
   <213> Artificial
<220>
   <223> Artificial construct
<400> 57
<210> 58
   <211> 324
   <212> PRT
   <213> Artificial
<220>
   <223> Artificial construct
<400> 58
   Arg His Arg Gln Pro Arg Gly Trp Glu Gln Leu Glu Gln Cys Gly Tyr
   1               5                   10                  15
   Pro Val Gln Arg Leu Val Ala Leu Tyr Leu Ala Ala Arg Leu Ser Trp
               20                  25                  30
   Asn Gln Val Asp Gln Val Ile Arg Asn Ala Leu Ala Ser Pro Gly Ser
           35                  40                  45
   Gly Gly Asp Leu Gly Glu Ala Ile Arg Glu Gln Pro Glu Gln Ala Arg
       50                  55                  60
   Leu Ala Leu Thr Leu Ala Ala Ala Glu Ser Glu Arg Phe Val Arg Gln
   65                  70                  75                  80
   Gly Thr Gly Asn Asp Glu Ala Gly Ala Ala Asn Gly Pro Ala Asp Ser
                   85                  90                  95
   Gly Asp Ala Leu Leu Glu Arg Asn Tyr Pro Thr Gly Ala Glu Phe Leu
               100                 105                 110
   Gly Asp Gly Gly Asp Ile Ser Phe Ser Thr Arg Gly Thr Gln Asn Trp
           115                 120                 125
   Thr Val Glu Arg Leu Leu Gln Ala His Arg Gln Leu Glu Glu Arg Gly
       130                 135                 140
   Tyr Val Phe Val Gly Tyr His Gly Thr Phe Leu Glu Ala Ala Gln Ser
   145                 150                 155                 160
   Ile Val Phe Gly Gly Val Arg Ala Arg Ser Gln Asp Leu Asp Ala Ile
                   165                 170                 175
   Trp Arg Gly Phe Tyr Ile Ala Gly Asp Pro Ala Leu Ala Tyr Gly Tyr
               180                 185                 190
   Ala Gln Asp Gln Glu Pro Asp Ala Arg Gly Arg Ile Arg Asn Gly Ala
           195                 200                 205
   Leu Leu Arg Val Tyr Val Pro Arg Ser Ser Leu Pro Gly Phe Tyr Arg
       210                 215                 220
   Thr Ser Leu Thr Leu Ala Ala Pro Glu Ala Ala Gly Glu Val Glu Arg
   225                 230                 235                 240
   Leu Ile Gly His Pro Leu Pro Leu Arg Leu Asp Ala Ile Thr Gly Pro
   245 250 255
   Glu Glu Glu Gly Gly Arg Leu Glu Thr Ile Leu Gly Trp Pro Leu Ala
   260 265 270
   Glu Arg Thr Val Val Ile Pro Ser Ala Ile Pro Thr Asp Pro Arg Asn
   275 280 285
   Val Gly Gly Asp Leu Asp Pro Ser Ser Ile Pro Asp Lys Glu Gln Ala
   290 295 300
   Ile Ser Ala Leu Pro Asp Tyr Ala Ser Gln Pro Gly Lys Pro Pro Arg
   305 310 315 320
   Lys Asp Glu Leu
<210> 59
   <211> 231
   <212> PRT
   <213> Artificial
<220>
   <223> Artificial construct
<400> 59
   Arg His Arg Gln Pro Arg Gly Trp Glu Gln Leu Tyr Pro Thr Gly Ala
   1 5 10 15
   Glu Phe Leu Gly Asp Gly Gly Asp Ile Ser Phe Ser Thr Arg Gly Thr
   20 25 30
   Gln Asn Trp Thr Val Glu Arg Leu Leu Gln Ala His Arg Gln Leu Glu
   35 40 45
   Glu Arg Gly Tyr Val Phe Val Gly Tyr His Gly Thr Phe Leu Glu Ala
   50 55 60
   Ala Gln Ser Ile Val Phe Gly Gly Val Arg Ala Arg Ser Gln Asp Leu
   65 70 75 80
   Asp Ala Ile Trp Arg Gly Phe Tyr Ile Ala Gly Asp Pro Ala Leu Ala
   85 90 95
   Tyr Gly Tyr Ala Gln Asp Gln Glu Pro Asp Ala Arg Gly Arg Ile Arg
   100 105 110
   Asn Gly Ala Leu Leu Arg Val Tyr Val Pro Arg Ser Ser Leu Pro Gly
   115 120 125
   Phe Tyr Arg Thr Ser Leu Thr Leu Ala Ala Pro Glu Ala Ala Gly Glu
   130 135 140
   Val Glu Arg Leu Ile Gly His Pro Leu Pro Leu Arg Leu Asp Ala Ile
   145 150 155 160
   Thr Gly Pro Glu Glu Glu Gly Gly Arg Leu Glu Thr Ile Leu Gly Trp
   165 170 175
   Pro Leu Ala Glu Arg Thr Val Val Ile Pro Ser Ala Ile Pro Thr Asp
   180 185 190
   Pro Arg Asn Val Gly Gly Asp Leu Asp Pro Ser Ser Ile Pro Asp Lys
   195 200 205
   Glu Gln Ala Ile Ser Ala Leu Pro Asp Tyr Ala Ser Gln Pro Gly Lys
   210 215 220
   Pro Pro Arg Lys Asp Glu Leu
   225 230
<210> 60
   <211> 929
   <212> PRT
   <213> Artificial
<220>
   <223> Artificial construct
<400> 60
   Arg Pro Asp Phe Cys Leu Glu Pro Pro Tyr Thr Gly Pro Cys Lys Ala
   1 5 10 15
   Arg Ile Ile Arg Tyr Phe Tyr Asn Ala Lys Ala Gly Leu Cys Gln Thr
   20 25 30
   Phe Val Tyr Gly Gly Cys Arg Ala Lys Arg Asn Asn Phe Lys Ser Ala
   35 40 45
   Glu Asp Cys Met Arg Thr Cys Gly Gly Ala Asn Tyr Ile Lys Gly Asp
   50 55 60
   Pro Tyr Phe Ala Glu Tyr Ala Thr Lys Leu Ser Phe Ile Leu Asn Pro
   65 70 75 80
   Ser Asp Ala Asn Asn Pro Ser Gly Glu Thr Ala Asn His Asn Asp Glu
                   85                  90                  95
   Ala Cys Asn Cys Asn Glu Ser Gly Ile Ser Ser Val Gly Gln Ala Gln
               100                 105                 110
   Thr Ser Gly Pro Ser Ser Asn Lys Thr Cys Ile Thr His Ser Ser Ile
           115                 120                 125
   Lys Thr Asn Lys Lys Lys Glu Cys Lys Asp Val Lys Leu Gly Val Arg
       130                 135                 140
   Glu Asn Asp Lys Asp Leu Lys Ile Cys Val Ile Glu Asp Thr Ser Leu
   145                 150                 155                 160
   Ser Gly Val Asp Asn Cys Cys Cys Gln Asp Leu Leu Gly Ile Leu Gln
                   165                 170                 175
   Glu Asn Cys Ser Asp Asn Lys Arg Gly Ser Ser Ser Asn Asp Ser Cys
               180                 185                 190
   Asp Asn Lys Asn Gln Asp Glu Cys Gln Lys Lys Leu Glu Lys Val Phe
           195                 200                 205
   Ala Ser Leu Thr Asn Gly Tyr Lys Cys Asp Lys Cys Lys Ser Gly Thr
       210                 215                 220
   Ser Arg Ser Lys Lys Lys Trp Ile Trp Lys Lys Ser Ser Gly Asn Glu
   225                 230                 235                 240
   Glu Gly Leu Gln Glu Glu Tyr Ala Asn Thr Ile Gly Leu Pro Pro Arg
                   245                 250                 255
   Thr Gln Ser Leu Tyr Leu Gly Asn Leu Pro Lys Leu Glu Asn Val Cys
               260                 265                 270
   Glu Asp Val Lys Asp Ile Asn Phe Asp Thr Lys Glu Lys Phe Leu Ala
           275                 280                 285
   Gly Cys Leu Ile Val Ser Phe His Glu Gly Lys Asn Leu Lys Lys Arg
       290                 295                 300
   Tyr Pro Gln Asn Lys Asn Ser Gly Asn Lys Glu Asn Leu Cys Lys Ala
   305                 310                 315                 320
   Leu Glu Tyr Ser Phe Ala Asp Tyr Gly Asp Leu Ile Lys Gly Thr Ser
                   325                 330                 335
   Ile Trp Asp Asn Glu Tyr Thr Lys Asp Leu Glu Leu Asn Leu Gln Asn
               340                 345                 350
   Asn Phe Gly Lys Leu Phe Gly Lys Tyr Ile Lys Lys Asn Asn Thr Ala
           355                 360                 365
   Glu Gln Asp Thr Ser Tyr Ser Ser Leu Asp Glu Leu Arg Glu Ser Trp
       370                 375                 380
   Trp Asn Thr Asn Lys Lys Tyr Ile Trp Thr Ala Met Lys His Gly Ala
   385                 390                 395                 400
   Glu Met Asn Ile Thr Thr Cys Asn Ala Asp Gly Ser Val Thr Gly Ser
                   405                 410                 415
   Gly Ser Ser Cys Asp Asp Ile Pro Thr Ile Asp Leu Ile Pro Gln Tyr
               420                 425                 430
   Leu Arg Phe Leu Gln Glu Trp Val Glu Asn Phe Cys Glu Gln Arg Gln
           435                 440                 445
   Ala Lys Val Lys Asp Val Ile Thr Asn Cys Lys Ser Cys Lys Glu Ser
       450                 455                 460
   Gly Asn Lys Cys Lys Thr Glu Cys Lys Thr Lys Cys Lys Asp Glu Cys
   465                 470                 475                 480
   Glu Lys Tyr Lys Lys Phe Ile Glu Ala Cys Gly Thr Ala Gly Gly Gly
                   485                 490                 495
   Ile Gly Thr Ala Gly Ser Pro Trp Ser Lys Arg Trp Asp Gln Ile Tyr
               500                 505                 510
   Lys Arg Tyr Ser Lys His Ile Glu Asp Ala Lys Arg Asn Arg Lys Ala
           515                 520                 525
   Gly Thr Lys Asn Cys Gly Thr Ser Ser Thr Thr Asn Ala Ala Ala Ser
       530                 535                 540
   Thr Asp Glu Asn Lys Cys Val Gln Ser Asp Ile Asp Ser Phe Phe Lys
   545                 550                 555                 560
   His Leu Ile Asp Ile Gly Leu Thr Thr Pro Ser Ser Tyr Leu Ser Asn
                   565                 570                 575
   Val Leu Asp Asp Asn Ile Cys Gly Ala Asp Lys Ala Pro Trp Thr Thr
               580                 585                 590
   Tyr Thr Thr Tyr Thr Thr Thr Glu Lys Cys Asn Lys Glu Arg Asp Lys
   595 600 605
   Ser Lys Ser Gln Ser Ser Asp Thr Leu Val Val Val Asn Val Pro Ser
   610 615 620
   Pro Leu Gly Asn Thr Pro Tyr Arg Tyr Lys Tyr Ala Cys Gln Cys Lys
   625 630 635 640
   Ile Pro Thr Asn Glu Glu Thr Cys Asp Asp Arg Lys Glu Tyr Met Asn
   645 650 655
   Gln Trp Ser Cys Gly Ser Ala Arg Thr Met Lys Arg Gly Tyr Lys Asn
   660 665 670
   Asp Asn Tyr Glu Leu Cys Lys Tyr Asn Gly Val Asp Val Lys Pro Thr
   675 680 685
   Thr Val Arg Ser Asn Ser Ser Lys Leu Asp Arg His Arg Gln Pro Arg
   690 695 700
   Gly Trp Glu Gln Leu Tyr Pro Thr Gly Ala Glu Phe Leu Gly Asp Gly
   705 710 715 720
   Gly Asp Ile Ser Phe Ser Thr Arg Gly Thr Gln Asn Trp Thr Val Glu
   725 730 735
   Arg Leu Leu Gln Ala His Arg Gln Leu Glu Glu Arg Gly Tyr Val Phe
   740 745 750
   Val Gly Tyr His Gly Thr Phe Leu Glu Ala Ala Gln Ser Ile Val Phe
   755 760 765
   Gly Gly Val Arg Ala Arg Ser Gln Asp Leu Asp Ala Ile Trp Arg Gly
   770 775 780
   Phe Tyr Ile Ala Gly Asp Pro Ala Leu Ala Tyr Gly Tyr Ala Gln Asp
   785 790 795 800
   Gln Glu Pro Asp Ala Arg Gly Arg Ile Arg Asn Gly Ala Leu Leu Arg
   805 810 815
   Val Tyr Val Pro Arg Ser Ser Leu Pro Gly Phe Tyr Arg Thr Ser Leu
   820 825 830
   Thr Leu Ala Ala Pro Glu Ala Ala Gly Glu Val Glu Arg Leu Ile Gly
   835 840 845
   His Pro Leu Pro Leu Arg Leu Asp Ala Ile Thr Gly Pro Glu Glu Glu
   850 855 860
   Gly Gly Arg Leu Glu Thr Ile Leu Gly Trp Pro Leu Ala Glu Arg Thr
   865 870 875 880
   Val Val Ile Pro Ser Ala Ile Pro Thr Asp Pro Arg Asn Val Gly Gly
   885 890 895
   Asp Leu Asp Pro Ser Ser Ile Pro Asp Lys Glu Gln Ala Ile Ser Ala
   900 905 910
   Leu Pro Asp Tyr Ala Ser Gln Pro Gly Lys Pro Pro Arg Lys Asp Glu
   915 920 925
   Leu
<210> 61
   <211> 1045
   <212> PRT
   <213> Artificial
<220>
   <223> Artificial construct
<400> 61
   Arg Pro Asp Phe Cys Leu Glu Pro Pro Tyr Thr Gly Pro Cys Lys Ala
   1               5                   10                  15
   Arg Ile Ile Arg Tyr Phe Tyr Asn Ala Lys Ala Gly Leu Cys Gln Thr
               20                  25                  30
   Phe Val Tyr Gly Gly Cys Arg Ala Lys Arg Asn Asn Phe Lys Ser Ala
           35                  40                  45
   Glu Asp Cys Met Arg Thr Cys Gly Gly Ala Asn Tyr Ile Lys Gly Asp
       50                  55                  60
   Pro Tyr Phe Ala Glu Tyr Ala Thr Lys Leu Ser Phe Ile Leu Asn Pro
   65                  70                  75                  80
   Ser Asp Ala Asn Asn Pro Ser Gly Glu Thr Ala Asn His Asn Asp Glu
                   85                  90                  95
   Ala Cys Asn Cys Asn Glu Ser Gly Ile Ser Ser Val Gly Gln Ala Gln
               100                 105                 110
   Thr Ser Gly Pro Ser Ser Asn Lys Thr Cys Ile Thr His Ser Ser Ile
   115 120 125
   Lys Thr Asn Lys Lys Lys Glu Cys Lys Asp Val Lys Leu Gly Val Arg
   130 135 140
   Glu Asn Asp Lys Asp Leu Lys Ile Cys Val Ile Glu Asp Thr Ser Leu
   145 150 155 160
   Ser Gly Val Asp Asn Cys Cys Cys Gln Asp Leu Leu Gly Ile Leu Gln
   165 170 175
   Glu Asn Cys Ser Asp Asn Lys Arg Gly Ser Ser Ser Asn Asp Ser Cys
   180 185 190
   Asp Asn Lys Asn Gln Asp Glu Cys Gln Lys Lys Leu Glu Lys Val Phe
   195 200 205
   Ala Ser Leu Thr Asn Gly Tyr Lys Cys Asp Lys Cys Lys Ser Gly Thr
   210 215 220
   Ser Arg Ser Lys Lys Lys Trp Ile Trp Lys Lys Ser Ser Gly Asn Glu
   225 230 235 240
   Glu Gly Leu Gln Glu Glu Tyr Ala Asn Thr Ile Gly Leu Pro Pro Arg
   245 250 255
   Thr Gln Ser Leu Tyr Leu Gly Asn Leu Pro Lys Leu Glu Asn Val Cys
   260 265 270
   Glu Asp Val Lys Asp Ile Asn Phe Asp Thr Lys Glu Lys Phe Leu Ala
   275 280 285
   Gly Cys Leu Ile Val Ser Phe His Glu Gly Lys Asn Leu Lys Lys Arg
   290 295 300
   Tyr Pro Gln Asn Lys Asn Ser Gly Asn Lys Glu Asn Leu Cys Lys Ala
   305 310 315 320
   Leu Glu Tyr Ser Phe Ala Asp Tyr Gly Asp Leu Ile Lys Gly Thr Ser
   325 330 335
   Ile Trp Asp Asn Glu Tyr Thr Lys Asp Leu Glu Leu Asn Leu Gln Asn
   340 345 350
   Asn Phe Gly Lys Leu Phe Gly Lys Tyr Ile Lys Lys Asn Asn Thr Ala
   355 360 365
   Glu Gln Asp Thr Ser Tyr Ser Ser Leu Asp Glu Leu Arg Glu Ser Trp
   370 375 380
   Trp Asn Thr Asn Lys Lys Tyr Ile Trp Thr Ala Met Lys His Gly Ala
   385 390 395 400
   Glu Met Asn Ile Thr Thr Cys Asn Ala Asp Gly Ser Val Thr Gly Ser
   405 410 415
   Gly Ser Ser Cys Asp Asp Ile Pro Thr Ile Asp Leu Ile Pro Gln Tyr
   420 425 430
   Leu Arg Phe Leu Gln Glu Trp Val Glu Asn Phe Cys Glu Gln Arg Gln
   435 440 445
   Ala Lys Val Lys Asp Val Ile Thr Asn Cys Lys Ser Cys Lys Glu Ser
   450 455 460
   Gly Asn Lys Cys Lys Thr Glu Cys Lys Thr Lys Cys Lys Asp Glu Cys
   465 470 475 480
   Glu Lys Tyr Lys Lys Phe Ile Glu Ala Cys Gly Thr Ala Gly Gly Gly
   485 490 495
   Ile Gly Thr Ala Gly Ser Pro Trp Ser Lys Arg Trp Asp Gln Ile Tyr
   500 505 510
   Lys Arg Tyr Ser Lys His Ile Glu Asp Ala Lys Arg Asn Arg Lys Ala
   515 520 525
   Gly Thr Lys Asn Cys Gly Thr Ser Ser Thr Thr Asn Ala Ala Ala Ser
   530 535 540
   Thr Asp Glu Asn Lys Cys Val Gln Ser Asp Ile Asp Ser Phe Phe Lys
   545 550 555 560
   His Leu Ile Asp Ile Gly Leu Thr Thr Pro Ser Ser Tyr Leu Ser Asn
   565 570 575
   Val Leu Asp Asp Asn Ile Cys Gly Ala Asp Lys Ala Pro Trp Thr Thr
   580 585 590
   Tyr Thr Thr Tyr Thr Thr Thr Glu Lys Cys Asn Lys Glu Arg Asp Lys
   595 600 605
   Ser Lys Ser Gln Ser Ser Asp Thr Leu Val Val Val Asn Val Pro Ser
       610                 615                 620
   Pro Leu Gly Asn Thr Pro Tyr Arg Tyr Lys Tyr Ala Cys Gln Cys Lys
   625                 630                 635                 640
   Ile Pro Thr Asn Glu Glu Thr Cys Asp Asp Arg Lys Glu Tyr Met Asn
                   645                 650                 655
   Gln Trp Ser Cys Gly Ser Ala Arg Thr Met Lys Arg Gly Tyr Lys Asn
               660                 665                 670
   Asp Asn Tyr Glu Leu Cys Lys Tyr Asn Gly Val Asp Val Lys Pro Thr
           675                 680                 685
   Thr Val Arg Ser Asn Ser Ser Lys Leu Asp Pro Glu Gly Gly Ser Leu
       690                 695                 700
   Ala Ala Leu Thr Ala His Gln Ala Cys His Leu Pro Leu Glu Thr Phe
   705                 710                 715                 720
   Thr Arg His Arg Gln Pro Arg Gly Trp Glu Gln Leu Glu Gln Cys Gly
                   725                 730                 735
   Tyr Pro Val Gln Arg Leu Val Ala Leu Tyr Leu Ala Ala Arg Leu Ser
               740                 745                 750
   Trp Asn Gln Val Asp Gln Val Ile Arg Asn Ala Leu Ala Ser Pro Gly
           755                 760                 765
   Ser Gly Gly Asp Leu Gly Glu Ala Ile Arg Glu Gln Pro Glu Gln Ala
       770                 775                 780
   Arg Leu Ala Leu Thr Leu Ala Ala Ala Glu Ser Glu Arg Phe Val Arg
   785                 790                 795                 800
   Gln Gly Thr Gly Asn Asp Glu Ala Gly Ala Ala Asn Gly Pro Ala Asp
                   805                 810                 815
   Ser Gly Asp Ala Leu Leu Glu Arg Asn Tyr Pro Thr Gly Ala Glu Phe
               820                 825                 830
   Leu Gly Asp Gly Gly Asp Ile Ser Phe Ser Thr Arg Gly Thr Gln Asn
           835                 840                 845
   Trp Thr Val Glu Arg Leu Leu Gln Ala His Arg Gln Leu Glu Glu Arg
       850                 855                 860
   Gly Tyr Val Phe Val Gly Tyr His Gly Thr Phe Leu Glu Ala Ala Gln
   865 870 875 880
   Ser Ile Val Phe Gly Gly Val Arg Ala Arg Ser Gln Asp Leu Asp Ala
   885 890 895
   Ile Trp Arg Gly Phe Tyr Ile Ala Gly Asp Pro Ala Leu Ala Tyr Gly
   900 905 910
   Tyr Ala Gln Asp Gln Glu Pro Asp Ala Arg Gly Arg Ile Arg Asn Gly
   915 920 925
   Ala Leu Leu Arg Val Tyr Val Pro Arg Ser Ser Leu Pro Gly Phe Tyr
   930 935 940
   Arg Thr Ser Leu Thr Leu Ala Ala Pro Glu Ala Ala Gly Glu Val Glu
   945 950 955 960
   Arg Leu Ile Gly His Pro Leu Pro Leu Arg Leu Asp Ala Ile Thr Gly
   965 970 975
   Pro Glu Glu Glu Gly Gly Arg Leu Glu Thr Ile Leu Gly Trp Pro Leu
   980 985 990
   Ala Glu Arg Thr Val Val Ile Pro Ser Ala Ile Pro Thr Asp Pro Arg
   995 1000 1005
   Asn Val Gly Gly Asp Leu Asp Pro Ser Ser Ile Pro Asp Lys Glu
   1010 1015 1020
   Gln Ala Ile Ser Ala Leu Pro Asp Tyr Ala Ser Gln Pro Gly Lys
   1025 1030 1035
   Pro Pro Arg Lys Asp Glu Leu
   1040 1045
<210> 62
   <211> 987
   <212> PRT
   <213> Artificial
<220>
   <223> Artificial construct
<400> 62
   Asn Tyr Ile Lys Gly Asp Pro Tyr Phe Ala Glu Tyr Ala Thr Lys Leu
   1 5 10 15
   Ser Phe Ile Leu Asn Pro Ser Asp Ala Asn Asn Pro Ser Gly Glu Thr
   20 25 30
   Ala Asn His Asn Asp Glu Ala Cys Asn Cys Asn Glu Ser Gly Ile Ser
   35 40 45
   Ser Val Gly Gln Ala Gln Thr Ser Gly Pro Ser Ser Asn Lys Thr Cys
   50 55 60
   Ile Thr His Ser Ser Ile Lys Thr Asn Lys Lys Lys Glu Cys Lys Asp
   65 70 75 80
   Val Lys Leu Gly Val Arg Glu Asn Asp Lys Asp Leu Lys Ile Cys Val
   85 90 95
   Ile Glu Asp Thr Ser Leu Ser Gly Val Asp Asn Cys Cys Cys Gln Asp
   100 105 110
   Leu Leu Gly Ile Leu Gln Glu Asn Cys Ser Asp Asn Lys Arg Gly Ser
   115 120 125
   Ser Ser Asn Asp Ser Cys Asp Asn Lys Asn Gln Asp Glu Cys Gln Lys
   130 135 140
   Lys Leu Glu Lys Val Phe Ala Ser Leu Thr Asn Gly Tyr Lys Cys Asp
   145 150 155 160
   Lys Cys Lys Ser Gly Thr Ser Arg Ser Lys Lys Lys Trp Ile Trp Lys
   165 170 175
   Lys Ser Ser Gly Asn Glu Glu Gly Leu Gln Glu Glu Tyr Ala Asn Thr
   180 185 190
   Ile Gly Leu Pro Pro Arg Thr Gln Ser Leu Tyr Leu Gly Asn Leu Pro
   195 200 205
   Lys Leu Glu Asn Val Cys Glu Asp Val Lys Asp Ile Asn Phe Asp Thr
   210 215 220
   Lys Glu Lys Phe Leu Ala Gly Cys Leu Ile Val Ser Phe His Glu Gly
   225 230 235 240
   Lys Asn Leu Lys Lys Arg Tyr Pro Gln Asn Lys Asn Ser Gly Asn Lys
   245 250 255
   Glu Asn Leu Cys Lys Ala Leu Glu Tyr Ser Phe Ala Asp Tyr Gly Asp
   260 265 270
   Leu Ile Lys Gly Thr Ser Ile Trp Asp Asn Glu Tyr Thr Lys Asp Leu
           275                 280                 285
   Glu Leu Asn Leu Gln Asn Asn Phe Gly Lys Leu Phe Gly Lys Tyr Ile
       290                 295                 300
   Lys Lys Asn Asn Thr Ala Glu Gln Asp Thr Ser Tyr Ser Ser Leu Asp
   305                 310                 315                 320
   Glu Leu Arg Glu Ser Trp Trp Asn Thr Asn Lys Lys Tyr Ile Trp Thr
                   325                 330                 335
   Ala Met Lys His Gly Ala Glu Met Asn Ile Thr Thr Cys Asn Ala Asp
               340                 345                 350
   Gly Ser Val Thr Gly Ser Gly Ser Ser Cys Asp Asp Ile Pro Thr Ile
           355                 360                 365
   Asp Leu Ile Pro Gln Tyr Leu Arg Phe Leu Gln Glu Trp Val Glu Asn
       370                 375                 380
   Phe Cys Glu Gln Arg Gln Ala Lys Val Lys Asp Val Ile Thr Asn Cys
   385                 390                 395                 400
   Lys Ser Cys Lys Glu Ser Gly Asn Lys Cys Lys Thr Glu Cys Lys Thr
                   405                 410                 415
   Lys Cys Lys Asp Glu Cys Glu Lys Tyr Lys Lys Phe Ile Glu Ala Cys
               420                 425                 430
   Gly Thr Ala Gly Gly Gly Ile Gly Thr Ala Gly Ser Pro Trp Ser Lys
           435                 440                 445
   Arg Trp Asp Gln Ile Tyr Lys Arg Tyr Ser Lys His Ile Glu Asp Ala
       450                 455                 460
   Lys Arg Asn Arg Lys Ala Gly Thr Lys Asn Cys Gly Thr Ser Ser Thr
   465                 470                 475                 480
   Thr Asn Ala Ala Ala Ser Thr Asp Glu Asn Lys Cys Val Gln Ser Asp
                   485                 490                 495
   Ile Asp Ser Phe Phe Lys His Leu Ile Asp Ile Gly Leu Thr Thr Pro
               500                 505                 510
   Ser Ser Tyr Leu Ser Asn Val Leu Asp Asp Asn Ile Cys Gly Ala Asp
           515                 520                 525
   Lys Ala Pro Trp Thr Thr Tyr Thr Thr Tyr Thr Thr Thr Glu Lys Cys
       530                 535                 540
   Asn Lys Glu Arg Asp Lys Ser Lys Ser Gln Ser Ser Asp Thr Leu Val
   545                 550                 555                 560
   Val Val Asn Val Pro Ser Pro Leu Gly Asn Thr Pro Tyr Arg Tyr Lys
                   565                 570                 575
   Tyr Ala Cys Gln Cys Lys Ile Pro Thr Asn Glu Glu Thr Cys Asp Asp
               580                 585                 590
   Arg Lys Glu Tyr Met Asn Gln Trp Ser Cys Gly Ser Ala Arg Thr Met
           595                 600                 605
   Lys Arg Gly Tyr Lys Asn Asp Asn Tyr Glu Leu Cys Lys Tyr Asn Gly
       610                 615                 620
   Val Asp Val Lys Pro Thr Thr Val Arg Ser Asn Ser Ser Lys Leu Asp
   625                 630                 635                 640
   Pro Glu Gly Gly Ser Leu Ala Ala Leu Thr Ala His Gln Ala Cys His
                   645                 650                 655
   Leu Pro Leu Glu Thr Phe Thr Arg His Arg Gln Pro Arg Gly Trp Glu
               660                 665                 670
   Gln Leu Glu Gln Cys Gly Tyr Pro Val Gln Arg Leu Val Ala Leu Tyr
           675                 680                 685
   Leu Ala Ala Arg Leu Ser Trp Asn Gln Val Asp Gln Val Ile Arg Asn
       690                 695                 700
   Ala Leu Ala Ser Pro Gly Ser Gly Gly Asp Leu Gly Glu Ala Ile Arg
   705                 710                 715                 720
   Glu Gln Pro Glu Gln Ala Arg Leu Ala Leu Thr Leu Ala Ala Ala Glu
                   725                 730                 735
   Ser Glu Arg Phe Val Arg Gln Gly Thr Gly Asn Asp Glu Ala Gly Ala
               740                 745                 750
   Ala Asn Gly Pro Ala Asp Ser Gly Asp Ala Leu Leu Glu Arg Asn Tyr
           755                 760                 765
   Pro Thr Gly Ala Glu Phe Leu Gly Asp Gly Gly Asp Ile Ser Phe Ser
   770 775 780
   Thr Arg Gly Thr Gln Asn Trp Thr Val Glu Arg Leu Leu Gln Ala His
   785 790 795 800
   Arg Gln Leu Glu Glu Arg Gly Tyr Val Phe Val Gly Tyr His Gly Thr
   805 810 815
   Phe Leu Glu Ala Ala Gln Ser Ile Val Phe Gly Gly Val Arg Ala Arg
   820 825 830
   Ser Gln Asp Leu Asp Ala Ile Trp Arg Gly Phe Tyr Ile Ala Gly Asp
   835 840 845
   Pro Ala Leu Ala Tyr Gly Tyr Ala Gln Asp Gln Glu Pro Asp Ala Arg
   850 855 860
   Gly Arg Ile Arg Asn Gly Ala Leu Leu Arg Val Tyr Val Pro Arg Ser
   865 870 875 880
   Ser Leu Pro Gly Phe Tyr Arg Thr Ser Leu Thr Leu Ala Ala Pro Glu
   885 890 895
   Ala Ala Gly Glu Val Glu Arg Leu Ile Gly His Pro Leu Pro Leu Arg
   900 905 910
   Leu Asp Ala Ile Thr Gly Pro Glu Glu Glu Gly Gly Arg Leu Glu Thr
   915 920 925
   Ile Leu Gly Trp Pro Leu Ala Glu Arg Thr Val Val Ile Pro Ser Ala
   930 935 940
   Ile Pro Thr Asp Pro Arg Asn Val Gly Gly Asp Leu Asp Pro Ser Ser
   945 950 955 960
   Ile Pro Asp Lys Glu Gln Ala Ile Ser Ala Leu Pro Asp Tyr Ala Ser
   965 970 975
   Gln Pro Gly Lys Pro Pro Arg Lys Asp Glu Leu
   980 985
<210> 63
   <211> 871
   <212> PRT
   <213> Artificial
<220>
   <223> Artificial construct
<210> 64
   <211> 1258
   <212> PRT
   <213> Artificial
<220>
   <223> Artificial construct
<400> 64
   Arg Pro Asp Phe Cys Leu Glu Pro Pro Tyr Thr Gly Pro Cys Lys Ala
   1 5 10 15
   Arg Ile Ile Arg Tyr Phe Tyr Asn Ala Lys Ala Gly Leu Cys Gln Thr
   20 25 30
   Phe Val Tyr Gly Gly Cys Arg Ala Lys Arg Asn Asn Phe Lys Ser Ala
   35 40 45
   Glu Asp Cys Met Arg Thr Cys Gly Gly Ala Asn His Ser Asp Ser Gly
   50 55 60
   Lys Tyr Asp Pro Cys Glu Lys Lys Leu Pro Pro Tyr Asp Asp Asn Asp
   65 70 75 80
   Gln Trp Lys Cys Gln Gln Asn Ser Ser Asp Gly Ser Gly Lys Pro Glu
                   85                  90                  95
   Asn Ile Cys Val Pro Pro Arg Arg Glu Arg Leu Cys Thr Tyr Asn Leu
               100                 105                 110
   Glu Asn Leu Lys Phe Asp Lys Ile Arg Asp Asn Asn Ala Phe Leu Ala
           115                 120                 125
   Asp Val Leu Leu Thr Ala Arg Asn Glu Gly Glu Lys Ile Val Gln Asn
       130                 135                 140
   His Pro Asp Thr Asn Ser Ser Asn Val Cys Asn Ala Leu Glu Arg Ser
   145                 150                 155                 160
   Phe Ala Asp Leu Ala Asp Ile Ile Arg Gly Thr Asp Gln Trp Lys Gly
                   165                 170                 175
   Thr Asn Ser Asn Leu Glu Lys Asn Leu Lys Gln Met Phe Ala Lys Ile
               180                 185                 190
   Arg Glu Asn Asp Lys Val Leu Gln Asp Lys Tyr Pro Lys Asp Gln Lys
           195                 200                 205
   Tyr Thr Lys Leu Arg Glu Ala Trp Trp Asn Ala Asn Arg Gln Lys Val
       210                 215                 220
   Trp Glu Val Ile Thr Cys Gly Ala Arg Ser Asn Asp Leu Leu Ile Lys
   225                 230                 235                 240
   Arg Gly Trp Arg Thr Ser Gly Lys Ser Asp Arg Lys Lys Asn Phe Glu
                   245                 250                 255
   Leu Cys Arg Lys Cys Gly His Tyr Glu Lys Glu Val Pro Thr Lys Leu
               260                 265                 270
   Asp Tyr Val Pro Gln Phe Leu Arg Trp Leu Thr Glu Trp Ile Glu Asp
           275                 280                 285
   Phe Tyr Arg Glu Lys Gln Asn Leu Ile Asp Asp Met Glu Arg His Arg
       290                 295                 300
   Glu Glu Cys Thr Arg Glu Asp His Lys Ser Lys Glu Gly Thr Ser Tyr
   305                 310                 315                 320
   Cys Ser Thr Cys Lys Asp Lys Cys Lys Lys Tyr Cys Glu Cys Val Lys
                   325                 330                 335
   Lys Trp Lys Thr Glu Trp Glu Asn Gln Glu Asn Lys Tyr Lys Asp Leu
   340 345 350
   Tyr Glu Gln Asn Lys Asn Lys Thr Ser Gln Lys Asn Thr Ser Arg Tyr
   355 360 365
   Asp Asp Tyr Val Lys Asp Phe Phe Glu Lys Leu Glu Ala Asn Tyr Ser
   370 375 380
   Ser Leu Glu Asn Tyr Ile Lys Gly Asp Pro Tyr Phe Ala Glu Tyr Ala
   385 390 395 400
   Thr Lys Leu Ser Phe Ile Leu Asn Pro Ser Asp Ala Asn Asn Pro Ser
   405 410 415
   Gly Glu Thr Ala Asn His Asn Asp Glu Ala Cys Asn Cys Asn Glu Ser
   420 425 430
   Gly Ile Ser Ser Val Gly Gln Ala Gln Thr Ser Gly Pro Ser Ser Asn
   435 440 445
   Lys Thr Cys Ile Thr His Ser Ser Ile Lys Thr Asn Lys Lys Lys Glu
   450 455 460
   Cys Lys Asp Val Lys Leu Gly Val Arg Glu Asn Asp Lys Asp Leu Lys
   465 470 475 480
   Ile Cys Val Ile Glu Asp Thr Ser Leu Ser Gly Val Asp Asn Cys Cys
   485 490 495
   Cys Gln Asp Leu Leu Gly Ile Leu Gln Glu Asn Cys Ser Asp Asn Lys
   500 505 510
   Arg Gly Ser Ser Ser Asn Asp Ser Cys Asp Asn Lys Asn Gln Asp Glu
   515 520 525
   Cys Gln Lys Lys Leu Glu Lys Val Phe Ala Ser Leu Thr Asn Gly Tyr
   530 535 540
   Lys Cys Asp Lys Cys Lys Ser Gly Thr Ser Arg Ser Lys Lys Lys Trp
   545 550 555 560
   Ile Trp Lys Lys Ser Ser Gly Asn Glu Glu Gly Leu Gln Glu Glu Tyr
   565 570 575
   Ala Asn Thr Ile Gly Leu Pro Pro Arg Thr Gln Ser Leu Tyr Leu Gly
   580 585 590
   Asn Leu Pro Lys Leu Glu Asn Val Cys Glu Asp Val Lys Asp Ile Asn
   595 600 605
   Phe Asp Thr Lys Glu Lys Phe Leu Ala Gly Cys Leu Ile Val Ser Phe
   610 615 620
   His Glu Gly Lys Asn Leu Lys Lys Arg Tyr Pro Gln Asn Lys Asn Ser
   625 630 635 640
   Gly Asn Lys Glu Asn Leu Cys Lys Ala Leu Glu Tyr Ser Phe Ala Asp
   645 650 655
   Tyr Gly Asp Leu Ile Lys Gly Thr Ser Ile Trp Asp Asn Glu Tyr Thr
   660 665 670
   Lys Asp Leu Glu Leu Asn Leu Gln Asn Asn Phe Gly Lys Leu Phe Gly
   675 680 685
   Lys Tyr Ile Lys Lys Asn Asn Thr Ala Glu Gln Asp Thr Ser Tyr Ser
   690 695 700
   Ser Leu Asp Glu Leu Arg Glu Ser Trp Trp Asn Thr Asn Lys Lys Tyr
   705 710 715 720
   Ile Trp Thr Ala Met Lys His Gly Ala Glu Met Asn Ile Thr Thr Cys
   725 730 735
   Asn Ala Asp Gly Ser Val Thr Gly Ser Gly Ser Ser Cys Asp Asp Ile
   740 745 750
   Pro Thr Ile Asp Leu Ile Pro Gln Tyr Leu Arg Phe Leu Gln Glu Trp
   755 760 765
   Val Glu Asn Phe Cys Glu Gln Arg Gln Ala Lys Val Lys Asp Val Ile
   770 775 780
   Thr Asn Cys Lys Ser Cys Lys Glu Ser Gly Asn Lys Cys Lys Thr Glu
   785 790 795 800
   Cys Lys Thr Lys Cys Lys Asp Glu Cys Glu Lys Tyr Lys Lys Phe Ile
   805 810 815
   Glu Ala Cys Gly Thr Ala Gly Gly Gly Ile Gly Thr Ala Gly Ser Pro
   820 825 830
   Trp Ser Lys Arg Trp Asp Gln Ile Tyr Lys Arg Tyr Ser Lys His Ile
   835 840 845
   Glu Asp Ala Lys Arg Asn Arg Lys Ala Gly Thr Lys Asn Cys Gly Thr
   850 855 860
   Ser Ser Thr Thr Asn Ala Ala Ala Ser Thr Asp Glu Asn Lys Cys Val
   865 870 875 880
   Gln Ser Asp Ile Asp Ser Phe Phe Lys His Leu Ile Asp Ile Gly Leu
   885 890 895
   Thr Thr Pro Ser Ser Tyr Leu Ser Asn Val Leu Asp Asp Asn Ile Cys
   900 905 910
   Gly Ala Asp Lys Ala Pro Trp Thr Thr Tyr Thr Thr Tyr Thr Thr Thr
   915 920 925
   Glu Lys Cys Asn Lys Glu Arg Asp Lys Ser Lys Ser Gln Ser Ser Asp
   930 935 940
   Thr Leu Val Val Val Asn Val Pro Ser Pro Leu Gly Asn Thr Pro Tyr
   945 950 955 960
   Arg Tyr Lys Tyr Ala Cys Gln Cys Lys Ile Pro Thr Asn Glu Glu Thr
   965 970 975
   Cys Asp Asp Arg Lys Glu Tyr Met Asn Gln Trp Ser Cys Gly Ser Ala
   980 985 990
   Arg Thr Met Lys Arg Gly Tyr Lys Asn Asp Asn Tyr Glu Leu Cys Lys
   995 1000 1005
   Tyr Asn Gly Val Asp Val Lys Pro Thr Thr Val Arg Ser Asn Ser
   1010 1015 1020
   Ser Lys Leu Asp Arg His Arg Gln Pro Arg Gly Trp Glu Gln Leu
   1025 1030 1035
   Tyr Pro Thr Gly Ala Glu Phe Leu Gly Asp Gly Gly Asp Ile Ser
   1040 1045 1050
   Phe Ser Thr Arg Gly Thr Gln Asn Trp Thr Val Glu Arg Leu Leu
   1055 1060 1065
   Gln Ala His Arg Gln Leu Glu Glu Arg Gly Tyr Val Phe Val Gly
   1070 1075 1080
   Tyr His Gly Thr Phe Leu Glu Ala Ala Gln Ser Ile Val Phe Gly
   1085 1090 1095
   Gly Val Arg Ala Arg Ser Gln Asp Leu Asp Ala Ile Trp Arg Gly
   1100 1105 1110
   Phe Tyr Ile Ala Gly Asp Pro Ala Leu Ala Tyr Gly Tyr Ala Gln
   1115 1120 1125
   Asp Gln Glu Pro Asp Ala Arg Gly Arg Ile Arg Asn Gly Ala Leu
   1130 1135 1140
   Leu Arg Val Tyr Val Pro Arg Ser Ser Leu Pro Gly Phe Tyr Arg
   1145 1150 1155
   Thr Ser Leu Thr Leu Ala Ala Pro Glu Ala Ala Gly Glu Val Glu
   1160 1165 1170
   Arg Leu Ile Gly His Pro Leu Pro Leu Arg Leu Asp Ala Ile Thr
   1175 1180 1185
   Gly Pro Glu Glu Glu Gly Gly Arg Leu Glu Thr Ile Leu Gly Trp
   1190 1195 1200
   Pro Leu Ala Glu Arg Thr Val Val Ile Pro Ser Ala Ile Pro Thr
   1205 1210 1215
   Asp Pro Arg Asn Val Gly Gly Asp Leu Asp Pro Ser Ser Ile Pro
   1220 1225 1230
   Asp Lys Glu Gln Ala Ile Ser Ala Leu Pro Asp Tyr Ala Ser Gln
   1235 1240 1245
   Pro Gly Lys Pro Pro Arg Lys Asp Glu Leu
   1250 1255
<210> 65
   <211> 1374
   <212> PRT
   <213> Artificial
<220>
   <223> Artificial construct
<400> 65
   Arg Pro Asp Phe Cys Leu Glu Pro Pro Tyr Thr Gly Pro Cys Lys Ala
   1               5                   10                  15
   Arg Ile Ile Arg Tyr Phe Tyr Asn Ala Lys Ala Gly Leu Cys Gln Thr
   20 25 30
   Phe Val Tyr Gly Gly Cys Arg Ala Lys Arg Asn Asn Phe Lys Ser Ala
   35 40 45
   Glu Asp Cys Met Arg Thr Cys Gly Gly Ala Asn His Ser Asp Ser Gly
   50 55 60
   Lys Tyr Asp Pro Cys Glu Lys Lys Leu Pro Pro Tyr Asp Asp Asn Asp
   65 70 75 80
   Gln Trp Lys Cys Gln Gln Asn Ser Ser Asp Gly Ser Gly Lys Pro Glu
   85 90 95
   Asn Ile Cys Val Pro Pro Arg Arg Glu Arg Leu Cys Thr Tyr Asn Leu
   100 105 110
   Glu Asn Leu Lys Phe Asp Lys Ile Arg Asp Asn Asn Ala Phe Leu Ala
   115 120 125
   Asp Val Leu Leu Thr Ala Arg Asn Glu Gly Glu Lys Ile Val Gln Asn
   130 135 140
   His Pro Asp Thr Asn Ser Ser Asn Val Cys Asn Ala Leu Glu Arg Ser
   145 150 155 160
   Phe Ala Asp Leu Ala Asp Ile Ile Arg Gly Thr Asp Gln Trp Lys Gly
   165 170 175
   Thr Asn Ser Asn Leu Glu Lys Asn Leu Lys Gln Met Phe Ala Lys Ile
   180 185 190
   Arg Glu Asn Asp Lys Val Leu Gln Asp Lys Tyr Pro Lys Asp Gln Lys
   195 200 205
   Tyr Thr Lys Leu Arg Glu Ala Trp Trp Asn Ala Asn Arg Gln Lys Val
   210 215 220
   Trp Glu Val Ile Thr Cys Gly Ala Arg Ser Asn Asp Leu Leu Ile Lys
   225 230 235 240
   Arg Gly Trp Arg Thr Ser Gly Lys Ser Asp Arg Lys Lys Asn Phe Glu
   245 250 255
   Leu Cys Arg Lys Cys Gly His Tyr Glu Lys Glu Val Pro Thr Lys Leu
   260 265 270
   Asp Tyr Val Pro Gln Phe Leu Arg Trp Leu Thr Glu Trp Ile Glu Asp
           275                 280                 285
   Phe Tyr Arg Glu Lys Gln Asn Leu Ile Asp Asp Met Glu Arg His Arg
       290                 295                 300
   Glu Glu Cys Thr Arg Glu Asp His Lys Ser Lys Glu Gly Thr Ser Tyr
   305                 310                 315                 320
   Cys Ser Thr Cys Lys Asp Lys Cys Lys Lys Tyr Cys Glu Cys Val Lys
                   325                 330                 335
   Lys Trp Lys Thr Glu Trp Glu Asn Gln Glu Asn Lys Tyr Lys Asp Leu
               340                 345                 350
   Tyr Glu Gln Asn Lys Asn Lys Thr Ser Gln Lys Asn Thr Ser Arg Tyr
           355                 360                 365
   Asp Asp Tyr Val Lys Asp Phe Phe Glu Lys Leu Glu Ala Asn Tyr Ser
       370                 375                 380
   Ser Leu Glu Asn Tyr Ile Lys Gly Asp Pro Tyr Phe Ala Glu Tyr Ala
   385                 390                 395                 400
   Thr Lys Leu Ser Phe Ile Leu Asn Pro Ser Asp Ala Asn Asn Pro Ser
                   405                 410                 415
   Gly Glu Thr Ala Asn His Asn Asp Glu Ala Cys Asn Cys Asn Glu Ser
               420                 425                 430
   Gly Ile Ser Ser Val Gly Gln Ala Gln Thr Ser Gly Pro Ser Ser Asn
           435                 440                 445
   Lys Thr Cys Ile Thr His Ser Ser Ile Lys Thr Asn Lys Lys Lys Glu
       450                 455                 460
   Cys Lys Asp Val Lys Leu Gly Val Arg Glu Asn Asp Lys Asp Leu Lys
   465                 470                 475                 480
   Ile Cys Val Ile Glu Asp Thr Ser Leu Ser Gly Val Asp Asn Cys Cys
                   485                 490                 495
   Cys Gln Asp Leu Leu Gly Ile Leu Gln Glu Asn Cys Ser Asp Asn Lys
               500                 505                 510
   Arg Gly Ser Ser Ser Asn Asp Ser Cys Asp Asn Lys Asn Gln Asp Glu
           515                 520                 525
   Cys Gln Lys Lys Leu Glu Lys Val Phe Ala Ser Leu Thr Asn Gly Tyr
   530 535 540
   Lys Cys Asp Lys Cys Lys Ser Gly Thr Ser Arg Ser Lys Lys Lys Trp
   545 550 555 560
   Ile Trp Lys Lys Ser Ser Gly Asn Glu Glu Gly Leu Gln Glu Glu Tyr
   565 570 575
   Ala Asn Thr Ile Gly Leu Pro Pro Arg Thr Gln Ser Leu Tyr Leu Gly
   580 585 590
   Asn Leu Pro Lys Leu Glu Asn Val Cys Glu Asp Val Lys Asp Ile Asn
   595 600 605
   Phe Asp Thr Lys Glu Lys Phe Leu Ala Gly Cys Leu Ile Val Ser Phe
   610 615 620
   His Glu Gly Lys Asn Leu Lys Lys Arg Tyr Pro Gln Asn Lys Asn Ser
   625 630 635 640
   Gly Asn Lys Glu Asn Leu Cys Lys Ala Leu Glu Tyr Ser Phe Ala Asp
   645 650 655
   Tyr Gly Asp Leu Ile Lys Gly Thr Ser Ile Trp Asp Asn Glu Tyr Thr
   660 665 670
   Lys Asp Leu Glu Leu Asn Leu Gln Asn Asn Phe Gly Lys Leu Phe Gly
   675 680 685
   Lys Tyr Ile Lys Lys Asn Asn Thr Ala Glu Gln Asp Thr Ser Tyr Ser
   690 695 700
   Ser Leu Asp Glu Leu Arg Glu Ser Trp Trp Asn Thr Asn Lys Lys Tyr
   705 710 715 720
   Ile Trp Thr Ala Met Lys His Gly Ala Glu Met Asn Ile Thr Thr Cys
   725 730 735
   Asn Ala Asp Gly Ser Val Thr Gly Ser Gly Ser Ser Cys Asp Asp Ile
   740 745 750
   Pro Thr Ile Asp Leu Ile Pro Gln Tyr Leu Arg Phe Leu Gln Glu Trp
   755 760 765
   Val Glu Asn Phe Cys Glu Gln Arg Gln Ala Lys Val Lys Asp Val Ile
   770 775 780
   Thr Asn Cys Lys Ser Cys Lys Glu Ser Gly Asn Lys Cys Lys Thr Glu
   785                 790                 795                 800
   Cys Lys Thr Lys Cys Lys Asp Glu Cys Glu Lys Tyr Lys Lys Phe Ile
                   805                 810                 815
   Glu Ala Cys Gly Thr Ala Gly Gly Gly Ile Gly Thr Ala Gly Ser Pro
               820                 825                 830
   Trp Ser Lys Arg Trp Asp Gln Ile Tyr Lys Arg Tyr Ser Lys His Ile
           835                 840                 845
   Glu Asp Ala Lys Arg Asn Arg Lys Ala Gly Thr Lys Asn Cys Gly Thr
       850                 855                 860
   Ser Ser Thr Thr Asn Ala Ala Ala Ser Thr Asp Glu Asn Lys Cys Val
   865                 870                 875                 880
   Gln Ser Asp Ile Asp Ser Phe Phe Lys His Leu Ile Asp Ile Gly Leu
                   885                 890                 895
   Thr Thr Pro Ser Ser Tyr Leu Ser Asn Val Leu Asp Asp Asn Ile Cys
               900                 905                 910
   Gly Ala Asp Lys Ala Pro Trp Thr Thr Tyr Thr Thr Tyr Thr Thr Thr
           915                 920                 925
   Glu Lys Cys Asn Lys Glu Arg Asp Lys Ser Lys Ser Gln Ser Ser Asp
       930                 935                 940
   Thr Leu Val Val Val Asn Val Pro Ser Pro Leu Gly Asn Thr Pro Tyr
   945                 950                 955                 960
   Arg Tyr Lys Tyr Ala Cys Gln Cys Lys Ile Pro Thr Asn Glu Glu Thr
                   965                 970                 975
   Cys Asp Asp Arg Lys Glu Tyr Met Asn Gln Trp Ser Cys Gly Ser Ala
               980                 985                 990
   Arg Thr Met Lys Arg Gly Tyr Lys  Asn Asp Asn Tyr Glu  Leu Cys Lys
           995                 1000                1005
   Tyr Asn  Gly Val Asp Val Lys  Pro Thr Thr Val Arg  Ser Asn Ser
       1010                 1015                 1020
   Ser Lys  Leu Asp Pro Glu Gly  Gly Ser Leu Ala Ala  Leu Thr Ala
           1025                1030                1035
   His Gln  Ala Cys His Leu Pro  Leu Glu Thr Phe Thr  Arg His Arg
       1040                1045                1050
   Gln Pro  Arg Gly Trp Glu Gln  Leu Glu Gln Cys Gly  Tyr Pro Val
       1055                1060                1065
   Gln Arg  Leu Val Ala Leu Tyr  Leu Ala Ala Arg Leu  Ser Trp Asn
       1070                1075                1080
   Gln Val  Asp Gln Val Ile Arg  Asn Ala Leu Ala Ser  Pro Gly Ser
       1085                1090                1095
   Gly Gly  Asp Leu Gly Glu Ala  Ile Arg Glu Gln Pro  Glu Gln Ala
       1100                1105                1110
   Arg Leu  Ala Leu Thr Leu Ala  Ala Ala Glu Ser Glu  Arg Phe Val
       1115                1120                1125
   Arg Gln  Gly Thr Gly Asn Asp  Glu Ala Gly Ala Ala  Asn Gly Pro
       1130                1135                1140
   Ala Asp  Ser Gly Asp Ala Leu  Leu Glu Arg Asn Tyr  Pro Thr Gly
       1145                1150                1155
   Ala Glu  Phe Leu Gly Asp Gly  Gly Asp Ile Ser Phe  Ser Thr Arg
       1160                1165                1170
   Gly Thr  Gln Asn Trp Thr Val  Glu Arg Leu Leu Gln  Ala His Arg
       1175                1180                1185
   Gln Leu  Glu Glu Arg Gly Tyr  Val Phe Val Gly Tyr  His Gly Thr
       1190                1195                1200
   Phe Leu  Glu Ala Ala Gln Ser  Ile Val Phe Gly Gly  Val Arg Ala
       1205                1210                1215
   Arg Ser  Gln Asp Leu Asp Ala  Ile Trp Arg Gly Phe  Tyr Ile Ala
       1220                1225                1230
   Gly Asp  Pro Ala Leu Ala Tyr  Gly Tyr Ala Gln Asp  Gln Glu Pro
       1235                1240                1245
   Asp Ala  Arg Gly Arg Ile Arg  Asn Gly Ala Leu Leu  Arg Val Tyr
       1250                1255                1260
   Val Pro Arg Ser Ser Leu Pro Gly Phe Tyr Arg Thr Ser Leu Thr
       1265                1270                1275
   Leu Ala Ala Pro Glu Ala Ala Gly Glu Val Glu Arg Leu Ile Gly
       1280                1285                1290
   His Pro Leu Pro Leu Arg Leu Asp Ala Ile Thr Gly Pro Glu Glu
       1295                1300                1305
   Glu Gly Gly Arg Leu Glu Thr Ile Leu Gly Trp Pro Leu Ala Glu
       1310                1315                1320
   Arg Thr Val Val Ile Pro Ser Ala Ile Pro Thr Asp Pro Arg Asn
       1325                1330                1335
   Val Gly Gly Asp Leu Asp Pro Ser Ser Ile Pro Asp Lys Glu Gln
       1340                1345                1350
   Ala Ile Ser Ala Leu Pro Asp Tyr Ala Ser Gln Pro Gly Lys Pro
       1355                1360                1365
   Pro Arg Lys Asp Glu Leu
       1370
<210> 66
   <211> 1200
   <212> PRT
   <213> Artificial
<220>
   <223> Artificial construct
<400> 66
   Asn His Ser Asp Ser Gly Lys Tyr Asp Pro Cys Glu Lys Lys Leu Pro
   1               5                   10                  15
   Pro Tyr Asp Asp Asn Asp Gln Trp Lys Cys Gln Gln Asn Ser Ser Asp
               20                  25                  30
   Gly Ser Gly Lys Pro Glu Asn Ile Cys Val Pro Pro Arg Arg Glu Arg
           35                  40                  45
   Leu Cys Thr Tyr Asn Leu Glu Asn Leu Lys Phe Asp Lys Ile Arg Asp
       50                  55                  60
   Asn Asn Ala Phe Leu Ala Asp Val Leu Leu Thr Ala Arg Asn Glu Gly
   65                  70                  75                  80
   Glu Lys Ile Val Gln Asn His Pro Asp Thr Asn Ser Ser Asn Val Cys
   85 90 95
   Asn Ala Leu Glu Arg Ser Phe Ala Asp Leu Ala Asp Ile Ile Arg Gly
   100 105 110
   Thr Asp Gln Trp Lys Gly Thr Asn Ser Asn Leu Glu Lys Asn Leu Lys
   115 120 125
   Gln Met Phe Ala Lys Ile Arg Glu Asn Asp Lys Val Leu Gln Asp Lys
   130 135 140
   Tyr Pro Lys Asp Gln Lys Tyr Thr Lys Leu Arg Glu Ala Trp Trp Asn
   145 150 155 160
   Ala Asn Arg Gln Lys Val Trp Glu Val Ile Thr Cys Gly Ala Arg Ser
   165 170 175
   Asn Asp Leu Leu Ile Lys Arg Gly Trp Arg Thr Ser Gly Lys Ser Asp
   180 185 190
   Arg Lys Lys Asn Phe Glu Leu Cys Arg Lys Cys Gly His Tyr Glu Lys
   195 200 205
   Glu Val Pro Thr Lys Leu Asp Tyr Val Pro Gln Phe Leu Arg Trp Leu
   210 215 220
   Thr Glu Trp Ile Glu Asp Phe Tyr Arg Glu Lys Gln Asn Leu Ile Asp
   225 230 235 240
   Asp Met Glu Arg His Arg Glu Glu Cys Thr Arg Glu Asp His Lys Ser
   245 250 255
   Lys Glu Gly Thr Ser Tyr Cys Ser Thr Cys Lys Asp Lys Cys Lys Lys
   260 265 270
   Tyr Cys Glu Cys Val Lys Lys Trp Lys Thr Glu Trp Glu Asn Gln Glu
   275 280 285
   Asn Lys Tyr Lys Asp Leu Tyr Glu Gln Asn Lys Asn Lys Thr Ser Gln
   290 295 300
   Lys Asn Thr Ser Arg Tyr Asp Asp Tyr Val Lys Asp Phe Phe Glu Lys
   305 310 315 320
   Leu Glu Ala Asn Tyr Ser Ser Leu Glu Asn Tyr Ile Lys Gly Asp Pro
   325 330 335
   Tyr Phe Ala Glu Tyr Ala Thr Lys Leu Ser Phe Ile Leu Asn Pro Ser
               340                 345                 350
   Asp Ala Asn Asn Pro Ser Gly Glu Thr Ala Asn His Asn Asp Glu Ala
           355                 360                 365
   Cys Asn Cys Asn Glu Ser Gly Ile Ser Ser Val Gly Gln Ala Gln Thr
       370                 375                 380
   Ser Gly Pro Ser Ser Asn Lys Thr Cys Ile Thr His Ser Ser Ile Lys
   385                 390                 395                 400
   Thr Asn Lys Lys Lys Glu Cys Lys Asp Val Lys Leu Gly Val Arg Glu
                   405                 410                 415
   Asn Asp Lys Asp Leu Lys Ile Cys Val Ile Glu Asp Thr Ser Leu Ser
               420                 425                 430
   Gly Val Asp Asn Cys Cys Cys Gln Asp Leu Leu Gly Ile Leu Gln Glu
           435                 440                 445
   Asn Cys Ser Asp Asn Lys Arg Gly Ser Ser Ser Asn Asp Ser Cys Asp
       450                 455                 460
   Asn Lys Asn Gln Asp Glu Cys Gln Lys Lys Leu Glu Lys Val Phe Ala
   465                 470                 475                 480
   Ser Leu Thr Asn Gly Tyr Lys Cys Asp Lys Cys Lys Ser Gly Thr Ser
                   485                 490                 495
   Arg Ser Lys Lys Lys Trp Ile Trp Lys Lys Ser Ser Gly Asn Glu Glu
               500                 505                 510
   Gly Leu Gln Glu Glu Tyr Ala Asn Thr Ile Gly Leu Pro Pro Arg Thr
           515                 520                 525
   Gln Ser Leu Tyr Leu Gly Asn Leu Pro Lys Leu Glu Asn Val Cys Glu
       530                 535                 540
   Asp Val Lys Asp Ile Asn Phe Asp Thr Lys Glu Lys Phe Leu Ala Gly
   545                 550                 555                 560
   Cys Leu Ile Val Ser Phe His Glu Gly Lys Asn Leu Lys Lys Arg Tyr
                   565                 570                 575
   Pro Gln Asn Lys Asn Ser Gly Asn Lys Glu Asn Leu Cys Lys Ala Leu
               580                 585                 590
   Glu Tyr Ser Phe Ala Asp Tyr Gly Asp Leu Ile Lys Gly Thr Ser Ile
   595 600 605
   Trp Asp Asn Glu Tyr Thr Lys Asp Leu Glu Leu Asn Leu Gln Asn Asn
   610 615 620
   Phe Gly Lys Leu Phe Gly Lys Tyr Ile Lys Lys Asn Asn Thr Ala Glu
   625 630 635 640
   Gln Asp Thr Ser Tyr Ser Ser Leu Asp Glu Leu Arg Glu Ser Trp Trp
   645 650 655
   Asn Thr Asn Lys Lys Tyr Ile Trp Thr Ala Met Lys His Gly Ala Glu
   660 665 670
   Met Asn Ile Thr Thr Cys Asn Ala Asp Gly Ser Val Thr Gly Ser Gly
   675 680 685
   Ser Ser Cys Asp Asp Ile Pro Thr Ile Asp Leu Ile Pro Gln Tyr Leu
   690 695 700
   Arg Phe Leu Gln Glu Trp Val Glu Asn Phe Cys Glu Gln Arg Gln Ala
   705 710 715 720
   Lys Val Lys Asp Val Ile Thr Asn Cys Lys Ser Cys Lys Glu Ser Gly
   725 730 735
   Asn Lys Cys Lys Thr Glu Cys Lys Thr Lys Cys Lys Asp Glu Cys Glu
   740 745 750
   Lys Tyr Lys Lys Phe Ile Glu Ala Cys Gly Thr Ala Gly Gly Gly Ile
   755 760 765
   Gly Thr Ala Gly Ser Pro Trp Ser Lys Arg Trp Asp Gln Ile Tyr Lys
   770 775 780
   Arg Tyr Ser Lys His Ile Glu Asp Ala Lys Arg Asn Arg Lys Ala Gly
   785 790 795 800
   Thr Lys Asn Cys Gly Thr Ser Ser Thr Thr Asn Ala Ala Ala Ser Thr
   805 810 815
   Asp Glu Asn Lys Cys Val Gln Ser Asp Ile Asp Ser Phe Phe Lys His
   820 825 830
   Leu Ile Asp Ile Gly Leu Thr Thr Pro Ser Ser Tyr Leu Ser Asn Val
   835 840 845
   Leu Asp Asp Asn Ile Cys Gly Ala Asp Lys Ala Pro Trp Thr Thr Tyr
   850 855 860
   Thr Thr Tyr Thr Thr Thr Glu Lys Cys Asn Lys Glu Arg Asp Lys Ser
   865 870 875 880
   Lys Ser Gln Ser Ser Asp Thr Leu Val Val Val Asn Val Pro Ser Pro
   885 890 895
   Leu Gly Asn Thr Pro Tyr Arg Tyr Lys Tyr Ala Cys Gln Cys Lys Ile
   900 905 910
   Pro Thr Asn Glu Glu Thr Cys Asp Asp Arg Lys Glu Tyr Met Asn Gln
   915 920 925
   Trp Ser Cys Gly Ser Ala Arg Thr Met Lys Arg Gly Tyr Lys Asn Asp
   930 935 940
   Asn Tyr Glu Leu Cys Lys Tyr Asn Gly Val Asp Val Lys Pro Thr Thr
   945 950 955 960
   Val Arg Ser Asn Ser Ser Lys Leu Asp Arg His Arg Gln Pro Arg Gly
   965 970 975
   Trp Glu Gln Leu Tyr Pro Thr Gly Ala Glu Phe Leu Gly Asp Gly Gly
   980 985 990
   Asp Ile Ser Phe Ser Thr Arg Gly Thr Gln Asn Trp Thr Val Glu Arg
   995 1000 1005
   Leu Leu Gln Ala His Arg Gln Leu Glu Glu Arg Gly Tyr Val Phe
   1010 1015 1020
   Val Gly Tyr His Gly Thr Phe Leu Glu Ala Ala Gln Ser Ile Val
   1025 1030 1035
   Phe Gly Gly Val Arg Ala Arg Ser Gln Asp Leu Asp Ala Ile Trp
   1040 1045 1050
   Arg Gly Phe Tyr Ile Ala Gly Asp Pro Ala Leu Ala Tyr Gly Tyr
   1055 1060 1065
   Ala Gln Asp Gln Glu Pro Asp Ala Arg Gly Arg Ile Arg Asn Gly
   1070 1075 1080
   Ala Leu Leu Arg Val Tyr Val Pro Arg Ser Ser Leu Pro Gly Phe
   1085 1090 1095
   Tyr Arg Thr Ser Leu Thr Leu Ala Ala Pro Glu Ala Ala Gly Glu
   1100 1105 1110
   Val Glu Arg Leu Ile Gly His Pro Leu Pro Leu Arg Leu Asp Ala
   1115 1120 1125
   Ile Thr Gly Pro Glu Glu Glu Gly Gly Arg Leu Glu Thr Ile Leu
   1130 1135 1140
   Gly Trp Pro Leu Ala Glu Arg Thr Val Val Ile Pro Ser Ala Ile
   1145 1150 1155
   Pro Thr Asp Pro Arg Asn Val Gly Gly Asp Leu Asp Pro Ser Ser
   1160 1165 1170
   Ile Pro Asp Lys Glu Gln Ala Ile Ser Ala Leu Pro Asp Tyr Ala
   1175 1180 1185
   Ser Gln Pro Gly Lys Pro Pro Arg Lys Asp Glu Leu
   1190 1195 1200
<210> 67
   <211> 1316
   <212> PRT
   <213> Artificial
<220>
   <223> Artificial construct
<400> 67
   Asn His Ser Asp Ser Gly Lys Tyr Asp Pro Cys Glu Lys Lys Leu Pro
   1               5                   10                  15
   Pro Tyr Asp Asp Asn Asp Gln Trp Lys Cys Gln Gln Asn Ser Ser Asp
               20                  25                  30
   Gly Ser Gly Lys Pro Glu Asn Ile Cys Val Pro Pro Arg Arg Glu Arg
           35                  40                  45
   Leu Cys Thr Tyr Asn Leu Glu Asn Leu Lys Phe Asp Lys Ile Arg Asp
       50                  55                  60
   Asn Asn Ala Phe Leu Ala Asp Val Leu Leu Thr Ala Arg Asn Glu Gly
   65                  70                  75                  80
   Glu Lys Ile Val Gln Asn His Pro Asp Thr Asn Ser Ser Asn Val Cys
                   85                  90                  95
   Asn Ala Leu Glu Arg Ser Phe Ala Asp Leu Ala Asp Ile Ile Arg Gly
               100                 105                 110
   Thr Asp Gln Trp Lys Gly Thr Asn Ser Asn Leu Glu Lys Asn Leu Lys
           115                 120                 125
   Gln Met Phe Ala Lys Ile Arg Glu Asn Asp Lys Val Leu Gln Asp Lys
       130                 135                 140
   Tyr Pro Lys Asp Gln Lys Tyr Thr Lys Leu Arg Glu Ala Trp Trp Asn
   145                 150                 155                 160
   Ala Asn Arg Gln Lys Val Trp Glu Val Ile Thr Cys Gly Ala Arg Ser
                   165                 170                 175
   Asn Asp Leu Leu Ile Lys Arg Gly Trp Arg Thr Ser Gly Lys Ser Asp
               180                 185                 190
   Arg Lys Lys Asn Phe Glu Leu Cys Arg Lys Cys Gly His Tyr Glu Lys
           195                 200                 205
   Glu Val Pro Thr Lys Leu Asp Tyr Val Pro Gln Phe Leu Arg Trp Leu
       210                 215                 220
   Thr Glu Trp Ile Glu Asp Phe Tyr Arg Glu Lys Gln Asn Leu Ile Asp
   225                 230                 235                 240
   Asp Met Glu Arg His Arg Glu Glu Cys Thr Arg Glu Asp His Lys Ser
                   245                 250                 255
   Lys Glu Gly Thr Ser Tyr Cys Ser Thr Cys Lys Asp Lys Cys Lys Lys
               260                 265                 270
   Tyr Cys Glu Cys Val Lys Lys Trp Lys Thr Glu Trp Glu Asn Gln Glu
           275                 280                 285
   Asn Lys Tyr Lys Asp Leu Tyr Glu Gln Asn Lys Asn Lys Thr Ser Gln
       290                 295                 300
   Lys Asn Thr Ser Arg Tyr Asp Asp Tyr Val Lys Asp Phe Phe Glu Lys
   305                 310                 315                 320
   Leu Glu Ala Asn Tyr Ser Ser Leu Glu Asn Tyr Ile Lys Gly Asp Pro
                   325                 330                 335
   Tyr Phe Ala Glu Tyr Ala Thr Lys Leu Ser Phe Ile Leu Asn Pro Ser
   340 345 350
   Asp Ala Asn Asn Pro Ser Gly Glu Thr Ala Asn His Asn Asp Glu Ala
   355 360 365
   Cys Asn Cys Asn Glu Ser Gly Ile Ser Ser Val Gly Gln Ala Gln Thr
   370 375 380
   Ser Gly Pro Ser Ser Asn Lys Thr Cys Ile Thr His Ser Ser Ile Lys
   385 390 395 400
   Thr Asn Lys Lys Lys Glu Cys Lys Asp Val Lys Leu Gly Val Arg Glu
   405 410 415
   Asn Asp Lys Asp Leu Lys Ile Cys Val Ile Glu Asp Thr Ser Leu Ser
   420 425 430
   Gly Val Asp Asn Cys Cys Cys Gln Asp Leu Leu Gly Ile Leu Gln Glu
   435 440 445
   Asn Cys Ser Asp Asn Lys Arg Gly Ser Ser Ser Asn Asp Ser Cys Asp
   450 455 460
   Asn Lys Asn Gln Asp Glu Cys Gln Lys Lys Leu Glu Lys Val Phe Ala
   465 470 475 480
   Ser Leu Thr Asn Gly Tyr Lys Cys Asp Lys Cys Lys Ser Gly Thr Ser
   485 490 495
   Arg Ser Lys Lys Lys Trp Ile Trp Lys Lys Ser Ser Gly Asn Glu Glu
   500 505 510
   Gly Leu Gln Glu Glu Tyr Ala Asn Thr Ile Gly Leu Pro Pro Arg Thr
   515 520 525
   Gln Ser Leu Tyr Leu Gly Asn Leu Pro Lys Leu Glu Asn Val Cys Glu
   530 535 540
   Asp Val Lys Asp Ile Asn Phe Asp Thr Lys Glu Lys Phe Leu Ala Gly
   545 550 555 560
   Cys Leu Ile Val Ser Phe His Glu Gly Lys Asn Leu Lys Lys Arg Tyr
   565 570 575
   Pro Gln Asn Lys Asn Ser Gly Asn Lys Glu Asn Leu Cys Lys Ala Leu
   580 585 590
   Glu Tyr Ser Phe Ala Asp Tyr Gly Asp Leu Ile Lys Gly Thr Ser Ile
           595                 600                 605
   Trp Asp Asn Glu Tyr Thr Lys Asp Leu Glu Leu Asn Leu Gln Asn Asn
       610                 615                 620
   Phe Gly Lys Leu Phe Gly Lys Tyr Ile Lys Lys Asn Asn Thr Ala Glu
   625                 630                 635                 640
   Gln Asp Thr Ser Tyr Ser Ser Leu Asp Glu Leu Arg Glu Ser Trp Trp
                   645                 650                 655
   Asn Thr Asn Lys Lys Tyr Ile Trp Thr Ala Met Lys His Gly Ala Glu
               660                 665                 670
   Met Asn Ile Thr Thr Cys Asn Ala Asp Gly Ser Val Thr Gly Ser Gly
           675                 680                 685
   Ser Ser Cys Asp Asp Ile Pro Thr Ile Asp Leu Ile Pro Gln Tyr Leu
       690                 695                 700
   Arg Phe Leu Gln Glu Trp Val Glu Asn Phe Cys Glu Gln Arg Gln Ala
   705                 710                 715                 720
   Lys Val Lys Asp Val Ile Thr Asn Cys Lys Ser Cys Lys Glu Ser Gly
                   725                 730                 735
   Asn Lys Cys Lys Thr Glu Cys Lys Thr Lys Cys Lys Asp Glu Cys Glu
               740                 745                 750
   Lys Tyr Lys Lys Phe Ile Glu Ala Cys Gly Thr Ala Gly Gly Gly Ile
           755                 760                 765
   Gly Thr Ala Gly Ser Pro Trp Ser Lys Arg Trp Asp Gln Ile Tyr Lys
       770                 775                 780
   Arg Tyr Ser Lys His Ile Glu Asp Ala Lys Arg Asn Arg Lys Ala Gly
   785                 790                 795                 800
   Thr Lys Asn Cys Gly Thr Ser Ser Thr Thr Asn Ala Ala Ala Ser Thr
                   805                 810                 815
   Asp Glu Asn Lys Cys Val Gln Ser Asp Ile Asp Ser Phe Phe Lys His
               820                 825                 830
   Leu Ile Asp Ile Gly Leu Thr Thr Pro Ser Ser Tyr Leu Ser Asn Val
           835                 840                 845
   Leu Asp Asp Asn Ile Cys Gly Ala Asp Lys Ala Pro Trp Thr Thr Tyr
       850                 855                 860
   Thr Thr Tyr Thr Thr Thr Glu Lys Cys Asn Lys Glu Arg Asp Lys Ser
   865                 870                 875                 880
   Lys Ser Gln Ser Ser Asp Thr Leu Val Val Val Asn Val Pro Ser Pro
                   885                 890                 895
   Leu Gly Asn Thr Pro Tyr Arg Tyr Lys Tyr Ala Cys Gln Cys Lys Ile
               900                 905                 910
   Pro Thr Asn Glu Glu Thr Cys Asp Asp Arg Lys Glu Tyr Met Asn Gln
           915                 920                 925
   Trp Ser Cys Gly Ser Ala Arg Thr Met Lys Arg Gly Tyr Lys Asn Asp
       930                 935                 940
   Asn Tyr Glu Leu Cys Lys Tyr Asn Gly Val Asp Val Lys Pro Thr Thr
   945                 950                 955                 960
   Val Arg Ser Asn Ser Ser Lys Leu Asp Pro Glu Gly Gly Ser Leu Ala
                   965                 970                 975
   Ala Leu Thr Ala His Gln Ala Cys His Leu Pro Leu Glu Thr Phe Thr
               980                 985                 990
   Arg His Arg Gln Pro Arg Gly Trp Glu Gln Leu Glu Gln Cys Gly Tyr
           995                 1000                 1005
   Pro Val Gln Arg Leu Val Ala Leu Tyr Leu Ala Ala Arg Leu Ser
       1010                 1015                 1020
   Trp Asn Gln Val Asp Gln Val Ile Arg Asn Ala Leu Ala Ser Pro
       1025                 1030                 1035
   Gly Ser Gly Gly Asp Leu Gly Glu Ala Ile Arg Glu Gln Pro Glu
       1040                 1045                 1050
   Gln Ala Arg Leu Ala Leu Thr Leu Ala Ala Ala Glu Ser Glu Arg
       1055                 1060                 1065
   Phe Val Arg Gln Gly Thr Gly Asn Asp Glu Ala Gly Ala Ala Asn
       1070                 1075                 1080
   Gly Pro Ala Asp Ser Gly Asp Ala Leu Leu Glu Arg Asn Tyr Pro
       1085                 1090                 1095
   Thr Gly  Ala Glu Phe Leu Gly  Asp Gly Gly Asp Ile  Ser Phe Ser
       1100                 1105                 1110
   Thr Arg  Gly Thr Gln Asn Trp  Thr Val Glu Arg Leu  Leu Gln Ala
       1115                 1120                 1125
   His Arg  Gln Leu Glu Glu Arg  Gly Tyr Val Phe Val  Gly Tyr His
       1130                 1135                 1140
   Gly Thr  Phe Leu Glu Ala Ala  Gln Ser Ile Val Phe  Gly Gly Val
       1145                 1150                 1155
   Arg Ala  Arg Ser Gln Asp Leu  Asp Ala Ile Trp Arg  Gly Phe Tyr
       1160                 1165                 1170
   Ile Ala  Gly Asp Pro Ala Leu  Ala Tyr Gly Tyr Ala  Gln Asp Gln
       1175                 1180                 1185
   Glu Pro  Asp Ala Arg Gly Arg  Ile Arg Asn Gly Ala  Leu Leu Arg
       1190                 1195                 1200
   Val Tyr  Val Pro Arg Ser Ser  Leu Pro Gly Phe Tyr  Arg Thr Ser
       1205                 1210                 1215
   Leu Thr  Leu Ala Ala Pro Glu  Ala Ala Gly Glu Val  Glu Arg Leu
       1220                 1225                 1230
   Ile Gly  His Pro Leu Pro Leu  Arg Leu Asp Ala Ile  Thr Gly Pro
       1235                 1240                 1245
   Glu Glu  Glu Gly Gly Arg Leu  Glu Thr Ile Leu Gly  Trp Pro Leu
       1250                 1255                 1260
   Ala Glu  Arg Thr Val Val Ile  Pro Ser Ala Ile Pro  Thr Asp Pro
       1265                 1270                 1275
   Arg Asn  Val Gly Gly Asp Leu  Asp Pro Ser Ser Ile  Pro Asp Lys
       1280                 1285                 1290
   Glu Gln  Ala Ile Ser Ala Leu  Pro Asp Tyr Ala Ser  Gln Pro Gly
       1295                 1300                 1305
   Lys Pro  Pro Arg Lys Asp Glu  Leu
       1310                 1315
<210> 68
   <211> 964
   <212> PRT
   <213> Artificial
<220>
   <223> Artificial construct
<400> 68
   Leu Ser Phe Ile Leu Asn Ser Ser Asp Ala Asn Asn Pro Ser Glu Lys
   1               5                   10                  15
   Ile Gln Lys Asn Asn Asp Glu Val Cys Asn Cys Asn Glu Ser Gly Ile
               20                  25                  30
   Ala Ser Val Glu Gln Glu Gln Ile Ser Asp Pro Ser Ser Asn Lys Thr
           35                  40                  45
   Cys Ile Thr His Ser Ser Ile Lys Ala Asn Lys Lys Lys Val Cys Lys
       50                  55                  60
   His Val Lys Leu Gly Val Arg Glu Asn Asp Lys Asp Leu Arg Val Cys
   65                  70                  75                  80
   Val Ile Glu His Thr Ser Leu Ser Gly Val Glu Asn Cys Cys Cys Gln
                   85                  90                  95
   Asp Phe Leu Arg Ile Leu Gln Glu Asn Cys Ser Asp Asn Lys Ser Gly
               100                 105                 110
   Ser Ser Ser Asn Gly Ser Cys Asn Asn Lys Asn Gln Glu Ala Cys Glu
           115                 120                 125
   Lys Asn Leu Glu Lys Val Leu Ala Ser Leu Thr Asn Cys Tyr Lys Cys
       130                 135                 140
   Asp Lys Cys Lys Ser Glu Gln Ser Lys Lys Asn Asn Lys Asn Trp Ile
   145                 150                 155                 160
   Trp Lys Lys Ser Ser Gly Lys Glu Gly Gly Leu Gln Lys Glu Tyr Ala
                   165                 170                 175
   Asn Thr Ile Gly Leu Pro Pro Arg Thr Gln Ser Leu Cys Leu Val Val
               180                 185                 190
   Cys Leu Asp Glu Lys Gly Lys Lys Thr Gln Glu Leu Lys Asn Ile Arg
           195                 200                 205
   Thr Asn Ser Glu Leu Leu Lys Glu Trp Ile Ile Ala Ala Phe His Glu
       210                 215                 220
   Gly Lys Asn Leu Lys Pro Ser His Glu Lys Lys Asn Asp Asp Asn Gly
   225 230 235 240
   Lys Lys Leu Cys Lys Ala Leu Glu Tyr Ser Phe Ala Asp Tyr Gly Asp
   245 250 255
   Leu Ile Lys Gly Thr Ser Ile Trp Asp Asn Glu Tyr Thr Lys Asp Leu
   260 265 270
   Glu Leu Asn Leu Gln Lys Ile Phe Gly Lys Leu Phe Arg Lys Tyr Ile
   275 280 285
   Lys Lys Asn Asn Thr Ala Glu Gln Asp Thr Ser Tyr Ser Ser Leu Asp
   290 295 300
   Glu Leu Arg Glu Ser Trp Trp Asn Thr Asn Lys Lys Tyr Ile Trp Leu
   305 310 315 320
   Ala Met Lys His Gly Ala Gly Met Asn Ser Thr Thr Cys Cys Gly Asp
   325 330 335
   Gly Ser Val Thr Gly Ser Gly Ser Ser Cys Asp Asp Ile Pro Thr Ile
   340 345 350
   Asp Leu Ile Pro Gln Tyr Leu Arg Phe Leu Gln Glu Trp Val Glu His
   355 360 365
   Phe Cys Lys Gln Arg Gln Glu Lys Val Lys Pro Val Ile Glu Asn Cys
   370 375 380
   Lys Ser Cys Lys Glu Ser Gly Gly Thr Cys Asn Gly Glu Cys Lys Thr
   385 390 395 400
   Glu Cys Lys Asn Lys Cys Glu Val Tyr Lys Lys Phe Ile Glu Asp Cys
   405 410 415
   Lys Gly Gly Asp Gly Thr Ala Gly Ser Ser Trp Val Lys Arg Trp Asp
   420 425 430
   Gln Ile Tyr Lys Arg Tyr Ser Lys Tyr Ile Glu Asp Ala Lys Arg Asn
   435 440 445
   Arg Lys Ala Gly Thr Lys Asn Cys Gly Pro Ser Ser Thr Thr Asn Ala
   450 455 460
   Ala Glu Asn Lys Cys Val Gln Ser Asp Ile Asp Ser Phe Phe Lys His
   465 470 475 480
   Leu Ile Asp Ile Gly Leu Thr Thr Pro Ser Ser Tyr Leu Ser Ile Val
   485 490 495
   Leu Asp Asp Asn Ile Cys Gly Ala Asp Lys Ala Pro Trp Thr Thr Tyr
   500 505 510
   Thr Thr Tyr Thr Thr Thr Glu Lys Cys Asn Lys Glu Thr Asp Lys Ser
   515 520 525
   Lys Leu Gln Gln Cys Asn Thr Ala Val Val Val Asn Val Pro Ser Pro
   530 535 540
   Leu Gly Asn Thr Pro His Gly Tyr Lys Tyr Ala Cys Gln Cys Lys Ile
   545 550 555 560
   Pro Thr Asn Glu Glu Thr Cys Asp Asp Arg Lys Glu Tyr Met Asn Gln
   565 570 575
   Trp Ser Cys Gly Ser Ala Arg Thr Met Lys Arg Gly Tyr Lys Asn Asp
   580 585 590
   Asn Tyr Glu Leu Cys Lys Tyr Asn Gly Val Asp Val Lys Pro Thr Thr
   595 600 605
   Val Arg Ser Asn Ser Ser Lys Leu Asp Pro Glu Gly Gly Ser Leu Ala
   610 615 620
   Ala Leu Thr Ala His Gln Ala Cys His Leu Pro Leu Glu Thr Phe Thr
   625 630 635 640
   Arg His Arg Gln Pro Arg Gly Trp Glu Gln Leu Glu Gln Cys Gly Tyr
   645 650 655
   Pro Val Gln Arg Leu Val Ala Leu Tyr Leu Ala Ala Arg Leu Ser Trp
   660 665 670
   Asn Gln Val Asp Gln Val Ile Arg Asn Ala Leu Ala Ser Pro Gly Ser
   675 680 685
   Gly Gly Asp Leu Gly Glu Ala Ile Arg Glu Gln Pro Glu Gln Ala Arg
   690 695 700
   Leu Ala Leu Thr Leu Ala Ala Ala Glu Ser Glu Arg Phe Val Arg Gln
   705 710 715 720
   Gly Thr Gly Asn Asp Glu Ala Gly Ala Ala Asn Gly Pro Ala Asp Ser
   725 730 735
   Gly Asp Ala Leu Leu Glu Arg Asn Tyr Pro Thr Gly Ala Glu Phe Leu
   740 745 750
   Gly Asp Gly Gly Asp Ile Ser Phe Ser Thr Arg Gly Thr Gln Asn Trp
   755 760 765
   Thr Val Glu Arg Leu Leu Gln Ala His Arg Gln Leu Glu Glu Arg Gly
   770 775 780
   Tyr Val Phe Val Gly Tyr His Gly Thr Phe Leu Glu Ala Ala Gln Ser
   785 790 795 800
   Ile Val Phe Gly Gly Val Arg Ala Arg Ser Gln Asp Leu Asp Ala Ile
   805 810 815
   Trp Arg Gly Phe Tyr Ile Ala Gly Asp Pro Ala Leu Ala Tyr Gly Tyr
   820 825 830
   Ala Gln Asp Gln Glu Pro Asp Ala Arg Gly Arg Ile Arg Asn Gly Ala
   835 840 845
   Leu Leu Arg Val Tyr Val Pro Arg Ser Ser Leu Pro Gly Phe Tyr Arg
   850 855 860
   Thr Ser Leu Thr Leu Ala Ala Pro Glu Ala Ala Gly Glu Val Glu Arg
   865 870 875 880
   Leu Ile Gly His Pro Leu Pro Leu Arg Leu Asp Ala Ile Thr Gly Pro
   885 890 895
   Glu Glu Glu Gly Gly Arg Leu Glu Thr Ile Leu Gly Trp Pro Leu Ala
   900 905 910
   Glu Arg Thr Val Val Ile Pro Ser Ala Ile Pro Thr Asp Pro Arg Asn
   915 920 925
   Val Gly Gly Asp Leu Asp Pro Ser Ser Ile Pro Asp Lys Glu Gln Ala
   930 935 940
   Ile Ser Ala Leu Pro Asp Tyr Ala Ser Gln Pro Gly Lys Pro Pro Arg
   945 950 955 960
   Lys Asp Glu Leu
<210> 69
   <211> 848
   <212> PRT
   <213> Artificial
<220>
   <223> Artificial construct
<400> 69
   Leu Ser Phe Ile Leu Asn Ser Ser Asp Ala Asn Asn Pro Ser Glu Lys
   1               5                   10                  15
   Ile Gln Lys Asn Asn Asp Glu Val Cys Asn Cys Asn Glu Ser Gly Ile
               20                  25                  30
   Ala Ser Val Glu Gln Glu Gln Ile Ser Asp Pro Ser Ser Asn Lys Thr
           35                  40                  45
   Cys Ile Thr His Ser Ser Ile Lys Ala Asn Lys Lys Lys Val Cys Lys
       50                  55                  60
   His Val Lys Leu Gly Val Arg Glu Asn Asp Lys Asp Leu Arg Val Cys
   65                  70                  75                  80
   Val Ile Glu His Thr Ser Leu Ser Gly Val Glu Asn Cys Cys Cys Gln
                   85                  90                  95
   Asp Phe Leu Arg Ile Leu Gln Glu Asn Cys Ser Asp Asn Lys Ser Gly
               100                 105                 110
   Ser Ser Ser Asn Gly Ser Cys Asn Asn Lys Asn Gln Glu Ala Cys Glu
           115                 120                 125
   Lys Asn Leu Glu Lys Val Leu Ala Ser Leu Thr Asn Cys Tyr Lys Cys
       130                 135                 140
   Asp Lys Cys Lys Ser Glu Gln Ser Lys Lys Asn Asn Lys Asn Trp Ile
   145                 150                 155                 160
   Trp Lys Lys Ser Ser Gly Lys Glu Gly Gly Leu Gln Lys Glu Tyr Ala
                   165                 170                 175
   Asn Thr Ile Gly Leu Pro Pro Arg Thr Gln Ser Leu Cys Leu Val Val
               180                 185                 190
   Cys Leu Asp Glu Lys Gly Lys Lys Thr Gln Glu Leu Lys Asn Ile Arg
           195                 200                 205
   Thr Asn Ser Glu Leu Leu Lys Glu Trp Ile Ile Ala Ala Phe His Glu
       210                 215                 220
   Gly Lys Asn Leu Lys Pro Ser His Glu Lys Lys Asn Asp Asp Asn Gly
   225                 230                 235                 240
   Lys Lys Leu Cys Lys Ala Leu Glu Tyr Ser Phe Ala Asp Tyr Gly Asp
                   245                 250                 255
   Leu Ile Lys Gly Thr Ser Ile Trp Asp Asn Glu Tyr Thr Lys Asp Leu
               260                 265                 270
   Glu Leu Asn Leu Gln Lys Ile Phe Gly Lys Leu Phe Arg Lys Tyr Ile
           275                 280                 285
   Lys Lys Asn Asn Thr Ala Glu Gln Asp Thr Ser Tyr Ser Ser Leu Asp
       290                 295                 300
   Glu Leu Arg Glu Ser Trp Trp Asn Thr Asn Lys Lys Tyr Ile Trp Leu
   305                 310                 315                 320
   Ala Met Lys His Gly Ala Gly Met Asn Ser Thr Thr Cys Cys Gly Asp
                   325                 330                 335
   Gly Ser Val Thr Gly Ser Gly Ser Ser Cys Asp Asp Ile Pro Thr Ile
               340                 345                 350
   Asp Leu Ile Pro Gln Tyr Leu Arg Phe Leu Gln Glu Trp Val Glu His
           355                 360                 365
   Phe Cys Lys Gln Arg Gln Glu Lys Val Lys Pro Val Ile Glu Asn Cys
       370                 375                 380
   Lys Ser Cys Lys Glu Ser Gly Gly Thr Cys Asn Gly Glu Cys Lys Thr
   385                 390                 395                 400
   Glu Cys Lys Asn Lys Cys Glu Val Tyr Lys Lys Phe Ile Glu Asp Cys
                   405                 410                 415
   Lys Gly Gly Asp Gly Thr Ala Gly Ser Ser Trp Val Lys Arg Trp Asp
               420                 425                 430
   Gln Ile Tyr Lys Arg Tyr Ser Lys Tyr Ile Glu Asp Ala Lys Arg Asn
           435                 440                 445
   Arg Lys Ala Gly Thr Lys Asn Cys Gly Pro Ser Ser Thr Thr Asn Ala
       450                 455                 460
   Ala Glu Asn Lys Cys Val Gln Ser Asp Ile Asp Ser Phe Phe Lys His
   465                 470                 475                 480
   Leu Ile Asp Ile Gly Leu Thr Thr Pro Ser Ser Tyr Leu Ser Ile Val
                   485                 490                 495
   Leu Asp Asp Asn Ile Cys Gly Ala Asp Lys Ala Pro Trp Thr Thr Tyr
               500                 505                 510
   Thr Thr Tyr Thr Thr Thr Glu Lys Cys Asn Lys Glu Thr Asp Lys Ser
           515                 520                 525
   Lys Leu Gln Gln Cys Asn Thr Ala Val Val Val Asn Val Pro Ser Pro
       530                 535                 540
   Leu Gly Asn Thr Pro His Gly Tyr Lys Tyr Ala Cys Gln Cys Lys Ile
   545                 550                 555                 560
   Pro Thr Asn Glu Glu Thr Cys Asp Asp Arg Lys Glu Tyr Met Asn Gln
                   565                 570                 575
   Trp Ser Cys Gly Ser Ala Arg Thr Met Lys Arg Gly Tyr Lys Asn Asp
               580                 585                 590
   Asn Tyr Glu Leu Cys Lys Tyr Asn Gly Val Asp Val Lys Pro Thr Thr
           595                 600                 605
   Val Arg Ser Asn Ser Ser Lys Leu Asp Arg His Arg Gln Pro Arg Gly
       610                 615                 620
   Trp Glu Gln Leu Tyr Pro Thr Gly Ala Glu Phe Leu Gly Asp Gly Gly
   625                 630                 635                 640
   Asp Ile Ser Phe Ser Thr Arg Gly Thr Gln Asn Trp Thr Val Glu Arg
                   645                 650                 655
   Leu Leu Gln Ala His Arg Gln Leu Glu Glu Arg Gly Tyr Val Phe Val
               660                 665                 670
   Gly Tyr His Gly Thr Phe Leu Glu Ala Ala Gln Ser Ile Val Phe Gly
           675                 680                 685
   Gly Val Arg Ala Arg Ser Gln Asp Leu Asp Ala Ile Trp Arg Gly Phe
       690                 695                 700
   Tyr Ile Ala Gly Asp Pro Ala Leu Ala Tyr Gly Tyr Ala Gln Asp Gln
   705                 710                 715                 720
   Glu Pro Asp Ala Arg Gly Arg Ile Arg Asn Gly Ala Leu Leu Arg Val
   725 730 735
   Tyr Val Pro Arg Ser Ser Leu Pro Gly Phe Tyr Arg Thr Ser Leu Thr
   740 745 750
   Leu Ala Ala Pro Glu Ala Ala Gly Glu Val Glu Arg Leu Ile Gly His
   755 760 765
   Pro Leu Pro Leu Arg Leu Asp Ala Ile Thr Gly Pro Glu Glu Glu Gly
   770 775 780
   Gly Arg Leu Glu Thr Ile Leu Gly Trp Pro Leu Ala Glu Arg Thr Val
   785 790 795 800
   Val Ile Pro Ser Ala Ile Pro Thr Asp Pro Arg Asn Val Gly Gly Asp
   805 810 815
   Leu Asp Pro Ser Ser Ile Pro Asp Lys Glu Gln Ala Ile Ser Ala Leu
   820 825 830
   Pro Asp Tyr Ala Ser Gln Pro Gly Lys Pro Pro Arg Lys Asp Glu Leu
   835 840 845
<210> 70
   <211> 808
   <212> PRT
   <213> Artificial
<220>
   <223> Artificial construct
<400> 70
   Asn Tyr Ile Lys Gly Asp Pro Tyr Phe Ala Glu Tyr Ala Thr Lys Leu
   1 5 10 15
   Ser Phe Ile Leu Asn Pro Ser Asp Ala Asn Asn Pro Ser Gly Glu Thr
   20 25 30
   Ala Asn His Asn Asp Glu Ala Cys Asn Cys Asn Glu Ser Gly Ile Ser
   35 40 45
   Ser Val Gly Gln Ala Gln Thr Ser Gly Pro Ser Ser Asn Lys Thr Cys
   50 55 60
   Ile Thr His Ser Ser Ile Lys Thr Asn Lys Lys Lys Glu Cys Lys Asp
   65 70 75 80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Cys Gln Asp
               100                 105                 110
   Leu Leu Gly Ile Leu Gln Glu Asn Cys Ser Asp Asn Lys Arg Gly Ser
           115                 120                 125
   Ser Ser Asn Asp Ser Cys Asp Asn Lys Asn Gln Asp Glu Cys Gln Lys
       130                 135                 140
   Lys Leu Glu Lys Val Phe Ala Ser Leu Thr Asn Gly Tyr Lys Cys Asp
   145                 150                 155                 160
   Lys Cys Lys Ser Gly Thr Ser Arg Ser Lys Lys Lys Trp Ile Trp Lys
                   165                 170                 175
   Lys Ser Ser Gly Asn Glu Glu Gly Leu Gln Glu Glu Tyr Ala Asn Thr
               180                 185                 190
   Ile Gly Leu Pro Pro Arg Thr Gln Ser Leu Tyr Leu Gly Asn Leu Pro
           195                 200                 205
   Lys Leu Glu Asn Val Cys Glu Asp Val Lys Asp Ile Asn Phe Asp Thr
       210                 215                 220
   Lys Glu Lys Phe Leu Ala Gly Cys Leu Ile Val Ser Phe His Glu Gly
   225                 230                 235                 240
   Lys Asn Leu Lys Lys Arg Tyr Pro Gln Asn Lys Asn Ser Gly Asn Lys
                   245                 250                 255
   Glu Asn Leu Cys Lys Ala Leu Glu Tyr Ser Phe Ala Asp Tyr Gly Asp
               260                 265                 270
   Leu Ile Lys Gly Thr Ser Ile Trp Asp Asn Glu Tyr Thr Lys Asp Leu
           275                 280                 285
   Glu Leu Asn Leu Gln Asn Asn Phe Gly Lys Leu Phe Gly Lys Tyr Ile
       290                 295                 300
   Lys Lys Asn Asn Thr Ala Glu Gln Asp Thr Ser Tyr Ser Ser Leu Asp
   305                 310                 315                 320
   Glu Leu Arg Glu Ser Trp Trp Asn Thr Asn Lys Lys Tyr Ile Trp Thr
                   325                 330                 335
   Ala Met Lys His Gly Ala Glu Met Asn Ile Thr Thr Cys Asn Ala Asp
               340                 345                 350
   Gly Ser Val Thr Gly Ser Gly Ser Ser Cys Asp Asp Ile Pro Thr Ile
           355                 360                 365
   Asp Leu Ile Pro Gln Tyr Leu Arg Phe Leu Gln Glu Trp Val Glu Asn
       370                 375                 380
   Phe Cys Glu Gln Arg Gln Ala Lys Val Lys Asp Val Ile Thr Asn Cys
   385                 390                 395                 400
   Lys Ser Cys Lys Glu Ser Gly Asn Lys Cys Lys Thr Glu Cys Lys Thr
                   405                 410                 415
   Lys Cys Lys Asp Glu Cys Glu Lys Tyr Lys Lys Phe Ile Glu Ala Cys
               420                 425                 430
   Gly Thr Ala Gly Gly Gly Ile Gly Thr Ala Gly Ser Pro Trp Ser Lys
           435                 440                 445
   Arg Trp Asp Gln Ile Tyr Lys Arg Tyr Ser Lys His Ile Glu Asp Ala
       450                 455                 460
   Lys Arg Asn Arg Lys Ala Gly Thr Lys Asn Cys Gly Thr Ser Ser Thr
   465                 470                 475                 480
   Thr Asn Ala Ala Ala Ser Thr Asp Glu Asn Lys Cys Val Gln Ser Asp
                   485                 490                 495
   Ile Asp Ser Phe Phe Lys His Leu Ile Asp Ile Gly Leu Thr Thr Pro
               500                 505                 510
   Ser Ser Tyr Leu Ser Asn Val Leu Asp Asp Asn Ile Cys Gly Ala Asp
           515                 520                 525
   Lys Ala Pro Trp Thr Thr Tyr Thr Thr Tyr Thr Thr Thr Glu Lys Cys
       530                 535                 540
   Asn Lys Glu Arg Asp Lys Ser Lys Ser Gln Ser Ser Asp Thr Leu Val
   545                 550                 555                 560
   Val Val Asn Val Pro Ser Pro Leu Gly Asn Thr Pro Tyr Arg Tyr Lys
                   565                 570                 575
   Tyr Arg His Arg Gln Pro Arg Gly Trp Glu Gln Leu Tyr Pro Thr Gly
                   580                 585                 590
   Ala Glu Phe Leu Gly Asp Gly Gly Asp Ile Ser Phe Ser Thr Arg Gly
               595                 600                 605
   Thr Gln Asn Trp Thr Val Glu Arg Leu Leu Gln Ala His Arg Gln Leu
       610                 615                 620
   Glu Glu Arg Gly Tyr Val Phe Val Gly Tyr His Gly Thr Phe Leu Glu
   625                 630                 635                 640
   Ala Ala Gln Ser Ile Val Phe Gly Gly Val Arg Ala Arg Ser Gln Asp
                   645                 650                 655
   Leu Asp Ala Ile Trp Arg Gly Phe Tyr Ile Ala Gly Asp Pro Ala Leu
               660                 665                 670
   Ala Tyr Gly Tyr Ala Gln Asp Gln Glu Pro Asp Ala Arg Gly Arg Ile
           675                 680                 685
   Arg Asn Gly Ala Leu Leu Arg Val Tyr Val Pro Arg Ser Ser Leu Pro
       690                 695                 700
   Gly Phe Tyr Arg Thr Ser Leu Thr Leu Ala Ala Pro Glu Ala Ala Gly
   705                 710                 715                 720
   Glu Val Glu Arg Leu Ile Gly His Pro Leu Pro Leu Arg Leu Asp Ala
                   725                 730                 735
   Ile Thr Gly Pro Glu Glu Glu Gly Gly Arg Leu Glu Thr Ile Leu Gly
               740                 745                 750
   Trp Pro Leu Ala Glu Arg Thr Val Val Ile Pro Ser Ala Ile Pro Thr
           755                 760                 765
   Asp Pro Arg Asn Val Gly Gly Asp Leu Asp Pro Ser Ser Ile Pro Asp
       770                 775                 780
   Lys Glu Gln Ala Ile Ser Ala Leu Pro Asp Tyr Ala Ser Gln Pro Gly
   785                 790                 795                 800
   Lys Pro Pro Arg Lys Asp Glu Leu
                   805
<210> 71
   <211> 393
   <212> PRT
   <213> Artificial
<220>
   <223> Artificial construct
<400> 71
   Met Gly Ala Asp Asp Val Val Asp Ser Ser Lys Ser Phe Val Met Glu
   1               5                   10                  15
   Asn Phe Ser Ser Tyr His Gly Thr Lys Pro Gly Tyr Val Asp Ser Ile
               20                  25                  30
   Gln Lys Gly Ile Gln Lys Pro Lys Ser Gly Thr Gln Gly Asn Tyr Asp
           35                  40                  45
   Asp Asp Trp Lys Gly Phe Tyr Ser Thr Asp Asn Lys Tyr Asp Ala Ala
       50                  55                  60
   Gly Tyr Ser Val Asp Asn Glu Asn Pro Leu Ser Gly Lys Ala Gly Gly
   65                  70                  75                  80
   Val Val Lys Val Thr Tyr Pro Gly Leu Thr Lys Val Leu Ala Leu Lys
                   85                  90                  95
   Val Asp Asn Ala Glu Thr Ile Lys Lys Glu Leu Gly Leu Ser Leu Thr
               100                 105                 110
   Glu Pro Leu Met Glu Gln Val Gly Thr Glu Glu Phe Ile Lys Arg Phe
           115                 120                 125
   Gly Asp Gly Ala Ser Arg Val Val Leu Ser Leu Pro Phe Ala Glu Gly
       130                 135                 140
   Ser Ser Ser Val Glu Tyr Ile Asn Asn Trp Glu Gln Ala Lys Ala Leu
   145                 150                 155                 160
   Ser Val Glu Leu Glu Ile Asn Phe Glu Thr Arg Gly Lys Arg Gly Gln
                   165                 170                 175
   Asp Ala Met Tyr Glu Tyr Met Ala Gln Ala Cys Ala Gly Asn Arg Val
               180                 185                 190
   Arg Arg Ser Val Gly Ser Ser Leu Ser Cys Ile Asn Leu Asp Trp Asp
           195                 200                 205
   Val Ile Arg Asp Lys Thr Lys Thr Lys Ile Glu Ser Leu Lys Glu His
       210                 215                 220
   Gly Pro Ile Lys Asn Lys Met Ser Glu Ser Pro Asn Lys Thr Val Ser
   225 230 235 240
   Glu Glu Lys Ala Lys Gln Tyr Leu Glu Glu Phe His Gln Thr Ala Leu
   245 250 255
   Glu His Pro Glu Leu Ser Glu Leu Lys Thr Val Thr Gly Thr Asn Pro
   260 265 270
   Val Phe Ala Gly Ala Asn Tyr Ala Ala Trp Ala Val Asn Val Ala Gln
   275 280 285
   Val Ile Asp Ser Glu Thr Ala Asp Asn Leu Glu Lys Thr Thr Ala Ala
   290 295 300
   Leu Ser Ile Leu Pro Gly Ile Gly Ser Val Met Gly Ile Ala Asp Gly
   305 310 315 320
   Ala Val His His Asn Thr Glu Glu Ile Val Ala Gln Ser Ile Ala Leu
   325 330 335
   Ser Ser Leu Met Val Ala Gln Ala Ile Pro Leu Val Gly Glu Leu Val
   340 345 350
   Asp Ile Gly Phe Ala Ala Tyr Asn Phe Val Glu Ser Ile Ile Asn Leu
   355 360 365
   Phe Gln Val Val His Asn Ser Tyr Asn Arg Pro Ala Tyr Ser Pro Gly
   370 375 380
   His Lys Thr Gln Pro Met His Glu Phe
   385 390
<210> 72
   <211> 1356
   <212> PRT
   <213> Artificial
<220>
   <223> Artificial construct
<400> 72
   Met Gly Ala Asp Asp Val Val Asp Ser Ser Lys Ser Phe Val Met Glu
   1               5                   10                  15
   Asn Phe Ser Ser Tyr His Gly Thr Lys Pro Gly Tyr Val Asp Ser Ile
               20                  25                  30
   Gln Lys Gly Ile Gln Lys Pro Lys Ser Gly Thr Gln Gly Asn Tyr Asp
           35                  40                  45
   Asp Asp Trp Lys Gly Phe Tyr Ser Thr Asp Asn Lys Tyr Asp Ala Ala
   50 55 60
   Gly Tyr Ser Val Asp Asn Glu Asn Pro Leu Ser Gly Lys Ala Gly Gly
   65 70 75 80
   Val Val Lys Val Thr Tyr Pro Gly Leu Thr Lys Val Leu Ala Leu Lys
   85 90 95
   Val Asp Asn Ala Glu Thr Ile Lys Lys Glu Leu Gly Leu Ser Leu Thr
   100 105 110
   Glu Pro Leu Met Glu Gln Val Gly Thr Glu Glu Phe Ile Lys Arg Phe
   115 120 125
   Gly Asp Gly Ala Ser Arg Val Val Leu Ser Leu Pro Phe Ala Glu Gly
   130 135 140
   Ser Ser Ser Val Glu Tyr Ile Asn Asn Trp Glu Gln Ala Lys Ala Leu
   145 150 155 160
   Ser Val Glu Leu Glu Ile Asn Phe Glu Thr Arg Gly Lys Arg Gly Gln
   165 170 175
   Asp Ala Met Tyr Glu Tyr Met Ala Gln Ala Cys Ala Gly Asn Arg Val
   180 185 190
   Arg Arg Ser Val Gly Ser Ser Leu Ser Cys Ile Asn Leu Asp Trp Asp
   195 200 205
   Val Ile Arg Asp Lys Thr Lys Thr Lys Ile Glu Ser Leu Lys Glu His
   210 215 220
   Gly Pro Ile Lys Asn Lys Met Ser Glu Ser Pro Asn Lys Thr Val Ser
   225 230 235 240
   Glu Glu Lys Ala Lys Gln Tyr Leu Glu Glu Phe His Gln Thr Ala Leu
   245 250 255
   Glu His Pro Glu Leu Ser Glu Leu Lys Thr Val Thr Gly Thr Asn Pro
   260 265 270
   Val Phe Ala Gly Ala Asn Tyr Ala Ala Trp Ala Val Asn Val Ala Gln
   275 280 285
   Val Ile Asp Ser Glu Thr Ala Asp Asn Leu Glu Lys Thr Thr Ala Ala
   290 295 300
   Leu Ser Ile Leu Pro Gly Ile Gly Ser Val Met Gly Ile Ala Asp Gly
   305 310 315 320
   Ala Val His His Asn Thr Glu Glu Ile Val Ala Gln Ser Ile Ala Leu
   325 330 335
   Ser Ser Leu Met Val Ala Gln Ala Ile Pro Leu Val Gly Glu Leu Val
   340 345 350
   Asp Ile Gly Phe Ala Ala Tyr Asn Phe Val Glu Ser Ile Ile Asn Leu
   355 360 365
   Phe Gln Val Val His Asn Ser Tyr Asn Arg Pro Ala Tyr Ser Pro Gly
   370 375 380
   His Lys Thr Gln Pro Met His Glu Phe His Ser Asp Ser Gly Thr Asn
   385 390 395 400
   Asp Pro Cys Asp Arg Ile Pro Pro Pro Tyr Gly Asp Asn Asp Gln Trp
   405 410 415
   Lys Cys Ala Ile Ile Leu Ser Lys Val Ser Glu Lys Pro Glu Asn Val
   420 425 430
   Phe Val Pro Pro Arg Arg Gln Arg Met Cys Ile Asn Asn Leu Glu Lys
   435 440 445
   Leu Asn Val Asp Lys Ile Arg Asp Lys His Ala Phe Leu Ala Asp Val
   450 455 460
   Leu Leu Thr Ala Arg Asn Glu Gly Glu Arg Ile Val Gln Asn His Pro
   465 470 475 480
   Asp Thr Asn Ser Ser Asn Val Cys Asn Ala Leu Glu Arg Ser Phe Ala
   485 490 495
   Asp Ile Ala Asp Ile Ile Arg Gly Thr Asp Leu Trp Lys Gly Thr Asn
   500 505 510
   Ser Asn Leu Glu Gln Asn Leu Lys Gln Met Phe Ala Lys Ile Arg Glu
   515 520 525
   Asn Asp Lys Val Leu Gln Asp Lys Tyr Pro Lys Asp Gln Asn Tyr Arg
   530 535 540
   Lys Leu Arg Glu Asp Trp Trp Asn Ala Asn Arg Gln Lys Val Trp Glu
   545 550 555 560
   Val Ile Thr Cys Gly Ala Arg Ser Asn Asp Leu Leu Ile Lys Arg Gly
   565 570 575
   Trp Arg Thr Ser Gly Lys Ser Asn Gly Asp Asn Lys Leu Glu Leu Cys
   580 585 590
   Arg Lys Cys Gly His Tyr Glu Glu Lys Val Pro Thr Lys Leu Asp Tyr
   595 600 605
   Val Pro Gln Phe Leu Arg Trp Leu Thr Glu Trp Ile Glu Asp Phe Tyr
   610 615 620
   Arg Glu Lys Gln Asn Leu Ile Asp Asp Met Glu Arg His Arg Glu Glu
   625 630 635 640
   Cys Thr Ser Glu Asp His Lys Ser Lys Glu Gly Thr Ser Tyr Cys Ser
   645 650 655
   Thr Cys Lys Asp Lys Cys Lys Lys Tyr Cys Glu Cys Val Lys Lys Trp
   660 665 670
   Lys Ser Glu Trp Glu Asn Gln Lys Asn Lys Tyr Thr Glu Leu Tyr Gln
   675 680 685
   Gln Asn Lys Asn Glu Thr Ser Gln Lys Asn Thr Ser Arg Tyr Asp Asp
   690 695 700
   Tyr Val Lys Asp Phe Phe Lys Lys Leu Glu Ala Asn Tyr Ser Ser Leu
   705 710 715 720
   Glu Asn Tyr Ile Lys Gly Asp Pro Tyr Phe Ala Glu Tyr Ala Thr Lys
   725 730 735
   Leu Ser Phe Ile Leu Asn Ser Ser Asp Ala Asn Asn Pro Ser Glu Lys
   740 745 750
   Ile Gln Lys Asn Asn Asp Glu Val Cys Asn Cys Asn Glu Ser Gly Ile
   755 760 765
   Ala Ser Val Glu Gln Glu Gln Ile Ser Asp Pro Ser Ser Asn Lys Thr
   770 775 780
   Cys Ile Thr His Ser Ser Ile Lys Ala Asn Lys Lys Lys Val Cys Lys
   785 790 795 800
   His Val Lys Leu Gly Val Arg Glu Asn Asp Lys Asp Leu Arg Val Cys
   805 810 815
   Val Ile Glu His Thr Ser Leu Ser Gly Val Glu Asn Cys Cys Cys Gln
   820 825 830
   Asp Phe Leu Arg Ile Leu Gln Glu Asn Cys Ser Asp Asn Lys Ser Gly
   835 840 845
   Ser Ser Ser Asn Gly Ser Cys Asn Asn Lys Asn Gln Glu Ala Cys Glu
   850 855 860
   Lys Asn Leu Glu Lys Val Leu Ala Ser Leu Thr Asn Cys Tyr Lys Cys
   865 870 875 880
   Asp Lys Cys Lys Ser Glu Gln Ser Lys Lys Asn Asn Lys Asn Trp Ile
   885 890 895
   Trp Lys Lys Ser Ser Gly Lys Glu Gly Gly Leu Gln Lys Glu Tyr Ala
   900 905 910
   Asn Thr Ile Gly Leu Pro Pro Arg Thr Gln Ser Leu Cys Leu Val Val
   915 920 925
   Cys Leu Asp Glu Lys Gly Lys Lys Thr Gln Glu Leu Lys Asn Ile Arg
   930 935 940
   Thr Asn Ser Glu Leu Leu Lys Glu Trp Ile Ile Ala Ala Phe His Glu
   945 950 955 960
   Gly Lys Asn Leu Lys Pro Ser His Glu Lys Lys Asn Asp Asp Asn Gly
   965 970 975
   Lys Lys Leu Cys Lys Ala Leu Glu Tyr Ser Phe Ala Asp Tyr Gly Asp
   980 985 990
   Leu Ile Lys Gly Thr Ser Ile Trp Asp Asn Glu Tyr Thr Lys Asp Leu
   995 1000 1005
   Glu Leu Asn Leu Gln Lys Ile Phe Gly Lys Leu Phe Arg Lys Tyr
   1010 1015 1020
   Ile Lys Lys Asn Asn Thr Ala Glu Gln Asp Thr Ser Tyr Ser Ser
   1025 1030 1035
   Leu Asp Glu Leu Arg Glu Ser Trp Trp Asn Thr Asn Lys Lys Tyr
   1040 1045 1050
   Ile Trp Leu Ala Met Lys His Gly Ala Gly Met Asn Ser Thr Thr
       1055                1060                1065
   Cys Cys Gly Asp Gly Ser Val Thr Gly Ser Gly Ser Ser Cys Asp
       1070                1075                1080
   Asp Ile Pro Thr Ile Asp Leu Ile Pro Gln Tyr Leu Arg Phe Leu
       1085                1090                1095
   Gln Glu Trp Val Glu His Phe Cys Lys Gln Arg Gln Glu Lys Val
       1100                1105                1110
   Lys Pro Val Ile Glu Asn Cys Lys Ser Cys Lys Glu Ser Gly Gly
       1115                1120                1125
   Thr Cys Asn Gly Glu Cys Lys Thr Glu Cys Lys Asn Lys Cys Glu
       1130                1135                1140
   Val Tyr Lys Lys Phe Ile Glu Asp Cys Lys Gly Gly Asp Gly Thr
       1145                1150                1155
   Ala Gly Ser Ser Trp Val Lys Arg Trp Asp Gln Ile Tyr Lys Arg
       1160                1165                1170
   Tyr Ser Lys Tyr Ile Glu Asp Ala Lys Arg Asn Arg Lys Ala Gly
       1175                1180                1185
   Thr Lys Asn Cys Gly Pro Ser Ser Thr Thr Asn Ala Ala Glu Asn
       1190                1195                1200
   Lys Cys Val Gln Ser Asp Ile Asp Ser Phe Phe Lys His Leu Ile
       1205                1210                1215
   Asp Ile Gly Leu Thr Thr Pro Ser Ser Tyr Leu Ser Ile Val Leu
       1220                1225                1230
   Asp Asp Asn Ile Cys Gly Ala Asp Lys Ala Pro Trp Thr Thr Tyr
       1235                1240                1245
   Thr Thr Tyr Thr Thr Thr Glu Lys Cys Asn Lys Glu Thr Asp Lys
       1250                1255                1260
   Ser Lys Leu Gln Gln Cys Asn Thr Ala Val Val Val Asn Val Pro
       1265                1270                1275
   Ser Pro Leu Gly Asn Thr Pro His Gly Tyr Lys Tyr Ala Cys Gln
   1280 1285 1290
   Cys Lys Ile Pro Thr Asn Glu Glu Thr Cys Asp Asp Arg Lys Glu
   1295 1300 1305
   Tyr Met Asn Gln Trp Ser Cys Gly Ser Ala Arg Thr Met Lys Arg
   1310 1315 1320
   Gly Tyr Lys Asn Asp Asn Tyr Glu Leu Cys Lys Tyr Asn Gly Val
   1325 1330 1335
   Asp Val Lys Pro Thr Thr Val Arg Ser Asn Ser Ser Lys Leu Asp
   1340 1345 1350
   Ser Gly Arg
   1355
<210> 73
   <211> 1364
   <212> PRT
   <213> Artificial
<220>
   <223> Artificial construct
<400> 73
   Met Gly Ala Asp Asp Val Val Asp Ser Ser Lys Ser Phe Val Met Glu
   1               5                   10                  15
   Asn Phe Ser Ser Tyr His Gly Thr Lys Pro Gly Tyr Val Asp Ser Ile
               20                  25                  30
   Gln Lys Gly Ile Gln Lys Pro Lys Ser Gly Thr Gln Gly Asn Tyr Asp
           35                  40                  45
   Asp Asp Trp Lys Gly Phe Tyr Ser Thr Asp Asn Lys Tyr Asp Ala Ala
       50                  55                  60
   Gly Tyr Ser Val Asp Asn Glu Asn Pro Leu Ser Gly Lys Ala Gly Gly
   65                  70                  75                  80
   Val Val Lys Val Thr Tyr Pro Gly Leu Thr Lys Val Leu Ala Leu Lys
                   85                  90                  95
   Val Asp Asn Ala Glu Thr Ile Lys Lys Glu Leu Gly Leu Ser Leu Thr
               100                 105                 110
   Glu Pro Leu Met Glu Gln Val Gly Thr Glu Glu Phe Ile Lys Arg Phe
           115                 120                 125
   Gly Asp Gly Ala Ser Arg Val Val Leu Ser Leu Pro Phe Ala Glu Gly
       130                 135                 140
   Ser Ser Ser Val Glu Tyr Ile Asn Asn Trp Glu Gln Ala Lys Ala Leu
   145                 150                 155                 160
   Ser Val Glu Leu Glu Ile Asn Phe Glu Thr Arg Gly Lys Arg Gly Gln
                   165                 170                 175
   Asp Ala Met Tyr Glu Tyr Met Ala Gln Ala Cys Ala Gly Asn Arg Val
               180                 185                 190
   Arg Arg Ser Val Gly Ser Ser Leu Ser Cys Ile Asn Leu Asp Trp Asp
           195                 200                 205
   Val Ile Arg Asp Lys Thr Lys Thr Lys Ile Glu Ser Leu Lys Glu His
       210                 215                 220
   Gly Pro Ile Lys Asn Lys Met Ser Glu Ser Pro Asn Lys Thr Val Ser
   225                 230                 235                 240
   Glu Glu Lys Ala Lys Gln Tyr Leu Glu Glu Phe His Gln Thr Ala Leu
                   245                 250                 255
   Glu His Pro Glu Leu Ser Glu Leu Lys Thr Val Thr Gly Thr Asn Pro
               260                 265                 270
   Val Phe Ala Gly Ala Asn Tyr Ala Ala Trp Ala Val Asn Val Ala Gln
           275                 280                 285
   Val Ile Asp Ser Glu Thr Ala Asp Asn Leu Glu Lys Thr Thr Ala Ala
       290                 295                 300
   Leu Ser Ile Leu Pro Gly Ile Gly Ser Val Met Gly Ile Ala Asp Gly
   305                 310                 315                 320
   Ala Val His His Asn Thr Glu Glu Ile Val Ala Gln Ser Ile Ala Leu
                   325                 330                 335
   Ser Ser Leu Met Val Ala Gln Ala Ile Pro Leu Val Gly Glu Leu Val
               340                 345                 350
   Asp Ile Gly Phe Ala Ala Tyr Asn Phe Val Glu Ser Ile Ile Asn Leu
           355                 360                 365
   Phe Gln Val Val His Asn Ser Tyr Asn Arg Pro Ala Tyr Ser Pro Gly
   370 375 380
   His Lys Thr Gln Pro Met His Glu Phe His Ser Asp Ser Gly Lys Tyr
   385 390 395 400
   Asp Pro Cys Glu Lys Lys Leu Pro Pro Tyr Asp Asp Asn Asp Gln Trp
   405 410 415
   Lys Cys Gln Gln Asn Ser Ser Asp Gly Ser Gly Lys Pro Glu Asn Ile
   420 425 430
   Cys Val Pro Pro Arg Arg Glu Arg Leu Cys Thr Tyr Asn Leu Glu Asn
   435 440 445
   Leu Lys Phe Asp Lys Ile Arg Asp Asn Asn Ala Phe Leu Ala Asp Val
   450 455 460
   Leu Leu Thr Ala Arg Asn Glu Gly Glu Lys Ile Val Gln Asn His Pro
   465 470 475 480
   Asp Thr Asn Ser Ser Asn Val Cys Asn Ala Leu Glu Arg Ser Phe Ala
   485 490 495
   Asp Leu Ala Asp Ile Ile Arg Gly Thr Asp Gln Trp Lys Gly Thr Asn
   500 505 510
   Ser Asn Leu Glu Lys Asn Leu Lys Gln Met Phe Ala Lys Ile Arg Glu
   515 520 525
   Asn Asp Lys Val Leu Gln Asp Lys Tyr Pro Lys Asp Gln Lys Tyr Thr
   530 535 540
   Lys Leu Arg Glu Ala Trp Trp Asn Ala Asn Arg Gln Lys Val Trp Glu
   545 550 555 560
   Val Ile Thr Cys Gly Ala Arg Ser Asn Asp Leu Leu Ile Lys Arg Gly
   565 570 575
   Trp Arg Thr Ser Gly Lys Ser Asp Arg Lys Lys Asn Phe Glu Leu Cys
   580 585 590
   Arg Lys Cys Gly His Tyr Glu Lys Glu Val Pro Thr Lys Leu Asp Tyr
   595 600 605
   Val Pro Gln Phe Leu Arg Trp Leu Thr Glu Trp Ile Glu Asp Phe Tyr
   610 615 620
   Arg Glu Lys Gln Asn Leu Ile Asp Asp Met Glu Arg His Arg Glu Glu
   625 630 635 640
   Cys Thr Arg Glu Asp His Lys Ser Lys Glu Gly Thr Ser Tyr Cys Ser
   645 650 655
   Thr Cys Lys Asp Lys Cys Lys Lys Tyr Cys Glu Cys Val Lys Lys Trp
   660 665 670
   Lys Thr Glu Trp Glu Asn Gln Glu Asn Lys Tyr Lys Asp Leu Tyr Glu
   675 680 685
   Gln Asn Lys Asn Lys Thr Ser Gln Lys Asn Thr Ser Arg Tyr Asp Asp
   690 695 700
   Tyr Val Lys Asp Phe Phe Glu Lys Leu Glu Ala Asn Tyr Ser Ser Leu
   705 710 715 720
   Glu Asn Tyr Ile Lys Gly Asp Pro Tyr Phe Ala Glu Tyr Ala Thr Lys
   725 730 735
   Leu Ser Phe Ile Leu Asn Pro Ser Asp Ala Asn Asn Pro Ser Gly Glu
   740 745 750
   Thr Ala Asn His Asn Asp Glu Ala Cys Asn Cys Asn Glu Ser Gly Ile
   755 760 765
   Ser Ser Val Gly Gln Ala Gln Thr Ser Gly Pro Ser Ser Asn Lys Thr
   770 775 780
   Cys Ile Thr His Ser Ser Ile Lys Thr Asn Lys Lys Lys Glu Cys Lys
   785 790 795 800
   Asp Val Lys Leu Gly Val Arg Glu Asn Asp Lys Asp Leu Lys Ile Cys
   805 810 815
   Val Ile Glu Asp Thr Ser Leu Ser Gly Val Asp Asn Cys Cys Cys Gln
   820 825 830
   Asp Leu Leu Gly Ile Leu Gln Glu Asn Cys Ser Asp Asn Lys Arg Gly
   835 840 845
   Ser Ser Ser Asn Asp Ser Cys Asp Asn Lys Asn Gln Asp Glu Cys Gln
   850 855 860
   Lys Lys Leu Glu Lys Val Phe Ala Ser Leu Thr Asn Gly Tyr Lys Cys
   865 870 875 880
   Asp Lys Cys Lys Ser Gly Thr Ser Arg Ser Lys Lys Lys Trp Ile Trp
                   885                 890                 895
   Lys Lys Ser Ser Gly Asn Glu Glu Gly Leu Gln Glu Glu Tyr Ala Asn
               900                 905                 910
   Thr Ile Gly Leu Pro Pro Arg Thr Gln Ser Leu Tyr Leu Gly Asn Leu
           915                 920                 925
   Pro Lys Leu Glu Asn Val Cys Glu Asp Val Lys Asp Ile Asn Phe Asp
       930                 935                 940
   Thr Lys Glu Lys Phe Leu Ala Gly Cys Leu Ile Val Ser Phe His Glu
   945                 950                 955                 960
   Gly Lys Asn Leu Lys Lys Arg Tyr Pro Gln Asn Lys Asn Ser Gly Asn
                   965                 970                 975
   Lys Glu Asn Leu Cys Lys Ala Leu Glu Tyr Ser Phe Ala Asp Tyr Gly
               980                 985                 990
   Asp Leu Ile Lys Gly Thr Ser Ile  Trp Asp Asn Glu Tyr  Thr Lys Asp
           995                 1000                1005
   Leu Glu  Leu Asn Leu Gln Asn  Asn Phe Gly Lys Leu  Phe Gly Lys
       1010                 1015                 1020
   Tyr Ile  Lys Lys Asn Asn Thr  Ala Glu Gln Asp Thr  Ser Tyr Ser
       1025                 1030                 1035
   Ser Leu  Asp Glu Leu Arg Glu  Ser Trp Trp Asn Thr  Asn Lys Lys
       1040                 1045                 1050
   Tyr Ile  Trp Thr Ala Met Lys  His Gly Ala Glu Met  Asn Ile Thr
       1055                 1060                 1065
   Thr Cys  Asn Ala Asp Gly Ser  Val Thr Gly Ser Gly  Ser Ser Cys
       1070                 1075                 1080
   Asp Asp  Ile Pro Thr Ile Asp  Leu Ile Pro Gln Tyr  Leu Arg Phe
       1085                 1090                 1095
   Leu Gln  Glu Trp Val Glu Asn  Phe Cys Glu Gln Arg  Gln Ala Lys
       1100                 1105                 1110
   Val Lys  Asp Val Ile Thr Asn  Cys Lys Ser Cys Lys  Glu Ser Gly
       1115                 1120                 1125
   Asn Lys Cys Lys Thr Glu Cys Lys Thr Lys Cys Lys Asp Glu Cys
   1130 1135 1140
   Glu Lys Tyr Lys Lys Phe Ile Glu Ala Cys Gly Thr Ala Gly Gly
   1145 1150 1155
   Gly Ile Gly Thr Ala Gly Ser Pro Trp Ser Lys Arg Trp Asp Gln
   1160 1165 1170
   Ile Tyr Lys Arg Tyr Ser Lys His Ile Glu Asp Ala Lys Arg Asn
   1175 1180 1185
   Arg Lys Ala Gly Thr Lys Asn Cys Gly Thr Ser Ser Thr Thr Asn
   1190 1195 1200
   Ala Ala Ala Ser Thr Asp Glu Asn Lys Cys Val Gln Ser Asp Ile
   1205 1210 1215
   Asp Ser Phe Phe Lys His Leu Ile Asp Ile Gly Leu Thr Thr Pro
   1220 1225 1230
   Ser Ser Tyr Leu Ser Asn Val Leu Asp Asp Asn Ile Cys Gly Ala
   1235 1240 1245
   Asp Lys Ala Pro Trp Thr Thr Tyr Thr Thr Tyr Thr Thr Thr Glu
   1250 1255 1260
   Lys Cys Asn Lys Glu Arg Asp Lys Ser Lys Ser Gln Ser Ser Asp
   1265 1270 1275
   Thr Leu Val Val Val Asn Val Pro Ser Pro Leu Gly Asn Thr Pro
   1280 1285 1290
   Tyr Arg Tyr Lys Tyr Ala Cys Gln Cys Lys Ile Pro Thr Asn Glu
   1295 1300 1305
   Glu Thr Cys Asp Asp Arg Lys Glu Tyr Met Asn Gln Trp Ser Cys
   1310 1315 1320
   Gly Ser Ala Arg Thr Met Lys Arg Gly Tyr Lys Asn Asp Asn Tyr
   1325 1330 1335
   Glu Leu Cys Lys Tyr Asn Gly Val Asp Val Lys Pro Thr Thr Val
   1340 1345 1350
   Arg Ser Asn Ser Ser Lys Leu Asp Ser Gly Arg
   1355 1360
<210> 74
   <211> 1013
   <212> PRT
   <213> Artificial
<220>
   <223> Artificial construct
<400> 74
   Met Gly Ala Asp Asp Val Val Asp Ser Ser Lys Ser Phe Val Met Glu
   1               5                   10                  15
   Asn Phe Ser Ser Tyr His Gly Thr Lys Pro Gly Tyr Val Asp Ser Ile
               20                  25                  30
   Gln Lys Gly Ile Gln Lys Pro Lys Ser Gly Thr Gln Gly Asn Tyr Asp
           35                  40                  45
   Asp Asp Trp Lys Gly Phe Tyr Ser Thr Asp Asn Lys Tyr Asp Ala Ala
       50                  55                  60
   Gly Tyr Ser Val Asp Asn Glu Asn Pro Leu Ser Gly Lys Ala Gly Gly
   65                  70                  75                  80
   Val Val Lys Val Thr Tyr Pro Gly Leu Thr Lys Val Leu Ala Leu Lys
                   85                  90                  95
   Val Asp Asn Ala Glu Thr Ile Lys Lys Glu Leu Gly Leu Ser Leu Thr
               100                 105                 110
   Glu Pro Leu Met Glu Gln Val Gly Thr Glu Glu Phe Ile Lys Arg Phe
           115                 120                 125
   Gly Asp Gly Ala Ser Arg Val Val Leu Ser Leu Pro Phe Ala Glu Gly
       130                 135                 140
   Ser Ser Ser Val Glu Tyr Ile Asn Asn Trp Glu Gln Ala Lys Ala Leu
   145                 150                 155                 160
   Ser Val Glu Leu Glu Ile Asn Phe Glu Thr Arg Gly Lys Arg Gly Gln
                   165                 170                 175
   Asp Ala Met Tyr Glu Tyr Met Ala Gln Ala Cys Ala Gly Asn Arg Val
               180                 185                 190
   Arg Arg Ser Val Gly Ser Ser Leu Ser Cys Ile Asn Leu Asp Trp Asp
           195                 200                 205
   Val Ile Arg Asp Lys Thr Lys Thr Lys Ile Glu Ser Leu Lys Glu His
       210                 215                 220
   Gly Pro Ile Lys Asn Lys Met Ser Glu Ser Pro Asn Lys Thr Val Ser
   225                 230                 235                 240
   Glu Glu Lys Ala Lys Gln Tyr Leu Glu Glu Phe His Gln Thr Ala Leu
                   245                 250                 255
   Glu His Pro Glu Leu Ser Glu Leu Lys Thr Val Thr Gly Thr Asn Pro
               260                 265                 270
   Val Phe Ala Gly Ala Asn Tyr Ala Ala Trp Ala Val Asn Val Ala Gln
           275                 280                 285
   Val Ile Asp Ser Glu Thr Ala Asp Asn Leu Glu Lys Thr Thr Ala Ala
       290                 295                 300
   Leu Ser Ile Leu Pro Gly Ile Gly Ser Val Met Gly Ile Ala Asp Gly
   305                 310                 315                 320
   Ala Val His His Asn Thr Glu Glu Ile Val Ala Gln Ser Ile Ala Leu
                   325                 330                 335
   Ser Ser Leu Met Val Ala Gln Ala Ile Pro Leu Val Gly Glu Leu Val
               340                 345                 350
   Asp Ile Gly Phe Ala Ala Tyr Asn Phe Val Glu Ser Ile Ile Asn Leu
           355                 360                 365
   Phe Gln Val Val His Asn Ser Tyr Asn Arg Pro Ala Tyr Ser Pro Gly
       370                 375                 380
   His Lys Thr Gln Pro Met His Glu Phe Leu Ser Phe Ile Leu Asn Ser
   385                 390                 395                 400
   Ser Asp Ala Asn Asn Pro Ser Glu Lys Ile Gln Lys Asn Asn Asp Glu
                   405                 410                 415
   Val Cys Asn Cys Asn Glu Ser Gly Ile Ala Ser Val Glu Gln Glu Gln
               420                 425                 430
   Ile Ser Asp Pro Ser Ser Asn Lys Thr Cys Ile Thr His Ser Ser Ile
           435                 440                 445
   Lys Ala Asn Lys Lys Lys Val Cys Lys His Val Lys Leu Gly Val Arg
   450 455 460
   Glu Asn Asp Lys Asp Leu Arg Val Cys Val Ile Glu His Thr Ser Leu
   465 470 475 480
   Ser Gly Val Glu Asn Cys Cys Cys Gln Asp Phe Leu Arg Ile Leu Gln
   485 490 495
   Glu Asn Cys Ser Asp Asn Lys Ser Gly Ser Ser Ser Asn Gly Ser Cys
   500 505 510
   Asn Asn Lys Asn Gln Glu Ala Cys Glu Lys Asn Leu Glu Lys Val Leu
   515 520 525
   Ala Ser Leu Thr Asn Cys Tyr Lys Cys Asp Lys Cys Lys Ser Glu Gln
   530 535 540
   Ser Lys Lys Asn Asn Lys Asn Trp Ile Trp Lys Lys Ser Ser Gly Lys
   545 550 555 560
   Glu Gly Gly Leu Gln Lys Glu Tyr Ala Asn Thr Ile Gly Leu Pro Pro
   565 570 575
   Arg Thr Gln Ser Leu Cys Leu Val Val Cys Leu Asp Glu Lys Gly Lys
   580 585 590
   Lys Thr Gln Glu Leu Lys Asn Ile Arg Thr Asn Ser Glu Leu Leu Lys
   595 600 605
   Glu Trp Ile Ile Ala Ala Phe His Glu Gly Lys Asn Leu Lys Pro Ser
   610 615 620
   His Glu Lys Lys Asn Asp Asp Asn Gly Lys Lys Leu Cys Lys Ala Leu
   625 630 635 640
   Glu Tyr Ser Phe Ala Asp Tyr Gly Asp Leu Ile Lys Gly Thr Ser Ile
   645 650 655
   Trp Asp Asn Glu Tyr Thr Lys Asp Leu Glu Leu Asn Leu Gln Lys Ile
   660 665 670
   Phe Gly Lys Leu Phe Arg Lys Tyr Ile Lys Lys Asn Asn Thr Ala Glu
   675 680 685
   Gln Asp Thr Ser Tyr Ser Ser Leu Asp Glu Leu Arg Glu Ser Trp Trp
   690 695 700
   Asn Thr Asn Lys Lys Tyr Ile Trp Leu Ala Met Lys His Gly Ala Gly
   705 710 715 720
   Met Asn Ser Thr Thr Cys Cys Gly Asp Gly Ser Val Thr Gly Ser Gly
   725 730 735
   Ser Ser Cys Asp Asp Ile Pro Thr Ile Asp Leu Ile Pro Gln Tyr Leu
   740 745 750
   Arg Phe Leu Gln Glu Trp Val Glu His Phe Cys Lys Gln Arg Gln Glu
   755 760 765
   Lys Val Lys Pro Val Ile Glu Asn Cys Lys Ser Cys Lys Glu Ser Gly
   770 775 780
   Gly Thr Cys Asn Gly Glu Cys Lys Thr Glu Cys Lys Asn Lys Cys Glu
   785 790 795 800
   Val Tyr Lys Lys Phe Ile Glu Asp Cys Lys Gly Gly Asp Gly Thr Ala
   805 810 815
   Gly Ser Ser Trp Val Lys Arg Trp Asp Gln Ile Tyr Lys Arg Tyr Ser
   820 825 830
   Lys Tyr Ile Glu Asp Ala Lys Arg Asn Arg Lys Ala Gly Thr Lys Asn
   835 840 845
   Cys Gly Pro Ser Ser Thr Thr Asn Ala Ala Glu Asn Lys Cys Val Gln
   850 855 860
   Ser Asp Ile Asp Ser Phe Phe Lys His Leu Ile Asp Ile Gly Leu Thr
   865 870 875 880
   Thr Pro Ser Ser Tyr Leu Ser Ile Val Leu Asp Asp Asn Ile Cys Gly
   885 890 895
   Ala Asp Lys Ala Pro Trp Thr Thr Tyr Thr Thr Tyr Thr Thr Thr Glu
   900 905 910
   Lys Cys Asn Lys Glu Thr Asp Lys Ser Lys Leu Gln Gln Cys Asn Thr
   915 920 925
   Ala Val Val Val Asn Val Pro Ser Pro Leu Gly Asn Thr Pro His Gly
   930 935 940
   Tyr Lys Tyr Ala Cys Gln Cys Lys Ile Pro Thr Asn Glu Glu Thr Cys
   945 950 955 960
   Asp Asp Arg Lys Glu Tyr Met Asn Gln Trp Ser Cys Gly Ser Ala Arg
   965 970 975
   Thr Met Lys Arg Gly Tyr Lys Asn Asp Asn Tyr Glu Leu Cys Lys Tyr
   980 985 990
   Asn Gly Val Asp Val Lys Pro Thr Thr Val Arg Ser Asn Ser Ser Lys
   995 1000 1005
   Leu Asp Ser Gly Arg
   1010
<210> 75
   <211> 1036
   <212> PRT
   <213> Artificial
<220>
   <223> Artificial construct
<400> 75
   Met Gly Ala Asp Asp Val Val Asp Ser Ser Lys Ser Phe Val Met Glu
   1 5 10 15
   Asn Phe Ser Ser Tyr His Gly Thr Lys Pro Gly Tyr Val Asp Ser Ile
   20 25 30
   Gln Lys Gly Ile Gln Lys Pro Lys Ser Gly Thr Gln Gly Asn Tyr Asp
   35 40 45
   Asp Asp Trp Lys Gly Phe Tyr Ser Thr Asp Asn Lys Tyr Asp Ala Ala
   50 55 60
   Gly Tyr Ser Val Asp Asn Glu Asn Pro Leu Ser Gly Lys Ala Gly Gly
   65 70 75 80
   Val Val Lys Val Thr Tyr Pro Gly Leu Thr Lys Val Leu Ala Leu Lys
   85 90 95
   Val Asp Asn Ala Glu Thr Ile Lys Lys Glu Leu Gly Leu Ser Leu Thr
   100 105 110
   Glu Pro Leu Met Glu Gln Val Gly Thr Glu Glu Phe Ile Lys Arg Phe
   115 120 125
   Gly Asp Gly Ala Ser Arg Val Val Leu Ser Leu Pro Phe Ala Glu Gly
   130 135 140
   Ser Ser Ser Val Glu Tyr Ile Asn Asn Trp Glu Gln Ala Lys Ala Leu
   145 150 155 160
   Ser Val Glu Leu Glu Ile Asn Phe Glu Thr Arg Gly Lys Arg Gly Gln
   165 170 175
   Asp Ala Met Tyr Glu Tyr Met Ala Gln Ala Cys Ala Gly Asn Arg Val
   180 185 190
   Arg Arg Ser Val Gly Ser Ser Leu Ser Cys Ile Asn Leu Asp Trp Asp
   195 200 205
   Val Ile Arg Asp Lys Thr Lys Thr Lys Ile Glu Ser Leu Lys Glu His
   210 215 220
   Gly Pro Ile Lys Asn Lys Met Ser Glu Ser Pro Asn Lys Thr Val Ser
   225 230 235 240
   Glu Glu Lys Ala Lys Gln Tyr Leu Glu Glu Phe His Gln Thr Ala Leu
   245 250 255
   Glu His Pro Glu Leu Ser Glu Leu Lys Thr Val Thr Gly Thr Asn Pro
   260 265 270
   Val Phe Ala Gly Ala Asn Tyr Ala Ala Trp Ala Val Asn Val Ala Gln
   275 280 285
   Val Ile Asp Ser Glu Thr Ala Asp Asn Leu Glu Lys Thr Thr Ala Ala
   290 295 300
   Leu Ser Ile Leu Pro Gly Ile Gly Ser Val Met Gly Ile Ala Asp Gly
   305 310 315 320
   Ala Val His His Asn Thr Glu Glu Ile Val Ala Gln Ser Ile Ala Leu
   325 330 335
   Ser Ser Leu Met Val Ala Gln Ala Ile Pro Leu Val Gly Glu Leu Val
   340 345 350
   Asp Ile Gly Phe Ala Ala Tyr Asn Phe Val Glu Ser Ile Ile Asn Leu
   355 360 365
   Phe Gln Val Val His Asn Ser Tyr Asn Arg Pro Ala Tyr Ser Pro Gly
   370 375 380
   His Lys Thr Gln Pro Met His Glu Phe Asn Tyr Ile Lys Gly Asp Pro
   385 390 395 400
   Tyr Phe Ala Glu Tyr Ala Thr Lys Leu Ser Phe Ile Leu Asn Pro Ser
   405 410 415
   Asp Ala Asn Asn Pro Ser Gly Glu Thr Ala Asn His Asn Asp Glu Ala
   420 425 430
   Cys Asn Cys Asn Glu Ser Gly Ile Ser Ser Val Gly Gln Ala Gln Thr
   435 440 445
   Ser Gly Pro Ser Ser Asn Lys Thr Cys Ile Thr His Ser Ser Ile Lys
   450 455 460
   Thr Asn Lys Lys Lys Glu Cys Lys Asp Val Lys Leu Gly Val Arg Glu
   465 470 475 480
   Asn Asp Lys Asp Leu Lys Ile Cys Val Ile Glu Asp Thr Ser Leu Ser
   485 490 495
   Gly Val Asp Asn Cys Cys Cys Gln Asp Leu Leu Gly Ile Leu Gln Glu
   500 505 510
   Asn Cys Ser Asp Asn Lys Arg Gly Ser Ser Ser Asn Asp Ser Cys Asp
   515 520 525
   Asn Lys Asn Gln Asp Glu Cys Gln Lys Lys Leu Glu Lys Val Phe Ala
   530 535 540
   Ser Leu Thr Asn Gly Tyr Lys Cys Asp Lys Cys Lys Ser Gly Thr Ser
   545 550 555 560
   Arg Ser Lys Lys Lys Trp Ile Trp Lys Lys Ser Ser Gly Asn Glu Glu
   565 570 575
   Gly Leu Gln Glu Glu Tyr Ala Asn Thr Ile Gly Leu Pro Pro Arg Thr
   580 585 590
   Gln Ser Leu Tyr Leu Gly Asn Leu Pro Lys Leu Glu Asn Val Cys Glu
   595 600 605
   Asp Val Lys Asp Ile Asn Phe Asp Thr Lys Glu Lys Phe Leu Ala Gly
   610 615 620
   Cys Leu Ile Val Ser Phe His Glu Gly Lys Asn Leu Lys Lys Arg Tyr
   625 630 635 640
   Pro Gln Asn Lys Asn Ser Gly Asn Lys Glu Asn Leu Cys Lys Ala Leu
   645 650 655
   Glu Tyr Ser Phe Ala Asp Tyr Gly Asp Leu Ile Lys Gly Thr Ser Ile
   660 665 670
   Trp Asp Asn Glu Tyr Thr Lys Asp Leu Glu Leu Asn Leu Gln Asn Asn
   675 680 685
   Phe Gly Lys Leu Phe Gly Lys Tyr Ile Lys Lys Asn Asn Thr Ala Glu
   690 695 700
   Gln Asp Thr Ser Tyr Ser Ser Leu Asp Glu Leu Arg Glu Ser Trp Trp
   705 710 715 720
   Asn Thr Asn Lys Lys Tyr Ile Trp Thr Ala Met Lys His Gly Ala Glu
   725 730 735
   Met Asn Ile Thr Thr Cys Asn Ala Asp Gly Ser Val Thr Gly Ser Gly
   740 745 750
   Ser Ser Cys Asp Asp Ile Pro Thr Ile Asp Leu Ile Pro Gln Tyr Leu
   755 760 765
   Arg Phe Leu Gln Glu Trp Val Glu Asn Phe Cys Glu Gln Arg Gln Ala
   770 775 780
   Lys Val Lys Asp Val Ile Thr Asn Cys Lys Ser Cys Lys Glu Ser Gly
   785 790 795 800
   Asn Lys Cys Lys Thr Glu Cys Lys Thr Lys Cys Lys Asp Glu Cys Glu
   805 810 815
   Lys Tyr Lys Lys Phe Ile Glu Ala Cys Gly Thr Ala Gly Gly Gly Ile
   820 825 830
   Gly Thr Ala Gly Ser Pro Trp Ser Lys Arg Trp Asp Gln Ile Tyr Lys
   835 840 845
   Arg Tyr Ser Lys His Ile Glu Asp Ala Lys Arg Asn Arg Lys Ala Gly
   850 855 860
   Thr Lys Asn Cys Gly Thr Ser Ser Thr Thr Asn Ala Ala Ala Ser Thr
   865 870 875 880
   Asp Glu Asn Lys Cys Val Gln Ser Asp Ile Asp Ser Phe Phe Lys His
   885 890 895
   Leu Ile Asp Ile Gly Leu Thr Thr Pro Ser Ser Tyr Leu Ser Asn Val
                   900                 905                 910
   Leu Asp Asp Asn Ile Cys Gly Ala Asp Lys Ala Pro Trp Thr Thr Tyr
               915                 920                 925
   Thr Thr Tyr Thr Thr Thr Glu Lys Cys Asn Lys Glu Arg Asp Lys Ser
           930                 935                 940
   Lys Ser Gln Ser Ser Asp Thr Leu Val Val Val Asn Val Pro Ser Pro
   945                 950                 955                 960
   Leu Gly Asn Thr Pro Tyr Arg Tyr Lys Tyr Ala Cys Gln Cys Lys Ile
                   965                 970                 975
   Pro Thr Asn Glu Glu Thr Cys Asp Asp Arg Lys Glu Tyr Met Asn Gln
               980                 985                 990
   Trp Ser Cys Gly Ser Ala Arg Thr  Met Lys Arg Gly Tyr  Lys Asn Asp
           995                 1000                1005
   Asn Tyr  Glu Leu Cys Lys Tyr  Asn Gly Val Asp Val  Lys Pro Thr
       1010                1015                1020
   Thr Val  Arg Ser Asn Ser Ser  Lys Leu Asp Ser Gly  Arg
       1025                1030                1035
<210> 76
   <211> 18
   <212> DNA
   <213> Artificial
<220>
   <223> Primer sequence
<400> 76
   aactacatca agggcgac 18
<210> 77
   <211> 21
   <212> DNA
   <213> Artificial
<220>
   <223> Primer sequence
<400> 77
   cttgttgata ttggtgtcgg t 21
<210> 78
   <211> 18
   <212> DNA
   <213> Artificial
<220>
   <223> Primer sequence
<400> 78
   cacagcgata gcggcaag 18
<210> 79
   <211> 18
   <212> DNA
   <213> Artificial
<220>
   <223> Primer sequence
<400> 79
   gtccagcttg ctggagtt 18
<210> 80
   <211> 18
   <212> DNA
   <213> Artificial
<220>
   <223> Primer sequence
<400> 80
   aactacatca agggcgac 18
<210> 81
   <211> 18
   <212> DNA
   <213> Artificial
<220>
   <223> Primer sequence
<400> 81
   gtccagcttg ctggagtt 18
<210> 82
   <211> 18
   <212> DNA
   <213> Artificial
<220>
   <223> Primer sequence
<400> 82
   aactacatca agggcgac 18
<210> 83
   <211> 18
   <212> DNA
   <213> Artificial
<220>
   <223> Primer sequence
<400> 83
   agcggcgttg gtggtgga 18
<210> 84
   <211> 18
   <212> DNA
   <213> Artificial
<220>
   <223> Primer sequence
<400> 84
   aactacatca agggcgac 18
<210> 85
   <211> 18
   <212> DNA
   <213> Artificial
<220>
   <223> Primer sequence
<400> 85
   gtacttgtac cggtaggg 18
<210> 86
   <211> 18
   <212> DNA
   <213> Artificial
<220>
   <223> Primer sequence
<400> 86
   cacagcgata gcggcaag 18
<210> 87
   <211> 18
   <212> DNA
   <213> Artificial
<220>
   <223> Primer sequence
<400> 87
   gtacttgtac cggtaggg 18
<210> 88
   <211> 18
   <212> DNA
   <213> Artificial
<220>
   <223> Primer sequence
<400> 88
   ctgaccaact gctacaag 18
<210> 89
   <211> 19
   <212> DNA
   <213> Artificial
<220>
   <223> Primer sequence
<400> 89
   ggtccagagg gtacagctt 19
<210> 90
   <211> 18
   <212> DNA
   <213> Artificial
<220>
   <223> Primer sequence
<400> 90
   ctgtccttca tcctgaac 18
<210> 91
   <211> 21
   <212> DNA
   <213> Artificial
<220>
   <223> Primer sequence
<400> 91
   ttcagcgttg ttgtactcgt a 21
<210> 92
   <211> 18
   <212> DNA
   <213> Artificial
<220>
   <223> Primer sequence
<400> 92
   ctgtccttca tcctgaac 18
<210> 93
   <211> 18
   <212> DNA
   <213> Artificial
<220>
   <223> Primer sequence
<400> 93
   gtccagaggg tacagctt 18
<210> 94
   <211> 18
   <212> DNA
   <213> Artificial
<220>
   <223> Primer sequence
<400> 94
   cactctgact ctggcacc 18
<210> 95
   <211> 24
   <212> DNA
   <213> Artificial
<220>
   <223> Primer sequence
<400> 95
   agaggacttc atcttgttgt tggt 24
<210> 96
   <211> 18
   <212> DNA
   <213> Artificial
<220>
   <223> Primer sequence
<400> 96
   ctgtccttca tcctgaac 18
<210> 97
   <211> 24
   <212> DNA
   <213> Artificial
<220>
   <223> Primer sequence
<400> 97
   agaggacttc atcttgttgt tggt 24
<210> 98
   <211> 18
   <212> DNA
   <213> Artificial
<220>
   <223> Primer sequence
<400> 98
   cactctgact ctggcacc 18
<210> 99
   <211> 18
   <212> DNA
   <213> Artificial
<220>
   <223> Primer sequence
<400> 99
   gtccagctta gaggagtt 18
<210> 100
   <211> 18
   <212> DNA
   <213> Artificial
<220>
   <223> Primer sequence
<400> 100
   ctgtccttca tcctgaac 18
<210> 101
   <211> 18
   <212> DNA
   <213> Artificial
<220>
   <223> Primer sequence
<400> 101
   gtccagctta gaggagtt 18
<210> 102
   <211> 18
   <212> DNA
   <213> Artificial
<220>
   <223> Primer sequence
<400> 102
   cactctgact ctggcacc 18
<210> 103
   <211> 18
   <212> DNA
   <213> Artificial
<220>
   <223> Primer sequence
<400> 103
   ggcggcgttg gtggtaga 18
<210> 104
   <211> 18
   <212> DNA
   <213> Artificial
<220>
   <223> Primer sequence
<400> 104
   ctgtccttca tcctgaac 18
<210> 105
   <211> 18
   <212> DNA
   <213> Artificial
<220>
   <223> Primer sequence
<400> 105
   ggcggcgttg gtggtaga 18
<210> 106
   <211> 18
   <212> DNA
   <213> Artificial
<220>
   <223> Primer sequence
<400> 106
   cactctgact ctggcacc 18
<210> 107
   <211> 18
   <212> DNA
   <213> Artificial
<220>
   <223> Primer sequence
<400> 107
   gtacttgtat ccgtgggg 18
<210> 108
   <211> 18
   <212> DNA
   <213> Artificial
<220>
   <223> Primer sequence
<400> 108
   ctgtccttca tcctgaac 18
<210> 109
   <211> 18
   <212> DNA
   <213> Artificial
<220>
   <223> Primer sequence
<400> 109
   gtacttgtat ccgtgggg 18
<210> 110
   <211> 18
   <212> DNA
   <213> Artificial
<220>
   <223> Primer sequence
<400> 110
   cacagcgata gcggcaag 18
<210> 111
   <211> 36
   <212> DNA
   <213> Artificial
<220>
   <223> Primer sequence
<400> 111
   ggtgtcgaag ttgatgtcgg gcagattgcc caggta 36
<210> 112
   <211> 18
   <212> DNA
   <213> Artificial
<220>
   <223> Primer sequence
<400> 112
   cacagcgata gcggcaag 18
<210> 113
   <211> 36
   <212> DNA
   <213> Artificial
<220>
   <223> Primer sequence
<400> 113
   agctgcggcc agattagcgc cctcgtggaa ggacac 36
<210> 114
   <211> 18
   <212> DNA
   <213> Artificial
<220>
   <223> Primer sequence
<400> 114
   cacagcgata gcggcaag 18
<210> 115
   <211> 36
   <212> DNA
   <213> Artificial
<220>
   <223> Primer sequence
<400> 115
   agcgcattca gctgcggcgt tggtcttgat ggagct 36
<210> 116
   <211> 18
   <212> DNA
   <213> Artificial
<220>
   <223> Primer sequence
<400> 116
   cacagcgata gcggcaag 18
<210> 117
   <211> 18
   <212> DNA
   <213> Artificial
<220>
   <223> Primer sequence
<400> 117
   gtccagcttg ctggagtt 18
<210> 118
   <211> 36
   <212> DNA
   <213> Artificial
<220>
   <223> Primer sequence
<400> 118
   gctaatctgg ccgcagctta cccccagaat aagaac 36
<210> 119
   <211> 18
   <212> DNA
   <213> Artificial
<220>
   <223> Primer sequence
<400> 119
   gtccagcttg ctggagtt 18
<210> 120
   <211> 36
   <212> DNA
   <213> Artificial
<220>
   <223> Primer sequence
<400> 120
   gccgcagctg aatgcgctga cgtgaagctg ggcgtg 36
<210> 121
   <211> 18
   <212> DNA
   <213> Artificial
<220>
   <223> Primer sequence
<400> 121
   gtccagcttg ctggagtt 18
<210> 122
   <211> 18
   <212> DNA
   <213> Artificial
<220>
   <223> Primer sequence
<400> 122
   cacagcgata gcggcaag 18
<210> 123
   <211> 18
   <212> DNA
   <213> Artificial
<220>
   <223> Primer sequence
<400> 123
   gtccagcttg ctggagtt 18
<210> 124
   <211> 18
   <212> DNA
   <213> Artificial
<220>
   <223> Primer sequence
<400> 124
   cacagcgata gcggcaag 18
<210> 125
   <211> 18
   <212> DNA
   <213> Artificial
<220>
   <223> Primer sequence
<400> 125
   gtccagcttg ctggagtt 18
<210> 126
   <211> 18
   <212> DNA
   <213> Artificial
<220>
   <223> Primer sequence
<400> 126
   cacagcgata gcggcaag 18
<210> 127
   <211> 18
   <212> DNA
   <213> Artificial
<220>
   <223> Primer sequence
<400> 127
   gtccagcttg ctggagtt 18
<210> 128
   <211> 640
   <212> PRT
   <213> Artificial Sequence
<220>
   <223> Artificial construct
<400> 128
   Asn Tyr Ile Lys Gly Asp Pro Tyr Phe Ala Glu Tyr Ala Thr Lys Leu
   1               5                   10                  15
   Ser Phe Ile Leu Asn Pro Ser Asp Ala Asn Asn Pro Ser Gly Glu Thr
               20                  25                  30
   Ala Asn His Asn Asp Glu Ala Cys Asn Cys Asn Glu Ser Gly Ile Ser
           35                  40                  45
   Ser Val Gly Gln Ala Gln Thr Ser Gly Pro Ser Ser Gln Lys Thr Cys
       50                  55                  60
   Ile Thr His Ser Ser Ile Lys Thr Asn Lys Lys Lys Glu Cys Lys Asp
   65                  70                  75                  80
   Val Lys Leu Gly Val Arg Glu Asn Asp Lys Asp Leu Lys Ile Cys Val
                   85                  90                  95
   Ile Glu Asp Thr Ser Leu Ser Gly Val Asp Asn Cys Cys Cys Gln Asp
   100 105 110
   Leu Leu Gly Ile Leu Gln Glu Asn Cys Ser Asp Asn Lys Arg Gly Ser
   115 120 125
   Ser Ser Asn Asp Ser Cys Asp Asn Lys Asn Gln Asp Glu Cys Gln Lys
   130 135 140
   Lys Leu Glu Lys Val Phe Ala Ser Leu Thr Asn Gly Tyr Lys Cys Asp
   145 150 155 160
   Lys Cys Lys Ser Gly Thr Ser Arg Ser Lys Lys Lys Trp Ile Trp Lys
   165 170 175
   Lys Ser Ser Gly Asn Glu Glu Gly Leu Gln Glu Glu Tyr Ala Asn Thr
   180 185 190
   Ile Gly Leu Pro Pro Arg Thr Gln Ser Leu Tyr Leu Gly Asn Leu Pro
   195 200 205
   Lys Leu Glu Asn Val Cys Glu Asp Val Lys Asp Ile Asn Phe Asp Thr
   210 215 220
   Lys Glu Lys Phe Leu Ala Gly Cys Leu Ile Val Ser Phe His Glu Gly
   225 230 235 240
   Lys Asn Leu Lys Lys Arg Tyr Pro Gln Asn Lys Asn Ser Gly Asn Lys
   245 250 255
   Glu Asn Leu Cys Lys Ala Leu Glu Tyr Ser Phe Ala Asp Tyr Gly Asp
   260 265 270
   Leu Ile Lys Gly Thr Ser Ile Trp Asp Asn Glu Tyr Thr Lys Asp Leu
   275 280 285
   Glu Leu Asn Leu Gln Asn Asn Phe Gly Lys Leu Phe Gly Lys Tyr Ile
   290 295 300
   Lys Lys Gln Asn Thr Ala Glu Gln Asp Thr Ser Tyr Ser Ser Leu Asp
   305 310 315 320
   Glu Leu Arg Glu Ser Trp Trp Asn Thr Asn Lys Lys Tyr Ile Trp Thr
   325 330 335
   Ala Met Lys His Gly Ala Glu Met Gln Ile Thr Thr Cys Asn Ala Asp
   340 345 350
   Gly Ser Val Thr Gly Ser Gly Ser Ser Cys Asp Asp Ile Pro Thr Ile
   355 360 365
   Asp Leu Ile Pro Gln Tyr Leu Arg Phe Leu Gln Glu Trp Val Glu Asn
   370 375 380
   Phe Cys Glu Gln Arg Gln Ala Lys Val Lys Asp Val Ile Thr Asn Cys
   385 390 395 400
   Lys Ser Cys Lys Glu Ser Gly Asn Lys Cys Lys Thr Glu Cys Lys Thr
   405 410 415
   Lys Cys Lys Asp Glu Cys Glu Lys Tyr Lys Lys Phe Ile Glu Ala Cys
   420 425 430
   Gly Thr Ala Gly Gly Gly Ile Gly Thr Ala Gly Ser Pro Trp Ser Lys
   435 440 445
   Arg Trp Asp Gln Ile Tyr Lys Arg Tyr Ser Lys His Ile Glu Asp Ala
   450 455 460
   Lys Arg Asn Arg Lys Ala Gly Thr Lys Asn Cys Gly Thr Ser Ser Thr
   465 470 475 480
   Thr Asn Ala Ala Ala Ser Thr Asp Glu Asn Lys Cys Val Gln Ser Asp
   485 490 495
   Ile Asp Ser Phe Phe Lys His Leu Ile Asp Ile Gly Leu Thr Thr Pro
   500 505 510
   Ser Ser Tyr Leu Ser Asn Val Leu Asp Asp Asn Ile Cys Gly Ala Asp
   515 520 525
   Lys Ala Pro Trp Thr Thr Tyr Thr Thr Tyr Thr Thr Thr Glu Lys Cys
   530 535 540
   Asn Lys Glu Arg Asp Lys Ser Lys Ser Gln Ser Ser Asp Thr Leu Val
   545 550 555 560
   Val Val Asn Val Pro Ser Pro Leu Gly Asn Thr Pro Tyr Arg Tyr Lys
   565 570 575
   Tyr Ala Cys Gln Cys Lys Ile Pro Thr Asn Glu Glu Thr Cys Asp Asp
   580 585 590
   Arg Lys Glu Tyr Met Asn Gln Trp Ser Cys Gly Ser Ala Arg Thr Met
   595 600 605
   Lys Arg Gly Tyr Lys Asn Asp Asn Tyr Glu Leu Cys Lys Tyr Asn Gly
   610 615 620
   Val Asp Val Lys Pro Thr Thr Val Arg Ser Asn Ser Ser Lys Leu Asp
   625 630 635 640
<210> 129
   <211> 116
   <212> PRT
   <213> Artificial Sequence
<220>
   <223> Artificial construct
<400> 129
   Gly Ala Met Val Asp Thr Leu Ser Gly Leu Ser Ser Glu Gln Gly Gln
   1 5 10 15
   Ser Gly Asp Met Thr Ile Glu Glu Asp Ser Ala Thr His Ile Lys Phe
   20 25 30
   Ser Lys Arg Asp Glu Asp Gly Lys Glu Leu Ala Gly Ala Thr Met Glu
   35 40 45
   Leu Arg Asp Ser Ser Gly Lys Thr Ile Ser Thr Trp Ile Ser Asp Gly
   50 55 60
   Gln Val Lys Asp Phe Tyr Leu Tyr Pro Gly Lys Tyr Thr Phe Val Glu
   65 70 75 80
   Thr Ala Ala Pro Asp Gly Tyr Glu Val Ala Thr Ala Ile Thr Phe Thr
   85 90 95
   Val Asn Glu Gln Gly Gln Val Thr Val Asn Gly Lys Ala Thr Lys Gly
   100 105 110
   Asp Ala His Ile
   115
<210> 130
   <211> 13
   <212> PRT
   <213> Artificial Sequence
<220>
   <223> Artificial Spytag sequence
<400> 130
   Ala His Ile Val Met Val Asp Ala Tyr Lys Pro Thr Lys
   1 5 10
<210> 131
   <211> 8
   <212> PRT
   <213> Artificial Sequence
<220>
   <223> Artificial Spytag sequence
<400> 131
   Ala His Ile Val Met Val Asp Ala
   1 5
<210> 132
   <211> 10
   <212> PRT
   <213> Artificial Sequence
<220>
   <223> The beta-strand of CnaB2
<400> 132
   Ala Thr His Ile Lys Phe Ser Lys Arg Asp
   1 5 10
<210> 133
   <211> 104
   <212> PRT
   <213> Artificial Sequence
<220>
   <223> SpyLigase sequence
<400> 133
   His His His His His His Asp Tyr Asp Gly Gln Ser Gly Asp Gly Lys
   1               5                   10                  15
   Glu Leu Ala Gly Ala Thr Met Glu Leu Arg Asp Ser Ser Gly Lys Thr
               20                  25                  30
   Ile Ser Thr Trp Ile Ser Asp Gly Gln Val Lys Asp Phe Tyr Leu Tyr
           35                  40                  45
   Pro Gly Lys Tyr Thr Phe Val Glu Thr Ala Ala Pro Asp Gly Tyr Glu
       50                  55                  60
   Val Ala Thr Ala Ile Thr Phe Thr Val Asn Glu Gln Gly Gln Val Thr
   65                  70                  75                  80
   Val Asn Gly Lys Ala Thr Lys Gly Gly Ser Gly Gly Ser Gly Gly Ser
                   85                  90                  95
   Gly Glu Asp Ser Ala Thr His Ile
               100
<210> 134
   <211> 16
   <212> PRT
   <213> Artificial Sequence
<220>
   <223> isopeptide Spy0128 sequence
<400> 134
   Thr Asp Lys Asp Met Thr Ile Thr Phe Thr Asn Lys Lys Asp Ala Glu
   1               5                   10                  15
<210> 135
   <211> 282
   <212> PRT
   <213> Artificial Sequence
<220>
   <223> Split-Spy0128 sequence
<400> 135
   Ala Thr Thr Val His Gly Glu Thr Val Val Asn Gly Ala Lys Leu Thr
   1               5                   10                  15
   Val Thr Lys Asn Leu Asp Leu Val Asn Ser Asn Ala Leu Ile Pro Asn
               20                  25                  30
   Thr Asp Phe Thr Phe Lys Ile Glu Pro Asp Thr Thr Val Asn Glu Asp
           35                  40                  45
   Gly Asn Lys Phe Lys Gly Val Ala Leu Asn Thr Pro Met Thr Lys Val
       50                  55                  60
   Thr Tyr Thr Asn Ser Asp Lys Gly Gly Ser Asn Thr Lys Thr Ala Glu
   65                  70                  75                  80
   Phe Asp Phe Ser Glu Val Thr Phe Glu Lys Pro Gly Val Tyr Tyr Tyr
                   85                  90                  95
   Lys Val Thr Glu Glu Lys Ile Asp Lys Val Pro Gly Val Ser Tyr Asp
               100                 105                 110
   Thr Thr Ser Tyr Thr Val Gln Val His Val Leu Trp Asn Glu Glu Gln
           115                 120                 125
   Gln Lys Pro Val Ala Thr Tyr Ile Val Gly Tyr Lys Glu Gly Ser Lys
   130 135 140
   Val Pro Ile Gln Phe Lys Asn Ser Leu Asp Ser Thr Thr Leu Thr Val
   145 150 155 160
   Lys Lys Lys Val Ser Gly Thr Gly Gly Asp Arg Ser Lys Asp Phe Asn
   165 170 175
   Phe Gly Leu Thr Leu Lys Ala Asn Gln Tyr Tyr Lys Ala Ser Glu Lys
   180 185 190
   Val Met Ile Glu Lys Thr Thr Lys Gly Gly Gln Ala Pro Val Gln Thr
   195 200 205
   Glu Ala Ser Ile Asp Gln Leu Tyr His Phe Thr Leu Lys Asp Gly Glu
   210 215 220
   Ser Ile Lys Val Thr Asn Leu Pro Val Gly Val Asp Tyr Val Val Thr
   225 230 235 240
   Glu Asp Asp Tyr Lys Ser Glu Lys Tyr Thr Thr Asn Val Glu Val Ser
   245 250 255
   Pro Gln Asp Gly Ala Val Lys Asn Ile Ala Gly Asn Ser Thr Glu Gln
   260 265 270
   Glu Thr Ser Thr Asp Lys Asp Met Thr Ile
   275 280
<210> 136
   <211> 93
   <212> PRT
   <213> Artificial Sequence
<220>
   <223> SpyCatcher-delta-N sequence
<400> 136
   Glu Asp Ser Ala Thr His Ile Lys Phe Ser Lys Arg Asp Glu Asp Gly
   1               5                   10                  15
   Lys Glu Leu Ala Gly Ala Thr Met Glu Leu Arg Asp Ser Ser Gly Lys
               20                  25                  30
   Thr Ile Ser Thr Trp Ile Ser Asp Gly Gln Val Lys Asp Phe Tyr Leu
           35                  40                  45
   Tyr Pro Gly Lys Tyr Thr Phe Val Glu Thr Ala Ala Pro Asp Gly Tyr
       50                  55                  60
   Glu Val Ala Thr Ala Ile Thr Phe Thr Val Asn Glu Gln Gly Gln Val
   65                  70                  75                  80
   Thr Val Asn Gly Lys Ala Thr Lys Gly Asp Ala His Ile
                   85                  90
<210> 137
   <211> 13
   <212> PRT
   <213> Artificial Sequence
<220>
   <223> Inversed spytaq sequence
<400> 137
   Lys Thr Pro Lys Tyr Ala Asp Val Met Val Ile His Ala
   1               5                   10

## Description

### FIELD OF THE INVENTION

The present invention provides compositions and methods for immunotherapeutic treatmentz of cancer using a chimeric antigen receptor (CAR) fused to VAR2CSA (VAR2-CAR) or a split-protein binding system (Spy-CAR), vectors encoding these constructs, and recombinant T-cells comprising the CARs.

### BACKGROUND OF THE INVENTION

In cell-based adoptive immunotherapy, immune cells isolated from a patient can be modified to express synthetic proteins that enable the cells to perform new therapeutic functions after they are subsequently transferred back into the patient. An example of such a synthetic protein is a chimeric antigen receptor (CAR). An example of a currently used CAR is a fusion of an extracellular target recognition domain (e.g., an antigen-binding ScFv chain), a transmembrane domain (e.g. from CD8 or CD28), and one or more intracellular signaling domains including CD28, OX40/4-1BB and CD3zeta. Upon antigen engagement, the intracellular signaling portion of the CAR can initiate an activation-related response in an immune cell; such are release of cytolytic molecules to induce tumor cell death, etc. Chimeric antigen receptors (CARs) are described in Porter et al., 2011, N. Engl. J. Med. 365:725-733; and Kalos et al., 2011, Sci. Transl. Med. 3:95ra73.

The split-protein binding system (SPBS) is characterized by the ability of two split-fragments of a protein to re-constitute into a stable covalently locked structure. An example of such a SPBS is the Spy-tag-Spy-Catcher system, which is comprised of the reactive sub-unit protein partners (Spy-Tag and Spy- Catcher) from the second immunoglobulin-like collagen adhesin domain (CnaB2) of the fibronectin-binding protein (FbaB) of *Streptococcus pyogenes* (PMID: 20235501). The SpyTag and SpyCatcher interact via a spontaneous isopeptide bond formation and is an example of a split-protein binding system (Zakeri, B. et al. PNAS. 2012). If one protein contain the SpyTag and another the SpyCather the two proteins will be spontaneously linked through a covalent interaction between the SpyTag and the SpyCather which ensures a high binding strength and a one-to-one interaction between the SpyTag and SpyCatcher linked proteins. The Spytag-SpyCatcher system, together with other split-protein binding systems, is described in Veggiani et al., 2014, Trends Biotechnol. Oct;32(10):506-12. The SpyCatcher protein-sequence (116 aa) displays some degree of immunogenicity *in vivo,* which can be reduced by truncating the N-terminal part of the protein (aa 1-24) (PMID: 25434527). While non-immunogenic, the AN-SpyCatcher sequence retains full binding capacity to the 13 aa Spytag sequence (PMID: 25434527) and can therefore be used for immuno-sensitive applications such as vaccine production or chimeric immune receptors.

Chondroitin sulfate A (CSA) belongs to the family of glycosaminoglycans (GAGs), which are linear polymers of alternating amino sugars and hexuronic acid residues, attached to proteoglycans. There are several types of GAGs including; chondroitin sulfate (CS), dermatan sulfate (DS or CSB), heparan sulfate (HS) and heparin. While the polysaccharide backbone of these GAGs is simple, considerable diversity arises in modifications such as sulfation and uronate epimerization. This is the basis for the wide variety in domain structure and biological activities of different GAGs. Abnormal or inappropriate expression of CSA has been reported in various human diseases including cancer. A CSA is chain is defined by being predominantly Chondroitin Sulfate A, however a CSA chain can also have a minority DS or CSC along the CSA chain.

The malaria parasite *Plasmodium falciparum* utilizes host cell proteoglycans in almost all stages of its complex life cycle. The sporozoite infects hepatocytes in the liver through surface-expressed circumsporozoite protein interacting with highly sulfated heparan sulfate proteoglycans (HSPG). Merozoite infection of the erythrocytes is mediated by EBA-175 binding to sialic acid on glycophorin A. In addition, a number of Plasmodium falciparum Erythrocyte Membrane Protein 1 (PfEMP1) proteins, mediating host endothelial adhesion, have been described as glycan-binding. One of these is VAR2CSA, which is a unique member of the PfEMP1 protein family. VAR2CSA binds with high affinity to a specific type of chondroitin sulfate A (CSA) attached to proteoglycans, so called Chondroitin Sulfate Proteoglycan (CSPG), in the intervillous spaces of the placenta. VAR2CSA is a large multidomain protein (350 kDa) expressed on the surface of *P. falciparum-infected* erythrocytes (IEs), and the VAR2CSA-CSA interaction is responsible for placenta specific sequestration in placental malaria (PM). Importantly, recombinant full-length VAR2CSA ecto-domain from FCR3 and 3D7 type parasites has shown affinity for CSA in the low nano-molar range.

WO 2013/117705 A1 discloses functional binding fragments comprising the minimal binding fragments of VAR2CSA as well as antibodies against them. It also discloses fusion proteins of VAR2CSA polypeptides with a therapeutic or diagnostic effector moiety, such as a cytotoxic moiety, a fluorescent label, and/or a radiolabel, for use in treatment of conditions associated with expression of CSA, such as inappropriate expression of CSA in cancer.

### OBJECT OF THE INVENTION

It is an object of embodiments of the invention to provide compositions and methods for immunotherapeutic treatment of diseases using a chimeric antigen receptor (CAR) fused with VAR2CSA (VAR2-CAR) or with a split-protein binding system (Spy-CAR), vectors encoding the same, and recombinant T-cells comprising the CAR. In some embodiments, the scFv portion of the CAR is substituted with one portion of a split-protein binding system such as SpyTag (SEQ ID NO: 130), SpyCatcher (SEQ ID NO: 129), or SpyCatcher-ΔN (SEQ ID NO: 136). In these embodiments, VAR2CSA peptides or fragments hereof mono- or bi-specific, are attached to a CAR through the split-protein binding system. The invention also relates to CARs specific to chondroitin sulfate A (CSA) modifications using VAR2CSA. In some embodiments, the scFv portion of the CAR is substituted with VAR2CSA variants comprising at least the minimal CSA binding domain ID1-ID2a-. Other objects of embodiments of the invention are to provide methods for deploying split-protein binding enabled CARs for treating conditions associated with inappropriate expression of an antigen, such as a disease-associated antigen, such as disease-associated CSA. Other objects of embodiments of the invention are to provide methods wherein VAR2CSA chimeric constructs, polypeptides or fragments thereof are used to target tissue or cells in humans having an expression, such as inappropriate expression of CSA. The invention also includes VAR2CSA-based bi-specific fusion proteins (bi-specific VAR2) able to engraft an immune response to cells and tissues, such as fusion proteins between VAR2CSA and immune cell-binding domains, such as a scFv anti-CD3 domain.

### SUMMARY OF THE INVENTION

It has been found by the present inventors that recombinant VAR2CSA binds with high affinity and specificity to placental-like CSA chains on a limited repertoire of disease associated proteoglycans. More importantly, the present inventors have found that placental-like CSA chains are uniquely re-expressed in virtually all types of human tumors and that recombinant VAR2CSA proteins bind with high and specific affinity to placental-like CSA on cancer cells and within primary cancer tissues. This function of VAR2CSA can be used to provide constructs suitable for immunotherapy, which constructs comprise elements or domains of a CAR, such as including at least a CD28 and a CD3zeta domain.

Accordingly, the present inventors suggest using this specific and high affinity binding between VAR2CSA and CSA for grafting an immune response to human tumors and metastasis with high or otherwise inappropriate expression of placental-like CSA-modified proteoglycans. This can be obtained through a direct genetic fusion between the CAR and VAR2CSA (VAR2-CAR). It can also be obtained by fusing on fragment of a split-protein binding system, such as the SpyTag or SpyCatcher, to the CAR (Spy-CAR) and subsequently attach a recombinant VAR2CSA protein fused to the corresponding SpyTag or SpyCatcher *in vitro* or *in vivo.* The Spy-CAR may be used in other scenarios where the attached targeting molecule is different from VAR2CSA, such as an antibody or a fragment of an antibody. Engraftment of immune cells to tumor tissue or other tissue with high or inappropriate expression of placental-like CSA may also be obtained by using a bi-specific VAR2CSA protein with dual specificity to placental-like CSA and immune cells, such as CD3 positive cells. This is obtained by fusing VAR2CSA with a single chain antibody (such as scFv anti-CD3) specific to a certain population of immune cells (such as CD3-positive cells). By combining the CAR technology with the Split protein system, it is possible to make a universal CAR that can be used as a personalized treatment by further treating the patient with a cancer targeting protein fused to the other part of the split protein. Dosing can be controlled, treatment can be stopped and treatment can be changed during the course of a treatment regimen. In particular by making a Split system CAR and a corresponding VAR2CSA split protein allows for perfect control of monitoring of VAR2CSA protein fold, binding affinity, binding specificity and secondary modifications during Good Manufacturing Practices prior to treatment of a patient.

So, in a first aspect the present invention relates to a polypeptide comprising i) a first polypeptide domain being a transmembrane domain and an endodomain of a chimeric antigen receptor (CAR), and ii) a second extracellular polypeptide domain being a VAR2CSA polypeptide.

It is to be understood that any of the constructs described herein including CARs and peptide parts of a split-protein binding system may be made in any orientation, forward or reverse translated in the chimeric construct. Accordingly and as an example a spytaq sequence may be AHIVMVDAYKPTK (SEQ ID NO: 130) or the inversed KTPKYADVMVIHA (SEQ ID NO: 137).

In some embodiments the isolated protein fragment of VAR2CSA according to the present invention comprises ID2a.

In a second aspect the present invention relates to an isolated nucleic acid molecule comprising a nucleotide sequences encoding a polypeptide of the invention.

In a third aspect the present invention relates to an expression vector comprising an isolated nucleic acid molecule comprising a nucleotide sequences encoding a polypeptide of the invention.

In a further aspect the present invention relates to a cell comprising a vector of the invention. In some embodiments, the cell is selected from the group consisting of an αβT cell, γδ T cell, a Natural Killer (NK) cell, a cytotoxic T lymphocyte (CTL), a regulatory T cell, or any combination thereof.

In a further aspect the present invention relates to a two-component therapeutic comprising
a) a first component being a polypeptide comprising the first polypeptide domain as defined in the first aspect of the invention and a peptide part of a split-protein binding system attached to the extracellular part of said first polypeptide domain and
b) a second component being a polypeptide comprising the second extracellular polypeptide domain as defined in the first aspect of the invention and a peptide part of a split-protein binding system; wherein said peptide parts of a split-protein binding system defined by components a) and b) are selected from K-Tag (SEO ID NO: 132), SpyCatcher (SEO ID NO: 129), SpyCatcher-ΔN (SEQ ID NO: 136), SpyTag (SEQ ID NO: 130), Minimal Spytag sequence (SEQ ID NO:131), split-Spy0128 (SEO ID NO: 135), isopeptide Spy0128 (SEO ID NO: 134), or any inverse sequence thereof, or a variant thereof with sequence identity of at least about 80%, such as at least about 82, 84, 86, 88, 90, 92, 94, 96, 98, or 99%, and spontaneously form an irreversible isopeptide bond.

In a further aspect the present invention relates to a vector encoding a polypeptide comprising the first polypeptide domain as defined in the first aspect of the invention and the second extracellular polypeptide domain as defined in the first aspect of the invention for use in a method of treating or preventing the occurrence of any indication or condition associated with expression, such as inappropriate expression of CSA, such as in cancer, in a subject; the method comprising: (a) culturing a population of cytotoxic lymphocytes under conditions promoting activation: (b) transfecting the lymphocyte population of (a) with said vector; (c) administering a therapeutically or prophylactically effective number of the transfected lymphocytes of (b) to a subject having or in risk at said indication; wherein the polypeptide comprising the domains of a CAR has binding specificity for the targeted moiety or can be bound by the targeted moiety; thereby treating or preventing said indication in said subject.

In a further aspect the present invention relates to a vector encoding a polypeptide comprising the first polypeptide domain as defined in the first aspect of the invention and a peptide part of a split-protein binding system attached to the extracellular part of said first polypeptide domain; for use in a method of treating or preventing the occurrence of any indication or condition associated with expression, such as inappropriate expression of CSA, such as in cancer, in a subject; the method comprising: (a) culturing a population of cytotoxic lymphocytes under conditions promoting activation; (b) transfecting the lymphocyte population of (a) with said vector; (c) administering a therapeutically or prophylactically effective number of the transfected lymphocytes of (b) to a subject having or in risk at said indication; and (d) administering a polypeptide comprising the second extracellular polypeptide domain as defined in the first aspect of the invention and a peptide part of a split-protein binding system, wherein said peptide parts of a split-protein binding system defined in the two different polypeptides used are selected from K-Tag (SEQ ID NO: 132), SpyCatcher (SEQ ID NO: 129), SpyCatcher-ΔN (SEQ ID NO: 136), SpyTag (SEQ ID NO: 130), Minimal Spytag sequence (SEO ID NO: 131), split-Spy0128 (SEO ID NO:135), isopeptide Spy0128 (SEO ID NO: 134), or any inverse sequence thereof, or a variant thereof with sequence identity of at least about 80%, such as at least about 82, 84, 86, 88, 90, 92, 94, 96, 98, or 99%, and spontaneously form an irreversible isopeptide bond; thereby treating or preventing said indication in said subject.

In a further aspect the present invention relates to a polypeptide according to the invention, or two-component therapeutic according to the invention for use as a medicament.

In a further aspect the present invention relates to a pharmaceutical composition comprising the polypeptide according to the invention, or two- component therapeutic according to the invention.

In a further aspect the present invention relates to a polypeptide according to the invention, or two- component therapeutic according to the invention, or pharmaceutical composition according to the invention for the treatment of any indications associated with a condition involving expression, such as inappropriate expression of CSA, such as in cancer.

Disclosed herein is further a polypeptide according to the invention, or two-component therapeutic according to the invention, or pharmaceutical composition according to the invention in combination with any other suitable anticancer agent.

In some embodiments the cancer is selected from CSA-expressing malignancies including carcinomas (including but not limited to Breast carcinoma, Pancreatic carcinoma, Ovarian carcinoma, Endometrial carcinoma, Hepatocellular carcinoma, Lung carcinoma, Colon carcinoma, Prostate carcinoma, Cervix carcinoma, Testis carcinoma, Basal cell skin carcinoma, Clear cell renal cell carcinoma, Head and neck squamous cell carcinoma, Skin squamous cell carcinoma, Vulvar kreatinized squamous cell carcinoma and Vulvar basal cell carcinoma), sarcomas (including but not limited to Fibrosarcoma, Dedifferentiated chondroand liposarcoma, Leiomyosarcoma, Liposarcoma, Myxoid liposarcoma, Uterine corpus leiomyosarcoma, Osteosarcoma, Ewing sarcoma and Rhabdomyosarcoma, Synovial sarcoma, Solitary Fibrous tumor), hematopoietic cancers (including but not limited to Chronic lymphatic leukaemia (CLL), Acute lymphatic leukaemia (ALL), Acute myeloid leukaemia (AML), B-cell, T-cell and large granular lymphoma), tumours of neuroepithelial tissue, such but not limited to Astrocytomas (Pleomorphic Xanthoastrocytoma, Fibrillary Astrocytomas, Anaplastic astrocytoma, Glioblastoma Multiforme), Oligodrendroglioma, Ependymoma, Choroid plexus turmor, Oligoastrocytoma, gliosarcoma, Ganglioglioma, Retinoblastoma, Neurocytoma, Neuroblastomas (Esthesioneuroblastoma and Ganglioneuroblastoma), Medulloblastoma, Atypical Teratoid Rhabdoid tumors and all types of neuroendocrine cancer, and any other CSA-expressing cancer.

It is to be understood that for a fusion or chimeric protein comprising a VAR2CSA polypeptide, any VAR2CSA sequences and polypeptides as defined herein may be used. Accordingly, this aspect is not limited to the use of minimal binding fragments. This applies whenever the term VAR2CSA sequence or polypeptide is used and is accordingly equally relevant when used for medical treatment or targeting.

### DETAILED DISCLOSURE OF THE INVENTION

In some embodiments, the polypeptides of the invention comprise the scFv portion of the CAR and a VAR2CSA peptide. In other embodiments, recombinant VAR2CSA peptides are attached to a CAR using a split-protein binding system. The invention also includes VAR2CSA-based fusion proteins able to recruit an immune response to cells and tissues expressing placental-like CSA through dual binding to placental-like CSA and immune cell-specific receptors (bi-specific VAR2).

### Mono- or bi-specific VAR2-CARs:

The polypeptide constructs according to the present invention may comprise VAR2CSA in conjunction to a transmembrane domain, and the endodomain of a CAR. In some aspects of the present invention, immune cells of a host are modified to express said polypeptide constructs according to the present invention comprising a CAR. Genetic engineering of human T lymphocytes to express tumor-directed chimeric antigen receptors (CAR) can produce anti-tumor effector cells that bypass tumor immune escape mechanisms that are due to abnormalities in protein-antigen processing and presentation. Moreover, these transgenic receptors can be directed to tumor-associated antigens that are not proteinderived. In certain embodiments of the disclosure there are CTLs that are modified to comprise at least a CAR.

In particular cases, the cytotoxic T lymphocytes (CTLs) include a receptor that is chimeric, non-natural and engineered at least in part by the hand of man. In particular cases, the polypeptide constructs according to the present invention, in addition to the CAR further comprises one, two, three, four, or more components, and in some embodiments the one or more components facilitate targeting or binding of the T lymphocyte to the tumor antigencomprising cancer cell, such as a VAR2CSA polypeptide. In specific embodiments, the polypeptide constructs according to the present invention comprises a CAR, as part or all of a cytoplasmic signaling domain, and/or part or all of one or more co-stimulatory molecules, for example endodomains of co-stimulatory molecules, and a VAR2CSA polypeptide alone (mono-specific) or fused with an antibody, single chain scFV or recombinant peptide specific for a tumor antigen (bi-specific VAR2-CAR). In certain aspects the antibody is directed at target antigens on the cell surface of cancer cells that are treatable by Temozolomide (TMZ), for example. In certain embodiments, a cytoplasmic signaling domain, such as those derived from the T cell receptor ζ-chain, is employed as at least part of the chimeric receptor in order to produce stimulatory signals for T lymphocyte proliferation and effector function following engagement of the chimeric receptor with the target antigen. Examples would include, but are not limited to, endodomains from co- stimulatory molecules such as CD27, CD28, 4- IBB, and OX40 or the signaling components of cytokine receptors such as IL7 and IL15. In particular embodiments, co- stimulatory molecules are employed to enhance the activation, proliferation, and cytotoxicity of T cells produced by the CAR after antigen engagement. In specific embodiments, the co- stimulatory molecules are CD28, OX40, and 4-1BB and cytokine and the cytokine receptors are IL7 and IL15.

The CAR may be first generation, second generation, or third generation (CAR in which signaling is provided by CD3ζ together with co- stimulation provided by CD28 and a tumor necrosis factor receptor (TNFr) domain, such as 4-1BB or OX40) or any other CAR comprised of at least one of these domains. The polypeptide construct according to the present invention VAR2-CAR may also contains a signal peptide (SP) so that the CAR part of the construct can be glycosylated and anchored in the cell membrane of the immune effector cell. The transmembrane domain (TD) connects the ectodomain to the endodomain and resides within the cell membrane when expressed by a cell and may be derived either from a natural or from a synthetic source. Where the source is natural, the domain may be derived from any membrane-bound or transmembrane protein. For example, the transmembrane region may be derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, or CD154. Alternatively the TD may be synthetic, in which case it will comprise predominantly hydrophobic residues such as leucine and valine. In some cases, a triplet of phenylalanine, tryptophan and valine will be found at each end of a synthetic TD.

A CAR may further comprise a hinge sequence. A hinge sequence is a short sequence of amino acids that facilitates protein flexibility (see, e.g., Woof et al, Nat. Rev. Immunol, 4(2): 89-99 (2004)). The hinge sequence may be positioned between the antigen recognition moiety and the TD. The hinge sequence can be any suitable sequence derived or obtained from any suitable molecule. In some embodiments, for example, the hinge sequence is derived from a CD8a molecule or a CD28 molecule, or derived from an IgG, such as the IgG1 hinge region.

The polypeptide constructs according to the present invention comprising a VAR2-CAR may have dual specificity to other disease-associated epitopes including, but not limited to, CD 19, CD20, CD22, Kappa or light chain, CD30, CD33, CD 123, CD38, ROR1, HER2/ErbB2, ErbB3/4, EGFR, EGFRvIII, EphA2, FAP, carcinoembryonic antigen, EGP2, EGP40, mesothelin, TAG72, PSMA, NKG2D ligands, B7-H6, IL-13 receptor cc2, IL-11 receptor a, MUC1, MUC16, CA9, GD2, GD3, HMW-MAA, CD171, Lewis Y, G250/CAIX, HLA-AI MAGE Al, HLA-A2 NY- ESO-1, PSC1, folate receptor- a, CD44v7/8, 8H9, NCAM, VEGF receptors, 5T4, Fetal AchR, NKG2D ligands, CD44v6, TEM1, TEM8, viral- associated antigens expressed by the tumor, or other tumor-associated antigens that are identified through genomic analysis and or differential expression studies of tumors.

The polypeptide constructs according to the present invention comprising the transmembrane domain, and the endodomain of a chimeric antigen receptor (CAR) may further comprise other regulatory elements or suicide gene systems, such as inducible caspase-9 (iCASP9) Clackson T. et al. (1998). Proc. Natl. Acad. Sci. U.S.A. 95 10437-10442; Straathof K. C. et al. (2005). Blood 105 4247-4254, or Sleeping Beauty (SB) transposon system Maiti SN et al, J Immunother. 2013 Feb;36(2): 112-23.

### THE SPLIT-PROTEIN BINDING SYSTEM (SPBS):

In the present invention, the SpyTag-SpyCatcher SPBS may be combined with CAR technology. Substituting the scFv domain of a CAR construct with a fragment of a SPBS, such as the SpyCatcher sequence, will allow for posttranslational attachment of a targeting unit (such as an scFv, and antibody or a recombinant protein) fused to the second half of the SPBS to the CAR. The interaction between SpyTag and SpyCatcher occurs when the unprotonated amine of Lys31 nucleophilically attacks the carbonyl carbon of Asp117, catalyzed by the neighboring Glu77. The minimal peptide to mediate this binding is AHIVMVDA (SEQ ID NO:131) whereas a c-terminal extension giving the sequence: AHIVMVDAYKPTK (SEQ ID NO: 130) provides the most optimal region, designated "SpyTag" (Zakeri et al PNAS 2012).

SpyCatcher is a part of the CnaB2 domain from the FbaB protein from Streptococcus pyogenes and binds SpyTag consisting of another part of the CnaB2 domain. When these two polypeptides of the CnaB2 domain are mixed, they will spontaneously form an irreversible isopeptide bond thereby completing the formation of the CnaB2 domain.

A K-Tag/SpyTag/SpyLigase system may also be used in the present invention. The CnaB2 domain from *Streptococcus pyogenes* can be used to generate a covalent peptide-peptide ligation system (Fierer JO. et al. 2014). This is done by splitting the CnaB2 into three parts a) the 13 amino acid SpyTag (SEQ ID NO: 130), b) the β-strand of CnaB2 (SEQ ID NO 132)) named K-Tag, and c) the SpyLigase (SEQ ID NO: 133) constructed from the remaining SpyCatcher polypeptide. By expressing the targeting protein with the small K-Tag fused at the C- or N-terminus and mixing that fusion protein with SpyTag-displaying T-Cells together with the SpyLigase, the K-Tag-fusion antigen will be attached to the SpyTag-VLPs by covalent ligation of the SpyTag with the K-Tag facilitated by the SpyLigase. Conversely, the K-Tag may also be inserted genetically into a CAR whereby the targeting antigen should then be fused to the SpyTag at the C- or N-terminus.

As part of a similar strategy for covalent coupling of targeting-antigens at the surface of T-cells, another pair of split-protein binding partners may be used in the present invention. The major pilin protein, Spy0128, from *Streptococcus pyogenes* can be split into two fragments (split-Spy0128 (residues 18-299 of Spy0128) (SEQ ID NO 135) and isopeptide (residues 293-308 of Spy0128 (TDKDMTITFTNKKDAE))) (SEQ ID NO 134), which together are capable of forming an intermolecular covalent complex (Zakeri, B. et al. 2010). In line with the described SpyTag-SpyCatcher strategy, the Spy0128 isopeptide can be inserted into the either the TCR or the targeting antigen and the same for the split-Spy0128 binding partner. Again simple mixing, or pre-injection of tagged targeting protein will result in a covalent interaction between the tagged CAR on a T-cell and the targeting antigen.

The Spy-CAR may also be used to construct bi- and multi-specific CARs by attaching K-tagged antibodies, K-tag-linked antibodies, K-tag-linked scFv chains, K-tagged recombinant proteins, or combinations hereof to the Spy-CAR obtaining bi- or multi-specific CAR constructs.

It is to be understood that the SpyTag may be replaced by a Spy0128 isopeptide, and the SpyCatcher replaced by split-Spy0128.

Currently used CARs are not easily pharmacologically controlled. The use of a split-protein binding system may facilitate CARs able to be activated and deactivated against a specific antigen *in vivo.*

One important aspect of the present invention is to i) collect, ii) modify and iii) infuse a population of T-cells from a subject with high or inappropriate expression of placental-like CSA, such as a cancer patient. In the collection step, T-cells are purified from a patient blood sample. In the modification step, the T-cell population is transfected with a vector encoding a CAR consisting of at least an extracellular split-protein binding domain (such as a spyTag or a SpyCatcher), a transmembrane domain (such as CD8 or CD28), and an activation domain (such as CD3ζ). The VAR2CSA polypeptide tagged, conjugated or fused to the other part of split-protein binding system is subsequently administered intravenously. This molecule will bind to the corresponding part of the split protein presented by the CAR, which will enable the CAR to bind the target of interest and activate. In the infusion step, recombinant T-cells expressing the CARs are injected into the patient establishing a population of T-cells able to react against the target.

This principle may be applied to any tumor binding molecule (including but not limited to antibodies, scFv single chains, and recombinant proteins) and will have the advantage that:
a) it is possible to target many different tumor antigens or targets with the same CAR infusion.
b) it is possible to revert the treatment by suspending infusion of spyTagged (or similar peptide) tumor-target molecules.
c) it is possible to use complex targeting agents such as recombinant VAR2CSA that are not easily made chimeric as part of a CAR construct.

### Bi-specific VAR2 proteins:

As VARCSA can be produced as a single chain recombinant protein, it can be fused with other single chain proteins able to recognize specific disease targets. For example, fusing VAR2CSA to a scFv chain targeting CD3 will be able to recruit CD3-expressing immune cells to cells and tissues expressing placental-like CSA. Accordingly, such a dual specificity VAR2CSA-based molecule may be used to graft a T-cell response to cells and tissues with expression of placental-like CSA. In some embodiments, VAR2CSA may be genetically fused to a single chain antibody such as an scFv chain or nanobody creating a bi-specific VAR2CSA protein. In some embodiments, such a bi-specific VAR2CSA protein may contain a split-protein binding tag, such as a SpyTag or a K-Tag. In some embodiments, a bi-specific VAR2CSA protein may be attached to a CAR through a split binding protein system.

### VAR2CSA polypeptides

The present invention is based on the fact that a part of a malaria protein, the so-called VAR2CSA, can bind to a cancer specific CSA and extra-cellular CSA-conjugated CSPG with very high specificity and very high binding strength. This part of the technology is described in WO2013117705 A1.

CSA interacts with many important factors such as growth hormones, cytokines, chemokines, and adhesion molecules and is thought to be involved in structural stabilization, cytokinesis, cell proliferation, differentiation, cell migration, tissue morphogenesis, organogenesis, infection, and wound repair. CS chains are composed of alternating units of N-acetyl-D-galactosamine (GalNAc) and glucuronic acid residues. Glucuronic acid can be sulfated at its C2 position and GalNAc can be sulfated at C4 and/or C6, giving rise to various disaccharide units. Varying modifications of the sugar backbone allows structural and functional heterogeneity of the CS chains. Placenta adhering *P. falciparum* parasites specifically associate with CS chains that are predominantly C4 sulfated.

Recombinant VAR2CSA protein has been shown to bind with unprecedented high affinity and specificity to CSA. This may be due to an interaction with CSA that is not only dependent on the charged sulfates but also on the CS backbone. The inventors of the present invention have demonstrated that CSA present in the placenta is very similar to the CSA presented on various cancer cells including cancer stem cells. This is substantiated by the fact that VAR2CSA expressing malaria parasites adhere specifically to CSA on C32 melanoma cells and to human brain cancer cells.

Accordingly, the current invention relies on the high affinity and specificity between VAR2CSA recombinant proteins and placental-like CSA. By tagging this protein the invention can be used in a wide range of applications including the tracking of metastases in vivo and to diagnose metastatic disease. By coupling VAR2CSA to an apoptotic or cytotoxic reagent the invention can be used to specifically target and eliminate cancer cells and cancer stem cells. By simple therapy using VAR2CSA recombinant protein it will be possible to neutralize the activity of CSA thereby inhibiting tumorigenesis and/or metastasis of CSA-expressing cancer cells. CSA can be present on a number of protein backbones, e.g. CSPG4, CD44, biglycan, decorin, versican, aggrecan (the major CSPG in cartilage), perlecan, syndecan,.

The present invention is envisioned to be particularly relevant to malignant melanoma cancer including cutaneous, ocular and conjuctival melanoma having CSPG4 with CSA chains on the surface of the melanoma cells. This GAG chain is believed to be involved in mitoses and metastases. However, CSPG4 is not only specific to melanoma. Micro- and tissue array analyses, performed by the inventors on data from large panels of human tissue and cell lines, suggest that CSPG4 and other types of CSA-containing proteoglycans may be present on a wide range of cancer types originating from all three cellular germ layers. These cancer types include carcinomas (Breast carcinoma, Pancreatic carcinoma, Ovarian carcinoma, Endometrial carcinoma, Hepatocellular carcinoma, Lung carcinoma, Colon carcinoma, Prostate carcinoma, Cervix carcinoma, Testis carcinoma, Basal cell skin carcinoma, Clear cell renal cell carcinoma, Kreatinized head and neck squamous cell carcinoma, Skin squamous cell carcinoma, Vulvar kreatinized squamous cell carcinoma and Vulvar basal cell carcinoma), sarcomas (Breast liposarcoma, Fibrosarcoma, Dedifferentiated chondro- and liposarcoma, Leiomyosarcoma, Liposarcoma, Myxoid liposarcoma, Uterine corpus leiomyosarcoma, Osteosarcoma, Ewing sarcoma and Rhabdomyosarcoma), hematopoietic cancers (Chronic lymphatic leukaemia (CLL), Acute lymphatic leukaemia (ALL), Acute myeloid leukaemia (AML), B-cell, T-cell and large granular lymphoma), tumours of neuroepithelial tissue, such as Astrocytomas (Pleomorphic Xanthoastrocytoma, Fibrillary Astrocytomas, Anaplastic astrocytoma, Glioblastoma Multiforme), Oligodrendroglioma, Ependymoma, Choroid plexus turmor, Oligoastrocytoma, gliosarcoma, Ganglioglioma, Retinoblastoma, Neurocytoma, Neuroblastomas (Esthesioneuroblastoma and Ganglioneuroblastoma), Medulloblastoma, Atypical Teratoid Rhabdoid tumors and all types of neuroendocrine cancer.

The inventors of the present invention have identified a malaria protein, VAR2CSA, which binds CSA in the intervillous spaces of the placenta with an affinity below 10 nM. Smaller recombinant parts of VAR2CSA have been produced at high yields that bind CSA with characteristics similar to that of the full-length and native VAR2CSA protein. The recombinant VAR2CSA protein does not bind other types of CS such as chondroitin sulfate C (C6S) or highly sulfated GAGs such as heparan sulfate (HS). Recombinant proteins can be produced to bind with high affinity to CSA in various expression systems, here among S2 cells, T.Ni cells, CHO cells and E. coli strains including BL21 and Shuffle cells (tm New England Biolabs).

The inventors of the present invention have also identified a single small (75 kDa) antigen that binds CSA with very high affinity (nM) and high specificity. Table 3 (See example 2) lists the CSA affinity of all the analyzed VAR2CSA proteins using biosensor technology.

The inventors of the present invention have shown that this VAR2CSA recombinant protein binds strongly at low concentrations to a wide range of cancer cell lines including cutaneous Melanoma (C32, MeWo), Lung carcinoma (A549), Breast carcinoma (HCC1395), Osterosarcoma (U2OS, MNNG/HOS), Rhabdomyosarcoma (RH30) and cutaneous T-cell lymphoma (Table 4 and 5). As a control molecule another VAR2CSA protein was used, which is identical to the minimal binding VAR2CSA construct except for a 151 amino acids truncation in the C-terminal part of the molecule. This truncation removes the CSA binding. Recombinant VAR2CSA binds all CSPG4 expressing cell lines and cancer cell lines expressing other CSPG molecules having CSA chains (e.g. T-cell lymphoma). Recombinant VAR2CSA protein fails to interact with human red blood cells and peripheral blood mononuclear cells (PBMC) (Table 4).

The inventors of the present invention have shown herein that malaria parasites adhere to cancer cells and primary human tumors, through a specific interaction between VAR2CSA and a placental-like CSA chain attached to a given proteoglycan. Thus, it is envisioned that recombinant VAR2CSA and conjugates thereof may be used to direct an immune response to the tumor environment.

The advantages of targeting CSA on tumors with VAR2CSA over other current therapies in development are numerous:
1) The interaction between VAR2CSA and CSA is of unprecedented high affinity and highly specific.
2) VAR2CSA is an evolutionary refined malaria protein and it is thus unlikely that therapy will break tolerance and cause autoimmune reactions in the patient.
3) VAR2CSA is a stable protein that is well characterized and can be highly expressed in organisms compatible with large-scale protein production.
4) VAR2CSA is a polymorphic protein with a number of serovariants. Repeated therapy could be offered by different serovariants to avoid issues with neutralizing antibodies.
5) VAR2CSA is naturally exposed extracellularly on the *P. falciparum-infected* the red blood cell and is thus by nature a stable protein in human serum and has been shown to be highly protease resistant.

The advantages of using a bi-specific VAR2CSA-based molecule, such as an VAR2CSA-scFv-CD3 fusion protein, are numerous:
1) VAR2CSA binds a common cancer-associated CSA modification expressed by multiple malignant disease indications, thus a bi-specific VAR2SCA fusion protein can graft a T-cell response directly to the CSA-positive tumor environment.
2) As the malignancy-associated CSA modification is present on several proteoglycans expressed in a given malignant disease, it is unlikely that a given cancer will develop resistance towards a bi-specific VAR2CSA fusion protein by down regulating a specific proteoglycan.
3) As the VAR2CSA sequence has been naturally optimized in evolution by the *P. falciparum* malaria parasite to evade human immune defense mechanisms and serum proteases, it is unlikely that a patient will develop immunological (or otherwise) resistance towards a bi-specific VAR2CSA-fusion protein.
4) Evident by the fact that VAR2CSA-expressing malaria parasites only sequester to the placenta (and cannot be found elsewhere in the human body/ vasculature), a bi-specific VAR2CSA-fusion protein will be predicted to have minimum adverse effects in a treatment context.

The advantages of using VAR2CSA-CARs over scFv-based CARs are numerous:
1) VAR2CSA binds a common cancer-associated CSA modification expressed by multiple malignant disease indications, thus a VAR2-CAR has a broad but cancer specific targeting range and may be used in several cancer indications.
2) As the malignancy-associated CSA modification is present on several proteoglycans expressed in a given malignant disease, it is unlikely that a given cancer will develop resistance towards a VAR2CSA-CAR by down regulating a specific proteoglycan.
3) As the VAR2CSA sequence has been naturally optimized in evolution by the *P. falciparum* malaria parasite to evade human immune defense mechanisms and serum proteases, it is unlikely that a patient will develop immunological (or otherwise) resistance towards a VAR2CSA-based CAR.
4) Evident by the fact that VAR2CSA-expressing malaria parasites only sequester to the placenta (and cannot be found elsewhere in the human body/ vasculature), a VAR2CSA-CAR will be predicted to have minimum adverse effects in a treatment context.

The advantages of using CARs with a split-protein binding system (Spy-CAR) over traditional first, second, and third generation CARs are numerous:
1) A Spy-CAR does not by itself pose a targeting sequence and will not by itself react against a human antigen.
2) A Spy-CAR can be 'armed' with VAR2CSA-Spy protein, which will enable the VAR2CSA-Spy-Spy-CAR to react against a selected disease target.
3) A Spy-CAR can be 'armed' with any given targeting molecule that is fused with, or conjugated to, a corresponding fragment of the split-protein binding system, which will enable the Targeting sequence-Spy-Spy-CAR to react against a selected disease target.
4) A Spy-CAR transduced T-cell can be 'armed' with VAR2CSA-Spy protein *in vitro,* which will enable the VAR2CSA-Spy-Spy-CAR to react against a selected disease target.
5) A Spy-CAR transduced T-cell can be 'armed' with any given targeting molecule that is fused with, or conjugated to, a corresponding fragment of the split-protein binding system, *in vitro,* which will enable the targeting sequence-Spy-Spy-CAR to react against a selected disease target.
6) A Spy-CAR transduced T-cell can be 'armed' with any given polymeric complex of targeting molecules attached to each other through the K-tag *in vitro,* which will enable the targeting sequence-Spy-Spy-CAR to react against a selected disease target.
7) A Spy-CAR transduced T-cell can be 'armed' with VAR2CSA-Spy protein *in vivo* by chasing Spy-CAR transduced T-cells with a targeting sequence fused with, or conjugated to, a corresponding fragment of the split-protein binding system, which will enable the VAR2CSA-Spy-Spy-CAR to react against a selected disease target.
8) A Spy-CAR transduced T-cell can be 'armed' with any given targeting molecule that is fused with, or conjugated to, a corresponding fragment of the split-protein binding system, *in vivo* by chasing Spy-CAR transduced T-cells with a targeting sequence fused with, or conjugated to, a corresponding fragment of the split-protein binding system, which will enable the targeting sequence-Spy-Spy-CAR to react against a selected disease target.
9) A Spy-CAR transduced T-cell can be 'armed' with any given polymeric complex of targeting molecules attached to each other through the K-tag, *in vitro* or *in vivo* by chasing Spy-CAR transduced T-cells with a targeting sequence fused with, or conjugated to, a corresponding fragment of the split-protein binding system, which will enable the targeting sequence-Spy-Spy-CAR to react against a selected disease target.
10) As activated cytotoxic T-cells have a limited lifespan, VAR2CSA-Spy-Spy-CAR transduced T-cells, or T-cells armed with any given targeting molecule that is fused with, or conjugated to, a corresponding fragment of the split-protein binding system, will de cleared from the organism over time, while maintaining 'un-armed' Spy-CAR-expressing memory T-cells. As such, the VAR2CSA-Spy-Spy-CAR 'armed' T-cell population will be eliminated over time by natural clearance in the spleen. In case of disease recurrence, persistent memory T-cells expressing only the Spy-CAR can then be 're-armed' *in vivo* by chasing with the same or a new disease targeting sequence fused to the corresponding fragment the split-protein binding system.
11) A spy-CAR can be armed with any cancer-targeting moiety that cannot be produced in the recombinant T-cell as a chimeric protein
12) During a CAR treatment period a spy-CAR can be 'armed' first with one spy-tagged protein and as resistance occurs, or if the cancer changes phenotype, or an immune response to the spy-tagged protein is induced, the spy-CAR can be armed with another second-line spy-tagged cancer targeting molecule.

### Definitions

The term "SpyTag" refers to a part of the CnaB2 domain from the FbaB protein from *Streptococcus pyogenes* optimized to bind SpyCatcher consisting of another part of the CnaB2 domain (SEQ ID NO: 130 and 131). The interaction occurs when the unprotonated amine of Lys31 nucleophilically attacks the carbonyl carbon of Asp117, catalyzed by the neighboring Glu77. The minimal peptide to mediate this binding is AHIVMVDA whereas a c-terminal extension giving the sequence: AHIVMVDAYKPTK provides the most optimal region, designated "SpyTag" (Zakeri, B. et al. PNAS. 2012).

The term "SpyCatcher" refers to a part of the CnaB2 domain from the FbaB protein from *Streptococcus pyogenes* optimized to bind SpyTag consisting of another part of the CnaB2 domain (SEQ ID NO: 129). SpyCatcher can be residue number 1-113 of CnaB2 and binding can be optimized by the following two mutations: I34E and M69Y (Zakeri, B. et al. PNAS, 2012).

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

The terms "antibodies" and "immunoglobulin" include antibodies or immunoglobulins of any isotype, fragments of antibodies which retain specific binding to antigen, including, but not limited to, Fab, Fv, scFv, and Fd fragments, chimeric antibodies, humanized antibodies, single-chain antibodies, and fusion proteins comprising an antigen-binding portion of an antibody and a non-antibody protein.

"Antibody fragments" comprise a portion of an intact antibody, for example, the antigen binding or variable region of the intact antibody. Examples of antibody fragments include Fab, Fab', F(ab')2, and Fv fragments; diabodies; linear antibodies (Zapata et al., Protein Eng. 8(10): 1057-1062 (1995)); single-chain antibody molecules; and multispecific antibodies formed from antibody fragments. Papain digestion of antibodies produces two identical antigen -binding fragments, called "Fab" fragments, each with a single antigen-binding site, and a residual "Fc" fragment, a designation reflecting the ability to crystallize readily.

The term "VAR2CSA polypeptide" as used herein refers to the extracellular part of a specific Erythrocyte Membrane Protein 1 (PfEMP1) protein expressed by *Plasmodium falciparum* interacting with chondroitin sulfate proteoglycans (CSPG) and characterized by having a sequence of SEQ ID NO:55 or SEQ ID NO:56 or fragments or variants thereof with the ability to bind chondroitin sulfate A (CSA) that could be presented on a proteoglycans (CSPG).

In some embodiments, the VAR2CSA polypeptide according to the present invention at least comprises the protein fragment of VAR2CSA, which fragment consist of a sequential amino acid sequence of a) ID1, and b) DBL2Xb.

In some embodiments, the VAR2CSA polypeptide according to the present invention at least comprises the protein fragment of VAR2CSA, which fragment consist of a sequential amino acid sequence of a) ID1, and b) DBL2Xb, and c) ID2a.

Included within the definition of a VAR2CSA polypeptide is polypetides described in Salanti A. et al Mol. Micro 2003 Jul;49(1): 179-91, in Khunrae P. et al, J Mol Biol. 2010 Apr 2;397(3): 826-34, in Srivastava A. et al, Proc Natl Acad Sci U S A. 2010 Mar 16;107(11):4884-9, in Dahlbäck M. et al, J Biol Chem. 2011 May 6;286(18):15908-17, or in Srivastava A. et al, PLoS One. 2011;6(5):e20270.

The term "ID1" as used herein refers to a domain of VAR2CSA characterized by having an amino acid sequence with at least 70 % sequence identity to an amino acid sequence identified by 1-152 of SEQ ID NO:1.

The term "DBL2Xb" as used herein refers to a domain of VAR2CSA characterized by having an amino acid sequence with at least 70 % sequence identity with to amino acid sequence identified by 153-577 of SEQ ID NO:1.

The term "ID2a" as used herein refers to a domain of VAR2CSA characterized by having an amino acid sequence of at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, or at least 62, such as the 63 consecutive amino acids from the N-terminal of amino acids 578-640 of SEQ ID NO:1 and with at least 70 % sequence identity to such a sequence of consecutive amino acids.

In some embodiments an amino acid sequence identity referred to herein of at least 70 % of any one sequence identified by SEQ ID NO:1-75 or a fragment thereof, refers to a sequence with at least 71, 72, 73, 74, 75, 76, 77, 78, 79, 80, 81, 8, 83, 84, 85, 86, 87, 88, 89, 90, 91, 92, 93, 94, 95, 96, 97, 98, or 99 % sequence identity to this sequence.

The terms "variant" or "variants", as used herein, refers to a VAR2CSA polypeptide having an amino acid sequence of SEQ ID NO:55 or SEQ ID NO:56 or a fragments a VAR2CSA polypeptide comprising an amino acid sequence of SEQ ID NO:1-54, which fragments or variants retain the ability to bind chondroitin sulfate A (CSA) on proteoglycans (CSPG), wherein one or more amino acids have been substituted by another amino acid and/or wherein one or more amino acids have been deleted and/or wherein one or more amino acids have been inserted in the polypeptide and/or wherein one or more amino acids have been added to the polypeptide. Such addition can take place either at the N-terminal end or at the C-terminal end or both. The "variant" or "variants" within this definition still have functional activity in terms of being able to bind chondroitin sulfate A (CSA). In some embodiment a variant has at least 70 %, such as at least 71, 72, 73, 74, 75, 76, 77, 78, 79, 80, 81, 8, 83, 84, 85, 86, 87, 88, 89, 90, 91, 92, 93, 94, 95, 96, 97, 98, or 99 % sequence identity with the sequence of SEQ ID NO: 1-75, and 128, such as the sequence of SEQ ID NO: 1, 3-5, 10, 11, 29, 34, 36-38, 41, 43-45, 48, 53-56, 60-70, 72-75, and 128.

The phrases "functional variant", "functional fragment", and "functional derivatives" as used herein refers to variants, fragments, truncated versions, as well as derivatives of SEQ ID NO:55 or SEQ ID NO:56, such as any one of SEQ ID NO:1, 3-5, 10, 11, 29, 34, 36-38, 41, 43-45, 48, 53-56, 60-70, 72-75, and 128, which polypeptides comprises essential binding sequence parts of SEQ ID NO:55 or SEQ ID NO:56 and at least possess the ability to bind chondroitin sulfate A (CSA). It is to be understood that a VAR2CSA functional variant or functional fragment may have two or three features selected from being a both a variant, and/or a fragment and/or a derivative.

A functional variant or fragment of a VAR2CSA polypeptide encompass those that exhibit at least about 25%, such as at least about 50%, such as at least about 75%, such as at least about 90% of the binding affinity of wild-type VAR2CSA polypeptide that has been produced in the same cell type, when tested in the assays as described herein.

The term "immunologic fragment" as used herein refers to fragment of an amino acid sequence that possess essentially the same functional activities and the same spatial orientation to be recognized by an antibody. Accordingly a specific antibody will bind both the polypeptide and immunologic fragments thereof.

The term "another amino acid" as used herein means one amino acid that is different from that amino acid naturally present at that position. This includes but is not limited to amino acids that can be encoded by a polynucleotide. In some embodiments the different amino acid is in natural L-form and can be encoded by a polynucleotide.

The term "derivative" as used herein, is intended to designate a VAR2CSA polypeptide exhibiting substantially the same or improved biological activity relative to wild-type VAR2CSA identified by SEQ ID NO:55 or SEQ ID NO:56, or a fragment thereof, in which one or more of the amino acids of the parent peptide have been chemically modified, e.g. by alkylation, PEGylation, acylation, ester formation or amide formation or the like.

The term "construct" is intended to indicate a polynucleotide segment which may be based on a complete or partial naturally occurring nucleotide sequence encoding the polypeptide of interest. The construct may optionally contain other polynucleotide segments. In a similar way, the term "amino acids which can be encoded by polynucleotide constructs" covers amino acids which can be encoded by the polynucleotide constructs defined above, i.e. amino acids such as Ala, Val, Leu, Ile, Met, Phe, Trp, Pro, Gly, Ser, Thr, Cys, Tyr, Asn, Glu, Lys, Arg, His, Asp and Gin.

The term "vector", as used herein, means any nucleic acid entity capable of the amplification in a host cell. Thus, the vector may be an autonomously replicating vector, i.e. a vector, which exists as an extra-chromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. The choice of vector will often depend on the host cell into which it is to be introduced. Vectors include, but are not limited to plasmid vectors, phage vectors, viruses or cosmid vectors. Vectors usually contain a replication origin and at least one selectable gene, i.e., a gene which encodes a product which is readily detectable or the presence of which is essential for cell growth.

As used herein the term "appropriate growth medium" means a medium containing nutrients and other components required for the growth of cells and the expression of the nucleic acid sequence encoding the VAR2CSA polypeptide of the invention.

In the present context, the term "treatment" is meant to include prevention, curing and alleviating the symptoms of a disease, disorder or condition involving expression, such as inappropriate expression of CSA, such as in cancer. Prophylactic and therapeutic administration of VAR2CSA polypeptide, conjugate or derivative according to the invention is thus included in the term "treatment".

The term "subject" as used herein means any animal, in particular mammals, such as humans, and may, where appropriate, be used interchangeably with the term "patient".

The term "sequence identity" as known in the art, refers to a relationship between the sequences of two or more polypeptide molecules or two or more nucleic acid molecules, as determined by comparing the sequences. In the art, "identity" also means the degree of sequence relatedness between nucleic acid molecules or between polypeptides, as the case may be, as determined by the number of matches between strings of two or more nucleotide residues or two or more amino acid residues. "Identity" measures the percent of identical matches between the smaller of two or more sequences with gap alignments (if any) addressed by a particular mathematical model or computer program (i.e., "algorithms").

The term "similarity" is a related concept, but in contrast to "identity", refers to a sequence relationship that includes both identical matches and conservative substitution matches. If two polypeptide sequences have, for example, (fraction (10/20)) identical amino acids, and the remainder are all non-conservative substitutions, then the percent identity and similarity would both be 50%. If, in the same example, there are 5 more positions where there are conservative substitutions, then the percent identity remains 50%, but the percent similarity would be 75% ((fraction (15/20))). Therefore, in cases where there are conservative substitutions, the degree of similarity between two polypeptides will be higher than the percent identity between those two polypeptides.

Conservative modifications to the amino acid sequence of SEQ ID NO: 1-56, 60-70, and 72-75, 128 (and the corresponding modifications to the encoding nucleotides) will produce VAR2CSA polypeptides having functional and chemical characteristics similar to those of naturally occurring VAR2CSA polypeptides. In contrast, substantial modifications in the functional and/or chemical characteristics of a VAR2CSA polypeptide may be accomplished by selecting substitutions in the amino acid sequence of SEQ ID NO: 1-56, 60-70, 72-75, and 128 that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis" (see, for example, MacLennan et al., 1998, Acta Physiol. Scand. Suppl. 643:55-67; Sasaki et al., 1998, Adv. Biophys. 35:1-24, which discuss alanine scanning mutagenesis).

Desired amino acid substitutions (whether conservative or non-conservative) can be determined by those skilled in the art at the time such substitutions are desired. For example, amino acid substitutions can be used to identify important residues of a polypeptide according to the present invention preferably containing VAR2CSA, or to increase or decrease the affinity of a polypeptide described herein.

Naturally occurring residues may be divided into classes based on common side chain properties:
1) hydrophobic: norleucine, Met, Ala, Val, Leu, Ile;
2) neutral hydrophilic: Cys, Ser, Thr, Asn, Gin;
3) acidic: Asp, Glu;
4) basic: His, Lys, Arg;
5) residues that influence chain orientation: Gly, Pro; and
6) aromatic: Trp, Tyr, Phe.

For example, non-conservative substitutions may involve the exchange of a member of one of these classes for a member from another class. Such substituted residues may be introduced into regions of the Plasmodium falciparum VAR2CSA polypeptide that are homologous with non-Plasmodium falciparum VAR2CSA polypeptides, or into the nonhomologous regions of the molecule.

In making such changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids may be substituted for other amino acids having a similar hydropathic index or score and still retain a similar biological activity. In making changes based upon the hydropathic index, the substitution of amino acids whose hydropathic indexes are within ±2 is preferred, those that are within ±1 are particularly preferred, and those within ±0.5 are even more particularly preferred.

It is also understood in the art that the substitution of like amino acids can be made effectively on the basis of hydrophilicity, particularly where the biologically functionally equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, i.e., with a biological property of the protein.

The following hydrophilicity values have been assigned to amino acid residues: arginine (+3.0); lysine ('3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids whose hydrophilicity values are within ±2 is preferred, those that are within ±1 are particularly preferred, and those within ±0.5 are even more particularly preferred. One may also identify epitopes from primary amino acid sequences on the basis of hydrophilicity. These regions are also referred to as "epitopic core regions."

A skilled artisan will be able to determine suitable variants of the polypeptide as set forth in SEQ ID NO:1-75, and 128 using well known techniques. For identifying suitable areas of the molecule that may be changed without destroying activity, one skilled in the art may target areas not believed to be important for activity. For example, when similar polypeptides with similar activities from the same species or from other species are known, one skilled in the art may compare the amino acid sequence of a VAR2CSA polypeptide to such similar polypeptides. With such a comparison, one can identify residues and portions of the molecules that are conserved among similar polypeptides. It will be appreciated that changes in areas of a VAR2CSA polypeptide that are not conserved relative to such similar polypeptides would be less likely to adversely affect the biological activity and/or structure of the VAR2CSA polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a VAR2CSA polypeptide that correspond to amino acid residues that are important for activity or structure in similar polypeptides. One skilled in the art may opt for chemically similar amino acid substitutions for such predicted important amino acid residues of VAR2CSA polypeptides and other polypeptides of the invention.

One skilled in the art can also analyze the three-dimensional structure and amino acid sequence in relation to that structure in similar polypeptides. In view of that information, one skilled in the art may predict the alignment of amino acid residues of a polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each desired amino acid residue. The variants can then be screened using activity assays as described herein. Such variants could be used to gather information about suitable variants. For example, if one discovered that a change to a particular amino acid residue resulted in destroyed, undesirably reduced, or unsuitable activity, variants with such a change would be avoided. In other words, based on information gathered from such routine experiments, one skilled in the art can readily determine the amino acids where further substitutions should be avoided either alone or in combination with other mutations.

A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol, 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins, which have a sequence identity of greater than 30%, or similarity greater than 40% often have similar structural topologies. The recent growth of the protein structural data base (PDB) has provided enhanced predictability of secondary structure, including the potential number of folds within a polypeptide's or protein's structure. See Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will gain dramatically in accuracy.

Additional methods of predicting secondary structure include "threading" (Jones, D., Curr. Opin. Struct. Biol., 7(3):377-87 (1997); Sippl et al., Structure, 4(1):15-9 (1996)), "profile analysis" (Bowie et al., Science, 253:164-170 (1991); Gribskov et al., Meth. Enzymol., 183:146-159 (1990); Gribskov et al., Proc. Nat. Acad. Sci., 84(13):4355-4358 (1987)), and "evolutionary linkage" (See Home, supra, and Brenner, supra).

Identity and similarity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math., 48:1073 (1988).

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., Nucl. Acid. Res., 12:387 (1984); Genetics Computer Group, University of Wisconsin, Madison, Wis.), BLASTP, BLASTN, and FASTA (Altschul et al., J. Mol. Biol., 215:403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

Certain alignment schemes for aligning two amino acid sequences may result in the matching of only a short region of the two sequences, and this small aligned region may have very high sequence identity even though there is no significant relationship between the two full length sequences. Accordingly, in a preferred embodiment, the selected alignment method (GAP program) will result in an alignment that spans at least 50 contiguous amino acids of the target polypeptide.

For example, using the computer algorithm GAP (Genetics Computer Group, University of Wisconsin, Madison, Wis.), two polypeptides for which the percent sequence identity is to be determined are aligned for optimal matching of their respective amino acids (the "matched span", as determined by the algorithm). A gap opening penalty (which is calculated as 3 times the average diagonal; the "average diagonal" is the average of the diagonal of the comparison matrix being used; the "diagonal" is the score or number assigned to each perfect amino acid match by the particular comparison matrix) and a gap extension penalty (which is usually {fraction (1/10)} times the gap opening penalty), as well as a comparison matrix such as PAM 250 or BLOSUM 62 are used in conjunction with the algorithm. A standard comparison matrix (see Dayhoff et al., Atlas of Protein Sequence and Structure, vol. 5, supp.3 (1978) for the PAM 250 comparison matrix; Henikoff et al., Proc. Natl. Acad. Sci USA, 89:10915-10919 (1992) for the BLOSUM 62 comparison matrix) is also used by the algorithm.

Preferred parameters for a polypeptide sequence comparison include the following:
Algorithm: Needleman et al., J. Mol. Biol, 48:443-453 (1970); Comparison matrix: BLOSUM 62 from Henikoff et al., Proc. Natl. Acad. Sci. USA, 89:10915-10919 (1992); Gap Penalty: 12, Gap Length Penalty: 4, Threshold of Similarity: 0.

The GAP program is useful with the above parameters. The aforementioned parameters are the default parameters for polypeptide comparisons (along with no penalty for end gaps) using the GAP algorithm.

Preferred parameters for nucleic acid molecule sequence comparisons include the following: Algorithm: Needleman et al., J. Mol Biol., 48:443-453 (1970); Comparison matrix: matches=+10, mismatch=0, Gap Penalty: 50, Gap Length Penalty: 3.

The GAP program is also useful with the above parameters. The aforementioned parameters are the default parameters for nucleic acid molecule comparisons.

Other exemplary algorithms, gap opening penalties, gap extension penalties, comparison matrices, thresholds of similarity, etc. may be used, including those set forth in the Program Manual, Wisconsin Package, Version 9, September, 1997. The particular choices to be made will be apparent to those of skill in the art and will depend on the specific comparison to be made, such as DNA to DNA, protein to protein, protein to DNA; and additionally, whether the comparison is between given pairs of sequences (in which case GAP or BestFit are generally preferred) or between one sequence and a large database of sequences (in which case FASTA or BLASTA are preferred).

The inventors of the present invention has now addressed and found the answers to the following key questions related to the molecular mechanism behind placental adhesion in PM: 1) is the described differential CSA adhesion related to the VAR2CSA sequence 2) what are the exact minimal structural requirements for VAR2CSA binding to CSA 3) what type of chemical interaction exists between VAR2CSA and CSA and finally 4) can this information be used to design an optimal vaccine antigen?

By expressing identical FCR3 and 3d7 VAR2CSA truncations, the present inventors showed that VAR2CSA bind CSA with similar affinity and specificity, regardless of parasite strain origin. These two sequences has a sequence identity of 79,6 %. The present inventors further demonstrate that the high CSA binding-affinity is retained in several shorter fragments, and that DBL2X, including small regions from the flanking interdomains, form a compact core that contains the high affinity CSA binding site. In silico the present inventors defined putative GAG binding sites in VAR2CSA and by deletion and substitution the present inventors showed that mutations in these sites have no effect on CSPG binding. Using the theory of polyelectrolyte-protein interactions the present inventors have shown that the VAR2CSA-CSA interaction may not, solely, be dependent on ionic interactions. Finally, the present inventors have shown that several short VAR2CSA fragments are capable of inducing the production of adhesion-blocking antibodies and that the anti-adhesive antibodies target the proposed CSA binding region. These data provide the first detailed insight into the biochemical nature of the interaction between a PfEMP1 molecule and its ligand.

### Preparation of polypeptides of the invention

The VAR2CSA polypeptides and other polypeptides of the invention described herein may be produced by means of recombinant nucleic acid techniques and as described in WO2013/117705. In general, a cloned wild-type VAR2CSA nucleic acid sequence is modified to encode the desired protein. This modified sequence is then inserted into an expression vector, which is in turn transformed or transfected into host cells. Higher eukaryotic cells, in particular cultured mammalian cells, may be used as host cells. Procaryotic cells such as Lactococcus lactis or E. coli can also be used to express the polypeptides as long as these prokaryotes are able to produce disulfide bonds or the protein is or may be refolded correctly. In addition, Yeast strains can also be used to express the protein, here among *Saccharomyces cerevisiae* and *P. Pichia.*

The amino acid sequence alterations may be accomplished by a variety of techniques. Modification of the nucleic acid sequence may be by site-specific mutagenesis. Techniques for site-specific mutagenesis are well known in the art and are described in, for example, Zoller and Smith (DNA 3:479-488, 1984) or "Splicing by extension overlap", Horton et al., Gene 77, 1989, pp. 61-68. Thus, using the nucleotide and amino acid sequences of VAR2CSA, one may introduce the alteration(s) of choice. Likewise, procedures for preparing a DNA construct using polymerase chain reaction using specific primers are well known to per-sons skilled in the art (cf. PCR Protocols, 1990, Academic Press, San Diego, California, USA).

The polypeptides of the present invention can also comprise non-naturally occurring amino acid residues. Non-naturally occurring amino acids include, without limitation, beta-alanine, desaminohistidine, trans-3-methylproline, 2,4-methanoproline, cis-4-hydroxyproline, trans-4-hydroxyproline, N-methylglycine, allo-threonine, methylthreonine, hydroxyethylcys-teine, hydroxyethylhomocysteine, nitroglutamine, homoglutamine, pipecolic acid, thiazolidine carboxylic acid, dehydroproline, 3- and 4-methylproline, 3,3-dimethylproline, tert-leucine, nor-valine, 2-azaphenylalanine, 3-azaphenylalanine, 4-azaphenylalanine, and 4-fluorophenylalanine. Several methods are known in the art for incorporating non-naturally occurring amino acid residues into polypeptides. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell-free system comprising an E. coli S30 extract and commercially available enzymes and other reagents. Polypeptides are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395-403, 1993).

The nucleic acid construct encoding the VAR2CSA polypeptides and other polypeptides of the invention of the invention may suitably be of genomic or cDNA origin, for instance obtained by preparing a genomic or cDNA library and screening for DNA sequences coding for all or part of the polypeptide by hybridization using synthetic oligonucleotide probes in accordance with standard techniques (cf. Sambrook et al., Molecular Cloning: A Laboratory Manual, 2nd. Ed. Cold Spring Harbor Labora-tory, Cold Spring Harbor, New York, 1989).

The nucleic acid construct encoding a VAR2CSA polypeptide may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. According to the phosphoamidite method, oligonucleotides are synthesised, e.g. in an automatic DNA synthesiser, purified, annealed, ligated and cloned in suitable vectors. The DNA sequences encoding the Plasmodium falciparum VAR2CSA polypeptides and other polypeptides of the invention may also be prepared by polymerase chain reaction using specific primers, for instance as described in US 4,683,202, Saiki et al., Science 239 (1988), 487 - 491, or Sambrook et al., supra.

Furthermore, the nucleic acid construct may be of mixed synthetic and genomic, mixed synthetic and cDNA or mixed genomic and cDNA origin prepared by ligating fragments of syn-thetic, genomic or cDNA origin (as appropriate), the fragments corresponding to various parts of the entire nucleic acid construct, in accordance with standard techniques.

The nucleic acid construct is preferably a DNA construct. DNA sequences for use in producing VAR2CSA polypeptides and other polypeptides according to the present invention will typically encode a pre-pro polypeptide at the amino-terminus of VAR2CSA to obtain proper posttranslational processing and secretion from the host cell.

The DNA sequences encoding the Plasmodium falciparum VAR2CSA polypeptides and other polypeptides according to the present invention are usually inserted into a recombinant vector which may be any vector, which may conveniently be subjected to recombinant DNA procedures, and the choice of vector will often depend on the host cell into which it is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the Plasmodium falciparum VAR2CSA polypeptides and other polypeptides according to the present invention is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the polypeptide.

Expression vectors for use in expressing VAR2CSA polypeptides and other polypeptides according to the present invention will comprise a promoter capable of directing the transcription of a cloned gene or cDNA. The promoter may be any DNA sequence, which shows transcriptional activity in the host cell of choice and may be derived from genes encoding proteins either homologous or heterologous to the host cell.

Examples of suitable promoters for directing the transcription of the DNA encoding the Plasmodium falciparum VAR2CSA polypeptide in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814), the CMV promoter (Boshart et al., Cell 41:521-530, 1985) or the adenovirus 2 major late promoter (Kaufman and Sharp, Mol. Cell. Biol, 2:1304-1319, 1982).

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the Autographa californica polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., Nature 304 (1983), 652 - 654) promoters.

Examples of suitable promoters for use in filamentous fungus host cells are, for instance, the ADH3 promoter (McKnight et al., The EMBO J. 4 (1985), 2093 - 2099) or the tpiA promoter. Examples of other useful promoters are those derived from the gene encoding A. oryzae TAKA amylase, Rhizomucor miehei aspartic proteinase, A. niger neutral alpha-amylase, A. niger acid stable alpha-amylase, A. niger or A. awamori glucoamylase (gluA), Rhizomucor miehei lipase, A. oryzae alkaline protease, A. oryzae triose phosphate isomerase or A. nidulans acetamidase. Preferred are the TAKA-amylase and gluA promoters. Suitable promoters are mentioned in, e.g. EP 238 023 and EP 383 779.

The DNA sequences encoding the Plasmodium falciparum VAR2CSA polypeptides and other polypeptides according to the present invention may also, if necessary, be operably connected to a suitable terminator, such as the human growth hormone terminator (Palmiter et al., Science 222, 1983, pp. 809-814) or the TPI1 (Alber and Kawasaki, J. Mol. Appl. Gen. 1, 1982, pp. 419-434) or ADH3 (McKnight et al., The EMBO J. 4, 1985, pp. 2093-2099) terminators. Expression vectors may also contain a set of RNA splice sites located downstream from the promoter and upstream from the insertion site for the VAR2CSA sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the human growth hormone gene terminator (DeNoto et al. Nucl. Acids Res. 9:3719-3730, 1981) or the polyadenylation signal from *Plasmodium falciparum,* human or bovine genes. The expression vectors may also include a noncoding viral leader sequence, such as the adenovirus 2 tripartite leader, located between the promoter and the RNA splice sites; and enhancer sequences, such as the SV40 enhancer.

To direct the Plasmodium falciparum VAR2CSA polypeptides and other polypeptides of the present invention into the secretory pathway of the host cells, a secretory signal sequence (also known as a leader sequence, prepro sequence or pre sequence) may be provided in the recombinant vector. The secretory signal sequence is joined to the DNA sequences encoding the Plasmodium falciparum VAR2CSA polypeptides and other polypeptides according to the present invention in the correct reading frame. Secretory signal sequences are commonly positioned 5' to the DNA sequence encoding the peptide. The secretory signal sequence may be that, normally associated with the protein or may be from a gene encoding another secreted protein.

For secretion from yeast cells, the secretory signal sequence may encode any signal peptide, which ensures efficient direction of the expressed Plasmodium falciparum VAR2CSA polypeptides and other polypeptides according to the present invention into the secretory pathway of the cell. The signal peptide may be naturally occurring signal peptide, or a functional part thereof, or it may be a synthetic peptide. Suitable signal peptides have been found to be the alpha-factor signal peptide (cf. US 4,870,008), the signal peptide of mouse salivary amylase (cf. O. Hagenbuchle et al., Nature 289, 1981, pp. 643-646), a modified carboxypeptidase signal peptide (cf. L.A. Valls et al., Cell 48, 1987, pp. 887-897), the yeast BAR1 signal peptide (cf. WO 87/02670), or the yeast aspartic protease 3 (YAP3) signal peptide (cf. M. Egel-Mitani et al., Yeast 6, 1990, pp. 127-137).

For efficient secretion in yeast, a sequence encoding a leader peptide may also be inserted downstream of the signal sequence and upstream of the DNA sequence encoding the Plasmodium falciparum VAR2CSA polypeptides and other polypeptides according to the present invention. The function of the leader peptide is to allow the expressed peptide to be directed from the endoplasmic reticulum to the Golgi apparatus and further to a secretory vesicle for secretion into the culture medium (i.e. exportation of the Plasmodium falciparum VAR2CSA polypeptides and other polypeptides according to the present invention across the cell wall or at least through the cellular membrane into the periplasmic space of the yeast cell). The leader peptide may be the yeast alpha-factor leader (the use of which is described in e.g. US 4,546,082, US 4,870,008, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378.

For use in filamentous fungi, the signal peptide may conveniently be derived from a gene encoding an Aspergillus sp. amylase or glucoamylase, a gene encoding a Rhizomucor miehei lipase or protease or a Humicola lanuginosa lipase. The signal peptide is preferably derived from a gene encoding A. oryzae TAKA amylase, A. niger neutral alpha-amylase, A. niger acidstable amylase, or A. niger glucoamylase. Suitable signal peptides are disclosed in, e.g. EP 238 023 and EP 215 594.

For use in insect cells, the signal peptide may conveniently be derived from an insect gene (cf. WO 90/05783), such as the lepidopteran Manduca sexta adipokinetic hormone precursor signal peptide (cf. US 5,023,328).

The procedures used to ligate the DNA sequences coding for the Plasmodium falciparum VAR2CSA polypeptides and other polypeptides according to the present invention, the promoter and optionally the terminator and/or secretory signal sequence, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841 - 845.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers are reviewed by Thilly (Mammalian Cell Technology, Butterworth Publishers, Stoneham, MA). The person skilled in the art will easily be able to choose suitable selectable markers.

Selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing a dicistronic message. Constructs of this type are known in the art (for example, Levinson and Simonsen, U.S. 4,713,339). It may also be advantageous to add additional DNA, known as "carrier DNA," to the mixture that is introduced into the cells.

After the cells have taken up the DNA, they are grown in an appropriate growth me-dium, typically 1-2 days, to begin expressing the gene of interest. As used herein the term "appropriate growth medium" means a medium containing nutrients and other components required for the growth of cells and the expression of the Plasmodium falciparum VAR2CSA polypeptide of interest. Media generally include a carbon source, a nitrogen source, essential amino acids, essential sugars, vitamins, salts, phospholipids, protein and growth factors. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby in-creasing expression levels. Clones of stably transfected cells are then screened for expression of the Plasmodium falciparum VAR2CSA polypeptide of interest.

The host cell into which the DNA sequences encoding the Plasmodium falciparum VAR2CSA polypeptides and other polypeptides according to the present invention is introduced may be any cell, which is capable of producing the posttranslational modified polypeptides and includes yeast, fungi and higher eukaryotic cells.

Examples of mammalian cell lines for use in the present invention are the COS-1 (ATCC CRL 1650), baby hamster kidney (BHK) and 293 (ATCC CRL 1573; Graham et al., J. Gen. Virol. 36:59-72, 1977) cell lines. A preferred BHK cell line is the tk- ts13 BHK cell line (Waechter and Baserga, Proc. Natl. Acad. Sci. USA 79:1106-1110, 1982), hereinafter referred to as BHK 570 cells. The BHK 570 cell line has been deposited with the American Type Culture Collection, 12301 Parklawn Dr., Rockville, Md. 20852, under ATCC accession number CRL 10314. A tk- ts13 BHK cell line is also available from the ATCC under accession number CRL 1632. In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77:4216-4220, 1980).

Examples of suitable yeasts cells include cells of Saccharomyces spp. or Schizosaccharomyces spp., in particular strains of Saccharomyces cerevisiae or Saccharomyces kluyveri. Methods for transforming yeast cells with heterologous DNA and producing heterologous poly-peptides there from are described, e.g. in US 4,599,311, US 4,931,373, US 4,870,008, 5,037,743, and US 4,845,075. Transformed cells are selected by a phenotype determined by a selectable marker, commonly drug resistance or the ability to grow in the absence of a particular nutrient, e.g. leucine. A preferred vector for use in yeast is the POT1 vector disclosed in US 4,931,373. The DNA sequences encoding the Plasmodium falciparum VAR2CSA polypeptides and other polypeptides according to the present invention may be preceded by a signal sequence and optionally a leader sequence, e.g. as described above. Further examples of suitable yeast cells are strains of Kluyveromyces, such as K. lactis, Hansenula, e.g. H. polymorpha, or Pichia, e.g. P. pastoris (cf. Gleeson et al., J. Gen. Microbiol. 132, 1986, pp. 3459-3465; US 4,882,279).

Examples of other fungal cells are cells of filamentous fungi, e.g. Aspergillus spp., Neurospora spp., Fusarium spp. or Trichoderma spp., in particular strains of A. oryzae, A. nidulans or A. niger. The use of Aspergillus spp. for the expression of proteins is described in, e.g., EP 272 277, EP 238 023, EP 184 438 The transformation of F. oxysporum may, for instance, be carried out as described by Malardier et al., 1989, Gene 78: 147-156. The transformation of Trichoderma spp. may be performed for instance as described in EP 244 234.

When a filamentous fungus is used as the host cell, it may be transformed with the DNA construct of the invention, conveniently by integrating the DNA construct in the host chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination.

Transformation of insect cells and production of heterologous polypeptides therein may be performed as described in US 4,745,051; US 4,879,236; US 5,155,037; 5,162,222; EP 397,485. The insect cell line used as the host may suitably be a Lepidoptera cell line, such as Spodoptera frugiperda cells or Trichoplusia ni cells (cf. US 5,077,214). Culture conditions may suitably be as described in, for instance, WO 89/01029 or WO 89/01028, or any of the aforementioned references.

The transformed or transfected host cell described above is then cultured in a suitable nutrient medium under conditions permitting expression of the Plasmodium falciparum VAR2CSA polypeptide after which all or part of the resulting peptide may be recovered from the culture. The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The Plasmodium falciparum VAR2CSA polypeptide produced by the cells may then be recovered from the culture medium by conventional procedures including separating the host cells from the medium by centrifugation or filtration, precipitating the proteinaqueous components of the supernatant or filtrate by means of a salt, e.g. ammonium sulfate, purification by a variety of chromatographic procedures, e.g. ion exchange chromatography, gelfiltration chromatography, affinity chromatography, or the like, dependent on the type of polypeptide in question.

Transgenic animal technology may be employed to produce the VAR2CSA polypeptides and other polypeptides of the invention. It is preferred to produce the proteins within the mammary glands of a host female mammal. Expression in the mammary gland and subsequent secretion of the protein of interest into the milk overcomes many difficulties encountered in isolating proteins from other sources. Milk is readily collected, available in large quantities, and biochemically well characterized. Furthermore, the major milk proteins are present in milk at high concentrations (typically from about 1 to 15 g/l).

From a commercial point of view, it is clearly preferable to use as the host a species that has a large milk yield. While smaller animals such as mice and rats can be used (and are preferred at the proof of principle stage), it is preferred to use livestock mammals including, but not limited to, pigs, goats, sheep and cattle. Sheep are particularly preferred due to such factors as the previous history of transgenesis in this species, milk yield, cost and the ready availability of equipment for collecting sheep milk (see, for example, WO 88/00239 for a comparison of factors influencing the choice of host species). It is generally desirable to select a breed of host animal that has been bred for dairy use, such as East Friesland sheep, or to introduce dairy stock by breeding of the transgenic line at a later date. In any event, animals of known, good health status should be used.

To obtain expression in the mammary gland, a transcription promoter from a milk protein gene is used. Milk protein genes include those genes encoding caseins (see U.S. 5,304,489), beta lactoglobulin, a lactalbumin, and whey acidic protein. The beta lactoglobulin (BLG) promoter is preferred. In the case of the ovine beta lactoglobulin gene, a region of at least the proximal 406 bp of 5' flanking sequence of the gene will generally be used, although larger portions of the 5' flanking sequence, up to about 5 kbp, are preferred, such as a ∼4.25 kbp DNA segment encompassing the 5' flanking promoter and non-coding portion of the beta lactoglobulin gene (see Whitelaw et al., Biochem. J. 286: 31 39 (1992)). Similar fragments of promoter DNA from other species are also suitable.

Other regions of the beta lactoglobulin gene may also be incorporated in constructs, as may genomic regions of the gene to be expressed. It is generally accepted in the art that constructs lacking introns, for example, express poorly in comparison with those that contain such DNA sequences (see Brinster et al., Proc. Natl. Acad. Sci. USA 85: 836 840 (1988); Palmiter et al., Proc. Natl. Acad. Sci. USA 88: 478 482 (1991); Whitelaw et al., Transgenic Res. 1: 3 13 (1991); WO 89/01343; and WO 91/02318). In this regard, it is generally preferred, where possible, to use genomic sequences containing all or some of the native introns of a gene encoding the protein or polypeptide of interest, thus the further inclusion of at least some introns from, e.g, the beta lactoglobulin gene, is preferred. One such region is a DNA segment that provides for intron splicing and RNA polyadenylation from the 3' non-coding region of the ovine beta lactoglobulin gene. When substituted for the natural 3' non-coding sequences of a gene, this ovine beta lactoglobulin segment can both enhance and stabilize expression levels of the protein or polypeptide of interest. Within other embodiments, the region surrounding the initiation ATG of the VAR2CSA sequence is replaced with corresponding sequences from a milk specific protein gene. Such replacement provides a putative tissue specific initiation environment to enhance expression. It is convenient to replace the entire VAR2CSA pre pro and 5' non-coding sequences with those of, for example, the BLG gene, although smaller regions may be replaced.

For expression of VAR2CSA polypeptides and other polypeptides according to the present invention in transgenic animals, a DNA segment encoding VAR2CSA is operably linked to additional DNA segments required for its expression to produce expression units. Such additional segments include the above mentioned promoter, as well as sequences that provide for termination of transcription and polyadenylation of mRNA. The expression units will further include a DNA segment encoding a secretory signal sequence operably linked to the segment encoding modified VAR2CSA. The secretory signal sequence may be a native secretory signal sequence or may be that of another protein, such as a milk protein (see, for example, von Heijne, Nucl. Acids Res. 14: 4683 4690 (1986); and Meade et al., U.S. 4,873,316).

Construction of expression units for use in transgenic animals is carried out by inserting a VAR2CSA sequence into a plasmid or phage vector containing the additional DNA segments, although the expression unit may be constructed by essentially any sequence of ligations. It is particularly convenient to provide a vector containing a DNA segment encoding a milk protein and to replace the coding sequence for the milk protein with that of a VAR2CSA variant; thereby creating a gene fusion that includes the expression control sequences of the milk protein gene. In any event, cloning of the expression units in plasmids or other vectors facilitates the amplification of the VAR2CSA sequence. Amplification is conveniently carried out in bacterial (e.g. E. coli) host cells, thus the vectors will typically include an origin of replication and a selectable marker functional in bacterial host cells. The expression unit is then introduced into fertilized eggs (including early stage embryos) of the chosen host species. Introduction of heterologous DNA can be accomplished by one of several routes, including microinjection (e.g. U.S. Patent No. 4,873,191), retroviral infection (Jaenisch, Science 240: 1468 1474 (1988)) or site directed integration using embryonic stem (ES) cells (reviewed by Bradley et al., Bio/Technology 10: 534 539 (1992)). The eggs are then implanted into the oviducts or uteri of pseudopregnant females and allowed to develop to term. Offspring carrying the introduced DNA in their germ line can pass the DNA on to their progeny in the normal, Mendelian fashion, allowing the development of transgenic herds. General procedures for producing transgenic animals are known in the art (see, for example, Hogan et al., Manipulating the Mouse Embryo: A Laboratory Manual, Cold Spring Harbor Laboratory, 1986; Simons et al., Bio/Technology 6: 179 183 (1988); Wall et al., Biol. Reprod. 32: 645 651 (1985); Buhler et al., Bio/Technology 8: 140 143 (1990); Ebert et al., Bio/Technology 9: 835 838 (1991); Krimpenfort et al., Bio/Technology 9: 844 847 (1991); Wall et al., J. Cell. Biochem. 49: 113 120 (1992); U.S. 4,873,191; U.S. 4,873,316; WO 88/00239, WO 90/05188, WO 92/11757; and GB 87/00458). Techniques for introducing foreign DNA sequences into mammals and their germ cells were originally developed in the mouse (see, e.g., Gordon et al., Proc. Natl. Acad. Sci. USA 77: 7380 7384 (1980); Gordon and Ruddle, Science 214: 1244 1246 (1981); Palmiter and Brinster, Cell 41: 343 345 (1985); Brinster et al., Proc. Natl. Acad. Sci. USA 82: 4438 4442 (1985); and Hogan et al. (ibid.)). These techniques were subsequently adapted for use with larger animals, including livestock species (see, e.g., WO 88/00239, WO 90/05188, and WO 92/11757; and Simons et al., Bio/Technology 6: 179 183 (1988)). To summarize, in the most efficient route used to date in the generation of transgenic mice or livestock, several hundred linear molecules of the DNA of interest are injected into one of the pro nuclei of a fertilized egg according to established techniques. Injection of DNA into the cytoplasm of a zygote can also be employed.

Production in transgenic plants may also be employed. Expression may be generalised or directed to a particular organ, such as a tuber (see, Hiatt, Nature 344:469 479 (1990); Edelbaum et al., J. Interferon Res. 12:449 453 (1992); Sijmons et al., Bio/Technology 8:217 221 (1990); and EP 0 255 378).

### Administration and pharmaceutical compositions

### Combination treatments

The polypeptide, derivative, or conjugate as defined in the present specification preferably including VAR2CSA may be administered simultaneously or sequentially with one or more other cancer agent, and/or be used in a combination treatment with other known therapies. The factors may be supplied in single-dosage form wherein the single-dosage form contains both compounds, or in the form of a kit-of-parts comprising a preparation of a VAR2CSA polypeptide as a first unit dosage form and a preparation of the one or more other compound as a second unit dosage form. Whenever a first or second or third, etc., unit dose is mentioned throughout this specification this does not indicate the preferred order of administration, but is merely done for convenience purposes.

Suitable other cancer agents or therapies that may be used in combination with a VAR2CSA polypeptide includes antibodies already on the market or in development, including Vemurafenib (Hoffmann-La Roche), human monoclonal antibodies against MCSP, Therapeutical (Micromet Inc) anti-MCSP using BiTE antibody platform technology, and Adoptive transfer of cytotoxic T cells with specificity for MCSP.

By "simultaneous" dosing of a preparation of a VAR2CSA polypeptide and a preparation of one or more other compound is meant administration of the compounds in single-dosage form, or administration of a first agent followed by administration of a second agent with a time separation of no more than 15 minutes, preferably 10, more preferred 5, more preferred 2 minutes. Either factor may be administered first.

By "sequential" dosing is meant administration of a first agent followed by administration of a second agent with a time separation of more than 15 minutes. Either of the two unit dosage form may be administered first. Preferably, both products are injected through the same intravenous access.

Another object of the present invention is to provide a pharmaceutical formulation comprising a polypeptide according to the present invention preferably containing VAR2CSA which is present in a serum/plasma concentration from 0 mg/ml to 1 mg/ml, and wherein the formulation has a pH from 2.0 to 10.0. The formulation may further comprise a buffer system, preservative(s), tonicity agent(s), chelating agent(s), stabilizers and surfactants. In some embodiments of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50 % w/w water. Likewise, the term "aqueous solution" is defined as a solution comprising at least 50 %w/w water, and the term "aqueous suspension" is defined as a suspension comprising at least 50 % w/w water.

In other embodiments the pharmaceutical formulation is a freeze-dried formulation, whereto the physician or the patient adds solvents and/or diluents prior to use.

In other embodiments the pharmaceutical formulation is a dried formulation (e.g. freeze-dried or spray-dried) ready for use without any prior dissolution.

In a further aspect the invention relates to a pharmaceutical formulation comprising an aqueous solution of a VAR2CSA containing polypeptide, and a buffer, wherein the VAR2CSA containing polypeptide is present in a serum/plasma concentration from 0-1 mg/ml or above, and wherein the formulation has a pH from about 2.0 to about 10.0.

In a other embodiments of the invention the pH of the formulation is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the formulation further comprises a pharmaceutically acceptable preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In some embodiments the sugar additive is sucrose. Sugar alcohol is defined as a C4-C8 hydrocarbon having at least one --OH group and includes, for example, mannitol, sorbitol, inositol, galactitol, dulcitol, xylitol, and arabitol. In some embodiments the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely affect the stabilizing effects achieved using the methods of the invention. In some embodiments, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well known to the skilled person. For convenience reference is made to Remington: The Science and Practice of Pharmacy, 19th edition, 1995.

In a further embodiment of the invention the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1 mg/ml to 5 mg/ml. In a further embodiment of the invention the chelating agent is present in a concentration from 0.1 mg/ml to 2 mg/ml. In a further embodiment of the invention the chelating agent is present in a concentration from 2 mg/ml to 5 mg/ml. Each one of these specific chelating agents constitutes an alternative embodiment of the invention. The use of a chelating agent in pharmaceutical compositions is well-known to the skilled person. For convenience reference is made to Remington: The Science and Practice of Pharmacy, 19th edition, 1995.

In a further embodiment of the invention the formulation further comprises a stabilizer. The use of a stabilizer in pharmaceutical compositions is well-known to the skilled person. For convenience reference is made to Remington: The Science and Practice of Pharmacy, 19th edition, 1995.

More particularly, compositions of the invention are stabilized liquid pharmaceutical compositions whose therapeutically active components include a polypeptide that possibly exhibits aggregate formation during storage in liquid pharmaceutical formulations. By "aggregate formation" is intended a physical interaction between the polypeptide molecules that results in formation of oligomers, which may remain soluble, or large visible aggregates that precipitate from the solution. By "during storage" is intended a liquid pharmaceutical composition or formulation once prepared, is not immediately administered to a subject.

Rather, following preparation, it is packaged for storage, either in a liquid form, in a frozen state, or in a dried form for later reconstitution into a liquid form or other form suitable for administration to a subject. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In some embodiments, amino acids to use in preparing the compositions of the invention are those carrying a charged side chain, such as arginine, lysine, aspartic acid, and glutamic acid. Any stereoisomer (i.e., L, D, or DL isomer) of a particular amino acid (e.g. glycine, methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In some embodiments the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D, or DL isomer) or combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 to about 100:1.

In a further embodiment of the invention the formulation further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation further comprises a surfactant. In a further embodiment of the invention the surfactant is selected from a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic® F68, poloxamer 188 and 407, Triton X-100), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium taurodihydrofusidate etc.), long-chain fatty acids and salts thereof C6-C12 (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesulfonates, 3-cholamido-1-propyldimethylammonio-1-propanesulfonate, cationic surfactants (quaternary ammonium bases) (e.g. cetyl-trimethylammonium bromide, cetylpyridinium chloride), non-ionic surfactants (eg. Dodecyl β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well known to the skilled person. For convenience reference is made to Remington: The Science and Practice of Pharmacy, 19th edition, 1995.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Pharmaceutical compositions containing a polypeptides according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Topical administration may be a particular advantage in the treatment of conditions associated with local inflammation, such as in the treatment of inflammation associated with burn or other conditions associated with the skin. Accordingly, in some embodiments administration is by topical administration.

In some particular embodiments, eye droplets may be used in conditions associated with the eye, such as keratitis, such as diffuse lamellar keratitis (DLK).

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of the polypeptides, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block copolymers thereof, polyethylene glycols, carrier proteins, for example albumin, gels, for example, thermogelling systems, for example block co-polymeric systems well known to those skilled in the art, micelles, liposomes, microspheres, nanoparticulates, virus like particles, bacteria like particles, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current invention are useful in the formulation of solids, semisolids, powder and solutions for pulmonary administration of the VAR2CSA containing polypeptide, using, for example a metered dose inhaler, dry powder inhaler and a nebulizer, all being devices well known to those skilled in the art.

Compositions of the current invention are specifically useful in the formulation of controlled, sustained, protracting, retarded, and slow release drug delivery systems. More specifically, but not limited to, compositions are useful in formulation of parenteral controlled release and sustained release systems (both systems leading to a many-fold reduction in number of administrations), well known to those skilled in the art. Even more preferably, are controlled release and sustained release systems administered subcutaneous. Without limiting the scope of the invention, examples of useful controlled release system and compositions are hydrogels, oleaginous gels, liquid crystals, polymeric micelles, microspheres, nanoparticles,

Methods to produce controlled release systems useful for compositions of the current invention include, but are not limited to, crystallization, condensation, co-crystallization, precipitation, co-precipitation, emulsification, dispersion, high pressure homogenisation, encapsulation, spray drying, microencapsulating, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Formulation and Delivery (MacNally, E.J., ed. Marcel Dekker, New York, 2000).

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition, which may be a solution or suspension for the administration of the polypeptide according to the present invention preferably containing VAR2CSA in the form of a nasal or pulmonal spray. As a still further option, the pharmaceutical compositions containing the polypeptide of the invention can also be adapted to transdermal administration, e.g. by needle-free injection or from a patch, optionally an iontophoretic patch, or transmucosal, e.g. buccal, administration.

The term "stabilized formulation" refers to a formulation with increased physical stability, increased chemical stability or increased physical and chemical stability.

The term "physical stability" of the protein formulation as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations is evaluated by means of visual inspection and/or turbidity measurements after exposing the formulation filled in suitable containers (e.g. cartridges or vials) to mechanical/physical stress (e.g. agitation) at different temperatures for various time periods. Visual inspection of the formulations is performed in a sharp focused light with a dark background. The turbidity of the formulation is characterized by a visual score ranking the degree of turbidity for instance on a scale from 0 to 3 (a formulation showing no turbidity corresponds to a visual score 0, and a formulation showing visual turbidity in daylight corresponds to visual score 3). A formulation is classified physical unstable with respect to protein aggregation, when it shows visual turbidity in daylight. Alternatively, the turbidity of the formulation can be evaluated by simple turbidity measurements well known to the skilled person. Physical stability of the aqueous protein formulations can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein formulation as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein formulation as well-known by the person skilled in the art. Most proteins are prone to deamidation, a process in which the side chain amide group in glutaminyl or asparaginyl residues is hydrolysed to form a free carboxylic acid. Other degradations pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein formulation can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized formulation" refers to a formulation with increased physical stability, increased chemical stability or increased physical and chemical stability. In general, a formulation must be stable during use and storage (in compliance with recommended use and storage conditions) until the expiration date is reached.

In some embodiments of the invention the pharmaceutical formulation comprising the VAR2CSA polypeptide is stable for more than 6 weeks of usage and for more than 3 years of storage. In other embodiments of the invention the pharmaceutical formulation comprising the VAR2CSA polypeptide is stable for more than 4 weeks of usage and for more than 3 years of storage. In a further embodiment of the invention the pharmaceutical formulation comprising the VAR2CSA polypeptide is stable for more than 4 weeks of usage and for more than two years of storage. In an even further embodiment of the invention the pharmaceutical formulation comprising the VAR2CSA polypeptide is stable for more than 2 weeks of usage and for more than two years of storage.

### Indications for use of polypeptides according to the invention

The polypeptides according to the present invention preferably containing VAR2CSAs may be used in a wide range of indications associated with expression, such as inappropriate expression of CSA, such as in various cancers, such as metastatic cancers including melanomas, such as C32 melanoma, sarcomas, lung carcinomas, oligodendrocytomas, human brain tumours including gliomas, leukaemia, such as lymphoblastic leukemia and acute myeloid leukemia, and carcinoma, such as squamous cell carcinomas and breast carcinomas, renal cell carcinomas, chondrosarcomas, and pancreatic cell carcinomas. The polypeptides may also be used for cancer stem cells and accordingly target the cells before development into a cancer. Other conditions associated with expression, such as inappropriate expression of CSA are conditions of the cartilage and/or the development of scar tissue.

### Specific embodiments of the invention

As described herein the present invention relates to a polypeptide comprising i) a first polypeptide domain being a transmembrane domain and an endodomain of a chimeric antigen receptor (CAR), and ii) a second extracellular polypeptide domain being a VAR2CSA polypeptide.

In some embodiments the polypeptide comprises a peptide part of a split-protein binding system which is selected from K-Tag (SEQ ID NO: 132), SpyCatcher (SEQ ID NO:129), SpyCatcher-ΔN (SEQ ID NO:136), SpyTag (SEQ ID NO:130), Minimal Spytag sequence (SEQ ID NO:131), split-Spy0128 (SEQ ID NO:135), isopeptide Spy0128 (SEQ ID NO:134), or any inverse sequence thereof, or a variant thereof with sequence identity of at least about 80%, such as at least about 82, 84, 86, 88, 90, 92, 94, 96, 98, or 99%.

In some embodiments the polypeptide further comprises one or more linker or hinge sequences, such as a GC linker.

In some embodiments the VAR2CSA polypeptide consist of SEQ ID NO:55 or SEQ ID NO:56 or fragments or variants thereof with the ability to bind chondroitin sulfate A (CSA) that could be presented on a proteoglycans (CSPG).

In some embodiments the VAR2CSA polypeptide is a fragment of VAR2CSA that consist of a sequential amino acid sequence of
a) ID1, and
b) DBL2Xb, and optionally
c) ID2a.

In some embodiments the VAR2CSA polypeptide binds chondroitin sulfate A (CSA) on proteoglycans (CSPG) with an affinity as measured by a KD lower than 100 nM, such as lower than 80 nM, such as lower than 70 nM, such as lower than 60 nM, such as lower than 50 nM, such as lower than 40 nM, such as lower than 30 nM, such as lower than 26 nM, such as lower than 24 nM, such as lower than 22 nM, such as lower than 20 nM, such as lower than 18 nM, such as lower than 16 nM, such as lower than 14 nM, such as lower than 12 nM, such as lower than 10 nM, such as lower than 9 nM, such as lower than 8 nM, such as lower than 7 nM, such as lower than 6 nM, or lower than 4nM.

In some embodiments the VAR2CSA polypeptide comprises an amino acid sequence having at least 70 % sequence identity with any one amino acid sequence of 1-577 of SEQ ID NO:1, 1-592 of SEQ ID NO:3, 1-579 of SEQ ID NO:4, 1-576 of SEQ ID NO:5, 1-586 of SEQ ID NO:10, 1-579 of SEQ ID NO:11, 1-565 of SEQ ID NO:29, 1-584 of SEQ ID NO:34, 1-569 of SEQ ID NO:36, 1-575 of SEQ ID NO:37, 1-592 of SEQ ID NO:38, 1-603 of SEQ ID NO:41, 1-588 of SEQ ID NO:43, 1-565 of SEQ ID NO:44, 1-589 of SEQ ID NO:45, 1-573 of SEQ ID NO:48, 1-583 of SEQ ID NO:53, 1-569 of SEQ ID NO:54, or 1-640 of SEQ ID NO:128.

In some embodiments the VAR2CSA polypeptide comprises an amino acid sequence having at least 70 % sequence identity with an amino acid sequence of 578-640 of SEQ ID NO:1, 593-656 of SEQ ID NO:3, 580-643 of SEQ ID NO:4, 577-640 of SEQ ID NO:5, 587-650 of SEQ ID NO:10, 580-643 of SEQ ID NO:11, 566-628 of SEQ ID NO:29, 585-647 of SEQ ID NO:34, 570-632 of SEQ ID NO:36, 576-639 of SEQ ID NO:37, 593-655 of SEQ ID NO:38, 604-667 of SEQ ID NO:41, 589-652 of SEQ ID NO:43, 566-628 of SEQ ID NO:44, 590-653 of SEQ ID NO:45, 574-637 of SEQ ID NO:48, 584-646 of SEQ ID NO:53, or 570-632 of SEQ ID NO:54.

In some embodiments the VAR2CSA polypeptide comprises an amino acid sequence having at least 70 % sequence identity with an amino acid sequence of SEQ ID NO:2, 6, 8, 9, 12, 13, 14, 15, 16, 17, 18, 19, 20, 21, 22, 23, 24, 25, 26, 27, 28, 30, 31, 32, 33, 35, 39, 40, 42, 46, 47, 49, 50, 51, 52, or 128.

In some embodiments the VAR2CSA polypeptide consists of an amino acid sequence having at least 70 % sequence identity with any one amino acid sequence of 1-577 of SEQ ID NO:1, 1-592 of SEQ ID NO:3, 1-579 of SEQ ID NO:4, 1-576 of SEQ ID NO:5, 1-586 of SEQ ID NO:10, 1-579 of SEQ ID NO:11, 1-565 of SEQ ID NO:29, 1-584 of SEQ ID NO:34, 1-569 of SEQ ID NO:36, 1-575 of SEQ ID NO:37, 1-592 of SEQ ID NO:38, 1-603 of SEQ ID NO:41, 1-588 of SEQ ID NO:43, 1-565 of SEQ ID NO:44, 1-589 of SEQ ID NO:45, 1-573 of SEQ ID NO:48, 1-583 of SEQ ID NO:53, 1-569 of SEQ ID NO:54, or 1-640 of SEQ ID NO:128.

In some embodiments the VAR2CSA polypeptide consists of an amino acid sequence selected from the list consisting of SEQ ID NO:1, 3-5, 10, 11, 29, 34, 36-38, 41, 43-45, 48, 53, 54, and 128.

In some embodiments the VAR2CSA polypeptide consists of an amino acid sequence having a length of less than 700 amino acids, such as less than 690 amino acids, such as less than 680 amino acids, such as less than 670 amino acids, such as less than 660 amino acids, such as less than 650 amino acids, such as less than 640 amino acids, such as less than 630 amino acids, such as less than 620 amino acids, such as less than 610 amino acids, such as less than 600 amino acids, such as less than 590 amino acids, such as less than 580 amino acids, such as less than 570 amino acids.

In some embodiments the VAR2CSA polypeptide has a molecular mass of less than about 100 kDa under non-reducing conditions on an SDS-PAGE.

In some embodiments the VAR2CSA polypeptide is non-glycosylated.

In some embodiments the transmembrane domain and/or endodomain of a chimeric antigen receptor (CAR) is derived from a from a 1st, 2nd, or 3rd generation CAR.

In some embodiments the polypeptide comprising a first polypeptide domain being a transmembrane domain and an endodomain of a chimeric antigen receptor (CAR), further comprises other regulatory elements or suicide gene systems, such as inducible Caspase-9 (iCASP9) or Sleeping Beauty (SB) transposon system.

In some embodiments the transmembrane domain and/or endodomain of a chimeric antigen receptor (CAR) comprises a CD28 and/or CD3 zeta signaling domain.

In some embodiments the transmembrane domain and/or endodomain of a chimeric antigen receptor (CAR) is as described in any one of WO2014127261, WO13126729, WO13126733, WO14055442, WO14055771, WO2014039523, WO14179759 and WO14138704.

Sequences, including sequences of VAR2CSA polypeptides:
>fcr3 745 amino acids | 640 aa; underlined sequence corresponds to the ID1 domain of FCR3, Sequence in bold corresponds to DBL2Xb domain of FCR3. Remaining sequence is ID2a (SEQ ID NO:1)
>gi|254952610|gb|ACT97135.1| VAR2CSA [Plasmodium falciparum] | 341 aa (SEQ ID NO:2)
>M24 745 amino acids | 656 aa (SEQ ID NO:3)
>KMWII 745 amino acids | 643 aa (SEQ ID NO:4)
>1248 745 amino acids | 640 aa (SEQ ID NO:5)
>gi|254952618|gb|ACT97139.| VAR2CSA Plasmodium falciparum] | 358 aa (SEQ ID NO:6)
>gi|254952592|gb|ACT97126.1| VAR2CSA [Plasmodium falciparum] | 333 aa (SEQ ID NO:7)
>gi|90193467|gb|ABD92329.1| erythrocyte membrane protein 1 [Plasmodium falciparum] | 269 aa (SEQ ID NO:8)
>gi|254952616|gb|ACT97138.1| VAR2CSA [Plasmodium falciparum] | 333 aa (SEQ ID NO:9)
>hb31 745 amino acids | 650 aa (SEQ ID NO:10)
>hb32 745 amino acids | 643 aa (SEQ ID NO:11)
>gi|90193475|gb|ABD92333.1| erythrocyte membrane protein 1 [Plasmodium falciparum] | 269 aa (SEQ ID NO:12)
>gi|254952600|gb|ACT97130.1| VAR2CSA [Plasmodium falciparum] | 344 aa (SEQ ID NO:13)
>gi|254952598|gb|ACT97129.1| VAR2CSA [Plasmodium falciparum] | 334 aa (SEQ ID NO:14)
>gi|254952596|gb|ACT97128.1| VAR2CSA [Plasmodium falciparum] | 332 aa (SEQ ID NO:15)
>gi|90193465|gb|ABD92328.1| erythrocyte membrane protein 1 [Plasmodium falciparum] | 267 aa (SEQ ID NO:16)
>gi|90193477|gb|ABD92334.1| erythrocyte membrane protein 1 [Plasmodium falciparum] | 263 aa (SEQ ID NO:17)
>gi|254952594|gb|ACT97127.1| VAR2CSA [Plasmodium falciparum] | 338 aa (SEQ ID NO:18)
>gi|254952602|gb|ACT97131.1| VAR2CSA [Plasmodium falciparum] | 341 aa (SEQ ID NO:19)
>gi|254952660|gb|ACT97160.1| VAR2CSA [Plasmodium falciparum] | 352 aa (SEQ ID NO:20)
>gi|254952652|gb|ACT97156.1| VAR2CSA [Plasmodium falciparum] | 344 aa (SEQ ID NO:21)
>gi|254952622|gb|ACT97141.1| VAR2CSA [Plasmodium falciparum] | 350 aa (SEQ ID NO:22)
>gi|254952626|gb|ACT97143.1| VAR2CSA [Plasmodium falciparum] | 359 aa (SEQ ID NO:23)
>gi|90193469|gb|ABD92330.1| erythrocyte membrane protein 1 [Plasmodium falciparum] | 270 aa (SEQ ID NO:24)
>gi|254952644|gb|ACT97152.1| VAR2CSA [Plasmodium falciparum] | 334 aa (SEQ ID NO:25)
>gi|254952642|gb|ACT97151.1| VAR2CSA [Plasmodium falciparum] | 351 aa (SEQ ID NO:26)
>gi|254952658|gb|ACT97159.1| VAR2CSA [Plasmodium falciparum] | 353 aa (SEQ ID NO:27)
>gi|254952640|gb|ACT97150.1| VAR2CSA [Plasmodium falciparum] | 327 aa (SEQ ID NO:28)
>dd2full 745 amino acids | 628 aa (SEQ ID NO:29)
>gi|254952636|gb|ACT97148.1| VAR2CSA [Plasmodium falciparum] | 350 aa (SEQ ID NO:30)
>gi|254952638|gb|ACT97149.1| VAR2CSA [Plasmodium falciparum] | 330 aa (SEQ ID NO:31)
>gi|254952628|gb|ACT97144.1| VAR2CSA [Plasmodium falciparum] | 334 aa (SEQ ID NO:32)
>gi|254952630|gb|ACT97145.1| VAR2CSA [Plasmodium falciparum] | 350 aa (SEQ ID NO:33)
>P13 745 amino acids | 647 aa (SEQ ID NO:34)
>gi|254952608|gb|ACT97134.1| VAR2CSA [Plasmodium falciparum] | 341 aa (SEQ ID NO:35)
>7g8 745 amino acids | 632 aa (SEQ ID NO:36)
>Indo 745 amino acids | 639 aa (SEQ ID NO:37)
>MC 745 amino acids | 655 aa (SEQ ID NO:38)
>gi|254952650|gb|ACT97155.1| VAR2CSA [Plasmodium falciparum] | 347 aa (SEQ ID NO:39)
>gi|254952648|gb|ACT97154.1| VAR2CSA [Plasmodium falciparum] | 335 aa (SEQ ID NO:40)
>ghana2 745 amino acids | 667 aa (SEQ ID NO:41)
>gi|254952634|gb|ACT97147.1| VAR2CSA [Plasmodium falciparum] | 348 aa (SEQ ID NO:42)
>ghana1 745 amino acids | 652 aa (SEQ ID NO:43)
>V1S1 745 amino acids | 628 aa (SEQ ID NO:44)
>raj116_var25 745 amino acids | 653 aa (SEQ ID NO:45)
>gi|31323048|gb|AAP37940.1| var2csa [Plasmodium falciparum] | 490 aa (SEQ ID NO:46)
>gi|254952620|gb|ACT97140.1| VAR2CSA [Plasmodium falciparum] | 335 aa (SEQ ID NO:47)
>T2C6 745 amino acids | 637 aa (SEQ ID NO:48)
>gi|254952632|gb|ACT97146.1| VAR2CSA [Plasmodium falciparum] | 330 aa (SEQ ID NO:49)
>gi|90193487|gb|ABD92339.1| erythrocyte membrane protein 1 [Plasmodium falciparum] | 269 aa (SEQ ID NO:50)
>gi|254952646|gb|ACT97153.1| VAR2CSA [Plasmodium falciparum] | 347 aa (SEQ ID NO:51)
>gi|90193485|gb|ABD92338.1| erythrocyte membrane protein 1 [Plasmodium falciparum] | 269 aa (SEQ ID NO:52)
>MTS1 745 amino acids | 646 aa (SEQ ID NO:53)
>Q8I639 (Q8I639_PLAF7) Plasmodium falciparum (isolate 3D7), 632 aa extracellular part (SEQ ID NO:54)
>Q8I639 (Q8I639_PLAF7) Plasmodium falciparum (isolate 3D7), complete 2730 aa extracellular part (SEQ ID NO:55)
>FCR3 (SEQ ID NO: 56) complete 2734 aa extracellular part (577 aa highlighted corr. ID1-DBL2b)
>BPTI, protease inhibitor (SEQ ID NO: 57)
   RPDFCLEPPYTGPCKARIIRYFYNAKAGLCQTFVYGGCRAKRNNFKSAEDCMRTCGGA
>PE38, *Pseudomonas* exotoxin A (SEQ ID NO:58), (underlining of KDEL represent a signal sequence, which may be optional for the constructs according to the present invention)
>PE38LR, variant of PE38 (SEQ ID NO: 59) (underlining of KDEL represent a signal sequence, which may be optional for the constructs according to the present invention)
Sequences of VAR2CSA polypeptides fused with truncated fragments of *Pseudomonas* exotoxin A (PE38)
   Fused VAR2CSA-PE38 proteins may have modifications such as a protease inhibitor (BPTI) in the N-terminal and/or an optimized PE38 sequence that is less immunogenic (PE38LR)
>BPTI-ID1-ID2aFCR3-PE38LR, (SEQ ID NO:60) underlined sequence corresponds to the ID1 domain of FCR3, sequence in bold corresponds to DBL2Xb domain of FCR3, underlined and bold sequence is ID2a
>BPTI-ID1-ID2aFCR3-PE38 (SEQ ID NO:61)
>ID1-ID2aFCR3-PE38 (SEQ ID NO:62)
>ID1-ID2aFCR3-PE38LR (SEQ ID NO:63)
>BPTI-DBL1-ID2aFCR3-PE38LR (SEQ ID NO:64)
>BPTI-DBL1-ID2aFCR3-PE38 (SEQ ID NO:65)
>DBL1-ID2aFCR3-PE38LR (SEQ ID NO:66)
>DBL1-ID2aFCR3-PE38 (SEQ ID NO:67)
>ID1-ID2a3D7-PE38 (SEQ ID NO:68)
>ID1-ID2a3D7-PE38LR (SEQ ID NO:69)
>ID1-DBL2bFCR3-PE38LR (SEQ ID NO:70)
>DT388, sequence of diphtheria toxin (SEQ ID NO:71)
Sequences of VAR2CSA polypeptides fused with truncated fragments of diphtheria toxin >DT388-DBL1-ID2a 3D7 (SEQ ID NO:72)
>DT388-DBL1-ID2a FCR3 (SEQ ID NO:73)
>DT388-ID1-ID2a 3D7 (SEQ ID NO:74)
>DT388-ID1-ID2a FCR3 (SEQ ID NO:75)
>idlid2a FCR3 3x Nglyc mutant (asn/N to Gln/Q) (SEQ ID NO:128)
> SpyCatcher (SEQ ID NO:129)
>Spytag (SEQ ID NO:130)
   AHIVMVDAYKPTK
>Minimal Spytag sequence (SEQ ID NO:131)
   AHIVMVDA
> The β-strand of CnaB2 (K-Tag) (SEQ ID NO:132)
   ATHIKFSKRD
SpyLigase (SEQ ID NO:133)
>isopeptide Spy0128 (SEQ ID NO:134)
   TDKDMTITFTNKKDAE
>Split-Spy0128 (SEQ ID NO:135)
> SpyCatcher-ΔN (SEQ ID NO:136)
>Inversed Spytag sequence (SEQ ID NO: 137)
   KTPKYADVMVIHA

### EXAMPLES

### METHODS

### METHOD 1 - Cloning and protein expression

VAR2CSA sequence fragments were amplified from codon optimized FCR3 (GenBank accession no. GU249598) or 3D7 (GenBank accession no. JQ247428) VAR2CSA genes using specific primers (Table 2). Simple fragments were amplified in a one-step PCR. Amino acid substitution constructs were made in a two-step PCR. First PCR amplified two fragments from the codon optimized FCR3 template, containing overlapping complimentary ends. Second PCR amplified the total construct, using the two overlapping fragments as template with primers specific for the outer borders. All fragments were sequenced for verification. Fragments were cloned into the baculovirus vector pAcGP67-A (BD Biosciences), modified to contain a V5 and His tag at the C-terminal or a pEt coli expression vector. The proteins were expressed in baculovirus-infected insect cells as soluble protein secreted into the cell culture supernatant or in E. coli Shuffle cells. Synthetic genes are designed using standard algorithms for gene desing taking into account secondary DNA and RNA structures, repetitive regions and codon choice for a given expression cell system. Synthetic genes are made for Spy-tag and Spy-catcher fusions as well as CAR designs.

### METHOD 2 - Protein Purification and SDS-PAGE

The filtered supernatant containing the secreted recombinant protein was dialyzed using an ÄKTA cross-flow (GE Healthcare). The dialysis was performed in 10 mM NaH2PO4 (pH 7.4, Sigma-Aldrich) and 500 mM NaCl. The resulting solution was filtered (0.2µm) and imidiazole was added to a final concentration of 15 mM. The protein was then purified on a 1-ml HisSelect column (H8286, Sigma-Aldrich). Bound protein was eluted with 10 mM NaH2PO4 (pH 7.4), 500 mM NaCl, and 500 mM imidiazole. Proteins needed for Quartz Crystal Microbalance measurements and SAXS were further purified to obtain monomers by size exclusion chromatography using a HiLoad 16/60 Superdex 200 column (GE Healthcare) in 20 mM Tris (pH 8) and 200 mM NaCl. The purity and structural integrity of the protein was verified by SDS-PAGE.

### METHOD 3 - ELISA

Falcon microtiter plates (351172, BD Biosciences) were incubated at a concentration of 3µg/ml for CSPG (bovine) (D8428, Sigma) or HSPG (H4777, Sigma) and 100µg/ml for CSA (C9819, Sigma), CSC (400675, Seikagaku), and CSB (C3788, Sigma) overnight at 4°C. The plates were then blocked with TSM binding buffer (20mM Tris, 150mM NaCl, 2mM CaCl₂, 0.05% Tween-20, 1% BSA, PH7.4 at 25°C) for 2 hours at 37°C on a shaker. A 2-fold dilution series (1.56mM-100mM) of protein was prepared in TSM binding buffer and added to the plates, which was incubated 1hr at 37°C on a shaker. All measurements were performed in triplicates. The plates were washed three times in TSM washing buffer (20mM Tris, 150mM NaCl, 2mM CaCl₂, 0.05% Tween-20, PH7.4 at 25°C). The plates were then incubated with 1:3000 anti-V5-HRP antibody (R96125, Invitrogen) in TSM binding buffer 1hr at 37°C on a shaker. The plates were washed three times in TSM washing buffer. Finally the plates were developed with o-phenylenediamine substrate (DAKO) for 15min. The reaction was quenched with 2.5M H₂SO₄. Absorbance was measured at 490nm.

### METHOD 4 - Quartz Crystal Microbalance (Attana A100)

Experiments were performed on an Attana A100 (Attana AB), using gold plated 10MHz, AT-cut quartz crystal, polystyrene chips (3611-3103 Attana AB). All buffers and reagents were filtered to 0.2µm. The ligand was CSPG (Bovine) (D8428, Sigma) or HSPG (H4777, Sigma), coated at a concentration of 100µg/ml. Coating was done in steady state by adding ligand solution and incubation 30 minutes at room temperature. This was followed by blocking the plate with PBS containing 0.1% Ig-free BSA (BSA-50, Rockland), 30 minutes at room temperature. The Attana A100 was washed with 1% SDS prior to every experiment, using the manufacturers predefined daily wash program. Following the wash, the running buffer was switched to PBS at a flow rate of 25µL/min, at 25°C, and the machine was allowed to stabilize at a maximum change in frequency of 0.5Hz/min. Once stabilized PBS was injected multiple times to show that the injection process minimally affected the baseline. Prior to sample injection PBS was injected as a blank. Analyte was injected in a 1:3 dilution series (0.25µg/ml-60µg/ml) starting with the lowest concentration. Association time was set to 84 seconds and disassociation time to 5 minutes. Due to high affinity of binding it was not possible to regenerate binding surface following injections. The data collected was processed in the Attester Evaluation software (Attana AB). Curves were fitted in a simple 1:1 model. kₒₙ and k_{off} were determined by curve fitting and K_{D} was calculated based K_{D} = k_{off} / kₒₙ.

### METHOD 5 - Cancer cell binding assays

Flow-cytometry (FCM) was used to test the reactivity of the VAR2CSA minimal binding polypeptide to CSPG expressed on the surface of various cell lines. Cells were cultured in RPMI supplemented with 10 % foetal calf serum (CHO cells, C32), Hams F12 (BeWo), kept in 5% carbon dioxide at 37°C or purified from a human blood sample in CPD buffer (red blood cells). Aliquots of cells (1×105) were sequentially exposed to the VAR2CSA minimal binding polypeptide (150, 75 or 37 nM) and α-V5-FITC (1:800)(Invitrogen) diluted in FACS2 (PBS+2% FCS) for 30 minutes at +4C in dark with smooth agitation. As negative controls a truncated version of the minimal binding polypeptide and FACS2 buffer were used. Intact cells were gated based on the forward and side scatter signal. Data were acquired using a FC500 flow-cytometer (Beckman Coulter) from a minimum of 5000 cells. All samples relating to a particular cell line were processed and analyzed in a single assay.

### METHOD 6 - Cancer cell binding assays

As an alternative to the flow-cytometry assay above, cells were incubated with VAR2CSA minimal binding polypeptide and α-V5-FITC (1:500)(Invitrogen) diluted in HBSS. VAR2CSA polypeptide was used at the same concentrations as written above. Following α-V5-FITC staining cells were washed 3 times in HBSS, collected in Enzyme-free cell detachment buffer (Invitrogen) and analyzed on a FACS Calibur (BD Biosciences) for FL-1 signal intensity.

Abbreviations CIDR, cysteine-rich inter-domain region; CSA, chondroitin sulfate A; CSPG, chondroitin sulfate proteoglycan; DBL, Duffy binding-like domain; FCM, flow-cytometry; FV2, full-length ecto-domain of the VAR2CSA protein without N-terminal segment; HSPG, heparan sulfate proteoglycan; ID, inter-domain; IE, P. falciparum-infected erythrocyte; NTS, N-terminal segment; PM, placental malaria; PfEMP1, Plasmodium falciparum erythrocyte membrane protein 1; PM, placental malaria.

### METHOD 7 - Staining of paraffin-embedded human tissue samples

The binding of recombinant VAR2CSA to primary cancer tissue obtained from human patients is investigated using immunohistochemistry (IHC). Paraffin embedded tissue spotted on glass slides subjected no antigen retrieval was incubated with 0.1-500 nM V5-VAR2CSA variants or V5-Control protein (DBL4) for 1 h in room temperature, washed for 8 minutes, incubated with 1:700 mouse anti-V5 antibody for 30 minutes, washed for 8 minutes. Bound anti-V5 was subsequently detected using UltraMap anti-mouse HRP using the Ventana Discovery XT platform.

### EXAMPLE 1 Production of truncated recombinant VAR2CSA proteins

All protein truncations were produced according to previously defined domain borders (Dahlbäck M, Jorgensen LM, Nielsen MA, Clausen TM, Ditlev SB, et al. J Biol Chem 286: 15908-15917). For the purpose of simplification we have divided the CIDR_{PAM} domain into two domains ID2a and ID2b, where ID2a is the N-terminal part of CIDR_{PAM} not containing the CIDR-like sequence and ID2b corresponds to the CIDR-like sequence. We also used a new DBL2X border incorporating 93 amino acids of ID2a. For simplification we call this border DBL2Xb, while the old border will be referred to as DBL2Xa. Primers used in cloning are listed in Table 2. Fragments were expressed in baculovirus-infected insect cells as soluble proteins as described in Method 1. Most proteins were produced based on the FCR3 genotype. Some FCR3 fragments did not express and these were instead made based on the 3D7 genotype. The proteins were used interchangeably in the analysis since we show that recombinant VAR2CSA from both genotypes bind equally to CSA. All proteins showed a shift in gel mobility when comparing reduced and non-reduced samples by SDS-PAGE (Method 2). This is consistent with the formation of intra-molecular disulfide bridges. Some proteins formed high-molecular weight complexes detected by non-reduced SDS-PAGE. This is probably due to the formation of inter-molecular disulfide bridges between unpaired cysteines. This was confirmed by reducing the complexes to monomeric protein using DTT.

**Table 2: Cloning Primers**

| **FCR3 Primers** | | |
|---|---|---|
| **Protein** | **Forward Primer** | **Reverse Primer** |
| ID1-ID2b | AACTACATCAAGGGCGAC (SEQ ID NO:76) | CTTGTTGATATTGGTGTCGGT (SEQ ID NO:77) |
| DBL1X-ID2a | CACAGCGATAGCGGCAAG (SEQ ID NO:78) | GTCCAGCTTGCTGGAGTT (SEQ ID NO:79) |
| ID1-ID2a | AACTACATCAAGGGCGAC (SEQ ID NO:80) | GTCCAGCTTGCTGGAGTT (SEQ ID NO:81) |
| ID1-DBL2Xa | AACTACATCAAGGGCGAC (SEQ ID NO:82) | AGCGGCGTTGGTGGTGGA (SEQ ID NO:83) |
| ID1-DBL2Xb | AACTACATCAAGGGCGAC (SEQ ID NO:84) | GTACTTGTACCGGTAGGG (SEQ ID NO:85) |
| DBL1X-DBL2Xb | CACAGCGATAGCGGCAAG (SEQ ID NO:86) | GTACTTGTACCGGTAGGG (SEQ ID NO:87) |

| **3d7 Primers** | | |
|---|---|---|
| **Protein** | **Forward Primer** | **Reverse Primer** |
| DBL2X-DBL4ε | CTGACCAACTGCTACAAG (SEQ ID NO:88) | GGTCCAGAGGGTACAGCTT (SEQ ID NO:89) |
| ID1-DBL3ε | CTGTCCTTCATCCTGAAC (SEQ ID NO:90) | TTCAGCGTTGTTGTACTCGTA (SEQ ID NO:91) |
| ID1-DBL4ε | CTGTCCTTCATCCTGAAC (SEQ ID NO:92) | GTCCAGAGGGTACAGCTT (SEQ ID NO:93) |
| DBL1X-ID2b | CACTCTGACTCTGGCACC (SEQ ID NO:94) | AGAGGACTTCATCTTGTTGTTGGT (SEQ ID NO:95) |
| ID1-ID2b | CTGTCCTTCATCCTGAAC (SEQ ID NO:96) | AGAGGACTTCATCTTGTTGTTGGT (SEQ ID NO:97) |
| DBL1X-ID2a | CACTCTGACTCTGGCACC (SEQ ID NO:98) | GTCCAGCTTAGAGGAGTT (SEQ ID NO:99) |
| ID1-ID2a | CTGTCCTTCATCCTGAAC (SEQ ID NO:100) | GTCCAGCTTAGAGGAGTT (SEQ ID NO:101) |
| DBL1X-DBL2Xa | CACTCTGACTCTGGCACC (SEQ ID NO:102) | GGCGGCGTTGGTGGTAGA (SEQ ID NO:103) |
| ID1-DBL2Xa | CTGTCCTTCATCCTGAAC (SEQ ID NO:104) | GGCGGCGTTGGTGGTAGA (SEQ ID NO:105) |
| DBL1X-DBL2Xb | CACTCTGACTCTGGCACC (SEQ ID NO:106) | GTACTTGTATCCGTGGGG (SEQ ID NO:107) |
| ID1-DBL2Xb | CTGTCCTTCATCCTGAAC (SEQ ID NO:108) | GTACTTGTATCCGTGGGG (SEQ ID NO:109) |

| **Mutating Putative CSA Binding Sites** | | |
|---|---|---|
| | **PCR1** | |
| | **Fragment 1** | |
| **Protein** | **Forward Reverse** | |
| DBL1X-ID2a (DSM Deletion) | CACAGCGATAGCGGCAAG (SEQ ID NO:110) | GGTGTCGAAGTTGATGTCGGGCAGATTGCCCAGGTA (SEQ ID NO:111) |
| Alanine sub. K(626,629,630), R(631) | CACAGCGATAGCGGCAAG (SEQ ID NO:112) | AGCTGCGGCCAGATTAGCGCCCTCGTGGAAGGACAC (SEQ ID NO:113) |
| Alanine sub. K(459,460,461,464) | CACAGCGATAGCGGCAAG (SEQ ID NO:114) | AGCGCATTCAGCTGCGGCGTTGGTCTTGATGGAGCT (SEQ ID NO:115) |

| | **Fragment 2** | |
|---|---|---|
| **Protein** | **Forward** | **Reverse** |
| DBL1X-ID2a (DSM Deletion) | CACAGCGATAGCGGCAAG (SEQ ID NO:116) | GTCCAGCTTGCTGGAGTT (SEQ ID NO:117) |
| Alanine sub. K(626,629,630), R(631) | GCTAATCTGGCCGCAGCTTACCCCCAGAATAAGAAC (SEQ ID NO:118) | GTCCAGCTTGCTGGAGTT (SEQ ID NO:119) |
| Alanine sub. K(459,460,461,464) | GCCGCAGCTGAATGCGCTGACGTGAAGCTGGGCGTG (SEQ ID NO:120) | GTCCAGCTTGCTGGAGTT (SEQ ID NO:121) |

| | **PCR2** | |
|---|---|---|
| | **Final Constuct** | |
| **Protein** | **Forward** | **Reverse** |
| DBL1X-ID2a (DSM Deletion) | CACAGCGATAGCGGCAAG (SEQ ID NO:122) | GTCCAGCTTGCTGGAGTT (SEQ ID NO:123) |
| Alanine sub. K(626,629,630), R(631) | CACAGCGATAGCGGCAAG (SEQ ID NO:124) | GTCCAGCTTGCTGGAGTT (SEQ ID NO:125) |
| Alanine sub. K(459,460,461,464) | CACAGCGATAGCGGCAAG (SEQ ID NO:126) | GTCCAGCTTGCTGGAGTT (SEQ ID NO:127) |

### EXAMPLE 2 VAR2CSA from FCR3 and 3D7 Binds CSA with similar Affinity and Specificity

FCR3 infected erythrocytes (IE) adhere much stronger to CSA *in vitro* compared to 3D7 or NF54 (IE). If the differences are related to the sequence differences of the expressed VAR2CSA the information could be used to define residues involved in the adhesion process. To test this, we produced a series of overlapping 3D7 VAR2CSA fragments, identical to the ones we have previously tested for FCR3.

The proteins were first screened for specific CSPG binding in a solid phase binding assay (ELISA) (described in Method 3). Proteins binding specifically to CSPG were then further purified by size exclusion chromatography, to obtain pure monomeric protein, and subjected to kinetic analysis on a Quartz Crystal Microbalance (Attana A100) (Method 2 and 4, respectively). The 3D7 VAR2CSA fragments showed binding characteristics very similar to their FCR3 counterparts in the solid state binding assay. The same is true in the kinetic analysis (Table 3). The sensorgrams show association and dissociation data collected at different protein concentrations. This allows determination of the association rate constant (kₒₙ), disassociation rate constant (k_{off}), and the equilibrium constant (K_{D}). Together with the peak response levels these parameters give estimation for the CSPG binding affinity. There is no apparent difference between 3D7 and FCR3 fragments. Some fragments show lower affinity, but this characteristic is maintained in the fragments counterpart. This indicates that the 3D7 and FCR3 VAR2CSA proteins fold and function in the same way.

**Table 3: CSA binding affinity of produced VAR2CSA proteins. Affinity is given as a K_{D} (nM) value determined in kinetics experiments using a quartz crystal microbalance biosensor (Attana A100). N/A: proteins for which no K_{D} value could be determined, due to a lack of binding to CSA.**

| | FCR3 | | 3D7 |
|---|---|---|---|
| VAR2CSA Fragment | Baculo | E. coli | Baculo |
| FV2 | 5.2* | | 8.2 |
| ID1-DBL4ε | 8.6* | | 9.4 |
| ID1-DBL3ε | 0.3* | | 8.5 |
| DBL2X-DBL4ε | 2.4* | | 1.2 |
| DBL1-ID2b | 1.5* | | |
| DBL1-ID2a | 8.0 | 3.5 | 29.5 |
| ID1-ID2a | 7.6 | 18.3 | 5.7 |
| DBL1X-DBL2Xb | | 14.6 | |
| DBL1X-DBL2Xa | N/A | | |
| ID1-DBL2Xb | | | 21.8 |
| ID1-DBL2Xa | | | N/A |

| | | | |
|---|---|---|---|
| * Proteins published in (Dahlbäck et al, JBC, 2011) | | | |

### EXAMPLE 3 - The core-CSA binding site lies within the DBL2X domain

It has been suggested that the minimal CSA binding region in VAR2CSA lies within DBL2X-ID2b, with the need of flanking domains for full affinity binding (Dahlbäck M, Jorgensen LM, Nielsen MA, Clausen TM, Ditlev SB, et al. J Biol Chem 286: 15908-15917). Here we have analyzed shorter fragments of VAR2CSA to further map the regions required for CSA binding.

The truncated proteins were first screened for binding to a CSA proteoglycan (CSPG) in ELISA and then further purified to obtain monomers for examination on the Quartz Crystal Microbalance (Methods 3, 2 and 4, respectively). The minimal binding region is ID1-DBL2Xb (Table 3). This region showed a binding affinity of 21.8 nM, which is comparable to that of full-length VAR2CSA.

Placental IEs are highly selective for low-sulfated placental CSPG. They do not adhere to any other glycosaminoglycans (GAG), such as heparan sulfate (HS). The same is true for the full-length recombinant VAR2CSA protein. The solid state binding assay showed that the VAR2CSA fragments, containing the minimal CSA binding region, bound specifically to CSA. To confirm this the minimal binding fragments were further tested for binding to a heparan sulfate proteoglycan (HSPG) on the Quartz Crystal Microbalance (Method 4). None of the fragments bound HSPG.

### EXAMPLE 4

### VAR2CSA minimal CSA binding region binds specifically to a wide panel of cancer cells

Many different cancer cells have been associated with high expression of the proteoglycan CSPG4. This molecule was initially described as a marker for melanoma but it has recently been found in many cancer forms, including cancer stem cells. The CS chain(s) attached to CSPG4 is known to be primarily CSA. One of the smallest VAR2CSA fragments (ID1-ID2a) was analyzed for binding to a large panel of various cancer cell lines by flow-cytometry (Method 5 and 6). The non-CSA binding protein ID1-DBL2Xa was used as a negative control. The VAR2CSA recombinant protein (ID1-ID2a) binds strongly at 75 nM to all cancer cell lines transcribing CSPG4 (microarray data) including cutaneous Melanoma (C32, MeWo), Lung carcinoma (A549), Breast carcinoma (HCC1395), Osterosarcoma (U2OS, MNNG/HOS), Rhabdomyosarcoma (RH30) (Table 4 and 5). This protein also binds strongly to cutaneous T-cell lymphoma, which does not express CSPG4 (Table 4). The negative control protein ID1-DBL2Xa did not bind to any of the cell lines tested (Table 4). In addition, ID1-ID2a did not interact with human red blood cells, which were used as control cells. Wild type and GAG-deficient Chinese hamster ovary (CHO) cells were also analyzed for ID1-ID2a interaction. The strong interaction seen for ID1-ID2a with wild-type CHO cells was completely abolished when analyzing the CHO-745 cell line, in which the GAG-synthesis is disrupted. The CSA specificity of the interaction was also verified by inhibiting VAR2CSA binding to cells by pre-mixing VAR2CSA with CSA, CSC or HS. CSC and HS did not have any effect on the binding, whereas CSA efficiently abrogated binding of VAR2CSA to the cancer cells.

Following these results, a larger panel of cancer cells were screened by flow cytometry (Table 6 and 7) using the DBL1-ID2a or ID1-ID2a fragment of VAR2CSA. The main purpose of this screening is to identify cell lines suitable for xenograft modeling *in vivo.*

### Table 4. Staining of cancer cell lines and negative control cells using the minimal binding domain of VAR2CSA (ID1-ID2a).

Cells were incubated with medium alone (blank) or recombinant proteins (ID1-DBL2 or ID1-ID2a) at 75 nM for 30 minutes, followed by incubation with anti-V5-FITC (Invitrogen) at 1:800, cells were washed thrice between each incubation. Shown are the mean FITC fluorescence values recorded from a minimum of 5000 cells using a FC500 flowcytometer (Becton Dickinson).

| **Cell type** | **Blank** | **ID1-DBL2Xa** | **ID1-ID2a** |
|---|---|---|---|
| C32 | 5,77 | 6,94 | 63,81 |
| MyLa 2059 | 5,61 | 5,61 | 145,35 |
| MyLa 1850 | 5,87 | 5,6 | 137,86 |
| Cho WT | 3,09 | 4,35 | 34,79 |
| Cho 745 | 4,24 | 4,29 | 4,38 |
| PBMC | 1,34 | 1,36 | 1,67 |
| Erythrocytes | 1,11 | 1,17 | 1,07 |

### Table 5. Staining of cancer cell lines using recombinant VAR2CSA

Cells were incubated with medium alone (blank) or recombinant proteins (DBL1-ID2a or ID1-ID2a) at 75 nM for 30 minutes, followed by incubation with anti-V5-FITC (Invitrogen) at 1:800, cells were washed thrice between each incubation. Shown are the medium score of FITC fluorescence intensity recorded from a minimum of 4 high power field images using a HAL100 Zeiss microscope. NS: No staining; +: weak; ++: medium; +++: strong; ++++: Very strong.

| **Cell type** | **Blank** | **DBL1-ID2a** |
|---|---|---|
| U2OS | NS | +++ |
| MG63 | NS | ++++ |
| MDA-MB-231 | NS | +++ |
| TC32 | NS | + |
| TC71 | NS | ++ |
| MNNG | NS | +++ |
| CHLA9 | NS | ++ |
| CHLA10 | NS | ++ |
| RH30 | NS | +++ |
| RH18 | NS | ++ |
| PC3 | NS | +++ |

**Table 6 Screening of diverse human cancer cell lines for binding of recombinant VAR2CSA (using DBL1-ID2a or ID1-ID2a).**

| Binding was measured by flow cytometry as described in METHOD 5 and 6. | | | | |
|---|---|---|---|---|
| NS: No staining; +: weak; ++: medium; +++: strong; ++++: Very strong. | | | | |
| Cell line | Control | 75 nM VAR2CSA | 150 nM VAR2CSA | Comments |
| MeWo | NS | +++ | ++++ | Melanoma (Fibroblast morphology, derived from lymphnode) |
| A549 | NS | +++ | +++ | Lung Adenocarcinoma (K-RasG12S) |
| HCC1395 | NS | +++ | ++++ | Invasive ductal breast carcinoma TNM stage 1 grade 3; no lymphnode metastasis; Her2-neg, ER-neg, PR-neg (Triple- negative) |
| RH30 | NS | +++ | ++++ | Rhabdomyosarcoma (TPp53 negativ; PAX7-FOXO1A fusion positive; highly genomic instable (>50 chromosome rearangements)) |
| MNNG | NS | +++ | +++ | Osteosarcoma from 13 year old female caucasian (TPR-Met positive) |
| U2OS | NS | +++ | +++ | Osterosarcoma from 15 year old female caucasian (IGF-R1 and IGFR-II positive; TPp53 wt, pRb wt, p16-neg; highly aneuploid) |
| H1792 | NS | ++ | ++ | Lung Adenocarcinoma (K-RasG12S: TPp53het)) |
| MDA-MD-435 | NS | ++ | +++ | Breast carcinoma of melanocytic origin (ER-neg, Her2-pos, PR-pos) |
| MG63 | NS | +++ | ++++ | Osteosarcoma |
| TC32 | NS | ++ | ++ | Ewing's sarcoma |
| CHLA9 | NS | ++ | ++ | Ewing's sarcoma |
| CHLA10 | NS | ++ | ++ | Ewing's sarcoma |
| TC71 | NS | ++ | ++ | Ewing's sarcoma |
| HOS | NS | +++ | ++++ | Osteosarcoma |
| PC3 | NS | ++ | ++ | Prostate carcinoma |
| SKNMC | NS | ++ | +++ | Ewing's sarcoma |
| MCF-7 | NS | + | ++ | Breast carcinoma |

**Table 7 Screening of more human cell cancer cell lines for binding of recombinant VAR2CSA (using DBL1-ID2a or ID1-ID2a)**

| Binding was measured by flow cytometry as described in METHOD 5 and 6. | | | |
|---|---|---|---|
| Values shown are mean fluorescence intensity using protein concentration of 200 nM. | | | |
| **Cell type** | **Negative control** | **ID1-ID2a** | **Comments** |
| GP202 | 21.63 | 111.37 | Gastric Carcinoma |
| NCI-N87 | 7.18 | 207.72 | Gastric Carcinoma |
| MKN45 | 4.22 | 55.4 | Gastric Carcinoma |
| MKN28 | 6.9 | 103.84 | Gastric Carcinoma |
| AGS | 7.25 | 18.21 | Gastric Carcinoma |
| KatoIII | 7.33 | 18.76 | Gastric Carcinoma |
| SNU-1 | 4.33 | 155.79 | Gastric Carcinoma |
| SNU-638 | 8.47 | 8.49 | Gastric Carcinoma |
| IPA220 | 7.72 | 13.67 | Gastric Carcinoma |
| MDA-231 | 3.39 | 63.43 | Triple negative Breast |
| T47D | 3.63 | 48.13 | Luminal Breast |
| LNCap | 6.58 | 24.86 | Prostate |
| PC3 | 5.2 | 29.82 | Prostate |
| Ovc316 | 1.89 | 7.24 | Ovarian cancer stem cells |
| **Cell type** | **Blank** | **DBL1-ID2a** | |
| NALM-6 | 6,19 | 8,22 | Acute lymphatic leukaemia (ALL) |
| 697 | 3,23 | 30,36 | ALL |
| AMO-1 | 2,68 | 35,22 | Myelomatosis |
| KMM-1 | 2,82 | 16,1 | Myelomatosis |
| MOLP-8 | 2,44 | 19,24 | Myelomatosis |
| KMS-12-PE | 3,02 | 7,14 | Myelomatosis |
| KMS-12-BM | 2,2 | 3,25 | Myelomatosis |
| U2932 | 4,24 | 16,83 | Diffuse Large B-cell lymphoma (DLBCL) |
| SU-DHL8 | ND | 3,75 | DLBCL |
| SU-DHL5 | 2,19 | 10,28 | DLBCL |
| Oci_Ly19 | 3,38 | 18,96 | DLBCL |
| HBL1 | 6,53 | 39,53 | DLBCL |
| Farage | 2,8 | 3,28 | DLBCL |
| RIVA | 2,26 | 3,32 | DLBCL |
| WSU-FSCCL | 4,89 | 22,32 | Low-grade follicular small cleaved cell lymphoma |
| U-698-M | 2,24 | 2,85 | Lymphoblastic lymphoma del(6)(q15q22) |

### EXAMPLE 5

### Recombinant VAR2CSA binds to cancer cells with high affinity

The binding affinity of the recombinant VAR2CSA fragment DBL1-ID2a to the cancer cell lines, C32 melanoma and two Cho cell lines (described in example 8) was analysed using a Quartz Crystal Microbalance biosensor (Attana Cell200). A 2-fold dilution series (25-400 nM) of the protein was analysed for binding to the cell surface, with regeneration of the binding surface in between each new protein injection. The binding affinity was estimated to lie in the nano-molar range (Table 8), which is similar to the binding affinity to pure receptor (Table 3).

**Table 8. Estimated binding affinity (K_{D}) of recombinant DBL1-ID2a (E. coli) to cancer cells expressing CSA (C32 and Cho WT) and lack of binding to a CSA-negative cell line (Cho 745)**

| N/A: K_{D} could not be determined due to lack of binding to the cells | |
|---|---|
| **Cell type** | **K_{D} (nM)** |
| C32 melanoma cells | 13 |
| Cho WT | 1.4 |
| Cho 745 | N/A |

### EXAMPLE 6

### Recombinant VAR2CSA protein binds to cancer tissue with high specificity

The binding of recombinant VAR2CSA to primary cancer tissue obtained from human patients is investigated using immunohistochemistry (IHC). The method was developed using human placenta tissue as positive control and Tonsil and liver tissue as negative control. The staining protocol was optimized on the Ventana Discovery XT platform with no epitope retrieval. Paraffin embedded tissue spotted on glass slides was incubated with 0.1-500 nM V5-VAR2CSA (ID1-ID2a) or V5-Control protein (DBL4) for 1 h in room temperature, washed for 8 minutes, incubated with 1:700 mouse anti-V5 antibody for 30 minutes, washed for 8 minutes. Bound anti-V5 was subsequently detected using UltraMap anti-mouse HRP. V5-VAR2CSA stains human placenta in 0.5 nM concentrations with no staining in Tonsil or normal liver. The staining can be completely blocked by adding 200 µg/µl CSA to the reaction buffer. V5-control protein does not stain human placenta tissue at any concentrations tested. A multi-organ tissue micro-array (TMA) representing 24 normal organs showed low or absent staining when stained with 1 nM V5-VAR2CSA, while cancer specimens of breast, colon, Rectum, Prostate, kidney, liver, bladder, pancreas, squamous cell, Lung, Gall bladder, Stomach, Testis, Ovary, Uterus, Adrenal gland, Thyroid and Thymus, hematopoietic system, and the connective tissue (sarcomas) stained positive with intensities equal or higher than human placenta positive control tissue (Table 9).

**Table 9. Detection of CSA on primary human tumor specimens using recombinant VAR2CSA. Table shows number of positive/ total number of cases stained as described in Example 10 for main cancer groups. Positive staining is defined as intensity equal or higher than that observed in placenta tissue.**

| **Cancer group** | **Positive ratio** |
|---|---|
| Bladder carcinoma | 44/56 |
| Prostate carcinoma | 71/76 |
| Breast carcinoma | 64/75 |
| Melanoma | 5/6 |
| Sarcoma | 23/25 |
| Esophagus Squamous cell carcinoma | 2/3 |
| Stomach Adenocarcinoma | 3/3 |
| Colon carcinoma | 2/3 |
| Rectal Adenocarcinoma | 3/3 |
| Liver carcinoma | 3/3 |
| Renal carcinoma | 3/3 |
| Lung carcinoma | 2/3 |
| Cervix carcinoma | 3/3 |
| Ovarian carcinoma | 2/3 |
| Diffuse B-cell lymphoma | 1/3 |
| Astrocytoma | 3/3 |
| Pancreatic carcinoma | 3/3 |

### EXAMPLE 7

### Identification of potential CSPG molecules that were targeted by VAR2CSA

Recombinant VAR2CSA protein (DBL1-ID2a) with a V5-tag was screened for binding to a panel of transfected HEK293 cells expressing >3000 human membrane receptors. A set of 25 receptors have been identified as potential targets of VAR2CSA (Table 10). The interaction between VAR2CSA and these receptors will be further verified by analysis of the binding specificity through inhibition with CSA and HS, both in the HEK293 system and in ELISA.

**Table 10. Receptors that were experimentally identified as potential targets of VAR2CSA.**

| **Gene ID** | **Name** | **UniProt/SwissProt** |
|---|---|---|
| BCAN | Brevican | PGCB HUMAN, Q96GW7 |
| BDKRB2 | Bradykinin receptor B2 | BKRB2 HUMAN, P30411 |
| CA9 | Carbonic anhydrase IX | CAH9 HUMAN, Q16790 |
| CCR10 | chemokine (C-C motif) receptor 10 | CCR10 HUMAN, P46092 |
| CD44 | CD44 molecule (Indian blood group) | CD44 HUMAN, P16070 |
| CDH8 | Cadherin 8, type 2 | CADH8 HUMAN, P55286 |
| CFB | Complement factor B | CFAB HUMAN, P00751 |
| GABBR2 | gamma-aminobutyric acid (GABA) B receptor, 2 | GABR2 HUMAN, O75899 |
| GPC3 | Glypican 3 | GPC3 HUMAN, P51654 |
| GPC5 | Glypican 5 | GPC5 HUMAN, P78333 |
| GPR65 | G-protein coupled receptor 65 | PSYR HUMAN, Q8IYL9 |
| GPRC5B | G protein-coupled receptor, family C, group 5, | GPC5B HUMAN, |
| | member B | Q9NZH0 |
| KCNA2 | potassium voltage-gated channel, shaker-related subfamily, member 2 | KCNA2 HUMAN, P16389 |
| PKD2 | polycystic kidney disease 2 (autosomal dominant) | PKD2 HUMAN, 013563 |
| PODXL2 | podocalyxin-like 2 | PDXL2 HUMAN, Q9NZ53 |
| PTPRG | protein tyrosine phosphatase, receptor type, G | PTPRG HUMAN, P23470 |
| S100A9 | S100 calcium binding protein A9 | S10A9 HUMAN, P06702 |
| SDC1 | Syndecan 1 | SDC1 HUMAN, P18827 |
| SDC4 | Syndecan 4 | SDC4 HUMAN, P31431 |
| STX2 | Syntaxin 2 | STX2 HUMAN, P32856 |
| STXBP5 | syntaxin binding protein 5 (tomosyn) | STXB5 HUMAN, Q5T5C0 |
| TGFBR3 | transforming growth factor, beta receptor III | TGBR3 HUMAN, 003167 |
| TMEFF1 | transmembrane protein with EGF-like and two follistatin-like domains 1 | TEFF1 HUMAN, Q8IYR6 |
| TMEFF2/TE NB2 | transmembrane protein with EGF-like and two follistatin-like domains 2 | TEFF2 HUMAN, Q9UIK5 |
| TMEM154 | Transmembrane protein 154 | (None) |
| THBD | Thrombomodulin | TRBM HUMAN, P07204 |
| CSPG5 | chondroitin sulfate proteoglycan 5 (neuroglycan C) | CSPG5 HUMAN, O95196 |
| STXBP5 | syntaxin binding protein 5 (tomosyn) | STXB5 HUMAN, Q5T5C0 |

### EXAMPLE 8

### Bi-specific VAR2CSA-scFv-anti-CD3 protein

DBL1-ID2a and ID1-ID2a VAR2CSA gene fragments have been fused to scFv sequences encoding single chain antibodies against CD3. These VAR2CSA-scFv proteins are expressed in E. coli or in insect cells. These VAR2CSA-scFv proteins are bi-specific towards CSA-expressing cancer cells and CD3-expressing T cells. As such, these proteins are able to bind CSA in the tumor environment through the VAR2CSA domain while recruiting CD3 positive T cells via the scFv-anti-CD3 domain.

### EXAMPLE 9

### VAR2CSA-Spy fusion proteins

DBL1-ID2a and ID1-ID2a VAR2CSA gene fragments have been fused to various variants of SpyTag, K-tag, SpyCatcher and SpyCather-ΔN (SEQ ID NO: 130, 129, 136). These VAR2CSA-Spy proteins are expressed in E. coli or in insect cells. The recombinant VAR2CSA-Spy protein can form an isopeptide bond with the corresponding split-protein binding partner. For example, VAR2CSA-SpyTag can bind to any molecule that has a SpyCatcher or AN-SpyCather sequence; VAR2CSA-SpyCatcher or VAR2CSA-ΔN-SpyCather can bind to any molecule that has a SpyTag sequence; VAR2CSA-SpyTag can bind to any molecule that has a K-Tag sequence in the presence of SpyLigase; or VAR2CSA-K-Tag can bind to any molecule that has a K-Tag sequence in the presence of SpyLigase. Furthermore, a VAR2CSA-K-Tag can be polymerized (attached to each other) through the K-Tag in the presence of SpyLigase, and subsequently combined with any protein expression a SpyCatcher, such as a Spy-CAR. As such, these proteins are able to bind any protein sequence having the corresponding sequence of the split-protein binding system. By simple mixing of the Spy-tagged VAR2CSA protein with Spy-catcher or Spy-catcher tagged VAR2CSA with Spy-tag we observed a rapid and effective coupling between the proteins as measured by size exclusion chromatography or SDS page. IV injection of Spy-tagged VAR2CSA into mice with established tumors followed by IV injection of fluorescence labeled Spy-catcher demonstrate by IVIS in vivo imaging and post mortem analyses that the isopeptide bond and pairing of Spy-tag and Spy-catcher does happen in vivo.

### EXAMPLE 10

### Bi-specific VAR2CSA-Spy-scFv-anti-CD3 fusion proteins

DBL1-ID2a and ID1-ID2a VAR2CSA gene fragments have been fused to various variants of SpyTag, K-tag, SpyCatcher and SpyCather-ΔN (SEQ ID NO: 130, 129, 136) and scFv sequences encoding single chain antibodies against CD3. These VAR2CSA-Spy proteins are expressed in E. coli or in insect cells. The recombinant VAR2CSA-Spy-scFv-anti-CD3 fusion proteins can form an isopeptide bond with the corresponding split-protein binding partner. For example, VAR2CSA-Spy-scFv-anti-CD3 can bind to any molecule that has a SpyCatcher or AN-SpyCather sequence; VAR2CSA-SpyCatcher-scFv-anti-CD3 or VAR2CSA-ΔN-SpyCather-scFv-anti-CD3 can bind to any molecule that has a SpyTag sequence; VAR2CSA-Spy-scFv-anti-CD3 can bind to any molecule that has a K-Tag sequence in the presence of SpyLigase; or VAR2CSA-K-Tag-scFv-anti-CD3 can bind to any molecule that has a K-Tag sequence in the presence of SpyLigase. Furthermore, a VAR2CSA-K-Tag-scFv-anti-CD3 can be polymerized (attached to each other) through the K-Tag in the presence of SpyLigase, and subsequently combined with any protein expression a SpyCatcher, such as a Spy-CAR. The VAR2CSA-Spy-scFv-anti-CD3 proteins are synthesized and validated as described in example 9.

### EXAMPLE 11

### Chimeric Antigen Receptors expressing VAR2CSA (VAR2-CAR)

CAR-encoding sequences are synthesized using codon-optimization algorithms (Optimizer, PMID: 17439967) and subcloned into "destination" vectors encoding second generation (CD28 transmembrane and intracellular T cell signaling domains and CD3-zeta chain intracellular T cell signaling domain); or third generation (CD8 transmembrane domain linked to CD28, 4-1BB, and CD3-zeta intracellular T cell signaling domains). Retroviral vector supernatants are created by transfecting 293GP packaging cells (Clonetech) with plasmids encoding CAR retroviral vectors and the RD114 envelope glycoprotein, collecting culture supernatants (s/n) 48-72 hours later. The culture supernatants are frozen or used immediately to transduce OKT3 and IL-2 activated human PBMC for 2 consecutive days (culture of lymphocytes on plates coated with RECTRONECTIN (Clonetech) pre-exposed to dilutions of vector containing s/n). CAR expression on transduced T cells is determined by flow cytometry. (Table 11).

**Table 11. Expression of VAR2-CAR on the plasma membrane of transduced T cells as detected by flow cytometry.**

| | **Percentage of positive cell** | **Percentage of Negative cell** |
|---|---|---|
| VAR2-CAR | 61.40% | 38.60% |

### EXAMPLE 12

### Chimeric Antigen Receptors expressing Spy-CAR

CAR-encoding sequences SpyTag, SpyCatcher, AN-SpyCatcher or K-Tag (SEQ ID NO: 130, 129, 136, or 132) are synthesized using codon-optimization algorithms (Optimizer, PMID: 17439967) and subcloned into "destination" vectors encoding second generation (CD28 transmembrane and intracellular T cell signaling domains and CD3-zeta chain intracellular T cell signaling domain); or third generation (CD8 transmembrane domain linked to CD28, 4-1BB, and CD3-zeta intracellular T cell signaling domains). Retroviral vector supernatants are created by transfecting 293GP packaging cells (Clonetech) with plasmids encoding CAR retroviral vectors and the RD114 envelope glycoprotein, collecting culture supernatants (s/n) 48-72 hours later. The culture supernatants are frozen or used immediately to transduce OKT3 and IL-2 activated human PBMC for 2 consecutive days (culture of lymphocytes on plates coated with RECTRONECTIN (Clonetech) pre-exposed to dilutions of vector containing s/n). CAR expression on transduced T cells is determined by flow cytometry (Table 12).

**Table 12. Expression of Spy-CAR on the plasma membrane of transduced T cells as detected by flow cytometry.**

| | **Percentage of positive cell** | **Percentage of Negative cell** |
|---|---|---|
| Spy-CAR | 64.82% | 35.18% |

### EXAMPLE 13

### Chimeric Antigen Receptors expressing Spy-CAR and subsequently linked to a VAR2-Spy fusion protein: VAR2-Spy-Spy-CARs

CAR-encoding sequences SpyTag, SpyCatcher, AN-SpyCatcher or K-tag (SEQ ID NO: 130, 129, 136, or 132) are synthesized using codon-optimization algorithms (Optimizer, PMID: 17439967 and subcloned into "destination" vectors encoding second generation (CD28 transmembrane and intracellular T cell signaling domains and CD3-zeta chain intracellular T cell signaling domain); or third generation (CD8 transmembrane domain linked to CD28, 4-1BB, and CD3-zeta intracellular T cell signaling domains). Retroviral vector supernatants are created by transfecting 293GP packaging cells (Clonetech) with plasmids encoding CAR retroviral vectors and the RD114 envelope glycoprotein, collecting culture supernatants (s/n) 48-72 hours later. The culture supernatants are frozen or used immediately to transduce OKT3 and IL-2 activated human PBMC for 2 consecutive days (culture of lymphocytes on plates coated with RECTRONECTIN (Clonetech) pre-exposed to dilutions of vector containing s/n). Spy-CAR transgenic T cells are subsequently incubated with at a corresponding VAR2CSA-Spy fusion protein (corresponding to the other half of the split-protein binding system of the Spy-CAR) to produce a fully functional VAR2CSA-Spy-Spy-CAR receptor. CAR expression on transduced T cells is determined by flow cytometry and/or co-immunofluorescence (Table 13).

**Table 13. Spy-CAR transgenic T cells are subsequently incubated with at a corresponding VAR2CSA-Spy fusion protein (corresponding to the other half of the split-protein binding system of the Spy-CAR) to produce an armed VAR2CSA-Spy-Spy-CAR receptor as measured by flow cytometry and immunofluorescence (IF) co-localization.**

| **VAR2-SPY attachment to SPY-CAR** | **Armed cells (Flow positive)** | **Un-armed cells (Flow negative)** | **VAR2-Spy/ Spy-CAR (IF co-localization)** |
|---|---|---|---|
| **Over expression** | 93.19% | 6.81% | 71.30% |
| **CTRL** | 6.93% | 93.07% | 1.38% |

### EXAMPLE 14

### Comparing and selecting VAR2-CARs

To normalize for the transduction efficiency of VAR2-CARs and VAR2-Spy-Spy-CARs, effector T cells are analyzed for percent CAR expression (transduction percentage). Based on this, an effector cell-to-target cell ratio (E:T ratio) is calculated. This is compared to % lysis. One Lytic Unit is defined as 30% lysis of target cells at an E:T ratio of 10: 1 (as defined in patent application WO 2013059593 A1). This functional "unit" definition quantifies the amount of lytic activity in each transduced effector cell population being compared. Lytic units represents normalized E:T ratios for the differences in transduction efficiency between constructs.

### EXAMPLE 15

### Lytic activity of different VAR2-CARs

To compare the lytic potential of different VAR2-CAR constructs, CARs encoding forward and reverse translated VAR2CSA are compared in ^{5 I}Cr release CTL assays or colorimetric cytotoxicity assay using five different CSA-expressing tumor cell lines (e.g. Colo205 colorectal cancer, REH leukemia, UM-UC13 bladder cancer, PC-3 prostate cancer, and RH30 pediatric rhabdomyosarcoma). As a control VAR2-CARs are also tested on non-CSA expressing non-tumorigenic HEK293 cells. The E:T ratios are normalized according to the percent transduction of each individual CAR construct as described in Example 14, thus lytic values are directly comparable. An example of lytic activity of a VAR2-CAR is shown in Table 14.

### EXAMPLE 16

### Lytic activity of different VAR2-Spy-Spy-CARs

To compare the lytic potential of different VAR2-Spy-Spy-CAR constructs, CARs encoding forward and reverse translated VAR2-Spy-Spy-CAR constructs are compared in ^{5 I}Cr release CTL assays or colorimetric cytotoxicity assay using five different CSA-expressing tumor cell lines (e.g. Colo205 colorectal cancer, REH leukemia, UM-UC13 bladder cancer, PC-3 prostate cancer, and RH30 pediatric rhabdomyosarcoma). As a control VAR2-Spy-Spy-CARs are also tested on non-CSA expressing non-tumorigenic HEK293 cells. The E:T ratios are normalized according to the percent transduction of each individual CAR construct as described in Example 14, thus lytic values are directly comparable. An example of lytic activity of a VAR2-Spy armed Spy-CAR is shown in Table 14.

**Table 14. T cells purified and transduced with either a VAR2-CAR or a Spy-CAR subsequently armed with VAR2-Spy as in Example 13. CARs were added to UC13 cells in the designated E:T ratios and T-cell mediated cytotoxicity was measured as described in Example 15 and 16.**

| **E:T ratio** | **40;1** | **20;1** | **10;1** | **5;1** | **2.5;1** |
|---|---|---|---|---|---|
| **VAR2-Spy-Spy-CAR (cytotoxicity)** | 99.82% | 53.47% | 21.37% | 11.65% | 9.02% |
| **VAR2-CAR (cytotoxicity)** | 74.43% | -19.05% | 2.87% | 5.21% | 3.55% |
| **CTRL (cytotoxicity)** | 27.33% | -24.95% | -11.41% | 0% | 11.75% |

### EXAMPLE 17

### Lytic activity of different VAR2-CARs and VAR2-Spy-Spy-CARs in vivo

NSG (NOD scid gamma), immune deficient mice are injected on day 0 with a CSA-positive human cancer cell line (e.g. Colo205 colorectal cancer, REH leukemia, UM-UC13 bladder cancer, PC-3 prostate cancer, and RH30 pediatric rhabdomyosarcoma) engineered to express luciferase. On Day 3, mice are treated with 1 x 10⁷ control T cells ("mock," untransduced) or 1 x 10⁷ T cells transduced with VAR2-CARs and VAR2-Spy-Spy-CARs. Tumor burden was measured over a time period of 30 days with bioluminescent imaging using IVIS. Mice are injected intraperitoneally (i.p.) with 3 mg D-luciferin (Caliper Life Sciences, Inc.) and 4 minutes post injection anesthetized mice are imaged with an exposure time of 10-60 seconds. LIVING IMAGE software is used to analyze the bioluminescent signals per each mouse as photons/s/cm /sr.

### ADDITIONAL REMARKS

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Moreover, any combination of the above-described elements in all possible variations thereof is encompassed by the invention unless otherwise indicated herein or otherwise clearly contradicted by context.

## Claims

1. A polypeptide comprising i) a first polypeptide domain being a transmembrane domain and an endodomain of a chimeric antigen receptor (CAR), and ii) a second extracellular polypeptide domain being a VAR2CSA polypeptide.

2. The polypeptide according to claim 1, wherein said polypeptide comprises a peptide part of a split-protein binding system selected from K-Tag (SEQ ID NO: 132), SpyCatcher (SEQ ID NO: 129), SpyCatcher-AN (SEQ ID NO: 136), SpyTag (SEQ ID NO: 130), Minimal Spytag sequence (SEQ ID NO: 131), split-Spy0128 (SEQ ID NO: 135), isopeptide Spy0128 (SEQ ID NO:134), or any inverse sequence thereof, or a variant thereof with sequence identity of at least about 80%, such as at least about 82, 84, 86, 88, 90, 92, 94, 96, 98, or 99%.

3. The polypeptide of any one of claims 1-2, wherein said transmembrane domain and/or endodomain of a chimeric antigen receptor (CAR) comprises a CD28 and/or CD3 zeta signaling domain.

4. The polypeptide according to any one of claims 1-3, wherein said polypeptide further comprises a signal peptide (SP) so that the CAR part of the construct can be glycosylated and anchored in the cell membrane of the immune effector cell.

5. The polypeptide according to any one of claims 1-4, wherein the scFv portion of the CAR is substituted with a VAR2CSA variant comprising at least the minimal CSA binding domain ID1-ID2a.

6. An isolated nucleic acid molecule comprising a nucleotide sequence encoding the polypeptide of claims 1-5.

7. An expression vector comprising an isolated nucleic acid molecule according to claim 6.

8. A cell comprising the vector of claim 7, such as any one selected from the group consisting of an αβT cell, γδT cell, a Natural Killer (NK) cell, a cytotoxic T lymphocyte (CTL), a regulatory T cell, or any combination thereof.

9. A two-component therapeutic comprising
a) a first component being a polypeptide comprising the first polypeptide domain as defined in claim 1 i) and a peptide part of a split-protein binding system attached to the extracellular part of said first polypeptide domain; and
b) a second component being a polypeptide comprising the second extracellular polypeptide domain as defined in claim 1 ii) and a peptide part of a split-protein binding system;
wherein said peptide parts of a split-protein binding system defined by components a) and b) are selected from K-Tag (SEQ ID NO:132), SpyCatcher (SEQ ID NO:129), SpyCatcher-AN (SEQ ID NO: 136), SpyTag (SEQ ID NO: 130), Minimal Spytag sequence (SEQ ID NO:131), split-Spy0128 (SEQ ID NO:135), isopeptide Spy0128 (SEQ ID NO:134), or any inverse sequence thereof, or a variant thereof with sequence identity of at least about 80%, such as at least about 82, 84, 86, 88, 90, 92, 94, 96, 98, or 99%, and spontaneously form an irreversible isopeptide bond.

10. The two-component therapeutic according to claim 9, wherein said polypeptides are as defined in any one of claims 1-5.

11. A vector encoding a polypeptide comprising the first polypeptide domain as defined in claim 1 i) and the second extracellular polypeptide domain as defined in claim 1 ii) for use in a method of treating or preventing the occurrence of any indication or condition associated with expression, such as inappropriate expression of CSA, such as in cancer, in a subject; the method comprising: (a) culturing a population of cytotoxic lymphocytes under conditions promoting activation; (b) transfecting the lymphocyte population of (a) with said vector; (c) administering a therapeutically or prophylactically effective number of the transfected lymphocytes of (b) to a subject having or in risk at said indication; wherein the polypeptide comprising the domains of a CAR has binding specificity for the targeted moiety or can be bound by the targeted moiety; thereby treating or preventing said indication in said subject.

12. A vector encoding a polypeptide comprising the first polypeptide domain as defined in claim 1 i) and a peptide part of a split-protein binding system attached to the extracellular part of said first polypeptide domain; for use in a method of treating or preventing the occurrence of any indication or condition associated with expression, such as inappropriate expression of CSA, such as in cancer, in a subject; the method comprising: (a) culturing a population of cytotoxic lymphocytes under conditions promoting activation; (b) transfecting the lymphocyte population of (a) with said vector; (c) administering a therapeutically or prophylactically effective number of the transfected lymphocytes of (b) to a subject having or in risk at said indication; and (d) administering a polypeptide comprising the second extracellular polypeptide domain as defined in claim 1 ii) and a peptide part of a split-protein binding system, wherein said peptide parts of a split-protein binding system defined in the two different polypeptides used are selected from K-Tag (SEQ ID NO:132), SpyCatcher (SEQ ID NO: 129), SpyCatcher-AN (SEQ ID NO: 136), SpyTag (SEQ ID NO: 130), Minimal Spytag sequence (SEQ ID NO:131), split-Spy0128 (SEQ ID NO:135), isopeptide Spy0128 (SEQ ID NO: 134), or any inverse sequence thereof, or a variant thereof with sequence identity of at least about 80%, such as at least about 82, 84, 86, 88, 90, 92, 94, 96, 98, or 99%, and spontaneously form an irreversible isopeptide bond; thereby treating or preventing said indication in said subject.

13. The polypeptide according to any one of claims 1-5, or the two-component therapeutic according to any one of claims 9-10, for use as a medicament.

14. A pharmaceutical composition comprising the polypeptide according to any one of claims 1-5, or two-component therapeutic according to any one of claims 9-10.

15. A polypeptide according to any one of claims 1-5, or two-component therapeutic according to any one of claims 9-10, or pharmaceutical composition according to claim 14 for the treatment of any indications associated with a condition involving expression, such as inappropriate expression of CSA, such as in cancer.

## Patentansprüche

1. Polypeptid umfassend i) eine erste Polypeptiddomäne, bei der es sich um eine Transmembrandomäne und eine Endodomäne eines chimären Antigenrezeptors (CAR) handelt, und ii) eine zweite extrazelluläre Polypeptiddomäne, bei der es sich um ein VAR2CSA-Polypeptid handelt.

2. Polypeptid nach Anspruch 1, wobei das Polypeptid einen Peptidteil eines Split-Protein-Bindungssystems umfasst, ausgewählt aus K-Tag (SEQ ID NR.: 132), SpyCatcher (SEQ ID NR.: 129), SpyCatcher-ΔN (SEQ ID NR.: 136), SpyTag (SEQ ID NR.: 130), einer minimalen Spytag-Sequenz (SEQ ID NR.: 131), Split-Spy0128 (SEQ ID NR.: 135), Isopeptid Spy0128 (SEQ ID NR.: 134) oder einer beliebigen inversen Sequenz davon oder einer Variante davon mit einer Sequenzidentität von mindestens etwa 80 %, wie etwa mindestens etwa 82, 84, 86, 88, 90, 92, 94, 96, 98 oder 99 %.

3. Polypeptid nach einem der Ansprüche 1 bis 2, wobei die Transmembrandomäne und/oder die Endodomäne eines chimären Antigenrezeptors (CAR) eine CD28- und/oder CD3-Zeta-Signalisierungsdomäne umfasst.

4. Polypeptid nach einem der Ansprüche 1 bis 3, wobei das Polypeptid ferner ein Signalpeptid (SP) umfasst, derart, dass der CAR-Teil des Konstrukts glykosyliert und in der Zellmembran der Immuneffektorzelle verankert werden kann.

5. Polypeptid nach einem der Ansprüche 1 bis 4, wobei der scFv-Abschnitt des CAR durch eine VAR2CSA-Variante umfassend mindestens die minimale CSA-Bindungsdomäne ID1-ID2a ersetzt ist.

6. Isoliertes Nukleinsäuremolekül umfassend eine Nukleotidsequenz, welche das Polypeptid nach Anspruch 1 bis 5 codiert.

7. Expressionsvektor umfassend ein isoliertes Nukleinsäuremolekül nach Anspruch 6.

8. Zelle umfassend den Vektor nach Anspruch 7, zum Beispiel vorzugsweise eine beliebige, aus der Gruppe bestehend aus einer abT-Zelle, gdT-Zelle, einer natürlichen Killer(NK)-Zelle, einem zytotoxischen T-Lymphozyten (CTL), einer regulatorischen T-Zelle oder einer beliebigen Kombination davon ausgewählte.

9. Zwei-Komponenten-Therapeutikum umfassend
a) eine erste Komponente, bei der es sich um ein Polypeptid umfassend die erste Polypeptiddomäne nach der Definition in Anspruch 1 i) und einen am extrazellulären Teil der ersten Polypeptiddomäne gebundenen Peptidteil eines Split-Protein-Bindungssystems handelt; und
b) eine zweite Komponente, bei der es sich um ein Polypeptid umfassend die zweite extrazelluläre Polypeptiddomäne nach der Definition in Anspruch 1 ii) und einen Peptidteil eines Split-Protein-Bindungssystems handelt;
wobei die Peptidteile eines Split-Protein-Bindungssystems, definiert durch die Komponenten a) und b), aus K-Tag (SEQ ID NR.: 132), SpyCatcher (SEQ ID NR.: 129), SpyCatcher-ΔN (SEQ ID NR.: 136), SpyTag (SEQ ID NR.: 130), einer minimalen Spytag-Sequenz (SEQ ID NR.: 131), Split-Spy0128 (SEQ ID NR.: 135), Isopeptid Spy0128 (SEQ ID NR.: 134) oder einer beliebigen inversen Sequenz davon oder einer Variante davon mit einer Sequenzidentität von mindestens etwa 80 %, wie etwa mindestens etwa 82, 84, 86, 88, 90, 92, 94, 96, 98 oder 99 % ausgewählt sind oder spontan eine irreversible Isopeptidbindung bilden.

10. Zwei-Komponenten-Therapeutikum nach Anspruch 9, wobei die Polypeptide der Definition in einem der Ansprüche 1 bis 5 entsprechen.

11. Vektor codierend ein Polypeptid umfassend die erste Polypeptiddomäne nach der Definition in Anspruch 1 i) und die zweite extrazelluläre Polypeptiddomäne nach der Definition in Anspruch 1 ii) zur Verwendung in einem Verfahren zum Behandeln oder Verhindern des Auftretens einer beliebigen Indikation oder eines Zustands, die bzw. der mit Expression verbunden ist, wie beispielsweise einer unangemessenen Expression von CSA, wie beispielsweise bei Krebs, in einem Individuum; wobei das Verfahren umfasst: (a) Kultivieren einer Population zytotoxischer Lymphozyten unter Bedingungen, welche eine Aktivierung begünstigen; (b) Transfizieren der Lymphozytenpopulation von (a) mit dem Vektor; (c) Verabreichen einer therapeutisch oder prophylaktisch wirksamen Anzahl der transfizierten Lymphozyten von (b) an ein Individuum mit der Indikation oder einem Risiko dafür; wobei das Polypeptid, das die Domänen eines CAR umfasst, Bindungsspezifität für die Zieleinheit aufweist oder von der Zieleinheit gebunden werden kann; wodurch die Indikation bei dem Individuum behandelt oder verhindert wird.

12. Vektor codierend ein Polypeptid umfassend die erste Polypeptiddomäne nach der Definition in Anspruch 1 i) und einen an den extrazellulären Teil der ersten Polypeptiddomäne gebundenen Peptidteil eines Split-Protein-Bindungssystems; zur Verwendung in einem Verfahren zum Behandeln oder Verhindern des Auftretens einer beliebigen Indikation oder eines Zustands, die bzw. der mit Expression verbunden ist, wie beispielsweise einer unangemessenen Expression von CSA, wie beispielsweise bei Krebs, in einem Individuum; wobei das Verfahren umfasst: (a) Kultivieren einer Population zytotoxischer Lymphozyten unter Bedingungen, welche eine Aktivierung begünstigen; (b) Transfizieren der Lymphozytenpopulation von (a) mit dem Vektor; (c) Verabreichen einer therapeutisch oder prophylaktisch wirksamen Anzahl der transfizierten Lymphozyten von (b) an ein Individuum mit der Indikation oder einem Risiko dafür; und (d) Verabreichen eines Polypeptids umfassend die zweite extrazelluläre Polypeptiddomäne nach der Definition in Anspruch 1 ii) und einen Peptidteil eines Split-Protein-Bindungssystems, wobei die Peptidteile eines Split-Protein-Bindungssystems, definiert durch die beiden verschiedenen verwendeten Polypeptide, aus K-Tag (SEQ ID NR.: 132), SpyCatcher (SEQ ID NR.: 129), SpyCatcher-ΔN (SEQ ID NR.: 136), SpyTag (SEQ ID NR.: 130), einer minimalen Spytag-Sequenz (SEQ ID NR.: 131), Split-Spy0128 (SEQ ID NR.: 135), Isopeptid Spy0128 (SEQ ID NR.: 134) oder einer beliebigen inversen Sequenz davon oder einer Variante davon mit einer Sequenzidentität von mindestens etwa 80 %, wie etwa mindestens etwa 82, 84, 86, 88, 90, 92, 94, 96, 98 oder 99 % ausgewählt sind und spontan eine irreversible Isopeptidbindung bilden; wodurch die Indikation bei dem Individuum behandelt oder verhindert wird.

13. Polypeptid nach einem der Ansprüche 1 bis 5 oder das Zwei-Komponenten-Therapeutikum nach einem der Ansprüche 9 bis 10 zur Verwendung als Medikament.

14. Pharmazeutische Zusammensetzung umfassend das Polypeptid nach einem der Ansprüche 1 bis 5 oder das Zwei-Komponenten-Therapeutikum nach einem der Ansprüche 9 bis 10.

15. Polypeptid nach einem der Ansprüche 1 bis 5 oder das Zwei-Komponenten-Therapeutikum nach einem der Ansprüche 9 bis 10 oder eine pharmazeutische Zusammensetzung nach Anspruch 14 für die Behandlung von jeglichen Indikationen, die mit einem Zustand verbunden sind, der Expression involviert, wie beispielsweise eine unangemessene Expression von CSA, wie beispielsweise bei Krebs.

## Revendications

1. Polypeptide comprenant i) un premier domaine polypeptidique étant un domaine transmembranaire et un endodomaine d'un récepteur antigénique chimérique (CAR), et ii) un second domaine polypeptidique extracellulaire étant un polypeptide VAR2CSA.

2. Polypeptide selon la revendication 1, dans lequel ledit polypeptide comprend une partie peptidique d'un système de liaison à une protéine de division sélectionnée parmi K-Tag (SEQ ID NO : 132), SpyCatcher (SEQ ID NO : 129), SpyCatcher-ΔN (SEQ ID NO : 136), SpyTag (SEQ ID NO : 130), la séquence Minimal Spytag (SEQ ID NO : 131), split-Spy0128 (SEQ ID NO : 135), l'isopeptide Spy0128 (SEQ ID NO : 134), ou une séquence inverse de ceux-ci, ou un variant de ceux-ci avec une identité de séquence d'au moins environ 80 %, par exemple d'au moins environ 82, 84, 86, 88, 90, 92, 94, 96, 98, ou 99 %.

3. Polypeptide selon l'une quelconque des revendications 1 et 2, dans lequel ledit domaine transmembranaire et/ou endodomaine d'un récepteur antigénique chimérique (CAR) comprend un domaine de signalisation CD28 et/ou CD3 zêta.

4. Polypeptide selon l'une quelconque des revendications 1 à 3, dans lequel ledit polypeptide comprend en outre un peptide signal (SP) de manière à ce que la partie CAR de la construction puisse être glycosylée et ancrée dans la membrane cellulaire de la cellule effectrice immunitaire.

5. Polypeptide selon l'une quelconque des revendications 1 à 4, dans lequel la partie scFv du CAR est substituée par un variant VAR2CSA comprenant au moins le domaine de liaison minimal à la CSA ID1-ID2A.

6. Molécule d'acide nucléique isolée comprenant une séquence nucléotidique codant pour le polypeptide selon les revendications 1 à 5.

7. Vecteur d'expression comprenant une molécule d'acide nucléique isolée selon la revendication 6.

8. Cellule comprenant le vecteur selon la revendication 7, par exemple l'une quelconque sélectionnée dans le groupe consistant en une cellule αβT, une cellule γδT, une cellule tueuse naturelle (NK), un lymphocyte T cytotoxique (CTL), une cellule T régulatrice ou toute combinaison de ceux-ci.

9. Produit thérapeutique à deux composants comprenant
a) un premier composant étant un polypeptide comprenant le premier domaine polypeptidique tel que défini dans la revendication 1 i) et une partie peptidique d'un système de liaison à une protéine de division fixée à la partie extracellulaire dudit premier domaine polypeptidique ; et
b) un second composant étant un polypeptide comprenant le second domaine polypeptidique extracellulaire tel que défini dans la revendication 1 ii) et une partie peptidique d'un système de liaison à une protéine de division ;
dans lequel lesdites parties peptidiques d'un système de liaison à une protéine de division définies par les composants a) et b) sont sélectionnées parmi K-Tag (SEQ ID NO : 132), SpyCatcher (SEQ ID NO : 129), SpyCatcher-ΔN (SEQ ID NO : 136), SpyTag (SEQ ID NO : 130), la séquence Minimal Spytag (SEQ ID NO : 131), split-Spy0128 (SEQ ID NO : 135), l'isopeptide Spy0128 (SEQ ID NO : 134), ou toute séquence inverse de ceux-ci, ou un variant de ceux-ci avec une identité de séquence d'au moins environ 80 %, par exemple d'au moins environ 82, 84, 86, 88, 90, 92, 94, 96, 98, ou 99 %, et forment spontanément une liaison isopeptidique irréversible.

10. Produit thérapeutique à deux composants selon la revendication 9, dans lequel lesdits polypeptides sont tels que définis dans l'une quelconque des revendications 1 à 5.

11. Vecteur codant pour un polypeptide comprenant le premier domaine polypeptidique tel que défini dans la revendication 1 i) et le second domaine polypeptidique extracellulaire tel que défini dans la revendication 1 ii) pour son utilisation dans un procédé de traitement ou de prévention de la survenue de toute indication ou affection associée à l'expression, par exemple une expression inappropriée de la CSA, par exemple dans un cancer, chez un sujet ; le procédé comprenant : (a) la culture d'une population de lymphocytes cytotoxiques dans des conditions promouvant l'activation ; (b) la transfection de la population de lymphocytes de (a) avec ledit vecteur ; (c) l'administration d'un nombre thérapeutiquement ou prophylactiquement efficace des lymphocytes transfectés de (b) à un sujet présentant ou à risque de ladite infection ; dans lequel le polypeptide comprenant les domaines d'un CAR présente une spécificité de liaison pour la fraction ciblée ou peut être lié par la fraction ciblée ; permettant ainsi le traitement ou la prévention de ladite indication chez ledit sujet.

12. Vecteur codant pour un polypeptide comprenant le premier domaine polypeptidique tel que défini dans la revendication 1 i) et une partie peptidique d'un système de liaison à une protéine de division fixée à la partie extracellulaire dudit premier domaine polypeptidique ; pour son utilisation dans un procédé de traitement ou de prévention de la survenue de toute indication ou affection associée à l'expression, par exemple une expression inappropriée de la CSA, par exemple dans un cancer, chez un sujet ; le procédé comprenant : (a) la culture d'une population de lymphocytes cytotoxiques dans des conditions promouvant l'activation ; (b) la transfection de la population de lymphocytes de (a) avec ledit vecteur ; (c) l'administration d'un nombre thérapeutiquement ou prophylactiquement efficace des lymphocytes transfectés de (b) à un sujet présentant ou à risque de ladite infection ; et (d) l'administration d'un polypeptide comprenant le second domaine polypeptidique extracellulaire tel que défini dans la revendication 1 ii) et une partie peptidique d'un système de liaison à une protéine de division, dans lequel lesdites parties peptidiques d'un système de liaison à une protéine de division définies dans les deux polypeptides différents utilisés sont sélectionnées parmi K-Tag (SEQ ID NO : 132), SpyCatcher (SEQ ID NO : 129), SpyCatcher-ΔN (SEQ ID NO : 136), SpyTag (SEQ ID NO : 130), la séquence Minimal Spytag (SEQ ID NO : 131), split-Spy0128 (SEQ ID NO : 135), l'isopeptide Spy0128 (SEQ ID NO : 134), ou toute séquence inverse de ceux-ci, ou un variant de ceux-ci avec une identité de séquence d'au moins environ 80 %, par exemple d'au moins environ 82, 84, 86, 88, 90, 92, 94, 96, 98, ou 99 %, et forment spontanément une liaison isopeptidique irréversible ; permettant ainsi le traitement ou la prévention de ladite indication chez ledit sujet.

13. Polypeptide selon d'une quelconque des revendications 1 à 5, ou produit thérapeutique à deux composants selon l'une quelconque des revendications 9 à 10, pour son utilisation en tant que médicament.

14. Composition pharmaceutique comprenant le polypeptide selon d'une quelconque des revendications 1 à 5, ou le produit thérapeutique à deux composants selon l'une quelconque des revendications 9 à 10.

15. Polypeptide selon d'une quelconque des revendications 1 à 5, ou produit thérapeutique à deux composants selon l'une quelconque des revendications 9 à 10, ou composition pharmaceutique selon la revendication 14 pour le traitement de toute indication associée à une affection impliquant l'expression, par exemple l'expression inappropriée de la CSA, par exemple dans un cancer.
